# EUROPÄISCHE PATENTANMELDUNG

(11) **EP 3 530 756 A1**
(43) Veröffentlichungstag der Anmeldung: **28.08.2019**
(21) Anmeldenummer: 18158518.3
(22) Anmeldetag: 26.02.2018
(51) Int. Cl.: C12Q 1/6858

(54) **VERFAHREN ZUR SELEKTIVEN AMPLIFIKATION VON NUKLEINSÄUREN UND KIT ZU DESSEN DURCHFÜHRUNG**

(71) Anmelder: AGCT GmbH, 23562 Luebeck (DE)
(72) Erfinder: BASLER, Norbert, 22927 Großhansdorf (DE); BECKER, Claus, 76470 Ötigheim (DE); CHERKASOV, Dmitry, 35039 Marburg (DE); HESS, Hans-Jörg, 13467 Berlin (DE); GRUNWALD, Christian, 35392 Gießen (DE); MÜLLER-HERMANN, Andreas, 80796 München (DE)
(74) Vertreter: Keller, Günter

(57) **Zusammenfassung**

Offenbart wird ein Verfahren zur Detektion einer Amplifikation von Nukleinsäuren, bei dem im Wesentlichen davon Gebrauch gemacht wird, dass eine vordefinierte Nukleinsäurekette (Zielsequenz) in Anwesenheit eines zielsequenzspezifischen Aktivator-Oligonukleotides vervielfältigt/amplifiziert werden kann. Das zielsequenzspezifische Aktivator-Oligonukleotid bewirkt die Trennung von neu synthetisierten komplementären Primer-Verlängerungsprodukten mittels Strangverdrängung, so dass sich an dem jeweiligen Matrizenstrang ein neues Primer-Oligonukleotid anlagern kann. Der so gebildete Komplex aus einem Primer-Oligonukleotid und einem Matrizenstrang kann eine neue Primer-Verlängerungsreaktion initiieren. Die so gebildeten Primer-Verlängerungsprodukte dienen wiederum als Matrizen, so dass eine exponentiell ablaufende Amplifikationsreaktion resultiert. Bei dem Verfahren wird eine selektive Amplifikation dadurch bewirkt, dass bei erstem, zweitem Primer sowie Aktivator Oligonukleotid Varianten eingesetzt werden, die sich in wenigstens einem Nukleotid von der Zielsequenz unterscheiden.

SEQUENCE LISTING

<110>  AGCT GmbH

<120>  Verfahren zur selektiven Amplifikation von Nucleinsäuren und Kit
       zu dessen Durchführung

<130>  PEP03004AGC

<160>  11

<170>  PatentIn version 3.5

<210>  1
<211>  38
<212>  DNA
<213>  Genicularia spirotaenia

<400>  1
gtaagagcag atccctggac aggcaaggaa tacaggta                              38


<210>  2
<211>  57
<212>  DNA
<213>  artificial sequence

<220>
<223>  first primer

<400>  2
aactcagaca agatgtgatt tttttacctg tatcucugau gcuuctacct gtattcc         57


<210>  3
<211>  70
<212>  DNA
<213>  artificial sequence

<220>
<223>  second primer

<400>  3
ctacagaact cagacaagat gtgaactaca atgttgctca tactacaatg tcacttactg      60

taagagcaga                                                             70


<210>  4
<211>  56
<212>  DNA
<213>  artificial sequence

<220>
<223>  activator oligonucleotid

<400>  4
uaaucuguaa gagcagaucc cuggacaggc aaggaauaca ggtagaagca tcagag          56


<210>  5
<211>  52
<212>  DNA

<213>  artificial sequence

<220>
<223>  matrix

<400>  5
aagctcgaca aaagaactca gagtgtgctc gacactacct gtattccttg cc          52


<210>  6
<211>  67
<212>  DNA
<213>  artificial sequence

<220>
<223>  activator oligonucleotid

<400>  6
gctcatacta caatgtcact tactgtaaga gcagatccct ggacaggcaa ggaatacagg    60

taaaaaa                                                              67


<210>  7
<211>  67
<212>  DNA
<213>  artificial sequence

<220>
<223>  matrix

<400>  7
gctcatacta caatgtcact tactgtaaga gcagatccct ggacaggcga ggaatacagg    60

taaaaaa                                                              67


<210>  8
<211>  70
<212>  DNA
<213>  artificial sequence

<220>
<223>  second primer

<400>  8
ctacagaact cagacaagat gtgaactaca atgttgctca tactacaatg tcacttactg    60

taagagcaga                                                           70


<210>  9
<211>  67
<212>  DNA
<213>  artificial sequence

<220>
<223>  matrix

<400>  9
gctcatacta caatgtcact tactgtaaga gcagatccct ggacaggcaa ggaatacagg    60

taaaaaa                                                              67

<210>  10
<211>  70
<212>  DNA
<213>  artificial sequence

<220>
<223>  second primer

<400>  10
ctacagaact cagacaagat gtgaactaca atgttgctca tactacaatg tcacttactg       60

taagagcaga                                                              70


<210>  11
<211>  60
<212>  DNA
<213>  artificial sequence

<220>
<223>  mismatch activator oligonucleotid

<400>  11
gctctaatct gtaagagcag atccctggac aggcaaagaa uacaggtaga agcatcagag       60

## Beschreibung

Die Synthese von Nukleinsäureketten nimmt heute eine zentrale Rolle in der Biotechnologie ein. Verfahren wie PCR haben sowohl die Forschungslandschaft als auch industrielle Applikationsfelder, wie Diagnostik in der Medizin und Lebensmittelindustrie, signifikant weiterentwickelt. Die Verbindung der PCR mit anderen Technologien, wie Sequenzierung, Real-Time-Nachweis, Microarray-Technologie, Mikrofluidic-Managment etc. hat zur technologischen Entwicklung der Basistechnologie beigetragen und einige Barrieren der Grundtechnologie der PCR teilweise überwinden können. Es wurden auch weitere Amplifikations-Verfahren, wie isothermale Amplifikationstechniken (LAMP, HDA, RPA, TMA, SDA etc.) entwickelt. Deren Einsatz wurde besonders für Bereich von POCT vorgesehen.

Trotz enormer Fortschritte auf diesem Gebiet, nimmt PCR die zentrale Rolle ein und bestimmt somit die einzelnen technologischen Barrieren der Applikationen.

Eine der Eigenschaften von verbreiteten Amplifikationsverfahren, wie PCR, besteht darin, dass während des Amplifikationsvorgangs der Nukleinsäure keine Kontrolle der amplifizierten Sequenzabschnitte zwischen beiden Primern stattfindet. Im Wesentlichen steht die Primer-Bindung im Fokus von Optimierungen von PCR-Amplifikationsreaktionen. Zu Beginn der PCR-Amplifikation und in ihrem Verlauf kommt es kontinuiertlich zu mehr oder weniger spezifischen Primer-Bindung und Initiierung der Synthese von Hauptprodukten und Nebenprodukten. Die Nebenprodukte können beispielsweise als Folge eines unspezifischen Primer-Verlängerungsereignisses in einem Synthese-Zyklus generiert werden. Bei ggf. erfolgenden Rücksynthese-Reaktion wird der unspezifisch verlängerte Primer als Matrize abgelesen, was in der Regel zu Ausbildung einer vollständigen Primer-Bindungsstelle führt. Somit erfolgt eine Übertragung einer fehlerhaften Sequenzinformation von einem Synthese-Zyklus auf den nächsten Synthese-Zyklus, was in Summe von Synthese-Zyklen nicht nur zu initialen Entstehung, sondern vor allem zur exponentiellen Vermehrung von Nebenprodukten führt.

Solche Nebenreaktionen können unter Umständen zur initialen Entstehung und exponentiellen Vermehrung von Fragmenten führen, welche die Hauptreaktion (Amplifikation einer Zielsequenz) stören bzw. zu Interfernzen in nachfolgenden Schritten der Analyse führen. Solche Nebenprodukte umfassen typischerweise Primersequenzen und entsprechende Primersequenzen, so dass ihre Amplifikation parallel zu der Hauptreaktion stattfinden kann. Anstatt einer Zielsequenz umfassen solche Nebenprodukte allerdings eine andere Nukleinsäuresequenz.

Vorrangig wird Spezifität der PCR Amplifikation durch Optimierung der Primer-Bindung an Zielsequenzen erreicht. Dabei können beispielsweise zusätzliche Oligonukleotide verwendet werden, welche an Primer teilweise binden können und somit bei Primer-Bindung an andere Nukleinsäureketten kompetetiv mitwirken. Solche Sonden binden in der Regel an einen Sequenz-Abschnitt des Primers und lassen am Primer einen einzelsträngigen Sequenzabschnitt frei, so dass der Primer mit diesem Abschnitt an die Zielnukleinsäure binden kann und eine Synthese Reaktion initiieren kann. Dabei soll die Spezifität einer Primer-Bindung dadurch verbessert werden, da Primer-Matrizen Mismatches durch solche Oligonukleotide kompetetiv verdrängt werden können. Im Ergebnis kann Spezifität der Initiierung von PCR-Reaktionen in der Regel verbessert werden. Die Wirkung solcher Oligonukleotide ist auf die Interaktion mit Primer-Sequenzen beschränkt. Solche zusätzlichen Oligonukleotide interagieren nicht mit der zu amplifizierenden Nukleinsäurekette in Abschnitten zwischen beiden Primern. Aufgrund eines molaren Überschusses an Primern kann es während einer Amplifikation dennoch zu unspezifischen Interaktionen von Primern mit Matrizen kommen. Falls es zu einem solchen unspezifischen Ereignis der Primer-Verlängerung kommt (Initiierung einer exponentiellen Nebenreaktion), kommt es zu Ausbildung einer vollfunktionsfähigen Primer-Bindungsstelle als Folge bzw. im Rahmen von Rücksynthese eines komplementären Stranges des Nebenproduktes. Das Vorliegen einer solchen vollständigen Primerbindungsstelle im Nebenprodukt führt zu einem Verlust der kompetetiven Wirkung von solchen zusätzlichen Oligonukleotiden auf Primer-Bindung. Kontrolle der Spezifität der Bindung eines Primers an die Matrize durch solche Oligonukleotide macht somit nur Initiierung einer Nebenreaktion unwahrscheinlicher, kann allerdings nach Entstehung eines Nebenproduktes seine expontielle Amplifikation kaum beeinflussen.

Im Allgemeinen kann eine Verbesserung der Spezifität der Synthese eines Amplifikationsverfahrens mit reduzierter Entstehung und Co-Amplifikation von Nebenprodukten, welche sich von Zielsequenz unterscheiden, beispielsweise zu einer Verbesserung von diagnostischen Verfahren beitragen.

Eine Aufgabe der vorliegenden Erfindung ist es, Verfahren und Komponenten bereitzustellen, welche die enzymatische Synthese und Amplifikation von Nukleinsäureketten ermöglichen. Es soll ein neues enzymatisches Verfahren und Komponenten für die Synthese von Nukleinsäureketten, sowie die Amplifikation von Nukleinsäureketten bereitgestellt werden.

Eine weitere Aufgabe der Erfindung ist es, ein Verfahren mit verbesserter Spezifität der Synthese von Zielnukleinsäureketten in einer exponentiellen Amplifikation bereitzustellen.

Insbesondere sollen damit Einzelnukleotid-Unterschiede, wie beispielsweise für Allel-Varianten bekannt, detektiert werden. Weiterhin sollen Sequenzunterschiede zwischen Sequenzen mit verbesserter Spezifität nachgewiesen werden, welche mehrere Nukleotide umfassen, z.B. Deletionen / Insertionen.

Eine weitere Aufgabe der Erfindung besteht darin, Verfahren bereitzustellen, welche in der Lage sind, mehrere Amplifikationsfragmente gleichzeitig in einem Ansatz zu amplifizieren. Damit soll eine Multiplex-Amplifikation mit verbesserter Spezifität ermöglicht werden

Eine weitere Aufgabe der Erfindung ist es, Mittel für die Umsetzung eines exponentiellen Amplifikationsverfahrens mit verbesserter Spezifität der Synthese zur Verfügung zu stellen.

Mit dem erfindungsgemäßen Verfahren sollen Nukleinsäureketten mit definierter Sequenz-Zusammensetzung synthetisiert bzw. amplifiziert werden können.

Die Aufgabe der Erfindung wird gelöst durch Bereitstellung von Amplifikationsverfahren und entsprechenden Mitteln zu seiner Ausführung. Die Ausführung des Amplifikationsverfahrens wurde in der PCT Anmeldung (PCT/EP2017/071011) und der europäischen Anmeldung (EP-A 16185624.0) bereits beschrieben. Zu Details der Ausführung der Amplifikation wird der Fachmann an diese Anmeldung verwiesen. Diese Anmeldung ist hiermit im vollen Umfang incorporiert ("incorporated by reference").

Vorzugsweise erfolgt die Amplifikation als exponentielle Amplifikation, bei welcher neusynthetisierte Produkte beider Primer (Primer-Verlängerungsprodukte) als Matrizen für weitere Syntheseschritte auftreten. Dabei werden Primersequenzen zumindest teilweise kopiert, so dass komplementäre Primer-Bindungsstellen entstehen, welche unmittelbar nach ihrer Synthese als Sequenzsegmente eines Doppelstranges vorliegen. Im Amplifikatonsverfahren erfolgen Synthese-Schritte von beiden Strängen und Doppelstrang-Öffnungs-Schritten der neusynthetisierten Sequenzabschnitte in gegenseitiger Abwechselung. Eine hinreichende Doppelstrang-Trennung nach einer Synthese stellt eine wichtige Voraussetzung für weitere Synthese dar. Insgesamt kann eine solche Abwechslung aus Synthese- und Doppelstrang-Trennungsschritten zu einer exponentiellen Amplifikation führen.

Im erfindungsgemäßen Amplifikationsverfahren erfolgt die Doppelstrang-Öffnung von Hauptprodukten der Amplifikation (Amplifikation von Zielsequenz umfassenden Nukleinsäureketten) unter anderem mittels eines Oligonukleotides, genannt Aktivator-Oligonukleotid. Das Aktivator-Oligonukleotid umfasst vorzugsweise Sequenzsegmente, welche der Zielsequenz entsprechen.

Im Einzelnen, wird die Strangtrennung erfindungsgemäß durch Einsatz von Aktivator-Oligonukleotide mit vordefinierten Sequenzen erreicht, welche vorzugsweise mittels einer sequenzabhängigen nukleinsäurevermittelten Strangverdrängung einen neusynthetisierten Doppelstrang bestehend aus beiden spezifischen Primer-Verlängerungsprodukten trennen. Die dabei entstehenden einzelsträngigen Segmente von Primer-Verlängerungsprodukten umfassen die Zielsequenz, sowie entsprechende Primer-Bindungsstellen, welche als Bindungsstellen für weitere Primer-Oligonukleotide dienen können, so dass eine exponentielle Amplifikation von zu amplifizierenden Nukleinsäureketten erreicht wird. Die Primer-Verlängerungsreaktionen und Strangverdrängungsreaktionen finden vorzugsweise im Ansatz gleichzeitig statt. Die Amplifikation erfolgt vorzugsweise unter Reaktionsbedingungen, welche eine spontane Trennung von beiden spezifischen synthetisierten Primer-Verlängerungsprodukten nicht zulassen.

Eine spezifische exponentielle Amplifikation einer Zielsequenz-umfassenden Nukleinsäurekette umfasst eine Wiederholung aus Synthese-Schritten und Doppelstrang-Öffnungs-Schritten (Aktivierungs-Schritten für Primer-Bindungsstellen) als zwingende Voraussetzung für Vermehrung der Nukleinsäurekette.

Die Öffnung von synthetisierten Doppelsträngen wird als Reaktions-Schritt umgesetzt, welcher sequenzspezifisch durch das 'Aktivator-Oligonukleotid beeinflusst werden soll.

Diese Öffnung kann vollständig erfolgen, bis hin zu Dissoziation von beiden komplementären Primer-Verlängerungsprodukten, oder auch partiell sein.

Das Aktivator-Oligonukleotid umfasst erfindungsgemäß Sequenzabschnitte, welche mit der Zielsequenz interagieren können und weitere Sequenzabschnitte, welche diese Interaktion herbeiführen, bzw. ermöglichen, bzw. begünstigen. Im Rahmen der Interaktion mit dem Aktivator-Oligonukleotid werden doppelsträngige Abschnitte der synthetisierten Primer-Verlängerungsprodukte via sequenzspezifischer Strangverdrängung in einzelsträngige Form überführt. Dieser Vorgang ist sequenzabhängig: erst wenn die Sequenz des synthetisieren Doppelstranges ein gewisses Maß an Komplementarität mit der entsprechenden Sequenz des Aktivator-Oligonukleotides aufweist, kommt es zu einer hinreichenden Doppelstrang-Öffnung, so dass die für die Fortführung der Synthese wesentlichen Sequenzabschnitte, wie z.B. Primer-Bindungsstellen, in einzelsträngige Form überführt werden, was einem "aktiven Zustand" entspricht. Das Aktivator-Oligonukleotid "aktiviert" somit spezifisch die neusynthetisierten Primer-Verlängerungsprodukte, welche die Zielsequenz umfassen, für weitere Synthese-Schritte.

Hingegen werden Sequenzabschnitte, welche keine Zielsequenz umfassen, nicht in einzelsträngigen Zustand überführt und verbleiben als Doppelstrang, was einem "inaktiven" Zustand entspricht. Die potenziellen Primerbindungsstellen sind in einem solchen Doppelstrang an Interaktion mit neuen Primern benachteiligt bzw. vollständig gehindert, so dass weitere Synthese-Schritte an solchen "nicht aktivierten" Strängen im Allgemeinen nicht stattfinden. Diese fehlende oder verminderte Aktivierung (d.h. Überführung in einen einzelsträngigen Zustand) von synthetisierten Nukleinsäuresträngen nach einem Synthese-Schritt führt dazu, dass im darauffolgenden Synthese-Schritt nur eine verminderte Menge an Primern an einer Primer-Verlängerungsreaktion erfolgreich teilnehmen kann.

Aufgrund einer anzustrebenden exponentiellen Amplifikation von Hauptprodukten (einer zu amplifizierenden Nukleinsäurekette, welche Zielsequenzen umfasst) werden mehrere Synthese-Schritte und Aktivierungs-Schritte (Doppelstrang-Öffnungs-Schritte) zu einem Amplifikations-Verfahren zusammengefasst und so lange ausgeführt, bzw. so oft wiederholt, bis die gewünschte Menge der spezifischen Nukleinsäurekette bereitgestellt ist.

Die Reaktionsbedingungen (z.B. Temperatur) werden dabei dermaßen gestaltet, dass eine spontane Trennung von komplementären Primer-Verlängerungsprodukten in Abwesenheit eines Aktivator-Oligonukleotdies unwahrscheinlich oder signifikant verlangsamt ist.

Die anzustrebende Erhöhung der Spezifität einer Amplifikation resultiert somit aus der Sequenzabhängigkeit der Trennung von komplementären Primer-Verlängerungsprodukten, welche eine Zielsequenz umfassen: Aktivator-Oligonukleotid ermöglicht bzw. begünstigt diese Doppelstrang-Trennung als Folge der Übereinstimmung seiner Sequenzabschnitte mit vorgegebenen Sequenzabschnitten der Primer-Verlängerungsprodukte. Diese Übereinstimmung wird nach jedem Synthese-Zyklus durch Aktivator-Oligonukleotid überprüft. Die exponentielle Amplifikation resultiert als Folge aus erfolgreichen Wiederholungen aus Synthese-Vorgängen und sequenzspezifischen Strangverdränungen durch Aktivator-Oligonukleotid, d.h. "Aktivierungen" (Doppelstrang-Öffnungen/Doppelstrang-Trennungen/Strangverdrängungs-Vorgängen resultierend in einzelsträngiger Form von entsprechenden Primer-Bindungsstellen) von neusynthetisierten Primer-Verlängerungsprodukten.

Vorwiegend sequenzspezifische Trennung des Doppelstranges unter Mitwirkung eines Aktivator-Oligonukleotides kann in Kombination mit sequenz-spezifischen oder weniger sequenz-spezifischen Primern Kombiniert werden.

Verwendung von vorwiegend spezifischen Aktivator-Oligonukleotiden, welche in der Lage sind, zwischen einzelnen Varianten zu unterscheiden, ermöglicht beispielsweise Zusammenstellung von Allel-Spezifischen bzw. Mutations-Spezifischen Assays.

Die Erfindung eignet sich besonders bei Amplifikation von Sequenzvarianten eines bekannten Lokus einer Zielsequenz. Ein solcher Lokus kann mehrere vorkommende Sequenzvariante einer Zielsequenz umfassen und ist somit ein polymorpher Lokus. Ein solcher polymorpher Lokus umfasst beispielsweise Einzelnukleotid-Polymorphismen.

Um die Sequenzspezifität einer Sequenzvariante eines Lokus der Zielsequenz zu erreichen, sollten Komponenten, welche für Spezifität der Amplifikation eine wesentliche Rolle spielen in eine spezifische Anordnung gebracht werden. Besonders vorteilhaft sind Anordnungen, welche eine bekannte Sequenzvariante eines Lokus umfassen, wobei ein vorwiegend Sequenz-spezifisches Aktivator-Oligonukleotid sowie entsprechende Primer entworfen werden können.

Die Position einer zu erwartenden Sequenzvariante im bekannten Lokus wird somit bei dem Design von Komponenten dahingehend berücksichtigt, dass zumindest das Aktivator-Oligonukleotid, besser Aktivator-Oligonukleotid in Kombination mit mindestens einem Primer, eine spezifische Sequenzzusammensetzung in einem Amplifikation-System aufweisen, welches für Amplifikation einer gewünschten Variante der Zielsequenz geeignet ist.

Die Aufgabe der Erfindung wird weiterhin dadurch gelöst, dass ein Amplifikations-System zumindest einen sequenz-spezifischen Primer (z.B. Allele-diskriminierender Primer) in Kombination mit jeweils einem spezifischen Aktivator-Oligonukleotiden (z.B. Allelespezifische Aktivator-Oligonukleotide) umfasst. Somit kann für jede spezifische Zusammensetzung einer Zielsequenz und ihre spezifische Varianten (z.B. Allele) jeweils eine spezifische Kombination aus mindestens einem spezifischen Primer und mindestens einem spezifischen Aktivator-Oligonukleotid bereitgestellt werden. Dabei resultiert eine spezifische Amplifikation einer der möglichen Sequenzvarianten einer Zielsequenz.

Weiterhin wird die Aufgaben der Erfindung dadurch gelöst, dass ein Amplifikations-System zumindest zwei, besser vier Sequenz-spezifische Primer (z.B. allel-diskriminierende Primer) in Kombination mit jeweils einem spezifischen Aktivator-Oligonukleotiden (z.B. Allelespezifische Aktivator-Oligonukleotide) umfasst. Somit kann für jede spezifische Zusammensetzung einer Zielsequenz und ihre spezifische Varienten (z.B. Allele) jeweils eine spezifische Kombination aus mindestens einem spezifischen Primer und mindestens einem spezifischen Aktivator-Oligonukleotid bereitgestellt werden, wobei für alle vier Nukleotid-Varianten an einer bestimmten Position ein spezifisches Primer-Aktivator-Oligonukleotid bereitgestellt wird. Bei Anwesenheit von mindestens einer Sequenzvariante der Zielsequenz im Reaktions-Ansatz resultiert eine spezifische Amplifikation einer der möglichen Sequenzvarianten einer Zielsequenz.

Weiterhin wird die Aufgabe der Erfindung dadurch gelöst, dass ein Amplifikations-System zumindest einen sequenz-spezifischen Primer (z.B. allel-diskriminierender Primer) in Kombination mit jeweils einem spezifischen Aktivator-Oligonukleotiden (z.B. allel-spezifische Aktivator-Oligonukleotide) und mindestens einen weiteren Primer (einen Kompetitor-Primer) umfasst, welcher zu anderen zu erwartenden Sequenzvarianten eine komplementäre Sequenzzusammensetzung aufweist. Somit kann für jede spezifische Zusammensetzung einer Zielsequenz und ihre spezifische Varienten (z.B. Allele) jeweils eine spezifische Kombination aus mindestens einem spezifischen Primer und mindesten einem spezifischen Aktivator-Oligonukleotid bereitgestellt werden. Dabei resultiert eine spezifische Amplifikation einer der möglichen Sequenzvarianten einer Zielsequenz.

Die Aufgabe der Erfindung wird weiterhin dadurch gelöst, dass beide Stränge einer doppelsträngigen Nukleinsäurekette analysiert werden, wobei in einer Reaktion die Komponenten eines Amplifikations-Systems für einen Strang einer Zielnukleinsäure bereitgestellt werden, welche als Start-Nukleinsäure verwendet wird, und in einem getrennten Ansatz werden Komponenten für den komplementären Strang der Zielnukleinsäurekette bereitgestellt, welche als Start-Nukleinsäue verwendet wird. Somit werden mindestens zwei Aktivator-Oligonukleotide verwendet, welche jeweils an einen Strang der Zielnukleinsäurekette binden können (als erstes Primer-Verlängerungsprodukt). Beide Aktivator-Oligonukleotide werden in getrennten Reaktions-Ansätzen verwendet.

Weiterhin wird die Aufgabe der Erfindung dadurch gelöst, dass ein Amplifikations-System zumindest einen Primer umfasst, welcher eine Amplifikation von mehreren potenziellen Sequenzvarianten unterstützen kann (z.B. ein zielsequenzspezifischer Primer, aber kein allel-diskriminierender Primer, ein zielsequenzspezifischer aber ein allel-unspezifischer Primer) in Kombination mit jeweils einem spezifischen Aktivator-Oligonukleotiden (z.B. allel-spezifische Aktivator-Oligonukleotide) bereitgestellt werden. Somit kann für jede spezifische Zusammensetzung einer Zielsequenz und ihre spezifische Varianten (z.B. Allele) jeweils eine spezifische Kombination aus mindestens einem allel-unspezifischen Primer und mindestens einem allel-spezifischen Aktivator-Oligonukleotid bereitgestellt werden. Dabei resultiert eine spezifische Amplifikation einer der möglichen Sequenzvarianten einer Zielsequenz.

Weiterhin wird die Aufgabe der Erfindung dadurch gelöst, dass zumindest ein Primer, welcher eine Amplifikation von mehreren Sequenzvarianten potenziell unterstützen kann (z.B. ein zielsequenzspezifischer aber nicht allel-diskriminierender Primer, allel-unspezifischer Primer) in Kombination mit einem Aktivator-Oligonukleotid verwendet wird, wobei das Aktivator-Oligonukleotid mindestens zwei Sequenzvarianten einer Zielsequenz amplifizieren kann. Bei Anwesenheit von mindestens einer der Varianten als Start-Nukleinsäurekette in der Reaktion kann somit eine von diesen beiden Varianten amplifiziert werden, unter Mitwirkung von nur einem Aktivator-Oligonukleotid. Ein solches Aktivator-Oligonukleotid ist somit in der Lage, Amplifikation von mindedesten zwei Sequenzvarianten unterstützen. Dadurch kann eine Amplifikation auch dann erfolgen, wenn eine Zielsequenz im Reaktions-Ansatz vorliegt, aber nicht genau bekannt ist, welche genaue Zusammensetzung einer polymorpher Lokus umfasst.

Die für eine Amplifikation erforderlichen Oligonukleotide (mindestens ein zielsequenzspezifischer erster Primer, mindestens ein zielsequenzspezifischer zweiter Primer, midnestens ein zielsequenzspezifisches Aktivator-Oligonukleotid) werden als zielsequenzspezifisches Amplifikations-System zusammengefasst.

Da im Ansatz auch mehrere Zielsequenzen vorliegen können, soll durch Verwendung von mehreren zielsequenzspezifischen Amplifikations-Systemen in einem Reaktions-Ansatz parallele Amplifikation von mehr als eine Zielsequenz ermöglicht werden, wobei bevorzugt jeweils zielsequenz-spezifische Komponenten kombiniert werden.

Der Einsatz von vordefiniertem Aktivator-Oligonukleotid ermöglicht somit eine sequenzabhängige Überprüfung der Inhalte von Primer-Verlängerungsprodukten zwischen einzelnen Synthese-Schritten während der exponentiellen Amplifikation und Herbeiführung einer Selektion bzw. einer Auswahl von Sequenzen für nachfolgende Synthese-Schritte. Dabei kann zwischen "aktiven", einzelsträngigen Zuständen von neusynthetisierten spezifischen Primer-Verlängerungsprodukten als Folge einer erfolgreichen Interaktion mit einem Aktivator-Oligonukleotid, und "inaktiven" doppelsträngigen Zuständen von neusynthetisierten unspezifischen Primer-Verlängerungsprodukten als Folge einer mangelhaften und/oder unzureichenden und/oder verminderten und/oder verlangsamten Interaktion mit einem Aktivator-Oligonukleotid unterschieden werden.

Für eine exponentielle Amplifikation ergeben sich daraus folgende Effekte:
Unter nicht-denaturierenden Bedingungen erfolgt die Trennung von spezifisch synthetisierten Strängen unter Mitwirkung von Aktivator-Oligonukleotid.
Exponentielle Amplifikation von Zielsequenz umfassenden Nukleinsäureketten erfolgt sequenzkontrolliert (Hauptreaktion). Diese Sequenz-Kontrolle erfolgt nach jedem Synthese-Schritt und schließt Sequenzsegmente ein, welche zwischen Primern liegen und eine Zielsequenz umfassen. Die erfolgreiche Überprüfung des Ergebnisses der Synthese nach jedem Synthese-Schritt resultiert in Trennng von beiden spezifischen Primer-Verlängerungsprodukten, was die Voraussetzung für weitere spezifische Synthese-Schritte darstellt.

Während der Amplifikation kann eine initiale Entstehung/Generierung von unspezifischen Primer-Verlängerungsprodukten nicht prinziell ausgeschlossen werden (Nebenprodukte). Aufgrund einer Matrizenabhängigkeit liegen solche unspezifischen Primer-Verlängerungsprodukte unmittelbar nach ihrer Synthese in der Regel in doppelsträngiger Form vor. Die Interaktion mit dem Aktivator-Oligonukleotid bleibt allerdings entweder vollständig aus oder ist eingeschränkt, so dass es nicht zu einer Strangtrennung kommt oder die Strangtrennung verlangsamt gegenüber der Hauptreaktion ist. Die Übertragung einer fehlerhaften Sequenzinformation von einem Synthese-Zyklus auf den nächsten findet somit nicht statt.

Durch die Wahl der Reaktions-Bedingungen und die Gestaltung von Aktivator-Oligonukleotid ist es somit möglich, einen gezielten Einfluss auf Effizienz der Regenerierung von korrekten Nukleinsäureketten-Matrizen zwischen einzelnen Synthese-Schritten im Rahmen eines Amplifikationsverfahrens auszuüben. Im Allgemeinen, je höher das Maß der Übereinstimmung der synthetisierten Sequenz mit der vorgegebenen Sequenz des Aktivator-Oligonukleotides, umso erfolgreicher ist die Trennung der synthetisierten Produkte, umso erfolgreicher ist die Regenerierung von korrekten Matrizen von einem Synthese-Schritt zum nächsten. Umgekehrt führt eine Sequenz-Abweichung bei Nebenprodukten zu einer insuffizienten Regenerierung von Matrizensträngen und somit zu einer Verlangsamung der Synthese-Initiierung bzw. zu Reduzierung der Ausbeute in jedem nachfolgenden Zyklus. Die gesamte exponentielle Amplifikation von Nebenprodukten verläuft entweder langsamer oder findet gar nicht statt und/oder bleibt auf einem nicht detektierbaren Niveau.

Das Verfahren ermöglicht somit eine Überprüfung der synthetisierten Sequenzen in Echtzeit, d.h. ohne Reaktions-Unterbrechung und stellt somit Potenzial für Entwicklung von homogenen Assays dar, bei welchen alle Komponenten des Assays bereits zu Beginn einer Reaktion im Reaktionsgemisch vorliegen.

### Begriff und Definitionen:

Im Rahmen der vorliegenden Erfindung haben die verwendeten Begriffe folgende Bedeutung:
Der Begriff **"Oligonukleotid"** wie er hier bezugnehmend auf Primer, Aktivator-Oligonukleotid, Sonden, zu amplifizierende Nukleinsäurekette verwendet wird, ist als ein Molekül definiert, das zwei oder mehr, bevorzugt mehr als drei Desoxyribonukleotide und/oder Ribonukleotide und/oder Nukleotid-Modifikationen und/oder Nicht-Nukleotid-Modifikationen umfasst. Seine Länge umfasst beispielsweise Bereiche zwischen 3 bis 300 Nukleotid-Einheiten oder ihrer Analoga, bevorzugt zwischen 5 bis 200 Nukleotid-Einheiten oder ihrer Analoga. Seine genaue Größe hängt von vielen Faktoren ab, die ihrerseits von der letztendlichen Funktion oder Verwendung der Oligonukleotide abhängen.

Der Begriff **"Primer",** wie er hier verwendet wird, betrifft ein Oligonukleotid, unabhängig davon, ob es natürlicherweise z. B. in einer gereinigten Restriktionsspaltung vorkommt oder synthetisch produziert wurde. Ein Primer ist fähig, als Initiationspunkt der Synthese zu wirken, wenn er unter Bedingungen eingesetzt wird, bei denen die Synthese eines zu einem Nukleinsäurestrang komplementären Primer-Verlängerungsproduktes induziert wird, d. h. in Gegenwart von Nukleotiden und einem induzierenden Agens, wie z.B. DNA-Polymerase bei einer geeigneten Temperatur und einem geeigneten pH-Wert. Der Primer ist für eine maximale Wirksamkeit bei der Amplifikation bevorzugt einzelsträngig. Der Primer muss genügend lang sein, um in Gegenwart des induzierenden Agens die Synthese des Verlängerungsproduktes zu initiieren. Die genaue Länge des Primers hängt von vielen Faktoren ab, einschließlich der Reaktions-Temperatur und der Primerquelle und der Anwendung des Verfahrens. Beispielsweise beträgt die Länge der Oligonukleotid-Primer bei diagnostischen Anwendungen, je nach Komplexität der Zielsequenz zwischen 5 bis 100 Nukleotide, vorzugsweise 6 bis 40 und besonders bevorzugt 7 bis 30 Nukleotide. Kurze Primer-Moleküle erfordern im allgemeinen für Ausübung ihrer Primer-Funktion niedrigere Reaktions-Temperaturen, um genügend stabile Komplexe mit der Matrize zu bilden, oder höhere Konzentrationen von anderen Reaktionskomponenten, beispielsweise von DNA-Polymerasen, so dass eine ausreichende Verlängerung von gebildeten Primer-Matrizen-Komplexen erfolgen kann.

Die hier verwendeten Primer sind so ausgewählt, das sie "im Wesentlichen" komplementär zu den verschiedenen Strängen jeder spezifischen zu amplifizierenden Sequenz sind. Das bedeutet, dass die Primer genügend komplementär sein müssen, um mit ihren betreffenden Strängen zu hybridisieren und eine Primer-Verlängerungsreaktion zu initiieren. Somit braucht die Primersequenz beispielsweise nicht die genaue Sequenz der Zielsequenz wiederzuspiegeln. Zum Beispiel kann ein nicht-komplementäres Nukleotidfragment an dem 5'-Ende des Primers angeheftet sein, wobei der Rest der Primersequenz komplementär zu dem Strang ist. In einer anderen Ausführungsform können einzelne nicht-komplementäre Basen oder längere nicht-komplementäre Sequenzen in einem Primer eingefügt sein, vorausgesetzt, dass die Primersequenz eine genügend große Komplementarität mit der zu amplifizierenden Sequenz des Stranges aufweist, um damit zu hybridisieren und so ein Primer-Matrizen-Komplex fähig zur Synthese des Verlängerungsproduktes zu erzeugen.

Im Rahmen der enzymatischen Synthese eines zu Matrize komplementären Stranges wird ein Primer-Verlängerungsprodukt erzeugt, was vollständig zum Matrizen-Strang komplementär ist.

Allel-spezifische Primer sind Primer, welche durch ihre Sequenzzusammensetzung in der Lage sind, bevorzugt unter stringenten Reaktionsbedingungen an jeweils einzelnen Sequenzvarianten einer Zielsequenz zu hybridisieren und durch eine Polymerase unter Verwendung der Zielsequenz verlängert zu werden. Einzelne allel-spezifische Primer können zu einer Gruppe zusammengefasst werden, welche sämtliche Varianten einer gemeinsamen Zielsequenz abdecken. Eine solche Gruppe von allel-spezifischen Primern umfasst zumindest zwei unterschiedliche allel-spezifische Primer, da ein polymorpher Lokus an einer vorgegeben Position in der Zielsequenz mindestens zwei Sequenzvarianten umfasst. Die allel-spezifischen Primer sind dahingehend designd, dass sie unter stringenten Reaktionsbedingungen vorzugsweise mit ihrer jeweils spezifischen perfekt-match Matrize Bindung eingehen und somit diese spezifische perfekt-Match Matrize zur Ausbildung der jeweiligen Primer-Verlängerungsprodukte unter katalytischer Wirkung der Polymerase verwenden. Vorzugsweise können 3'-terminale Segmente von allel-spezifischen Primern zur Diskriminierung von Varianten von Zielsequenzen verwendet werden und dabei dermaßen in ihrer Sequenzzusammensetzung an die jeweiligen Varianten angepasst, dass solche Primer einen perfekt-match Doppelstrang unter stringenten Bedingungen mit der jeweiligen Variante ausbilden. Solche perfekt-match Doppelstränge können in der Regel von einer Polymerase gut erkannt werden und unter geeigneten Reaktionsbedingungen kommt es zu einer Primer-Verlängerung. Bei der Interaktion eines allel-spezifischen Primers mit einer anderen Variante eienr Zielsequenz entsteht somit ein Mismatch- Doppelstrang. Solche Mismatches führen in der Regel zu einer Verzögerung der Verlängerung durch eine Polymerase bzw. zu einer Verlangsamung der Gesamtreaktion. In einer Ausführungsform können allel-spezifischen Primer im 3'-Segment mindestens eine phosphoro-thioat-Bindung umfassen, welche allel-spezifische Primer vor 3'-5'-Nuklease-Abbau durch eine Polymerase schützt.

Mehrere Allel-spezifische Primer umfassen somit Sequenzsegmente, welche für eine Gruppe von allel-spezifischen Primern im Wesentlichen identisch bzw. einheitlich sind, sowie Sequenzsegmente, welche in den Primern einer Gruppe unterschiedlich sind und charakteristisch für jeweilige Sequenzvariante einer Zielsequenz. Unter Einbeziehung von einheitlichen Sequenzsegmenten können solche Primer an die jeweilige Zielsequenz unter Reaktionsbedingungen hybridisieren. Unter Einbeziehung von charakteristischen Sequenzsegmenten kann ein jeweiliger Primer spezifisch an eine Sequenzvariante der Zielsequenz unter Ausbildung eines perfekt-match Doppelstranges binden. Vorzugsweise sind die Primer dermaßen konstruiert, dass unter verwendeten Reaktionsbedingungen die Bindung an eine Zielsequenz unter Ausbildung eines perfekt-match Doppelstranges bevorzugt wird und die Bindung an einer Zielsequenz unter Ausbildung eines Mismatch-Doppelstranges weniger bevorzugt wird.

In einer Ausführungsform wird das erste Primer-Oligonukleotid als allel-spezifischer Primer in Kombination mit einem entsprechenden allel-spezifischen Aktivator-Oligonukleotid bereitgestellt. In einer weiteren Ausführungsform wird das zweite Primer-Oligonukleotid als allel-spezifischer Primer in Kombination mit einem entsprechenden allel-spezifischen Aktivator-Oligonukleotid bereitgestellt.

### Tm-Schmelztemperatur

Als Schmelztemperatur eines komplementären oder partiell komplementären Doppelstranges wird im Allgemeinen ein Messwert einer Reaktionstemperatur verstanden, bei welchem ca. die Hälfte der Stränge als Doppelstrang vorliegt und die andere Hälfte als Einzelstrang vorliegt. Das System (Assoziation und Dissoziation von Strängen) befindet sich im Gleichgewicht.

Aufgrund einer Vielzahl von Faktoren, welche die Tm eines Doppelstranges beeinflussen können (z.B. Sequenzlänge, CG-Gehalt der Sequenz, Puffer-Bedingungen, Konzentration von divalenten Metal-Kationen etc.) sollte die Bestimmung der Tm einer zu amplifizierenden Nukleinsäure unter gleichen Bedingungen erfolgen, wie die beabsichtigte Amplifikationsreaktion.

Wegen Abhängigkeit der messbaren Schmelztemperatur von multiplen Reaktions-Parametern, z.B. von jeweiligen Puffer-Bedingungen und jeweiligen Konzentrationen der Reaktionsteilnehmer, wird unter Schmelztemperatur ein Wert verstanden, welcher in gleichem Reaktionspuffer gemessen wurde wie die exponentielle Amplifikation, bei Konzentrationen von beiden komplementären Komponenten eines Doppelstranges von etwa 0,1µmol/l bis etwa 10 µmol/l, bevorzugt in Konzentration von etwa 0,3 µmol/ bis ca. 3 µmol/l, vorzugsweise bei ca. 1 µmol/l. Bei dem jeweiligen Wert der Schmelztemperatur handelt sich um einen Richtwert, welcher mit der Stabilität eines jeweiligen Doppelstranges korreliert.

Eine Schmelztemperatur für einen Doppelstrang kann grob abgeschätzt werden. Mehrere kommerzielle Anbieter ermöglichen eine theoretische Berechnung einer zu erwartenden Schmelztemperatur. Beispielsweise kann Software-Packet OligoAnalyzer 3.1 (online zugängig bei IDT (Intergrated DNA-Technologies) zur Abschätzung der Stärke der Bindungen einzelner Oligonukleotide verwendet werden.

Die **Desoxyribonukleosid-Triphosphate** (dNTPs) dATP, dCTP, dGTP und TTP (oder dUTP, oder dUTP/TTP-Gemisch) werden in adäquaten Mengen zum Synthesegemisch gegeben. In einer Ausführungsform können zusätzlich zu dNTPs mindestens eine weitere Art von dNTP-Analoga zum Synthesegemisch zugegeben werden. Diese dNTP-Analoga umfassen in einer Ausführungsform beispielsweise eine charakteristische Markierung (z.B. Biotin oder Fluoreszenzfarbstoff), so dass wenn in Nukleinsäurestrang eingebaut, auch diese Markierung in den Nukleinsäurestrang integriert ist. In einer anderen Ausführungsform umfassen diese dNTP-Analoga mindestens eine Modifikation von Zucker-Phosphat-Anteil des Nukleotids, z.B. alpha-Phosphorothioat-2'-Desoxyribonukleosid-Triphosphate (oder andere Modifikationen, welche einem Nukleinsäurestrang eine Nuklease-Resistenz verleihen), 2',3'-Dideoxy-Ribonukleosid-Triphosphate, Azyklo-Nukleosid-Triphosphate (oder andere zur Termination einer Synthese führende Modifikationen). In einer weiteren Ausführungsform umfassen diese dNTP-Analoga mindestens eine Modifikation von Nukleobase, z.B. Iso-Cytosine, Iso-Guanosine (oder auch andere Modifikationen der Nukleobasen des erweiterten genetischen Alphabets), 2-Amino-Adenosine, 2-Thiouridine, Inosine, 7-deazy-adenosine, 7-deaza-guanosine, 5-Me-Cytosine, 5-Propyl-Uridine, 5-Propyl-Cytosine (oder auch andere Modifikationen von Nukleobasen, welche gegenüber natürlichen Nukleobasen durch eine Polymerase eingebaut werden können und zur Änderung der Strang-Stabilität führen). In einer weiteren Ausführungsform umfasst ein dNTP-Analog sowohl eine Modifikation der Nukleobase als auch eine Modifikation des Zucker-Phosphat-Anteils. In einer weiteren Ausführungsform wird statt mindestens eines natürlichen dNTP-Substrates mindestens eine weitere Art von dNTP-Analoga zum Synthesegemisch zugegeben.

Das die Nukleinsäure-Synthese induzierende Agens kann eine Enzyme einschließende Verbindung oder ein System sein, das so wirkt, dass dadurch die Synthese der Primer-Verlängerungsprodukte bewirkt wird. Geeignete Enzyme für diesen Zweck umfassen z.B. matrizenabhängige **DNA-Polymerasen** wie Bst Polymerase und ihre Modifikationen, Vent Polymerase und andere - bevorzugt hitzestabile DNA-Polymerasen, die den Einbau der Nukleotide in der korrekten Weise ermöglichen, wodurch die Primer-Verlängerungsprodukte gebildet werden, die zu jedem synthetisierten Nukleinsäurestrang komplementär sind. Im Allgemeinen wird die Synthese am 3'-Ende eines jeden Primers initiiert und schreitet dann in 5'- Richtung entlang des Matrizenstranges fort, bis die Synthese beendet ist oder unterbrochen wird.

Bevorzugt werden Polymerasen verwendet, welche zu Strangverdrängung fähig sind. Dazu zählen beispielsweise großes Fragment der Bst Polymerase oder ihre Modifikationen (z.B. Bst 2.0 DNA Polymerase), Klenow Fragment, Vent exo minus Polymerase, Deepvent exo minus DNA Polymerase, großes Fragment der Bsu DNA Polymerase, großes Fragment der Bsm DNA Polymerase.
In einer Ausführungsform werden bevorzugt Polymerasen eingesetzt, welche keine 5'-3'-Exonuklease-Aktivität aufweisen, bzw. keine 5'-3'-FEN-Aktivität aufweisen.

In einer Ausführungsform werden mindestens zwei verschiedene Polymerasen eingesetzt, beispielsweise Polymerasen welche zu Strangverdrängung fähig sind und solche, welche eine 3'-5'-Proofreading Aktivität aufweisen.

In bevorzugten Ausführungsformen werden Polymerasen mit einer Hotstart-Funktion eingesetzt, welche erst beim Erreichen einer bestimmten Temperatur ihre Funktion entfalten können.

### Erstes Primer-Oligonukleotid:

Das erste Primer-Oligonukleotid umfasst einen ersten Primer-Bereich und einen zweiten Bereich. Der erste Primer-Bereich ist in der Lage, an eine im Wesentlichen komplementäre Sequenz innerhalb der zu amplifizierenden Nukleinsäure oder ihrer Äquivalente zu binden und eine Primer-Verlängerungsreaktion zu initiieren. Der zweite Bereich umfasst einen Polynukleotid-Schwanz, welcher in der Lage ist, ein Aktivator-Oligonukleotid zu binden und damit eine räumliche Nähe zwischen dem Aktivator-Oligonukleotid und den anderen Teilen des ersten Primer-Verlängerungsproduktes zu bewirken, welche ausreichend ist, um eine Strangverdrängung durch das Aktivator-Oligonukleotid zu initiieren. Der zweite Bereich des ersten Primer-Oligonukleotids umfasst weiterhin mindestens eine Modifikation (eine Nukleotid-Modifikation oder eine nicht-Nukleotid-Modifikation), welche die Polymerase am Kopieren des Polynukleotid-Schwanzes hindert, indem die Fortführung der Polymerasenabhängigen Synthese gehemmt wird. Diese Modifikation ist beispielsweise am Übergang zwischen dem ersten und dem zweiten Bereich des ersten Primer-Oligonukleotid lokalisiert. Der erste Primer-Bereich des ersten Primer-Oligonukleotides ist folglich durch eine Polymerase kopierbar, so dass eine komplementäre Sequenz zu diesem Bereich während der Synthese des zweiten Primer-Verlängerungsproduktes von der Polymerase erzeugt werden kann. Der Polynukleotid-Schwanz des zweiten Bereichs des ersten Primer-Oligonukleotides wird vorzugsweise durch die Polymerase nicht kopiert. In einer Ausführungsform wird dies durch die Modifikation im zweiten Bereich erreicht, welche die Polymerase vor dem Polynukleotid-Schwanz stoppt. In einer weiteren Ausführungsform
wird dies durch Nukleotid-Modifikationen im zweiten Bereich erreicht, wobei der gesamte Polynukleotid-Schwanz im Wesentlichen aus solchen Nukleotid-Modifikationen besteht und somit für Polymerase unkopierbar ist.

In einer Ausführungsform ist ein jedes erstes Primer-Oligonukleotid spezifisch für je eine zu amplifizierende Nukleinsäure.

In einer Ausführungsform ist ein jedes erste Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das erste Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

In einer Ausführungsform wird mindestens ein erstes Primer-Oligonukleotid als allel-spezifischer Primer in Kombination mit mindestens einem entsprechenden allel-spezifischen Aktivator-Oligonukleotid bereitgestellt.

### Zweites Primer-Oligonukleotid:

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren. Dieses zweite Primer-Oligonukleotid ist somit in der Lage, an das 3'-Segment eines ersten spezifischen Primer-Verlängerungsproduktes des ersten Primer-Oligonukleotids zu binden und eine Polymerase-abhängige Synthese eines zweiten Primer-Verlängerungsproduktes zu initiieren.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden.

In einer Ausführungsform ist ein jedes zweite Primer-Oligonukleotid spezifisch für je eine zu amplifizierende Nukleinsäure.

In einer Ausführungsform ist ein jedes zweite Primer-Oligonukleotid spezifisch für mindestens zwei der zu amplifizierenden Nukleinsäuren, welche jeweils im Wesentlichen unterschiedliche Sequenzen umfassen.

In einer Ausführungsform ist das zweite Primer-Oligonukleotid mit einem charakteristischen Marker markiert, z.B. einem Fluoreszenzfarbstoff (z.B. TAMRA, Fluoreszein, Cy3, Cy5) oder einem Affinitätsmarker (z.B. Biotin, Digoxigenin) oder einem zusätzlichen Sequenzfragment, z.B. zur Bindung einer spezifischen Oligonukleotid-Sonde für Detektion oder Immobilisierung oder zur Barcode-Markierung.

In einer Ausführungsform wird mindestens ein zweites Primer-Oligonukleotid als allel-spezifischer Primer in Kombination mit mindestens einem entsprechenden allel-spezifischen Aktivator-Oligonukleotid bereitgestellt.

### Primer-Verlängerungsprodukt:

Ein Primer-Verlängerungsprodukt (auch Primer-Extensionsprodukt genannt) entsteht durch enzymatische (Polymerase-abhängige) Verlängerung eines Primer-Oligonukleotides als Ergebnis einer matrizenabhängigen Synthese, welche durch eine Polymerase katalysiert wird.

Ein Primer-Verlängerungsprodukt umfasst die Sequenz des Primer-Oligonukleotid in seinem 5'-Segment und die Sequenz des Verlängerungsproduktes (auch ExtensionsProdukt genannt), welches durch eine Polymerase in matrizenabhängiger Form synthetisiert wurde. Das durch die Polymerase synthetisierte Verlängerungsprodukt ist komplementär zum Matrizenstrang, an welchem es synthetisiert wurde.

Ein spezifisches Primer-Verlängerungsprodukt (Hauptprodukt) umfasst Sequenzen der zu amplifizierenden Nukleinsäurekette. Es ist Ergebnis einer spezifischen Synthese bzw. einer korrekten Ausführung einer beabsichtigten Primer-Verlängerungsreaktion bei welcher die spezifisch zu amplifizierende Nukleinsäurekette als Matrize dient. In einer bevorzugten Ausführungsform stimmt die Sequenz der synthetisierten Primer-Verlängerungsprodukte mit der zu erwartenden Sequenz einer zu amplifizierenden Nukleinsäure komplett überein. In einer anderen Ausführungsform können Abweichungen in der erhaltenen Sequenz von der theoretisch zu erwartenden Sequenz toleriert werden. In einer Ausführungsform liegt das Ausmaß der Übereinstimmung der erhaltenen Sequenz infolge einer Amplifikation mit der Sequenz der theoretisch zu erwartenden zu amplifizierenden Nukleinsäure beispielsweise zwischen 90 % und 100 %, bevorzugt liegt die Übereinstimmung bei über 95 %, idealerweise liegt die Übereinstimmung über 98 % (gemessen am Anteil der synthetisierten Basen).

Die Länge des Verlängerungsproduktes eines spezifischen Primer-Verlängerungsproduktes kann zwischen 10 und 300 Nukleotiden, besser zwischen 10 und 180 Nukleotiden liegen, bevorzugt zwischen 20 und 120 Nukleotiden, besonders bevorzugt zwischen 30 und 80 Nukleotiden.

Ein unspezifisches Primer-Verlängerungsprodukt (Nebenprodukt) umfasst beispielsweise Sequenzen, welche als Ergebnis einer unspezifischen bzw. inkorrekten bzw. nicht beabsichtigten Primer-Verlängerungsreaktion entstanden sind. Diese schließen beispielsweise Primer-Verlängerungsprodukte ein, welche als Folge eines falschen Initüerungs-Ereignisses (falsches Primings) oder als Folge anderer Nebenreaktionen, einschließlich polymerase-abhängigen Sequenzveränderungen wie Basen-Substitution, Deletion etc., entstanden sind. Das Ausmaß an Sequenzabweichungen von unspezifischen Primer-Verlängerungsprodukten übersteigt im Allgemeinen die Fähigkeit von Aktivator-Oligonukleotide, solche doppelsträngige Nebenprodukte erfolgreich von ihren Matrizen zu verdrängen, so dass Amplifikation von solchen Nebenprodukten langsamer verläuft oder vollständig ausbleibt. Das Ausmaß der Akzeptanz bzw. die Toleranzgrenze zu Abweichungen sind abhängig beispielsweise von Reaktionstemperatur und Art und Weise der Sequenabweichung. Beispiele für unspezifische Primer-Verlängerungsprodukte bilden Primer-Dimere oder Sequenzvarianten, welche nicht der zu amplifizierenden Nukleinsäure entsprechen, z.B. Sequenzen, welche keine Zielsequenz umfassen.

Die Beurteilung einer hinreichenden Spezifität der Amplifikation hängt häufig mit der Aufgabenstellung zusammen. In vielen Amplifikationsverfahren kann ein gewisses Maß der Unspezifität der Amplifikationsreaktion toleriert werden, solange das gewünschte Ergebnis erreicht werden kann. In einer bevorzugten Ausführungsform beträgt der Anteil von zu amplifizierenden Nukleinsäureketten am Gesamtergebnis der Reaktion mehr als als 1 %, besser mehr als 10 %, noch bevorzugter mehr als 30 % gemessen an Gesamtmenge von neusynthetisierten Stränge.

### Die zu amplifizierende Nukleinsäure

Die zu amplifizierende Nukleinsäure stellt eine Nukleinsäurekette dar, welche sequenzspezifisch oder zumindest vorwiegend sequenzspezifisch mit Hilfe der exponentiellen Amplifikation unter Einsatz von Primern und Aktivator-Oligonukleoid durch die Polymerase amplifiziert werden soll.

Die Länge der zu amplifizierenden Nukleinsäure kann zwischen 20 und 300 Nukleotiden, besser zwischen 30 und 200 Nukleotiden liegen, bevorzugt zwischen 40 und 150 Nukleotiden, besonders bevorzugt zwischen 50 und 100 Nukleotiden.

Die zu amplifizierende Nukleinsäurekette kann eine oder mehrere Zielsequenzen oder ihre Äquivalente umfassen. Weiterhin kann eine zu amplifizierende Nukleinsäure die im Wesentlichen zu einer Zielsequenz komplementären Sequenzen umfassen, welche mit ähnlicher Effizienz wie eine Zielsequenz in einer Amplifikationsreaktion vermehrt werden und Zielsequenz oder ihre Abschnitte umfasst. Zusätzlich zu einer Zielsequenz kann die zu amplifizierende Nukleinsäure weitere Sequenzsegmente einschließen, beispielsweise Primer-Sequenzen, Sequenzen mit Primer-Bindungsstellen und /oder Sequenzsegmente für Bindung von Detektions-Sonden, und / oder Sequenzsegmente für Sequenz-Kodierung von Strängen durch Barcode-Sequenzen und / oder Sequenzsegmente für Bindung an eine feste Phase. Die Primer-Sequenzen oder ihre Sequenzanteile, sowie Primer-Bindungsstellen oder ihre Sequenzanteile können beispielsweise zu Sequenzabschnitten einer Zielsequenz gehören.

In einer Ausführungsform entspricht die zu amplifizierende Nukleinsäure der Zielsequenz.

In einer anderen Ausführungsform bildet die Zielsequenz einen Teil der Sequenz der zu amplifizierenden Nukleinsäurekette. Eine solche Zielsequenz kann von 3'-Seite und/oder von 5'-Seite von weiteren Sequenzen flankiert sein. Diese weiteren Sequenzen können beispielsweise Bindungsstellen für Primer oder ihre Anteile umfassen, und/oder Primer-Sequenzen oder ihre Anteile umfassen, und/oder Bindungsstellen für Detektions-Sonden umfassen, und/oder Adaptor-Sequenzen für komplementäre Bindung an eine feste Phase (z.B. Im Rahmen von Microarrays, oder Bead-basierten Analysen) umfassen und/oder Barcoding-Sequenzen für eine digitale Signatur von Sequenzen umfassen.

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt. Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurekette von Bedeutung sind.

In einer bevorzugten Ausführungsform entspricht die initiale Matrize (Start-Nukleinsäurekette), welche einer Amplifikationsreaktion zu Beginn zugeführt wird, bzw. welche in das Reaktionsgemisch gegeben wird, der Sequenz-Zusammensetzung der zu amplifizierenden Nukleinsäurekette.

In anfänglichen Stadien der Amplifikations-Reaktion und in ihrem weiteren Verlauf binden die jeweiligen Primer an die entsprechenden Bindungsstellen in der Start-Nukleinsäurekette und initiieren die Synthese von spezifischen Primer-Verlängerungsprodukten. Solche spezifischen Primer-Verlängerungsprodukte akkumulieren im Verlauf der Amplifikation in exponentieller Art und Weise und übernehmen zunehmend die Rolle von Matrizen für Synthese komplementärer Primer-Verlängerungsprodukte bei einer exponentiellen Amplifikation.

Durch die wiederholten matrizen-abhängige Synthese-Vorgänge im Rahmen einer exponentiellen Amplifikation wird somit die zu amplifizierende Nukleinsäurekette gebildet.

Gegen Ende einer Amplifikationsreaktion kann das Hauptprodukt der Reaktion (die zu amplifizierende Nukleinsäure) vorwiegend einzelsträngig sein oder vorwiegend einen komplementären Doppelstrang bilden. Dies kann beispielsweise durch die relative Konzentrationen von beiden Primern und entsprechende Reaktionebedingungen bestimmt werden.

Äquivalente der zu amplifizierenden Nukleinsäure umfassen Nukleinsäuren mit im Wesentlichen identischen Informationsgehalt. Beispielsweise haben komplementäre Stränge einer zu amplifizierenden Nukleinsäure identischen Informationsgehalt und können als äquivalent bezeichnet werden.

### Zielsequenz

Eine Zielsequenz ist in einer Ausführungsform ein Segment einer zu amplifizierenden Nukleinsäurekette, welches als charakteristische Sequenz der zu amplifizierenden Nukleinsäure dienen kann. Diese Zielsequenz kann als Marker für die Anwesenheit oder Abwesenheit einer anderen Nukleinsäure dienen. Diese andere Nukleinsäure dient somit als Quelle der Zielsequenz und kann beispielsweise eine genomische DNA oder RNA oder Teile der genomischen DNA oder RNA (z.B. mRNA), oder Äquivalente der genomischen DNA oder RNA eines Organismus sein (z.B. cDNA, modifizierte RNA wie rRNA, tRNA, microRNA etc.), oder definierte Veränderungen der genomischen DNA oder RNA eines Organismus umfassen, beispielsweise Mutationen (z.B. Deletionen, Insertionen, Substitutionen, Additionen, Sequenzvermehrung, z.B. Repeat-Vermehrung im Rahmen von Mikrosatellit-Instabilität), Splice-Varianten, Rearrangement-varianten (z.B. T-Zell-Rezeptor Varianten) usw. Die einzelnen Zielsequenzen können für ein phänotypisches Merkmal stehen, beispielsweise für Antibiotika-Resistenz oder prognostische Information haben und somit für diagnostische Assays/Tests vom Interesse sein. Als Quelle/Ursprung einer Zielsequenz kann eine solche Nukleinsäure beispielsweise die Zielsequenz als Sequenz-Element ihres Stranges umfassen. Eine Zielsequenz kann somit als charakteristischer Marker für einen bestimmten Sequenzinhalt einer anderen Nukleinsäure dienen.

Die Zielsequenz kann einzelsträngig oder doppelsträngig sein. Sie kann mit der zu amplifizierenden Nukleinsäure im Wesentlichen identisch sein oder nur einen Teil der zu amplifizierenden Nukleinsäure darstellen.

Äquivalente der Zielsequenz umfassen Nukleinsäuren mit im wesentlichen identischen Informationsgehalt. Beispielsweise haben komplementäre Stränge einer Zielsequenz identischen Informationsgehalt und können als äquivalent bezeichnet werden, RNA und DNA Varianten einer Sequenz sind ebenfalls Beispiele für äquivalenten Informationsgehalt.

Im Rahmen der Material-Vorbereitung vor Amplifikations-Reaktion kann eine solche Zielsequenz aus ihrer ursprünglichen Sequenzumgebung isoliert werden und für die Amplifikationsreaktion vorbereitet werden.

In einer bevorzugten Ausführungsform umfasst eine zu amplifizierende Nukleinsäure eine Zielsequenz. In einer Ausführungsform entspricht die Zielsequenz der zu amplifizierenden Nukleinsäure. In einer weiteren bevorzugten Ausführungsform umfasst eine Start-Nukleinsäurekette eine Zielsequenz. In einer Ausführungsform entspricht die Zielsequenz einer Start-Nukleinsäurekette.

Allel-Varianten einer Zielsequenz/Polymorpher Lokus einer Zielsequenz

Eine Zielsequenz (z.B. ein charakteristisches Segment einer DNA oder RNA) kann mehrere in der Natur vorkommende Sequenzvarianten oder künstlich eingeführte Sequenzveränderungen umfassen. Solche Varianten werden häufig als Polymorphismen eines Lokus oder Allel-Sequenzen genannt. Weiterhin können diese Varianten einer Zielsequenz spezifische Veränderungen des natürlichen Zustandes der DNA abbilden, welche z.B. durch Mutationen/Mutagenese enstehen und bei Vermehrung einer Zell-masse charakteristische Merkmale eines solchen klonalen Vermehrungvorgangs darstellen (z.B. bei Krebs-Erkrankungen oder bei biotechnologischen verwertbaren Stämmen). Bei einzelnen Allel-Sequenzen einer Zielsequenz kann es sich dabei um Sequenzunterschiede handeln, welche einzelne Nukleotid-Positionen umfassen (z.B. A-> G oder T->C Substitutionen oder Einzelnukleotid-Insertionen bzw. Deletionen) oder ein Sequenzunterschied kann auch mehrere Nukleotide umfassen (z.B. 2 bis 200 Nukleotide). Dabei kann es sich beispielsweise um Deletionen / Insertionen / Transversionen / Duplikationen etc. handeln. Ein Sequenzsegment umfassend solche Sequenzvarianten einer Zielsequenz wird häufig als polymorpher Lokus genannt. Eine Zielsequenz kann einen oder auch mehrere polymorphe Loci umfassen.

Die Sequenz-Positionen von einzelnen Allel-Varianten einer Zielsequenz sind in der Regel bekannt und erlauben somit ein Design von sequenzspezifischen Reaktions-Komponenten, z.B. Aktivator-Oligonukleotide oder von Kombinationen umfassend ein Aktivator-Oligonukleotid und einen ersten Primer oder ein Aktivator-Oligonukleotid und einen zweiten Primer.

Die Ausführungsformen von Zielsequenzen:
In einer Ausführungsform umfasst eine Zielsequenz, welche mehrere Sequenzvarianten in einem polymorphen Lokus umfassen kann, weiterhin mindestens ein erstes Zielsequenzsegment, welches für alle Zielsequenzvarianten einer Zielsequenz (Zielsequenz-Gruppe) charakteristisch und einheitlich ist. In einer weiteren Ausführungsform umfasst eine Zielsequenz mindestens zwei Zielsequenzsegmente (ein erstes Zielsequenzsegment und ein zweites Zielsequenzsegment, jedes dieser Zielsequenzsegmente für alle Zielsequenzvarianten einer Zielsequenz (Zielsequenz-Gruppe) charakteristisch und einheitlich ist. Solche einheitliche Zielsequenzsegmente sind vorzugsweise auf beiden Seiten eines polymorphen Lokus lokalisiert und flankieren somit einen polymorpehen Lokus (mit Sequenzvarianten) einer Zielsequenz von beiden Seiten. Vorzugsweise unterscheiden sich das erste einheitliche Zielsequenzsegment in seiner Sequenzzusammensetzung von der Sequenzzusammensetzung des zweiten einheitlichen Zielsequenzsegments in mindestens einer Nukleotid-Position.

Die Länge eines polymorphen Lokus einer Zielsequenz kann Bereiche von einem Nukleotid bis zu 200 Nukleotiden, besser von einem Nukleotid bis zu 100 Nukleotiden, bevorzugt von einem Nukleotid bis zu 50 Nukleotiden, besonders bevorzugt von einem Nukleotid bis zu 20 Nukleotiden umfassen. Die Längen von einem einheitlichen Sequenzsegmenten (erstes und zweite einheitliches Sequenzsegment) kann zumindest für eines der beiden Segmente folgende Bereiche umfassen: von 6 bis 300 Nukleotiden, besser von 10 bis 200 Nukleotiden, bevorzugt von 15 bis 100 Nukleotiden, besonders bevorzugt von 20 bis 100 Nukleotiden.

In einer Ausführungsform können einzelne Sequenz-Varianten einer Zielsequenz-Gruppe zusammengefasst werden, wobei solche Varianten mindestens ein für diese Zielsequenz (Zielsequenz-Gruppe) einheitliches und spezifisches Sequenzsegmenten definiert sind. Einzelne Sequenzvarianten einer solchen Zielsequenz (Zielsequenz-Gruppe) unterscheiden sich somit durch Sequenz-Segmente des polymorphen Lokus.

In einer weiteren Ausführungsform können einzelne Sequenz-Varianten einer Zielsequenz-Gruppe zusammengefasst werden, wobei solche Varianten mindestens zwei für diese Zielsequenz (Zielsequenz-Gruppe) einheitliche und spezifische Sequenzsegmente definiert sind. Einzelne Sequenzvarianten einer solchen Zielsequenz (Zielsequenz-Gruppe) unterscheiden sich somit durch Sequenz-Segmente des polymorphen Lokus. Vorzugsweise liegt der polymorphe Lokus zwischen beiden einheitlichen Sequenzsegmenten.

In einer Ausführungsform können zwei unterschiedliche Zielsequenzen in einer Amplifikations-Reaktion jeweils spezifisch amplifiziert werden.

Unterschiedliche Zielsequenzen sind dadurch charakterisiert, dass sie vorzugsweise durch für jede Zielsequenz spezifische und charakteristische Sequenzen unter einander differenzierbar sind. Vorzugsweise sind unterschiedliche Zielsequenzen in ihrer gesamten Länge durch jeweils spezifische und charakteristische Sequenzen differenzierbar und somit keine Sequenzsegmente umfassen, welche für beide Zielsequenzen identisch sind. In einer weiteren Ausführungsform können unterschiedliche Zielsequenzen mindesten ein Sequenzsegment umfassen, welches für mindestens zwei unterschiedliche Zielsequenzen im Wesentlichen ähnlich oder sogar identisch st. Die Länge eines solchen Sequenzsegmentes beträgt gleich / oder weniger als 19 Nukleotide (gezählt als aneinander gekoppelte Nukleotid-Positionen), besser gleich / oder weniger als 14 Nukleotide (als aneinander gekoppelte Nukleotid-Positionen), bevorzugt gleich / oder weniger als 9 Nukleotide (als aneinander gekoppelte Nukleotid-Positionen), besonders bevorzugt gleich / oder weniger als 5 Nukleotide (als aneinander gekoppelte Nukleotid-Positionen)

### Start-Nukleinsäurekette

Damit die Amplifikation starten kann, muss zu Beginn der Reaktion eine Nukleinsäurekette in das Reaktionsgemisch gegeben werden, welche als initiale Matrize für die Synthese der zu amplifizierenden Nukleinsäurekette auftritt. Diese Nukleinsäurekette wird als Start-Nukleinsäurekette bezeichnet. Diese Start-Nukleinsäurekette gibt die Anordnung einzelner Sequenz-Elemente vor, welche für die Bildung / Synthese / exponentielle Amplifikation einer zu amplifizierenden Nukleinsäurkette von Bedeutung sind.

Eine solche Start-Nukleinsäurekette kann einzelsträngig oder doppelsträngig zu Beginn der Reaktion vorliegen. Wenn die komplementären Stränge der Start-Nukleinsäurekette voneinander getrennt sind, können die Stränge unabhängig davon, ob die Nukleinsäure ursprünglich doppel- oder einzelsträngig vorlag, als Matrize für die Synthese von spezifischen komplementären Primer-Verlängerungsprodukte dienen.

Die Start-Nukleinsäurekette umfasst in einer Ausführungsform eine Zielsequenz oder ihre Teil-Segmente, welche zumindest eine zu erwartende Sequenz-Variante eines polymorphen Lokus der Zielsequenz umfassen. Eine Start-Nukleinsäurekette umfasst in einer weiteren Ausführungsform eine Zielsequenz oder ihre Teil-Segmente, welche zumindest eine zu erwartende Sequenz-Variante eines polymorphen Lokus der Zielsequenz und mindestens ein einheitliches Sequenzsegment einer Zielsequenz umfassen.

Eine Start-Nukleinsäurekette umfasst in einer weiteren Ausführungsform eine Zielsequenz oder ihre Teil-Segmente, welche zumindest eine zu erwartende Sequenz-Variante eines polymorphen Lokus der Zielsequenz und mindestens zwei einheitliche Sequenzsegmente einer Zielsequenz umfassen, wobei einheitliche Sequenzsegmente einer Zielsequenz den polymorphen Lokus mit möglichen Sequenzvarianten auf beiden Seiten flankieren.

Eine Start-Nukleinsäure umfasst weiterhin zumindest ein vorwiegend einzelsträngiges Sequenzsegment, zu weilchem mindestens eines der Primer des Amplifikations-Systems mit seinem 3'-Segment vorwiegend komplementär binden kann, so dass verwendete Polymerase einen solchen Primer, wenn hybridisiert an die Start-Nukleinsäurekette, matrizenspezifische unter Einbau von dNTPs verlängern kann.

### Aktivator-Oligonukleotid:

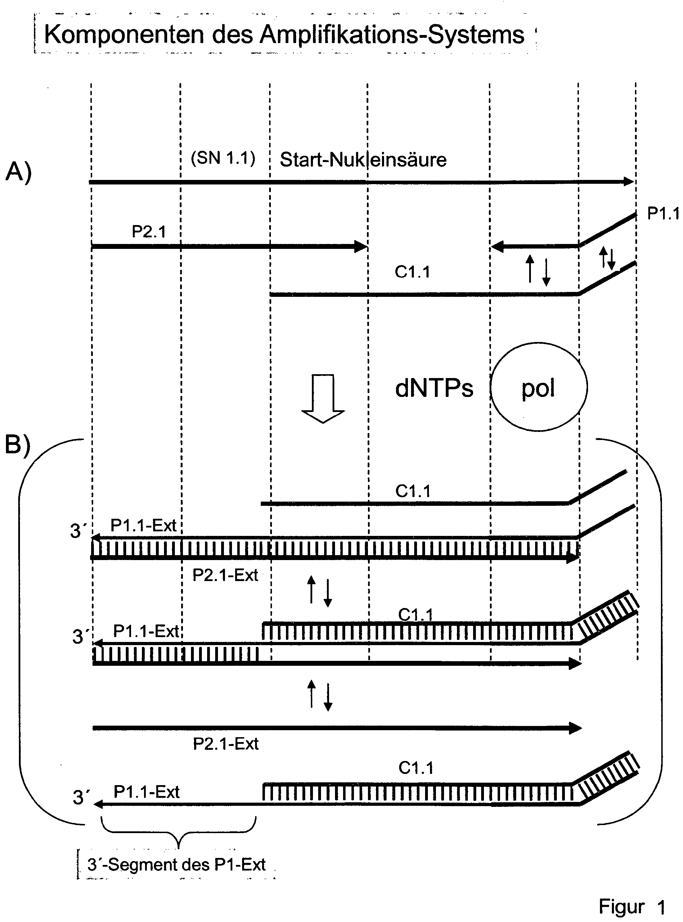

Das Aktivator-Oligonukleotid (Fig. 1) ist eine bevorzugt einzelsträngige Nukleinsäurekette, welche eine vordefinierte im wesentlichen komplementäre Sequenz in einem Teil des ersten Primer-Verlängerungsprodukts einschließt, welches im Rahmen der Amplifikation der zu amplifizierenden Nukleinsäure spezifisch generiert wird. Dadurch kann das Aktivator-Oligonukleotid an das erste Primer-Oligonukleotid und mindestens an das 5'-Segment des spezifischen Verlängerungsprodukts des ersten Primer-Oligonukleotid im Wesentlichen komplementär binden. Das Aktivator-Oligonukleotid umfasst in seinen inneren Sequenzsegment in einer Ausführungsform Nukleotid-Modifikationen, welche die Polymerase daran hindern, einen komplementären Strang unter Verwendung des Aktivator-Oligonukleotides als Matrize zu synthetisieren, wenn das erste Primer-Oligonukleotid an das Aktivator-Oligonukleotid komplementär gebunden ist. Das Aktivator-Oligonukleotid ist weiterhin in der Lage, unter den gewählten Reaktionsbedingungen das zweite spezifische Primer-Verlängerungsprodukt vollständig oder teilweise aus der Bindung mit dem ersten spezifischen Primer-Verlängerungsprodukt via Strangverdrängung zu verdrängen. Dabei lagert sich das Aktivator-Oligonukleotid mit seinen komplementären Bereichen an das erste spezifische Primer-Verlängerungsprodukt an. Bei erfolgreicher Bindung zwischen dem Aktivator-Oligonukleotid und dem ersten spezifischen Primer-Verlängerungsprodukt führt dies zu Wiederherstellung eines einzelsträngigen Zustandes des 3'-ständigen Segments des zweiten spezifischen Primer-Verlängerungsproduktes, welches für die Bindung des ersten Primer-Oligonukleotides geeignet ist, so dass eine neue Primer-Verlängerungsreaktion stattfinden kann. Während der Synthese des zweiten Primer-Verlängerungsproduktes kann das Aktivator-Oligonukleotid aus der Bindung mit dem ersten Primer-Verlängerungsprodukt mittels Strangverdrängung beispielsweise durch Polymerase und/oder durch das zweite Primer-Oligonukleotid getrennt werden.

Zielsequenz-spezifisches Aktivator-Oligonukleotid wird vorzugsweise dermaßen konstruiert, dass es in der Lage ist, bei Amplifikation von bevorzugt einer definierten spezifischen Sequenz-Variante einer vordefinierten Zielsequenz mitzuwirken.

Allel-spezifisches Aktivator-Oligonukleotid wird vorzugsweise dermaßen konstruiert, dass es in der Lage ist, die Amplifikation einer spezifischen Sequenz-Varianten einer Zielsequenz (z.B. Allel 1) bevorzugt zu bewirken, wobei bei einer anderen Sequenz-Variante dergleichen Zielsequenz (z.B. Allel 2) verläuft die Amplifikation mit verminderter Effizienz bzw. findet in der vorgegebener Zeit gar nicht statt, bzw. resultiert in einer nicht für eine Detektion hinreichender Ausbeute an Amplifikationsprodukten. Somit ergeben sich messbare Unterschiede im Verlauf einer Amplifikations-Reaktion, welche davon abhängen, ob die Sequenz eines für eine Allel-Variante spezifischen Aktivator-Oligonukleotids mit der Sequenz einer zu Beginn der Reaktion bereitgestellte Nukleinsäurekette, welche als Start-Nukleionsäure dient und die Zielsequenz mit einem polymorphen Lokus umfasst, spezifisch übereinstimmt oder nicht. Bei Übereinstimmung bzw. perfekt-match Komplementarität zwischen der Sequenzzusammensetzung des polymorphen Lokus einer Start-Nukleinsäurekette und einem korrespondierenden Sequenzsegment im Aktivator-Oligonukleotid erfolgt eine spezifische charakteristische Amplifikation. Bei fehlender Übereinstimmung bzw. Mismatch Komplementarität zwischen der Sequenzzusammensetzung des polymorphen Lokus einer Start-Nukleinsäurekette und einem korrespondierenden Sequenzsegment im Aktivator-Oligonukleotid verläuft die Amplifikation anders als eine spezifische charakteristische Amplifikation.

Ein Allel-spezifisches Aktivator-Oligonukleotid wird somit nicht nur zielsequenzspezifisch, sonder auch allel-spezifisch bereitgestellt. Je nach Komplexität eines polymorphen Lokus einer Zielsequenz können somit mehrere, für jeweilige allel-Variante einer Zielsequenz spezifische Aktivator-Oligonukleotide konstruiert werden.

Vorzugsweise umfasst ein Aktivator-Oligonukleotid mindestens ein Sequenzsegment, welches zu einer spezifischen Allel-Variante einer Zielsequenz komplementäre Sequenzzusammensetzung aufweist. Weiterhin umfasst ein Aktivator-Oligonukleotid vorzugsweise mindestens ein Sequenzsegment, welches für alle Sequenzvarianten einer Zielsequenz einheitliche komplementäre Sequenz umfasst. In einer Ausführungsform umfasst ein Aktivator-Oligonukleotid, welches mindestens ein Sequenzsegment umfasst, welches komplementär an eine Variante eines polymorphen Lokus einer Zielsequenz komplementär binden kann.

In einer Ausführungsform werden mehrere Aktivator-Oligonukleotide bereitgestellt, welche jeweils allel-spezifische Sequenzsegmente umfassen.

Die Länge eines zum polymorphen Lokus einer Start-Nukleinsäureke komplementären Sequenzsegmentes eines Aktivator-Oligonukleotide kann Bereiche von einem Nukleotid bis zu 100 Nukleotiden, besser von einem Nukleotid bis zu 50 Nukleotiden, bevorzugt von einem Nukleotid bis zu 20 Nukleotiden umfassen. Die Länge von mindestens einem Sequenzsegment eines Aktivator-Oligonukleotides, welches zu einheitlichen Sequenzsegmenten einer Zielsequenz (erstes und zweite einheitliches Sequenzsegment einer Start-Nukleinsäure) komplementär ist, kann folgende Bereiche umfassen: von 4 bis 100 Nukleotide, besser von 6 bis 100 Nukleotide, bevorzugt von 8 bis 50 Nukleotide.

In einer Ausführungsform wird ein Sequenzsegment eines Aktivator-Oligonukleotides, welches zu mindestens einer Variante eines polymophen Lokus einer Zielsequenz komplementär ist, im dritten Bereich des Aktivator-Oligonukleotides lokalisiert.

In einer weiteren Ausführungsform wird ein Sequenzsegment eines Aktivator-Oligonukleotides, welches zu mindestens einer Variante eines polymophen Lokus einer Zielsequenz komplementär ist, im zweiten Bereich des Aktivator-Oligonukleotides lokalisiert.

In einer weiteren Ausführungsform wird ein Sequenzsegment eines Aktivator-Oligonukleotides, welches zu mindestens einer Variante eines polymophen Lokus einer Zielsequenz komplementär ist, teilweise und/oder vollständig im zweiten und im dritten Bereich des Aktivator-Oligonukleotides lokalisiert.

Die Ausbildung von Basenpaaren unter zwei Nukleinsäuresträngen kann im Wesentlichen komplementär erfolgen, das bedeutet, dass zwischen 70 % und 100 %, besser zwischen 90 % und 100 %, bevorzugt zwischen 95 % und 100 % von Nukleotiden eines Stranges komplementär aneinander binden können. Bespielsweise kann eine Nukleotid-Position in einem Strang von 20 Nukleotiden keine komplementäre Bindung aufweisen, das entspricht 95 % Komplementarität.

Bei Perfekt-Match-Bindung von zwei Sequenzen erfolgt vorzugsweise Ausbildung von komplementären Basenpaarungen zu 100% zwischen beiden Strängen einer Duplex. Eine perfekt-match Duplex, umfasst somit einen Mismatch zwischen Strängen.

### Amplifikations-System:

Ein Amplifikations-System umfasst mindestens ein zielsequenz-spezifisches Aktivator-Oligonukleotid, mindesten ein erstes Primer-Oligonukleotid und mindestens ein zweites Primer-Oligonukleotid, sowie mindestens eine matrizenabhängige Polymerase, welche in der Lage ist, unter Verwendung einer Start-Nukleinsäurekette als Matrize, welche mindestens eine charakteristische Zielsequenz oder Zielsequenz-Variante umfasst, unter geeigneten Reaktionsbedingungen eine vorwiegend spezifische Amplifikation der zu amplifizierenden Nukleinsäurekette zu unterstützen, wobei vorwiegend zielsequenz spezifische erste Primer-Verlängerungsprodukte und vorwiegend spezifische zweite Primer-Verlängerungsprodukte synthetisiert werden.

Ein allel-spezifisches Amplifikations-System umfasst mindestens ein allel--spezifisches Aktivator-Oligonukleotid, mindestens ein erstes Primer-Oligonukleotid und mindestens ein zweites Primer-Oligonukleotid (Primer können je nach Design zielsequenzspezifisch und / oder allel-spezifisch sein), sowie mindestens eine matrizenabhängige Polymerase, welche in der Lage ist, unter Verwendung einer Start-Nukleinsäurekette als Matrize, welche mindestens eine Zielsequenz-Variante umfasst, unter geeigneten Reaktionsbedingungen eine vorwiegend spezifische Amplifikation der Zielsequenz-Variante (ein Allel) der zu amplifizierenden Nukleinsäurekette zu unterstützen, wobei vorwiegend spezifische erste Primer-Verlängerungsprodukte und vorwiegend spezifische zweite Primer-Verlängerungsprodukte synthetisiert werden. Vorzugsweise sind beide Primer-Verlängerungsprodukte allel-spezifisch.

Ein Amplifikations-System kann mehrere allel-spezifische Amplifikations-Systeme umfassen, wobei jedes allel-spezifisches Amplifikations-System bevorzugt mindestens eine der Zielsequenz-Variante amplifiziert.

### Strangverdrängung (Strand Displacement):

Hiermit wird ein Vorgang bezeichnet, welcher durch Einwirkung eines geeigneten Mittels zu einer vollständigen oder partiellen Trennung eines ersten Doppelstranges (bestehend beispielsweise aus A1 und B1-Strang) führt, und zur gleichzeitigen / parallelen Ausbildung eines neuen zweiten Doppelstranges, wobei mindestens einer der Stränge (A1 oder B1) an der Ausbildung dieses neuen zweiten Stranges beteiligt sind. Man kann hier zwei Formen der Strangverdrängung unterschieden.

In einer ersten Form der Strangverdrängung kann die Ausbildung eines neuen zweiten Doppelstranges unter Verwendung eines bereits existierenden komplementären Stranges erfolgen, welcher zu Beginn der Reaktion im Allgemeinen in einzelsträngiger Form vorliegt. Dabei wirkt das Mittel der Strangverdrängung (beispielsweise ein vorgebildeter einzelsträngiger Strang C1, welcher eine komplementäre Sequenz zum Strang A1 aufweist, auf den ersten bereits ausgebildeten Doppelstrang (A1 und B1) und geht mit dem Strang A1 eine komplementäre Bindung ein, wodurch der Strang B1 aus der Bindung mit dem Strang A1 verdrängt wird. Falls die Verdrängung des B1 vollständig abläuft, so ist das Ergebnis der C1-Wirkung ein neuer Doppelstrang (A1:C1) und ein einzelsträngiger Strang B1. Falls die Verdrängung von B1 unvollständig abläuft, so hängt das Ergebnis von mehreren Faktoren ab. Beispielsweise kann ein Komplex aus partiell doppelsträngigen A1:B1 und A1:C1 als Zwischenprodukt vorliegen.

In einer zweiten Form der Strangverdrängung kann die Ausbildung eines neuen zweiten Doppelstranges unter einer gleichzeitig ablaufenden enzymatischen Synthese des komplementären Stranges erfolgen, wobei ein Strang des ersten vorgebildeten Doppelstranges als Matrize für die Synthese durch die Polymerase auftritt. Dabei wirkt das Mittel der Strangverdrängung (beispielsweise Polymerase mit einer Strangverdrängungs-Fähigkeit), auf den ersten bereits vorgebildeten Dopp⁻ Istrang (A1 und B1) und synthetisiert einen zu Strang A1 neuen komplementären Strang D1, wobei gleichzeitig der Strang B1 aus der Bindung mit dem Strang A1 verdrängt wird.

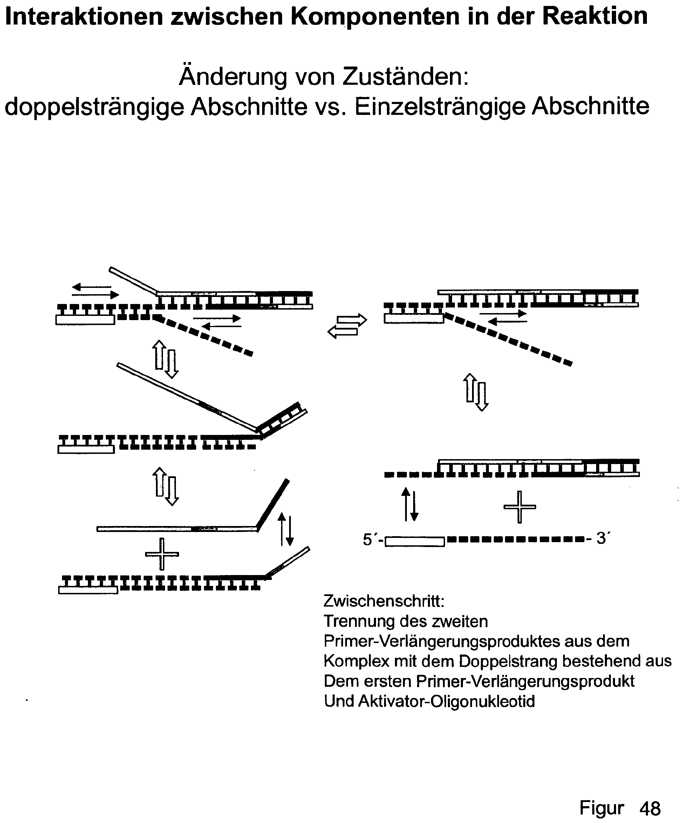

Unter dem Begriff "nukleinsäurevermittelte Strang-Verdrängung" wird eine Summe/Reihe von Zwischenschritten zusammengefasst, welche untereinander im Gleichgewicht stehen können, und im Ergebnis zur vorübergehenden oder dauerhaften Öffnung einer ersten vorgeformten Duplex (bestehend aus komplementären Strängen A1 und B1) und Ausbildung einer neuen zweiten Duplex (bestehend aus komplementären Strängen A1 und C1), wobei A1 und C1 einander komplementär sind. Dieser Vorgang ist im Fig. 48 veranschaulicht (s.u.).

Es ist bekannt, dass eine wesentliche strukturelle Voraussetzung für die Initiierung einer Strangverdrängung die Herbeiführung einer räumlichen Nähe zwischen einem Duplex-Ende (vorgeformter erster Duplex aus A1 und B1) und einem einzelsträngigen Strang (C1) ist, welcher die Strangverdrängung initiiert (wobei A1 und C1 einen komplementären Strang bilden können). Eine solche räumliche Nähe kann vorzugsweise mittels eines einzelsträngigen Überhanges (in der Literatur sind Beispiele mit kurzen Überhängen bekannt, im Englischen genannt "Toehold") herbeigeführt werden, welcher den einzelsträngigen Strang (C1) vorübergehend oder dauerhaft komplementär bindet, und somit komplementäre Seqmente des Stranges C1 und A1 in hinreichende Nähe bringt, so dass eine erfolgreiche Strangverdrängung des Stranges B1 initiiert werden kann. Die Effizienz der Initiierung der nukleinsäurevermittelten Strang-Verdrängung ist im Allgemeinen um so höher, je näher die komplementäre Segmente des Stranges A1 und C1 zueinander positioniert werden.

Eine weitere wesentliche strukturelle Voraussetzung für die effiziente Fortführung einer nukleinsäurevermittelten Strangverdrängung in inneren Segmenten liegt in hoher Komplementarität zwischen Strängen (z.B. zwischen A1 und C1), welche einen neuen Doppelstrang ausbilden müssen. So können beispielsweise einzelne Nukleiotid-Mutationen (in C1) zur Unterbrechung einer Strangverdrängung (beschrieben z.B. für Branch-Migration) führen.

Die vorliegende Erfindung macht Gebrauch von der Fähigkeit komplementärer Nukleinsäuren zu einer sequenzabhängigen nukleinsäurevermittelten Strangverdrängung.

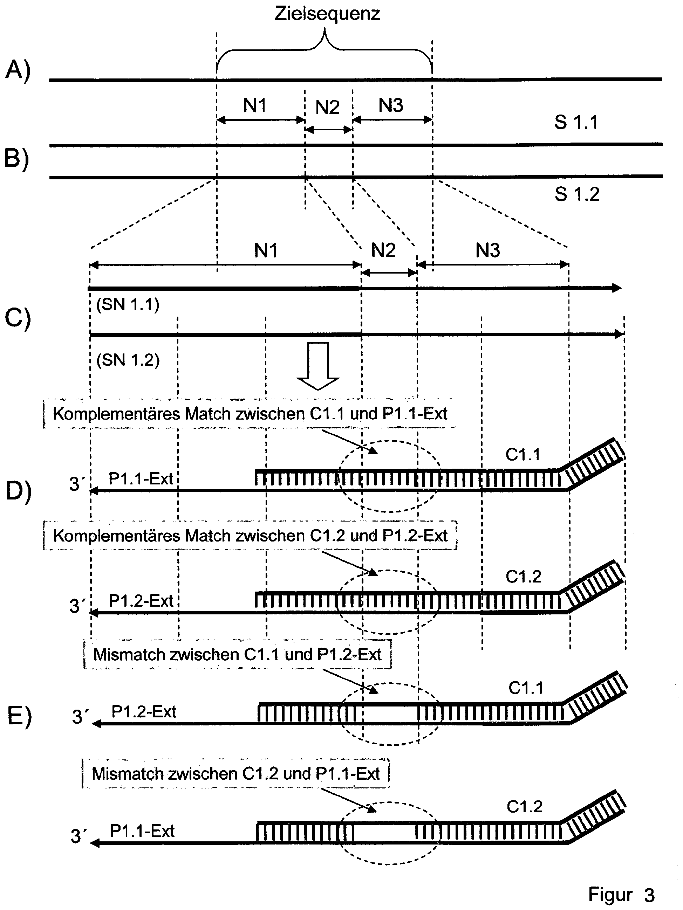

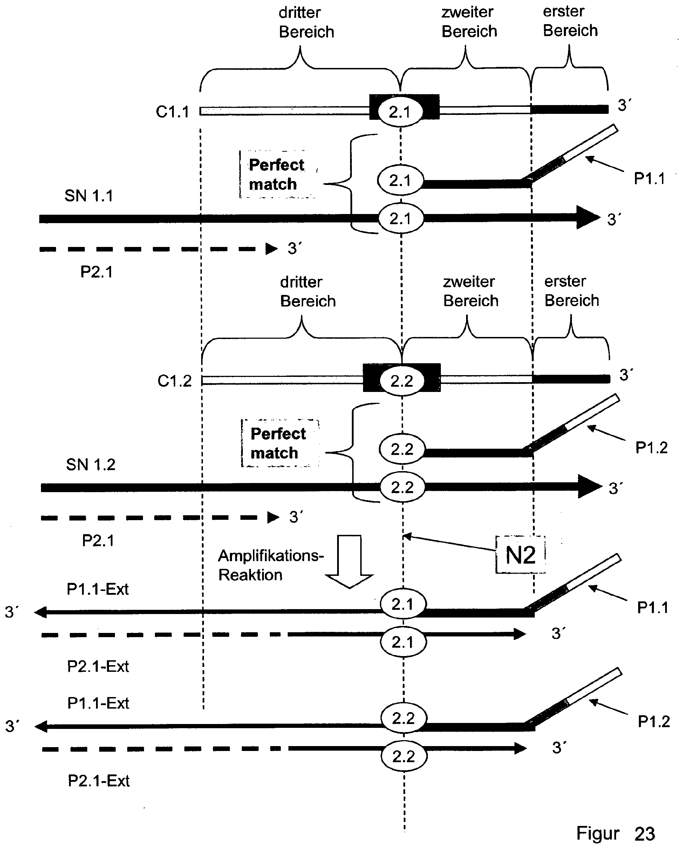

Bevorzugte Ausführungsformen der Erfindung werden in den Figuren und Beispielen näher erläutert.
Fig. 1A
   Zeigt schematisch eine Start-Nukleinsäurekette und Komponenten des Amplifikationssystems im Ansatz vor Amplifikationsstart:
   Start-Nukleinsäure (Start-NS), welche eine Zielsequenz umfasst.
   Primer 1 (P1.1), Primer 2 (P2.1), ein Aktivator-Oligonukleotid (C1.1).
   Komponenten für Primer-Verlängerungsreaktion: Polymerase (Pol) und dNTPs
Fig. 1 B
   Zeigt schematisch Ergebnis der Amplifikation:
   Primer-Verlängerungsprodukt P1.1-Ext ausgehnd von P1.1, Primer-Verlängerungsprodukt 2.1-Ext. Diese Produkte (P1.1-Ext, P2.1-Ext) können untereinader und mit Aktovator-Oligonukleotid unterschiedliche Komplexformen ausbilden (je nach Konzentrations-Verhältnis und Reaktionsbedingugnen). Im Einzelnen, können diese Formen Komplese aus P1.1-Ext / C1.1 und / oder P1.1-Ext und / oder P1.1-Ext / C1.1/ P2.1-Ext umfassen.
   Das P1-Ext umfasst ein 3'-Segment, welches nicht vom Aktivator-Oligonukleotid komplementär gebunden wird. Dieses Segment dient als Bindungspartner für die Oligonukleotid-Sonde.
Fig. 2A bis 2C
   Zeigt schematisch Topographie einer Zielnukleinsäurekette mit einem polymorphen Lokus (N2) und zwei einheitlichen Zielsequenzsegmenten (N1 und N3). Die Start-Nukleionsäureketten (SN 1.1 und SN 1.2) zeigen schematisch Topographie einer Zielnukleinsäuresequenz innerhalb von Start-Nukleinsäuren. Beide Start-Nukleionsäureketten umfassen Zielsequenzsegmentene N1 und N3, welche bei beiden Start-Nukleionsäureketten identisch und einheitlich sind. Das jeweilige Sequenzsegment N2 ist bei jeder Start-Nukleinsäure charakteristisch und sezifisch, so dass beide Start-Nukleisäuren dadurch unterschieden werden können.
   In dieser Ausführungsform umfasst eine Zielsequenz, welche mehrere Sequenzvarianten in einem polymorphen Lokus (N2) umfassen kann, weiterhin mindestens ein erstes Zielsequenzsegment (N1), welches für alle Zielsequenzvarianten einer Zielsequenz (Zielsequenz-Gruppe umfassend hier SN 1.1 und SN 1.2) charakteristisch und einheitlich ist. Weiterhin umfasst eine Zielsequenz mindestens ein zweites Zielsequenzsegment (N3), welches für alle Zielsequenzvarianten einer Zielsequenz (Zielsequenz-Gruppe umfassend hier SN 1.1 und SN 1.2) charakteristisch und einheitlich ist. Solche einheitliche Zielsequenzsegmente sind vorzugsweise auf beiden Seiten eines polymorphen Lokus lokalisiert und flankieren somit einen polymorpehen Lokus (mit Sequenzvarianten) einer Zielsequenz von beiden Seiten. Vorzugsweise unterscheiden sich das erste einheitliche Zielsequenzsegment (N1) in seiner Sequenzzusammensetzung von der Sequenzzusammensetzung des zweiten einheitlichen Zielsequenzsegments (N3) in mindestens einer Nukleotid-Position.

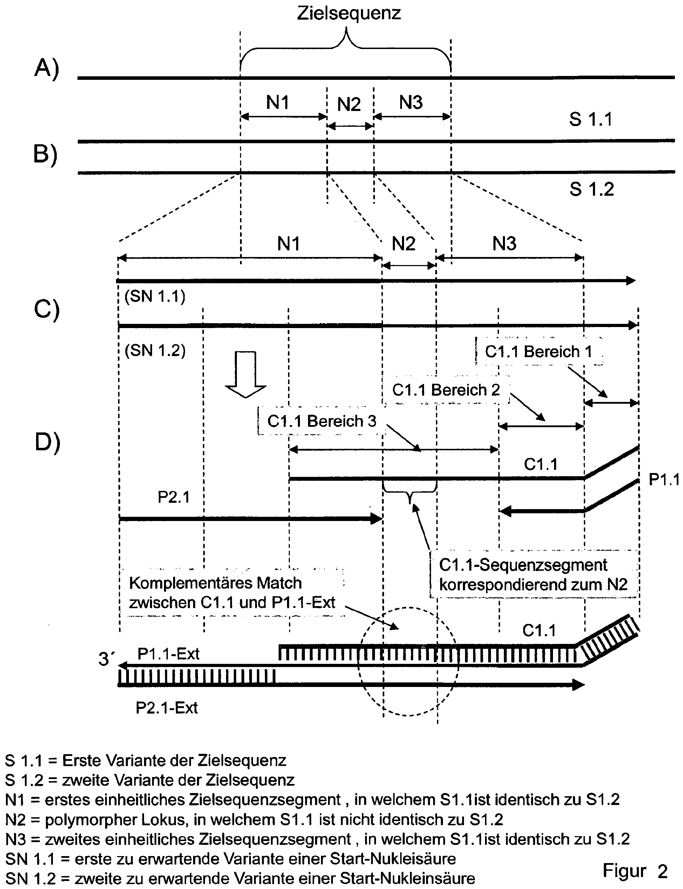

Fig. 2D: Während einer Amplifikation erfolgt exponentielle Vermehrung einer zu amplifizierenden Nukleionsäurekette unter Mitwirkung eines Aktivator-Oligonukleotdies C1,.1, so dass eine Verlängerung eines ersten Primers (P1.1) und eines zweiten Primers (P2.1) erfolgt und in Synthese von Primer-Verlängerungsprodukten resultiert (P1.1-Ext und P2.1-Ext), welche Segmente N1, N2 und N3 einer Zielnukleinsäurekette umfassen.
   Das Aktivator-Oligonukleotid umfasst dabei ein zum polymorphen Lokus einer Zielsequenz korrespondierendes Sequenzsegment. Die Sequenzzusammensetzung dieses Sequenzsegmentes des Aktivator-Oligonukleoides ist charakteristisch und spezifisch für eine der Sequenz-Varianten der Zielsequenz in diesem Lokus, so dass dieses Segment komplementär an das korrespondierende Segment des ersten Primer-Verlängerungsprodutkes binden kann, welches beispielsweise aus dem Kopier-Vorgang einer spezifischen Start-Nukleinsäurekette resultiert. Dadurch kann das Aktivator-Oligonukleotid bei einer für diese Sequenz-Variante spezifische Strangtrennung mitwirken, was in einer Amplifikation resultiert.
   Bei dieser Ausführungsform ist Aktivator-Oligonukleoitid dermaßen angeordnet, dass ein zum N2 der Zielsequenz korrespondierendes Sequenzsegment des Aktivator-Oligonukleotides in seinem dritten Bereich liegt und sich mit keiner der Primer-Sequenzen überschneidet.
Fig. 3 (A-C)
   Zeigt schematisch Topographie einer Zielnukleinsäurekette mit einem polymorphen Lokus (N2) und zwei einheitlichen Zielsequenzsegmenten (N1 und N3) wie in Fig. 2 dargestellt. Fig. 3D zeigt das Ergebnis einer Verwendung von zwei jeweils für eine Sequenz-Variante charakteristischen und spezifischen Aktivator-Oligonukleotide (C1.1 und C1.2), wobei jedes dieser Aktivator-Oligonukleotide einen zum polymorphen Lokus der Zielsequenz (N2) korrespondierendes Sequenzsegment umfasst, wobei C1.1 und C1.2 sich in diesem Sequenzsegment unterscheiden. Daraus folgt eine spezifische und charakteristische Paarbildung einer spezifischen und charakteristischen Sequenz-Variante (einer Allel-Variante) einer spezifischen Zielsequenz und eines für diese Sequenz-Variante charakteristischen und spezifischen Aktivator-Oligonukleotides.
   Die spezifischen Aktivator-Oligonukleotide sind in der Lage nur mit einem Strang einer zu amplifizierenden Nukleinsäurekette (mit dem jeweiligen ersten Primer-Verlängerungsprodukten) umfassend jeweils nur eine spezifischen Sequenz-Variante einen komplementären Strang auszubilden, bei welchem das zum N2 korrespondierende Segment des Aktivator-Oligonukleotides einen perfekt-match Doppelstrang mit dem ersten Primer-Verlängerungsprodukt ausbilden kann (das C1.1 mit P1.1-Ext und C1.2 mit P1.2-Ext). und damit bei der Strang-Trennung effektiv mitwirken.
Fig. 3E zeigt schematisch ein Konstrukt umfassend C1.1 und P1.2-Ext oder C1.2 und P1.1-Ext. Bei solchen Duplexen würde ein Mismatch zwischen einem Aktivator-Oligonukleotid und einem ersten Primer-Verlängerungsprodukt entstehen und zwar im Sequenz-Segment, welches mit dem Lokus N2 der Zielsequenz korrespondiert. Solche Mismatcheinschließende Komplexe entstehen in erster Linie in Abwesenheit eines Kompetitor-Stranges (in diesem Fall fehlt das komplementäre P1.1-Ext bzw. P1.2-Ext). Aus diesem Grund kann in Abwesenheit eines zweiten komplementären Primer-Verlängerungsproduktes eine Ausbildung von Duplex zwischen dem 5'-Ende eines Aktivator-Oligonukleotides und korrespondierenden Anteilen des ersten Primer-Verlängerungsproduktes erfolgen.

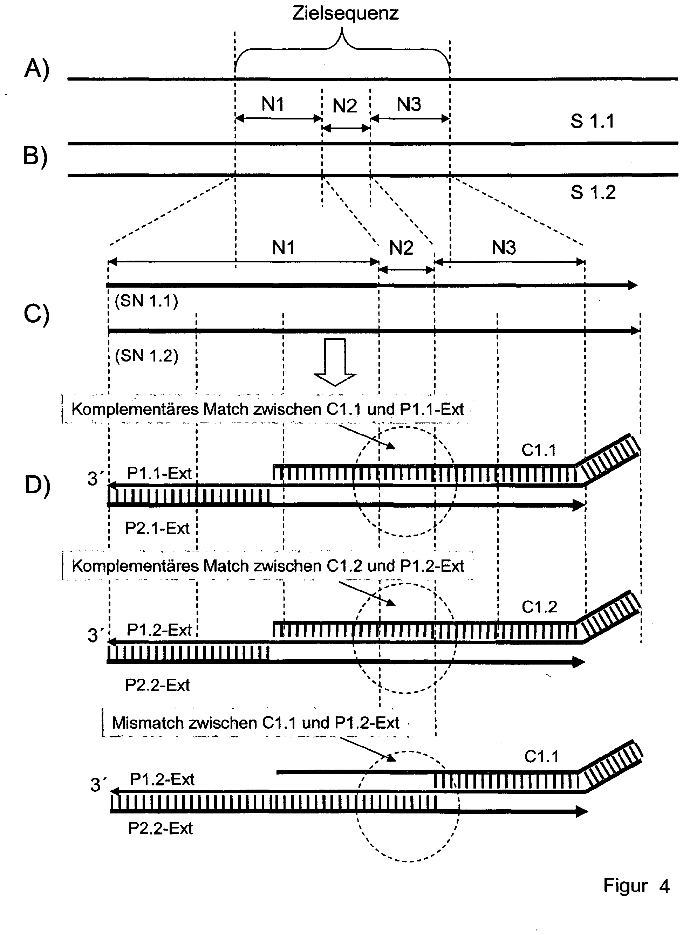

Fig. 4D fasst schematisch zusammen, welche Wirkung die Anwesenheit des vollkomplementären Stranges auf die mögliche Mismatch-Duplex der Fig. 3E hat: Wegen vollkomplementären Natur des von einer Polymerase synthetisierten Primer-Verlängerungsprodutkes, umfassen beide während der Synthese entstandenen Primer-Verlängerungsprodukte jeweils voll-komplementäre Sequenzen. Bindung von solchen vollkomplementären Sequenzen an einander erfolgt effektiver, als bei Sequenzen, welche ein Mismatch umfassen. Aus diesem Grund erfolgt in Anwesenheit eines zweiten komplementären Primer-Verlängerungsproduktes keine Ausbildung von Duplex zwischen dem 5'-Ende eines Aktivator-Oligonukleotides und korrespondierenden Anteilen des ersten Primer-Verlängerungsproduktes. Die Strang-Trennung verläuft somit nur über eine kürzere Distanz und ist weniger Effektiv, als bei vollkomplementären Strängen. Das resultiert in einer Verlangsamung bzw. sogar Unterbrechung einer Amplifikation.

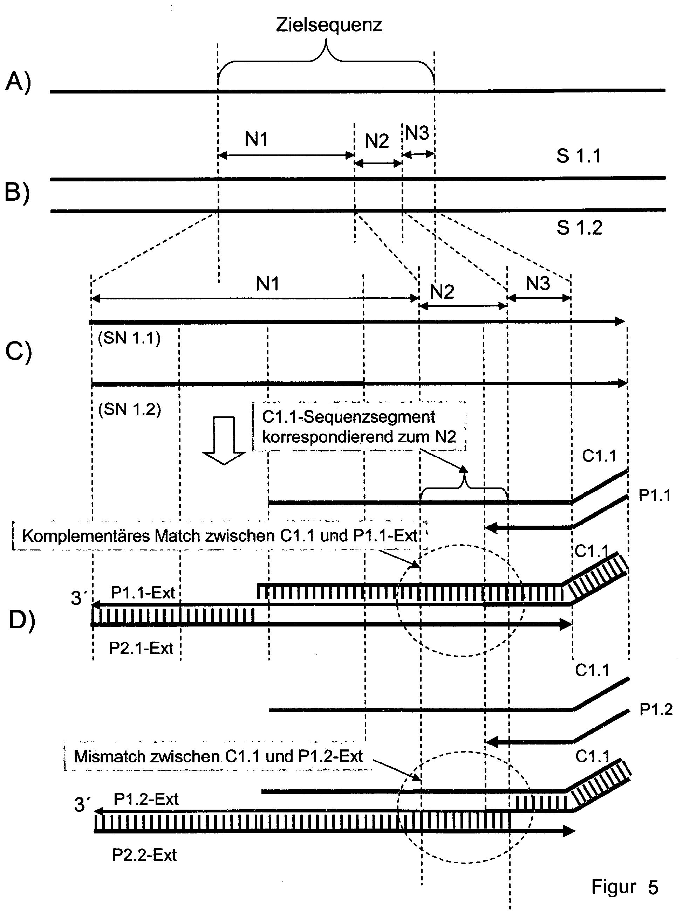

Fig. 5 zeigt schematisch eine andere Topographie eines N2 einer Zielnukleinsäurekette in Bezug auf Aktivator-Oligonukleotid und den ersten Primer.
   Bei diesem Ausführungsbeispiel umfasst das zum N2 korrespondierende Sequenzsegment des Aktivator-Oligonukleotides teilweise den dritten Bereich als auch teilweise den zweiten Bereich. Auch das 3'-Segment des ersten Primers wird sequenzspezifisch und charakteristisch für eine spezifische und charakteristische Sequenz-Variante der Zielnukleionsäure gestaltet. An der Allel-Diskriminierung nehmen also sowohl Aktivator-Oligonukleotid als auch das erste Primer-Oligonukleotid teil. Für jede spezifische Sequenzvariante des polymorphen Lokus kann somit sowohl ein spezifisches Aktivator-Oligonukleotid als auch ein spezifisches erstes Primer-Oligonukleotid konstruiert werden. Unter Verwendung von spezifischen und charakteristischen Primern sowie geeigneten Reaktionsbedingungen erfolgt Synthese von Primer-Verlängerungsprodukten in spezifischer Art und Weise, so dass P1.1 spezifisch verlängert wird und dabei ein P1.1-Ext resultiert, und P1.2 wird spezifisch verlängert und dabei P1.2-Ext resultiert. Aufgrund gleicher Gestaltung von zweiten Bereichen bei P1.1 und P1.2 kann eine Bindung von C1.1 an P1.2-Ext erfolgen und Strang-Verdrängung zwar initiiert werden, dennoch kann eine vollständige Strang-Trennung nicht erfolgen, da P1.2-Ext kein vollkomplementäres Sequenzsegment im zu N2 korrespondierenden Sequenzsegment umfasst.

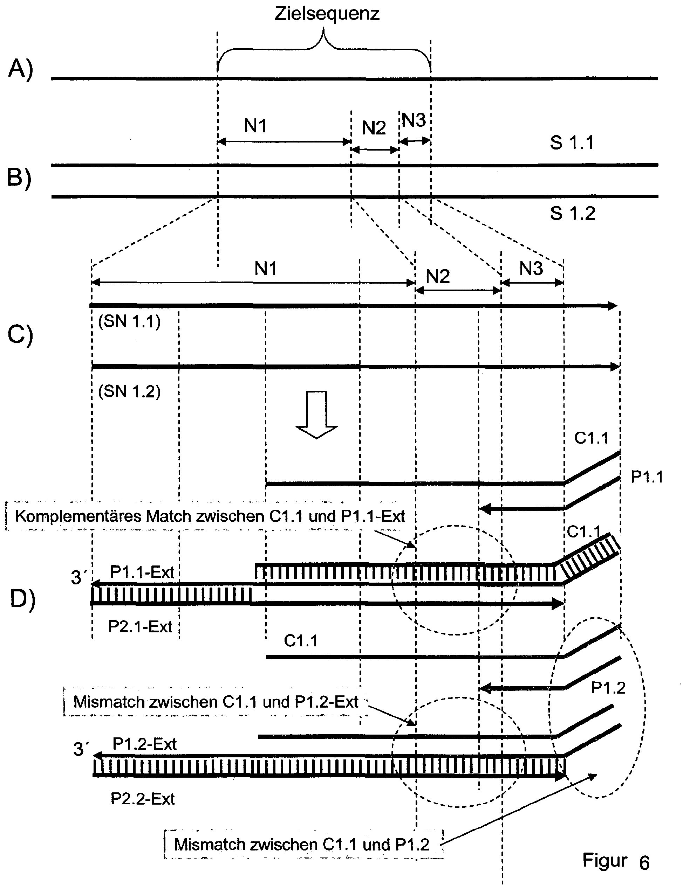

Fig. 6 zeigt schematisch eine andere Topographie eines N2 einer Zielnukleinsäurekette in Bezug auf Aktivator-Oligonukleotid und den ersten Primer, wobei durch die Sequenzspezifische Kodierung der komplementären Anteile des zweiten Primer-Bereiches zu entsprechenden komplementären Anteilen des ersten Bereichs des Aktivator-Oligonukleotides, die Interaktion zwischen einem spezifischen P1.2-Ext und einem spezifischen C1.1 reduziert werden kann. Daraus ergibt sich ein Möglichkeit für ein vielfaches Multiplexing, wobei jedes Paar umfassend ein zumindest ein erstes Primer-Oligonukleotid und das Aktivator-Oligonukleotid eine für sie charakteristische Sequenzzusammensetzung einer Duplex umfassend den ersten Bereich des Aktivator-Oligonukleotides und den zweiten Bereich des ersten Primers umfasst.

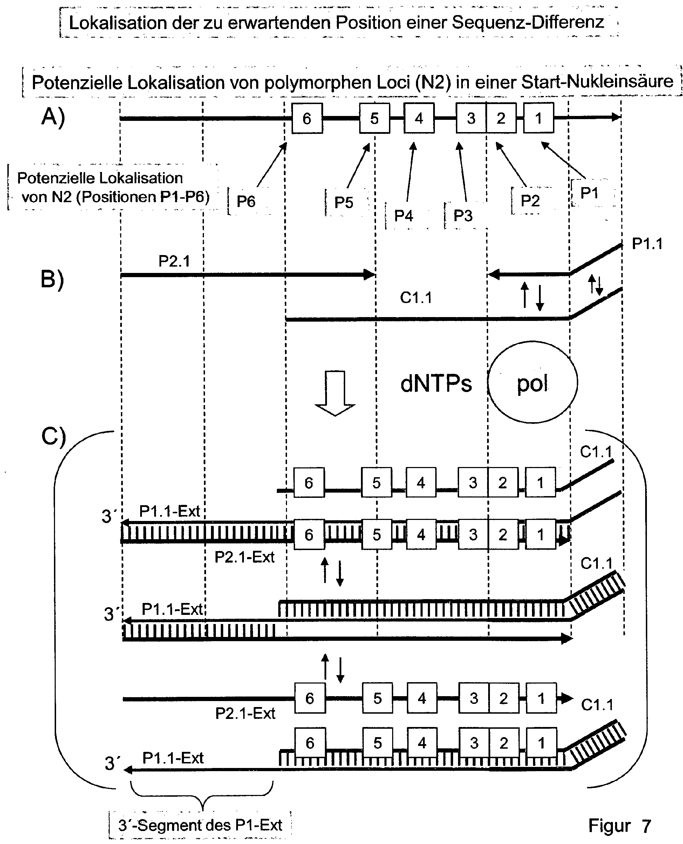

Fig. 7 zeigt schematisch potenzielle Positionen eines polymorphen Lokus innerhalb einer Zielsequenz (Position 1 bis 6) und ihre korrespondierenden Sequenzsegment-Positionen in einzelnen Komponenten eines Amplifikations-Systems. Die hier schematisch dargestellten polymorphen Loki einer Zielsequenz umfassen 2 bis 50 Nukleotide, besser 4 bis 30 Nukleotide welche für eine Allel-Variante einer Zielsequenz charakteristisch und spezifisch sind.
   Insgesamt kann die Anordnung von einzelnen Amplifikations-Komponenten dermaßen gestaltet werden, dass mehrere Varianten/Kombinationen möglich sind:
   **Anordnung 1 (P1):** polymorpher Lokus (N2) der Zielsequenz überlagert den ersten Primer (ersten Bereich) und Aktivator-Oligonukleotid (zweiten Bereich). Diese Komponenten des Amplifikations-Systems können somit spezifisch und charakteristisch für jeweilige Sequenzvariate der Zielsequenz konstruiert werden. Bei dieser Anordnung wird ein zielsequenzspezifischer zweiter Primer verwendet, welcher aber nicht Sequenz-Varianten spezifisch ist.
   **Anordnung 2 (P2):** polymorpher Lokus (N2) der Zielsequenz überlagert den ersten Primer (ersten Bereich) und Aktivator-Oligonukleotid (zweiten Bereich). Diese Komponenten des Amplifikations-Systems können somit spezifisch und charakteristisch für jeweilige Sequenzvariate der Zielsequenz konstruiert werden. Bei dieser Anordnung wird ein Zielsequenzspezifischer zweiter Primer verwendet, welcher aber nicht Sequenz-Varianten spezifisch ist. Anordnung P2 unterscheidet sich von P1 vor allem dadurch, dass N2 vorwiegend im 3'-Terminalen Sequenz-Segment des ersten Primers liegt. Dadurch kann ggf. eine bessere Spezifität der Amplifikation erzeicht werden.
   **Anordnung 3 (P3):** polymorpher Lokus (N2) der Zielsequenz überlagert nur das Aktivator-Oligonukleotid (dritter Bereich), wobei das korrespondierende Sequenzsegment des Aktivator-Oligonukleotides im 5'-Segment des synthetisierten Anteils des ersten Primer-Verlängerungsproduktes liegt. Das Aktivator-Oligonukleotid des Amplifikations-Systems kann somit spezifisch und charakteristisch für jeweilige Sequenzvariate der Zielsequenz konstruiert werden. Bei dieser Anordnung wird ein zielsequenzspezifischer erster und zweiter Primer verwendet, welche aber nicht Sequenz-Varianten spezifisch sind. Aufgrund der möglichen Nähe der zweiten Blockierungs-Einheit kann Bindung des Aktivator-Oligonukleotide an das erste Primer-Verlängerungsprodukt durch Nukleotid-Modifikationen der zweiten Blockierungs-Einheit beeinflusst werden.
   **Anordnung 4 (P4):** polymorpher Lokus (N2) der Zielsequenz überlagert nur das Aktivator-Oligonukleotid (dritter Bereich). Das Aktivator-Oligonukleotid des Amplifikations-Systems kann somit spezifisch und charakteristisch für jeweilige Sequenzvariate der Zielsequenz konstruiert werden. Bei dieser Anordnung wird ein zielsequenzspezifischer erster und zweiter Primer verwendet, welche aber nicht Sequenz-Varianten spezifisch sind. Dieses Sequenzsegment des Aktivator-Oligonukleotides liegt in 5'-Richtung von der zweiten Blockierungs-Einheit und kann mehrere DNA-Nukleotid-Monomere umfassen, z.B. von 5 bis 30.
   **Anordnung 5 (P5):** polymorpher Lokus (N2) der Zielsequenz überlagert den zweiten Primer und Aktivator-Oligonukleotid (dritter Bereich). Diese Komponenten des Amplifikations-Systems können somit spezifisch und charakteristisch für jeweilige Sequenzvariate der Zielsequenz konstruiert werden. Bei dieser Anordnung wird ein zielsequenzspezifischer erster Primer verwendet, welcher aber nicht Sequenz-Varianten spezifisch ist.
   **Anordnung 6 (P6):** polymorpher Lokus (N2) der Zielsequenz überlagert den zweiten Primer und Aktivator-Oligonukleotid (dritten Bereich, nahe dem 5'-Ende). Diese Komponenten des Amplifikations-Systems können somit spezifisch und charakteristisch für jeweilige Sequenzvariate der Zielsequenz konstruiert werden. Bei dieser Anordnung wird ein zielsequenzspezifischer erster Primer verwendet, welcher aber nicht Sequenz-Varianten spezifisch ist.
   Aus den Design-Unterschieden einzelner Elemente ergeben sich auch Unterschiede für während einer Amplifikation synthetisierte Primer-Verlängerungsprodukte (Fig. 7C).

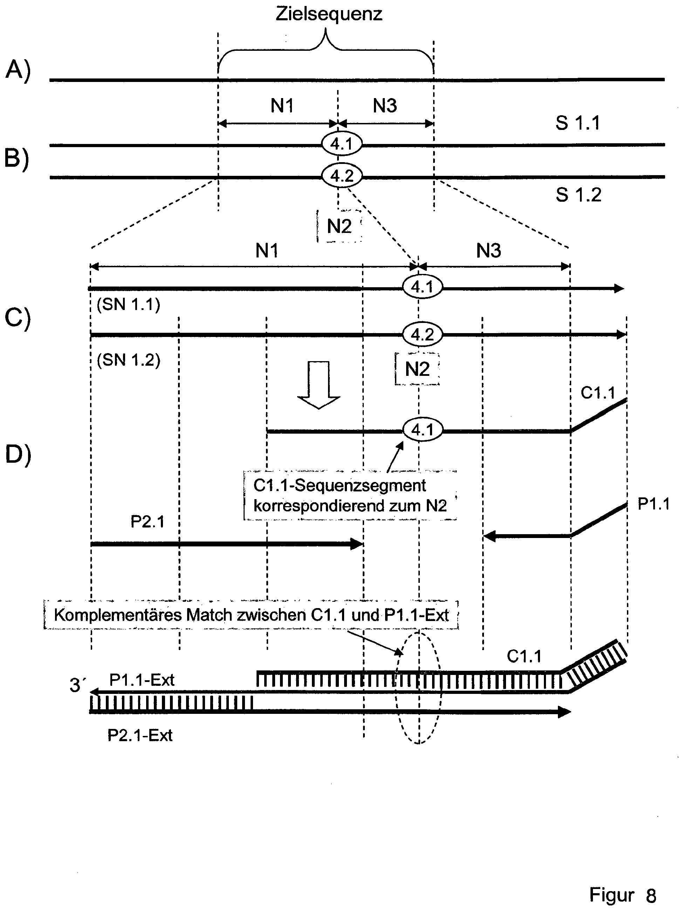

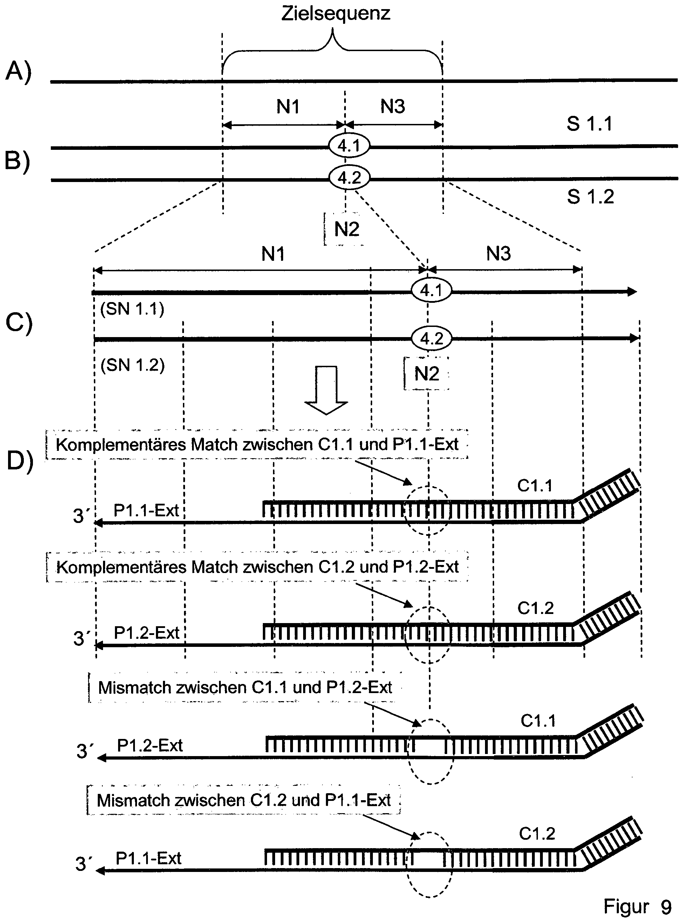

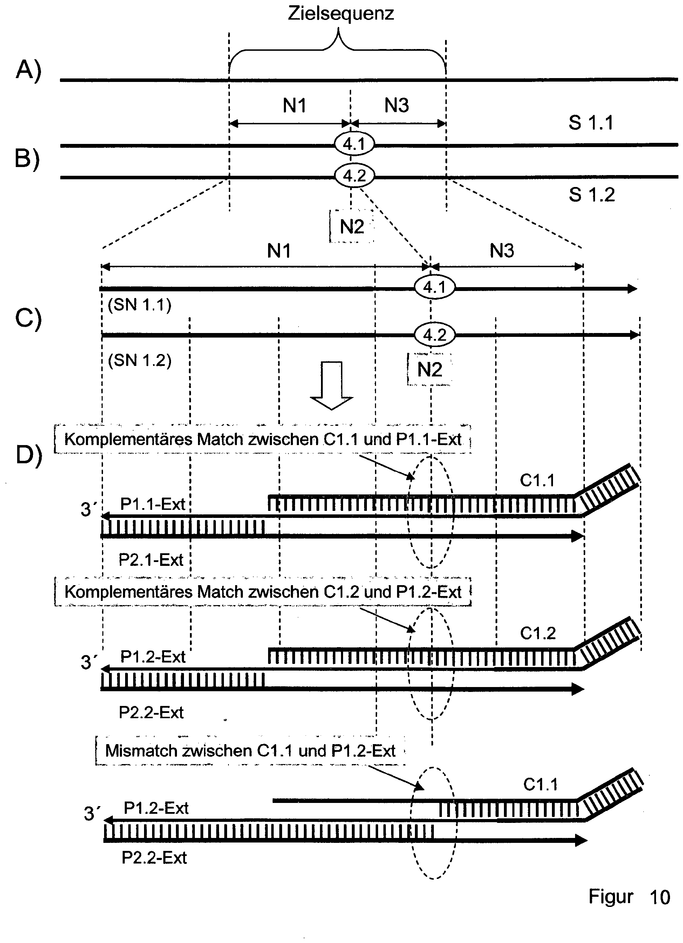

Fig. 8-10 zeigen schematisch die Topographie einer Zielnukleinsäurekette mit einem polymorphen Lokus (N2) und zwei einheitlichen Zielsequenzsegmenten (N1 und N3) wie in Fig. 2 dargestellt, wobei der polymorphe Lokus nur eine Nukleotid-Position umfasst (z.B. ein SNV oder ein SNP oder eine Punkt-Mutation etc.). Die Unterschiede zwischen einzelnen Sequenzvarianten betragen somit lediglich nur ein Nukleotid (hier als 4.1 und 4.2 bezeichnet). Ein solcher Lokus (N2) kann zu ähnlichen Anordnungen einzelner Komponenten führen wie ein Lokus mit mindestens 2 Nukeotiden. Bei einer Bindung des Aktivator-Oligonukleotides an ein komplementäres erstes Primer-Verlängerungsprodukt (perfekt-Match) kann somit eine Amplifikation erfolgen. Bei der Ausbildung eines Mismatches wird Amplifikation gehemmt oder verlangsamt bzw. sogar unterbrochen. Ähnlich wie bei längeren N2 kann es bei einzelnen Nukleotid-Mismatches zwischen dem Aktivator-Oligonukleotid und einem ersten Primer-Verlängerungsprodukt zu Unterbrechung/Verlangsamung der Strang-Trennung kommen.

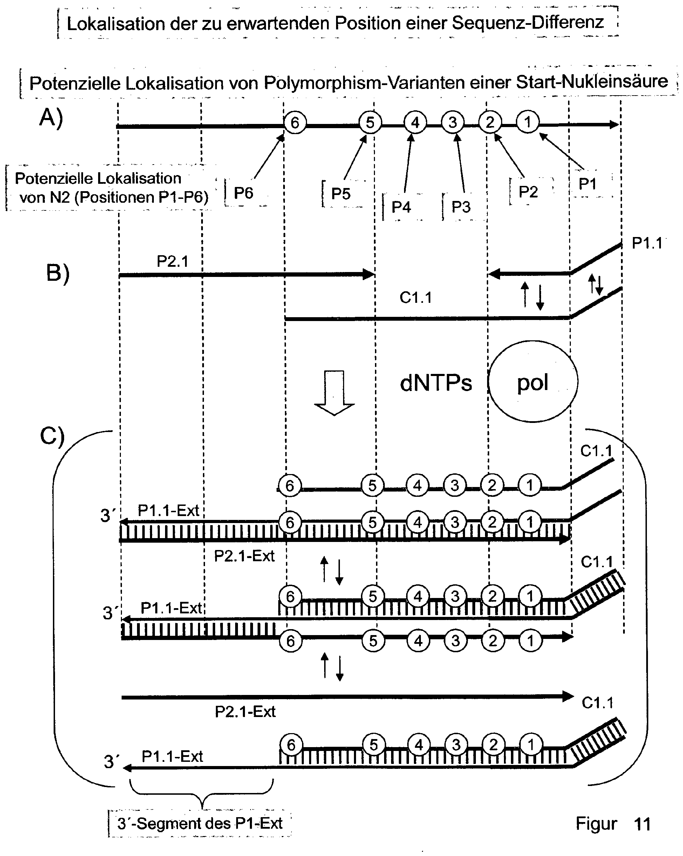

Fig. 11 zeigt schematisch potenzielle Positionen eines polymorphen Lokus mit Sequenz-Varianz von nur einem Nukleotid innerhalb einer Zielsequenz (Position 1 bis 6) und ihre korrespondierenden Sequenzsegment-Positionen in einzelnen Komponenten eines Amplifikations-Systems.
   Insgesamt kann die Anordnung von einzelnen Amplifikations-Komponenten dermaßen gestaltet werden, dass mehrere Varianten/Kombinationen möglich sind:
   **Anordnung 1 (P1):** polymorpher Lokus (N2) der Zielsequenz überlagert den ersten Primer (ersten Bereich) und Aktivator-Oligonukleotid (zweiten Bereich). Diese Komponenten des Amplifikations-Systems können somit spezifisch und charakteristisch für die jeweilige Sequenzvarianten der Zielsequenz konstruiert werden. Bei dieser Anordnung wird ein Zielsequenzspezifischer zweiter Primer verwendet, welcher aber nicht Sequenz-Varianten spezifisch ist.
   **Anordnung 2 (P2):** polymorpher Lokus (N2) der Zielsequenz überlagert den ersten Primer (ersten Bereich) und Aktivator-Oligonukleotid (zweiten Bereich). Diese Komponenten des Amplifikations-Systems können somit spezifisch und charakteristisch für jeweilige Sequenzvarianten der Zielsequenz konstruiert werden. Bei dieser Anordnung wird ein Zielsequenzspezifischer zweiter Primer verwendet, welcher aber nicht Sequenz-Varianten spezifisch ist. Anordnung P2 unterscheidet sich von P1 vor allem dadurch, dass N2 vorwiegend im 3'-terminalen Sequenz-Segment des ersten Primers liegen kann oder sogar das 3'-terminale Nukleotid umfasst. Dadurch kann ggf. eine weitere Erhöhnung der Spezifität der Amplifikation erzeicht werden.
   **Anordnung 3 (P3):** polymorpher Lokus (N2) der Zielsequenz überlagert nur das Aktivator-Oligonukleotid (dritten Bereich), wobei das korrespondierende Sequenzsegment des Aktivator-Oligonukleotides im 5'-Segment des synthetisierten Anteils des ersten Primer-Verlängerungsproduktes liegt. Das Aktivator-Oligonukleotid des Amplifikations-Systems kann somit spezifisch und charakteristisch für jeweilige Sequenzvariante der Zielsequenz konstruiert werden. Bei dieser Anordnung wird ein zielsequenzspezifischer erster und zweiter Primer verwendet, welche aber nicht Sequenz-Varianten spezifisch sind. Aufgrund der möglichen Nähe der zweiten Blockierungs-Einheit kann Bindung des Aktivator-Oligonukleotide an das erste Primer-Verlängerungsprodukt durch Nukleotid-Modifikationen der zweiten Blockierungs-Einheit beeinflusst werden.
   **Anordnung 4 (P4):** polymorpher Lokus (N2) der Zielsequenz überlagert nur das Aktivator-Oligonukleotid (dritten Bereich). Das Aktivator-Oligonukleotid des Amplifikations-Systems kann somit spezifisch und charakteristisch für jeweilige Sequenzvariante der Zielsequenz konstruiert werden. Bei dieser Anordnung wird ein zielsequenzspezifischer erster und zweiter Primer verwendet, welche aber nicht Sequenz-Varianten spezifisch sind. Dieses Sequenzsegment des Aktivator-Oligonukleotides liegt in 5'-Richtung von der zweiten Blockierungs-Einheit. Dieses Segment des Aktivator-Oligonukleotides kann mehrere DNA-Nukleotid-Monomere umfassen, z.B. von 5 bis 30, wobei das N2-korrespondierende Sequenzsegment von mindestens 3 bis 15 DNA-Nukleotid-Bausteinen auf beiden Seiten flankiert sein kann.
   **Anordnung 5 (P5):** polymorpher Lokus (N2) der Zielsequenz überlagert den zweiten Primer und Aktivator-Oligonukleotid (dritter Bereich). Diese Komponenten des Amplifikations-Systems können somit spezifisch und charakteristisch für jeweilige Sequenzvariante der Zielsequenz konstruiert werden. Die Anordnung kann dermaßen gestaltet werden, dass das 3'-terminale Nukleotid des zweiten Primers mit dem N2-Lokus korrespondiert. Bei dieser Anordnung wird ein zielsequenzspezifischer erster Primer verwendet, welcher aber nicht Sequenz-Varianten spezifisch ist.
   **Anordnung 6 (P6):** polymorpher Lokus (N2) der Zielsequenz überlagert den zweiten Primer und Aktivator-Oligonukleotid (dritter Bereich, nahe dem 5'-Ende). Diese Komponenten des Amplifikations-Systems können somit spezifisch und charakteristisch für jeweilige Sequenzvariante der Zielsequenz konstruiert werden. Bei dieser Anordnung wird ein zielsequenzspezifischer erster Primer verwendet, welcher aber nicht Sequenz-Varianten spezifisch ist.
   Aus den Design-Unterschieden einzelner Elemente ergeben sich auch Unterschiede für während einer Amplifikation synthetisierten Primer-Verlängerungsprodukte (Fig. 11C).

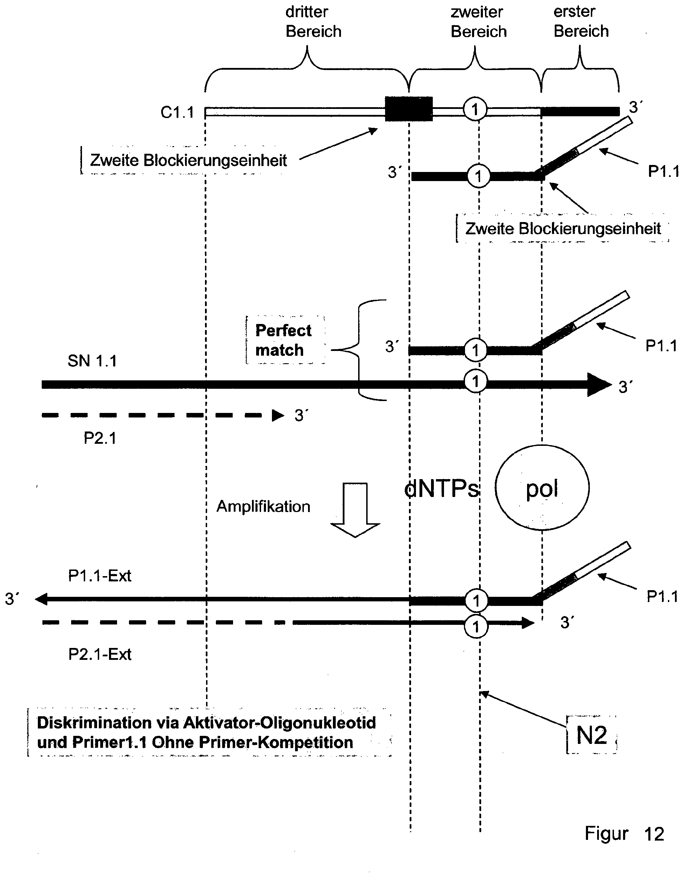

Fig. 12 zeigt schematisch eine Ausführungsform mit Topographie eines spezifischen Amplifikations-Systems und einer Start-Nukleisäurekette mit einem polymorphen Lokus (N2) in einer Ausführungsform, bei welcher das zu N2 korrespondierende Segment des Aktivator-Oligonukleotide im zweiten Bereich liegt. Ein spezifischer P1.1 kann unter Reaktionsbedingungen bevorzugt spezifisch an die komplemenäre Position einer spezifischen Sequenz-Variante der Start-Nukleinsäure (SN 1.1) binden und die Synthese des P1.1-Ext initiieren. Im Verlauf der Amplifikation kommt es dabei zu Ausbildung von P1.1-Ext und P2.1-Ext. Es wird kein Kompetitor-Primer verwendet.

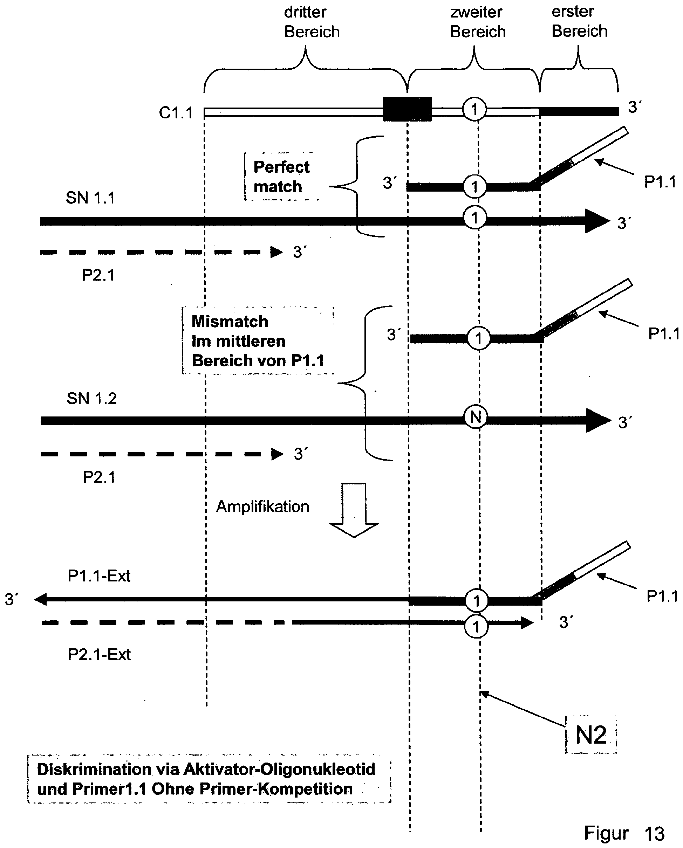

Fig. 13 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotides im zweiten Bereich liegt. Ein spezifischer P1.1 kann unter Reaktionsbedingungen bevorzugt spezifisch an die komplementäre Position einer spezifischen Sequenz-Variante der Start-Nukleinsäure (SN 1.1) binden und die Synthese des P1.1-Ext initiieren. Im Verlauf der Amplifikation kommt es dabei zur Ausbildung von P1.1-Ext und P2.1-Ext. Es wird kein Kompetitor-Primer verwendet. Die Bindung an eine andere Sequenz-Variante (SN 1.2) erfolgt weniger effizient aufgrund des Mismatches mit Position (N) innerhalb der Pimer-Bindungsstelle der SN 1.2. Die Amplifikation wird somit weniger effizient gestartet.

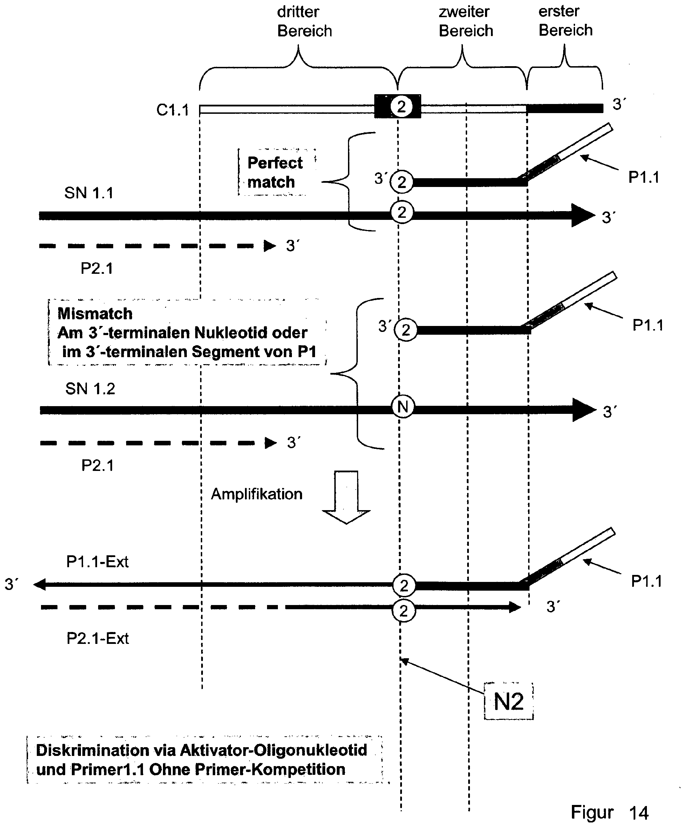

Fig. 14 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotide im zweiten Bereich liegt (2). N2-Lokus umfasst auch das 3'-Terminale Nukleotid und / oder das 3'-terminales Segment des ersten Primers. Ein spezifischer P1.1, mit einem komplementären 3'-terminalen Nukleotid kann unter Reaktionsbedingungen bevorzugt spezifisch an die komplemenäre Position einer spezifischen Sequenz-Variante der Start-Nukleinsäure (SN 1.1) binden und die Synthese des P1.1-Ext initiieren. Im Verlauf der Amplifikation kommt es dabei zur Ausbildung von P1.1-Ext und P2.1-Ext. Es wird kein Kompetitor-Primer verwendet. Die Bindung und Initiierung der Primer-Verlängerung an einer anderen Sequenz-Variante (SN 1.2) erfolgt weniger effizient aufgrund des Mismatches mit Position (N) innerhalb der Pimer-Bindungsstelle der SN 1.2. Die Amplifikation wird somit weniger effizient gestartet.

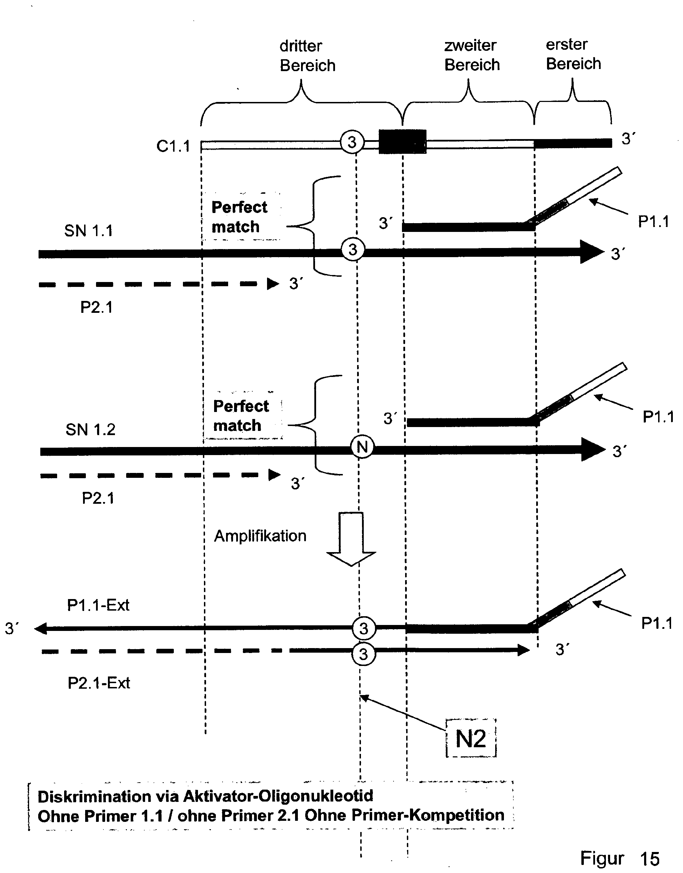

Fig. 15 zeigt schematisch eine Ausführungsform mit Topographie eines spezifischen Amplifikations-Systems und einer Start-Nukleisäurekette mit einem polymorphen Lokus (N2) in einer Ausführungsform, bei welcher das zu N2 korrespondierende Segment des Aktivator-Oligonukleotide im dritten Bereich liegt (3). Es werden zielsequenzspezifische aber nicht Sequenz-Varianten spezifische erster und zweiter Primer verwendet. Ein für alle Sequenz-Varianten einer Zielnukleinsäure einheitlicher P1.1 und P2.1, kann an die Start-Nukleinsäurekette binden und die Synthese des P1.1-Ext bzw. P2.1-Ext initiieren. Im Verlauf der Amplifikation kommt es dabei zu Ausbildung von P1.1-Ext und P2.1-Ext. Es wird kein Kompetitor-Primer verwendet. Die Trennung von beiden synthetisierten komplementären Primer-Verlängerungsprodukten P1.1-Ext und P2.1-Ext erfolgt vorzugsweise spezifisch durch komplementäre Bindung des P1.1-Ext an das Aktivator-Oligonukleotid. Die Trennung von Strängen einer anderen Sequenz-Variante, welche unter Verwendung von (SN 1.2) synthetisiert wurden, erfolgt weniger effizient aufgrund des Mismatches mit Position (N) zum korrespondierenden Sequenz-Segment des Aktivator-Oligonukleotides. Die Amplifikation von SN 1.2 Sequenz-Variante erfolgt somit weniger effizient bzw. nur unwesentlich bzw. gar nicht. Die Position des Sequenz-Segmentes (3), welches zu N2 der Zielsequenz korrespondiert, liegt in der Nähe der zweiten Blockierungseinheit, so dass Interaktion zwischen dem Aktivator-Oligonukleotid und dem jeweiligen ersten Primer-Verlängerungsprodukt von verwendeten Modifikationen in der zweiten Blockierungseinheit beeinflusst werden kann.

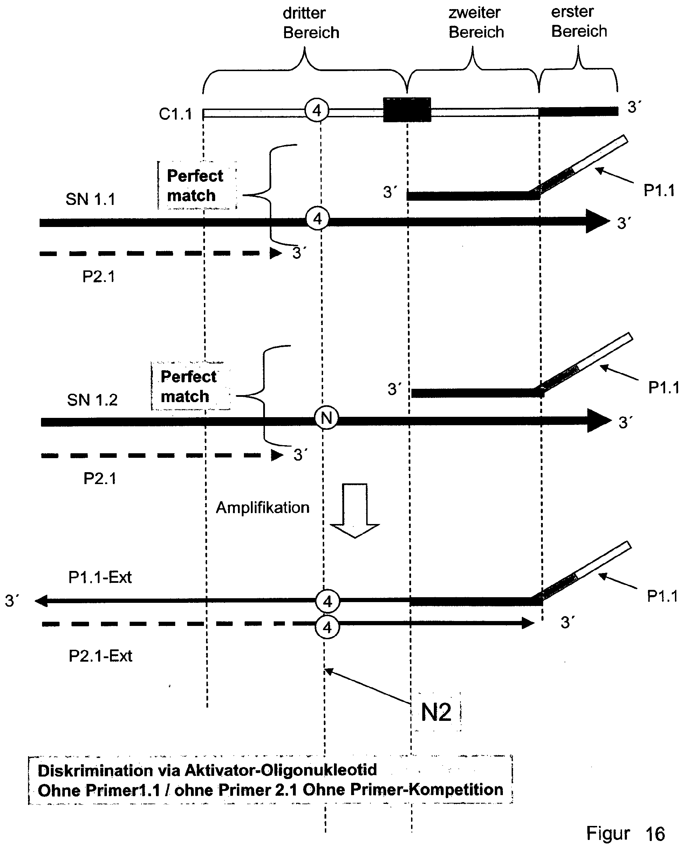

Fig. 16 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotides im dritten Bereich liegt (4). Es werden zielsequenzspezifische aber nicht sequenz-Varianten spezifische erster und zweiter Primer verwendet. Ein für alle Sequenz-Varianten einer Zielnukleinsäure einheitlicher P1.1 und P2.1, kann an die Start-Nukleinsäurekette binden und die Synthese des P1.1-Ext bzw. P2.1-Ext initiieren. Im Verlauf der Amplifikation kommt es dabei zur Ausbildung von P1.1-Ext und P2.1-Ext. Es wird kein Kompetitor-Primer verwendet. Die Trennung von beiden synthetisierten komplementären Primer-Verlängerungsprodukten P1.1-Ext und P2.1-Ext erfolgt vorzugsweise spezifisch durch komplementäre Bindung des P1.1-Ext an das Aktivator-Oligonukleotid. Die Trennung von Strängen einer anderen Sequenz-Variante, welche unter Verwendung von (SN 1.2) synthetisiert wurden, erfolgt weniger effizient aufgrund des Mismatches mit Position (N) zum korrespondierenden Sequenz-Segment des Aktivator-Oligonukleotides. Die Amplifikation von SN 1.2 Sequenz-Variante erfolgt somit weniger effizient bzw. nur unwesentlich bzw. gar nicht. Die Position des Sequenz-Segmentes (4), welches zu N2 der Zielsequenz korrespondiert, liegt in einer Entfernung von der zweiten Blockierungseinheit, so dass Interaktion zwischen dem Aktivator-Oligonukleotid und dem jeweiligen ersten Primer-Verlängerungsprodukt von verwendeten Modifikationen in der zweiten Blockierungseinheit im Wesentlichen unbeeinflusst bleibt. Das Segment des Aktivator-Oligonukleotides, welches zu N2 korrespondiert besteht vorzugsweise aus DNA-Monomeren, wobei zwischen 4 bis 20 DNA Monomeren verwendet werden und mindestens 4 bis 10 DNA-Monomere um die Position-4 auf beiden Seiten lokalisiet sind.

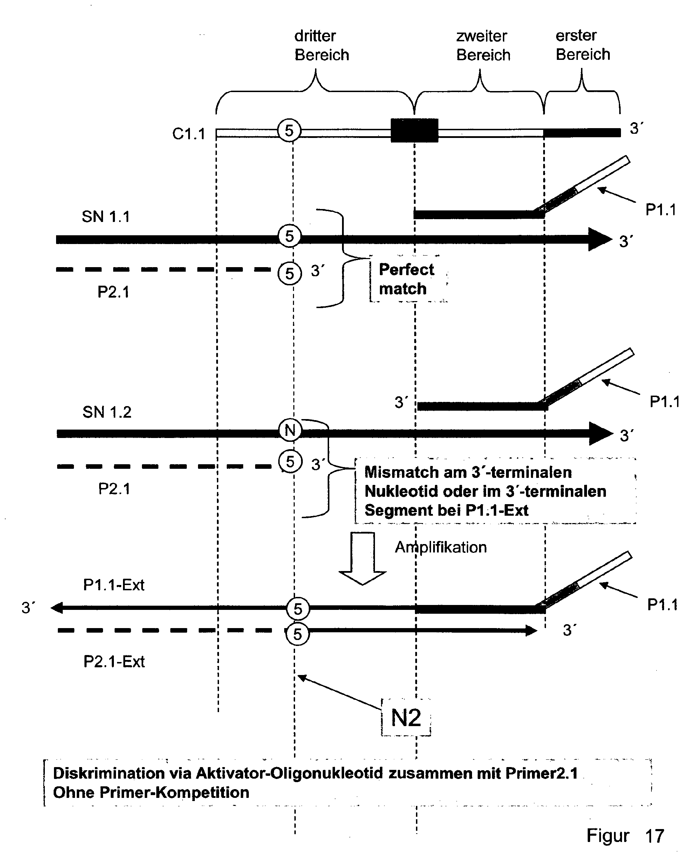

Fig. 17 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotides im dritten Bereich liegt (5). N2-Lokus umfasst auch das 3'-Terminale Nukleotid des zweiten Primers. Ein spezifischer P2.1, mit einem komplementären 3'-terminalen Nukleotid kann unter Reaktionsbedingungen bevorzugt spezifisch an die komplemenäre Position einer spezifischen Sequenz-Variante ausgehend von komplementären zu Start-Nukleinsäure (SN 1.1) Strängen binden und die Synthese des P2.1-Ext vorzugsweise spezifisch initiieren. Im Verlauf der Amplifikation kommt es dabei zu Ausbildung von P1.1-Ext und P2.1-Ext. Es wird kein Kompetitor-Primer verwendet. Die Bindung an eine andere Sequenz-Variante (SN 1.2) erfolgt weniger effizient aufgrund des Mismatches mit Position (N) innerhalb der Pimer-Bindungsstelle der SN 1.2. Die Amplifikation wird somit weniger effizient gestartet.

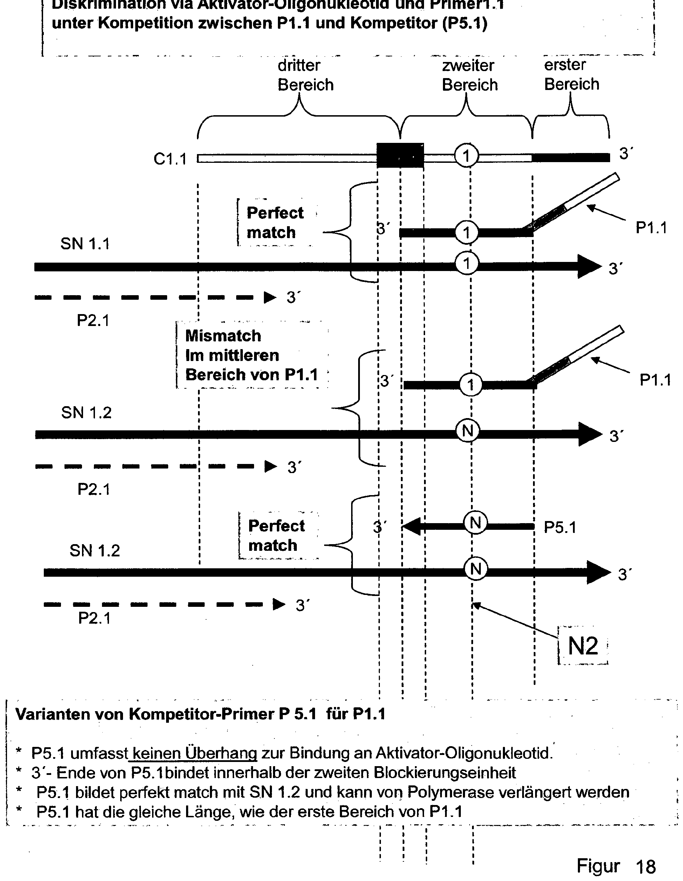

Fig. 18 zeigt schematisch eine Ausführungsform mit Topographie eines spezifischen Amplifikations-Systems und einer Start-Nukleisäurekette mit einem polymorphen Lokus (N2) in einer Ausführungsform, bei welcher das zu N2 korrespondierende Segment des Aktivator-Oligonukleotide im zweiten Bereich liegt. Ein spezifischer P1.1 kann unter Reaktionsbedingungen bevorzugt spezifisch an die komplemenäre Position einer spezifischen Sequenz-Variante der Start-Nukleinsäure (SN 1.1) binden und die Synthese des P1.1-Ext initiieren. Im Verlauf der Amplifikation kommt es dabei zu Ausbildung von P1.1-Ext und P2.1-Ext. Die Bindung an eine andere Sequenz-Variante (SN 1.2) erfolgt weniger Effizient aufgrund des Mismatches mit Position (N) innerhalb der Pimer-Bindungsstelle der SN 1.2. Die Amplifikation wird somit weniger effizient gestartet.
   Zur Erhöhung der Spezifität wird ein Kompetitor-Primer (P 5.1) in die Reaktion zugegeben, welcher in der Lage ist, vorwiegend komplementär an die Sequenz-Varianten der Zielsequenz zu binden, dessen Amplifikation unterdrückt werden muss. Aufgrund einer komplementären Bindung mit solchen Primer-Bindungsstellen kann ein Kompetitor-Primer bevorzugt binden und von Polymerase verlängert werden. Das dabei resultierende Produkt blockiert die einzelsträngigen Primer-Bindungsstellen für eine Interaktion des ersten Amplifikations-Primers. In einer Ausführungsform bindet das 3'-Ende eines Kompetitor-Primer innerhalb der zweiten Blockierungs-Einheit des Aktivator-Oligonukleotides, so dass keine Verlängerung dieses Primers am Aktivator-Oligonukleotid erfolgen kann. Der Kompetitor-Primer umfasst keinen zweiten Bereich und kann somit nicht mit dem ersten Bereich des Aktivator-Oligonukleotid interagieren. Dadurch wird verhindert, dass das Verlängerungsprodukt des Kompetitor-Oligonukleotides von Matrize unter Mitwirkung eines Aktivator-Oligonukleotides abgelöst wird.

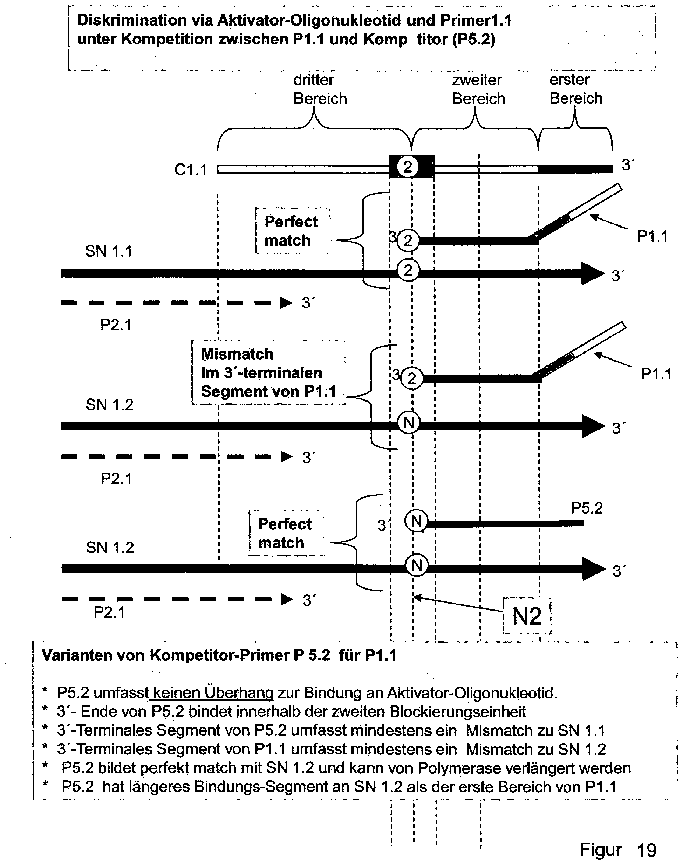

Fig. 19 zeigt schematisch eine Ausführungsform mit Topographie eines spezifischen Amplifikations-Systems und einer Start-Nukleisäurekette mit einem polymorphen Lokus (N2) in einer Ausführungsform, bei welcher das zu N2 korrespondierende Segment der Aktivator-Oligonukleotide im zweiten Bereich liegt (2). N2-Lokus umfasst auch das 3'-Terminale Nukleotid des ersten Primers. Ein spezifischer P1.1, mit einem komplementären 3'-terminalen Nukleotid kann unter Reaktionsbedingungen bevorzugt spezifisch an die komplemenäre Position einer spezifischen Sequenz-Variante der Start-Nukleinsäure (SN 1.1) binden und die Synthese des P1.1-Ext initiieren. Im Verlauf der Amplifikation kommt es dabei zu Ausbildung von P1.1-Ext und P2.1-Ext. Die Bindung an eine andere Sequenz-Variante (SN 1.2) und die Verlängerung durch eine Polymerase erfolgt weniger effizient aufgrund des Mismatches mit Position (N) innerhalb der Pimer-Bindungsstelle der SN 1.2. Die Amplifikation wird somit weniger effizient gestartet.
   Zur Erhöhung der Spezifität wird ein Kompetitor-Primer (P 5.2) in die Reaktion zugegeben, welcher in der Lage ist, vorwiegend komplementär an die Sequenz-Varianten der Zielsequenz zu binden, dessen Amplifikation unterdrückt werden muss. Aufgrund einer komplementären Bindung mit solchen Primer-Bindungsstellen kann ein Kompetitor-Primer bevorzugt an bestimmte Sequenz-Varianten binden (hier mit N bezeichnet) und von Polymerase verlängert werden. Das dabei resultierende Produkt blockiert die einzelsträngigen Primer-Bindungsstellen für eine Interaktion des ersten Amplifikations-Primers.
   In einer Ausführungsform bindet das 3'-Ende eines Kompetitor-Primer innerhalb der zweiten Blockierungs-Einheit des Aktivator-Oligonukleotides, so dass keine Verlängerung dieses Primers am Aktivator-Oligonukleotid erfolgen kann. Der Kompetitor-Primer umfasst keinen zweiten Bereich und kann somit nicht mit dem ersten Bereich des Aktivator-Oligonukleotid interagieren. Dadurch wird verhindert, dass das Verlängerungsprodukt des Kompetitor-Oligonukleotides der Matrize abgelöst wird.

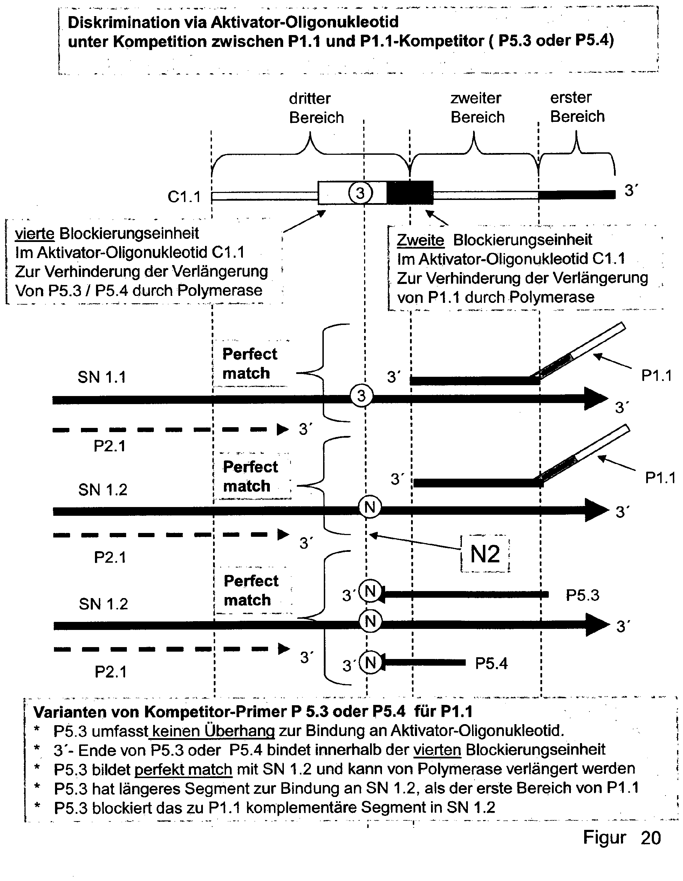

Fig.20 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotide im dritten Bereich liegt (3). Es werden zielsequenzspezifische aber nicht sequenz-Varianten spezifische erster und zweiter Primer verwendet. Ein für alle Sequenz-Varianten einer Zielnukleinsäure einheitlicher P1.1 und P2.1, kann an die Start-Nukleinsäurekette binden und die Synthese des P1.1-Ext bzw. P2.1-Ext initiieren. Im Verlauf der Amplifikation kommt es dabei zu Ausbildung von P1.1-Ext und P2.1-Ext. Die Trennung von beiden synthetisierten komplementären Primer-Verlängerungsprodukten P1.1-Ext und P2.1-Ext erfolgt vorzugsweise spezifisch durch komplementäre Bindung des P1.1-Ext an das Aktivator-Oligonukleotid. Die Trennung von Strängen einer anderen Sequenz-Variante, welche unter Verwendung von (SN 1.2) synthetisiert wurden, erfolgt weniger effizient aufgrund des Mismatches mit Position (N) zum korrespondierenden Sequenz-Segment des Aktivator-Oligonukleotides. Die Amplifikation von SN 1.2 Sequenz-Variante erfolgt somit weniger effizient bzw. nur unwesentlich bzw. gar nicht. Die Position des Sequenz-Segmentes (3), welches zu N2 der Zielsequenz korrespondiert, liegt in der Nähe der zweiten Blockierungseinheit, so dass Interaktion zwischen dem Aktivator-Oligonukleotid und dem jeweiligen ersten Primer-Verlängerungsprodukt von verwendeten Modifikationen in der zweiten Blockierungseinheit beeinflusst werden kann.
   Zur Erhöhung der Spezifität wird ein Kompetitor-Primer (P 5.3 oder P5.4)in die Reaktion zugegeben, welches in der Lage ist, vorwiegend komplementär an die Sequenz-Varianten der Zielsequenz bindet (N), dessen Amplifikation unterdrückt werden muss. Aufgrund einer komplementären Bindung mit solchen Primer-Bindungsstellen kann ein Kompetitor-Primer bevorzugt an bestimmte Sequenz-Varianten binden (hier mit N bezeichnet) und von Polymerase verlängert werden. Das dabei resultierende Produkt blockiert die einzelsträngigen Primer-Bindungsstellen für eine Interaktion des ersten Amplifikations-Primers.
   In einer Ausführungsform ist das Kompetitor-Primer-Oligonukleotid (P 5.3) länger als der erste Bereich des ersten Primer-Oligonukleotid, so dass sein 3'-Ende innerhalb der vierten Blockeirungs-Einheit des Aktivator-Oligonukleotides bindet (vierte Blockierungseinheit ist analog zu zweiten Blockeirungseinheit zusammengesetzt und blockiert eine Primer-Verlängerung am Aktivator-Oligonukleotid), so dass keine Verlängerung dieses Primers am Aktivator-Oligonukleotid erfolgen kann. Der Kompetitor-Primer kann das Segment der Matrize, welches P1.1 vorwiegend komplementär binden kann, vollständig (P 5.3) oder teilweise (P 5.4) abdecken. Der Kompetitor-Primer umfasst keinen zweiten Bereich und kann somit nicht mit dem ersten Bereich des Aktivator-Oligonukleotid interagieren. Dadurch wird verhindert, dass das Verlängerungsprodukt des Kompetitor-Oligonukleotides von der Matrize unter Mitwirkung eines Aktivator-Oligonukleotides abgelöst wird.

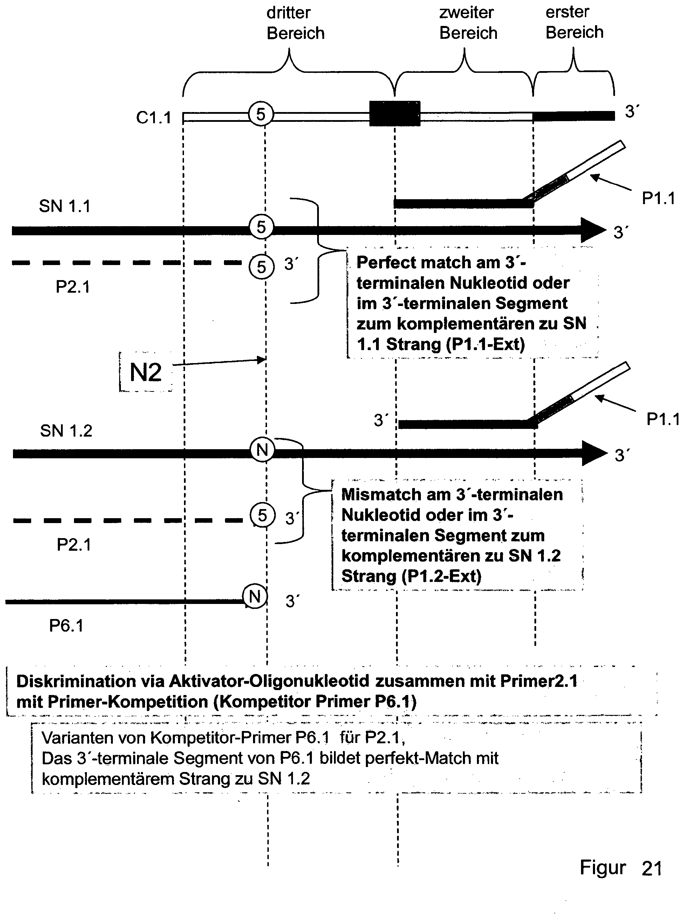

Fig. 21 zeigt schematisch eine Ausführungsform mit Topographie eines spezifischen Amplifikations-Systems und einer Start-Nukleinsäurekette mit einem polymorphen Lokus (N2) in einer Ausführungsform, bei welcher das zu N2 korrespondierende Segment des Aktivator-Oligonukleotide im dritten Bereich liegt (5). N2-Lokus umfasst auch das 3'-Terminale Nukleotid des zweiten Primers. Ein spezifischer P2.1, mit einem komplementären 3'-terminalen Nukleotid kann unter Reaktionsbedingungen bevorzugt spezifisch an die komplemenäre Position einer spezifischen Sequenz-Variante ausgehend von komplementären zu Start-Nukleinsäure (SN 1.1) strängen binden und die Synthese des P2.1-Ext vorzugsweise spezifisch initiieren. Im Verlauf der Amplifikation kommt es dabei zu Ausbildung von P1.1-Ext und P2.1-Ext. Die Bindung an eine andere Sequenz-Variante (SN 1.2) erfolgt weniger effizient aufgrund des Mismatches mit Position (N) innerhalb der Pimer-Bindungsstelle der SN 1.2. Die Amplifikation wird somit weniger effizient gestartet.
   Zur Erhöhung der Spezifität wird ein Kompetitor-Primer (P6.1) in die Reaktion zugegeben, welches in der Lage ist, vorwiegend komplementär an die Sequenz-Varianten der Zielsequenz zu binden, dessen Amplifikation unterdrückt werden muss. Aufgrund einer komplementären Bindung mit solchen Primer-Bindungsstellen kann ein Kompetitor-Primer bevorzugt an bestimmte Sequenz-Varianten binden (hier mit N bezeichnet) und von Polymerase verlängert werden. Das dabei resultierende Produkt blockiert die einzelsträngigen Primer-Bindungsstellen für eine Interaktion des ersten Amplifikations-Primers.

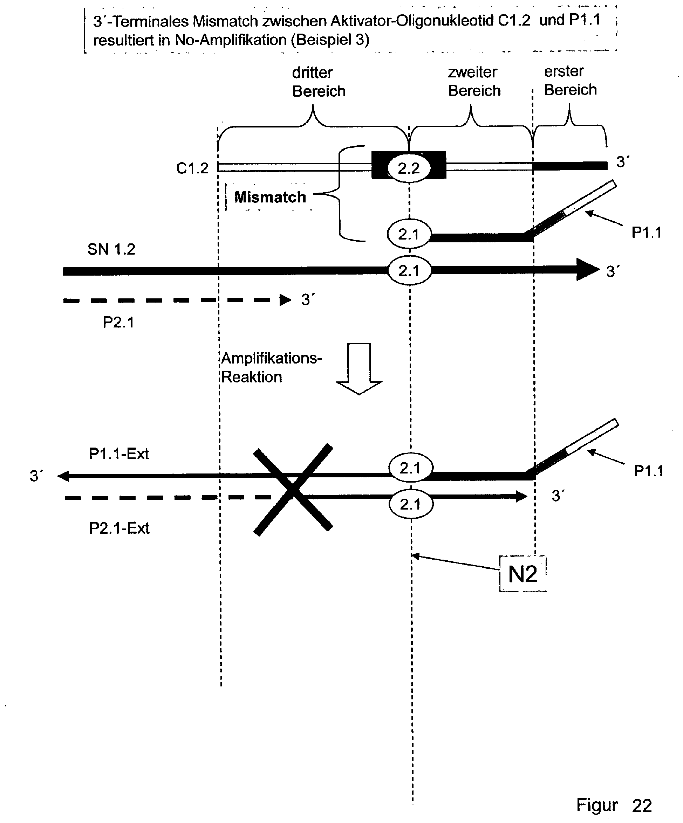

Fig. 22 - 23 zeigen schematisch eine Ausführungsform und die Erfordernis einer Übereinstimmung in der Topographie eines spezifischen Amplifikations-Systems:
   Bei einem Mismatch zwischen einem ersten Primer (hier P1.1 mit perfekt-Match Position zu Matrize) und einem in der Sequenz abweichenden Aktivator-Oligonukleotid (hier C1.2 mit einem Mismatch zum 3'-Ende des ersten Primers) kommt es nicht zu einer exponentiellen Amplifikation. Die Strang-Verdrängung wird bei jeder Interaktion zwischen dem Primer und dem Aktivator-Oligonukleotid durch einen solchen Mismatch bebindert, auch wenn die erste Primer-Verlängerung erfolgreich verläuft.
      Daraus ergeben sich spezifische Kombinationen (Fig. 23) zwischen einem spezifischen und charakteristischen Primer und einem spezifischen und charakteristischen Aktivator-Oligonukleotid.

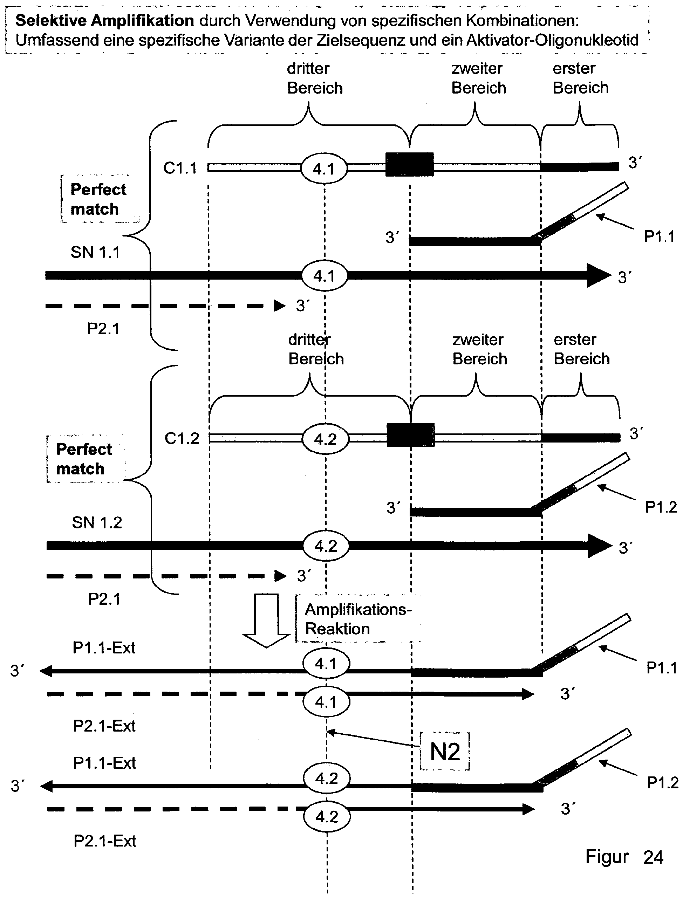

Fig. 24 teigt schematisch eine selektive Amplifikation von mehreren Sequenz-Varianten bei Verwendung von mehreren selektiven Aktivator-Oligonukleotiden.
Fig. 25 bis 28 zeigen schematisch das Zusammenwirken von Komponenten bei einer exponentiellen Amplifikation einer zu amplifizierenden Nukleinsäurekette in einzelnen Schritten.

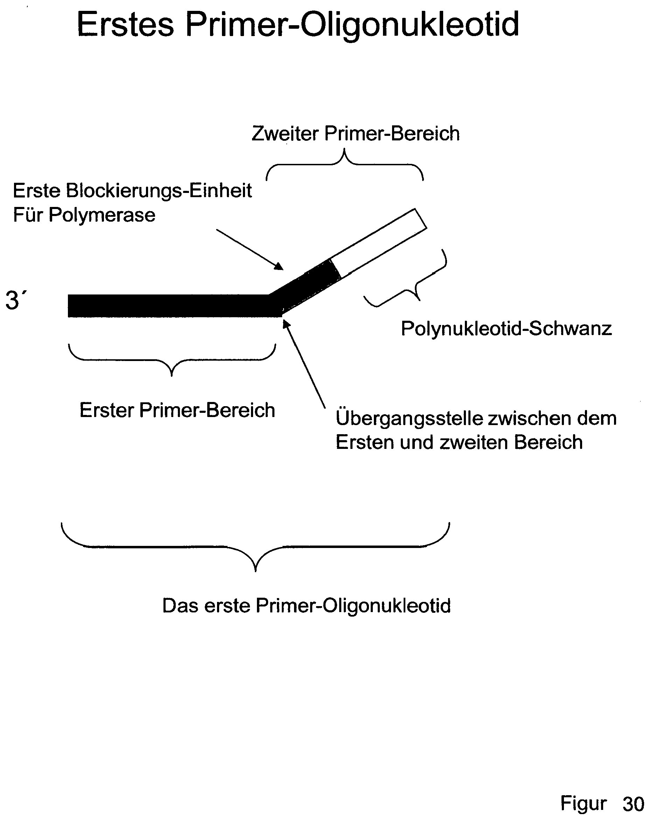

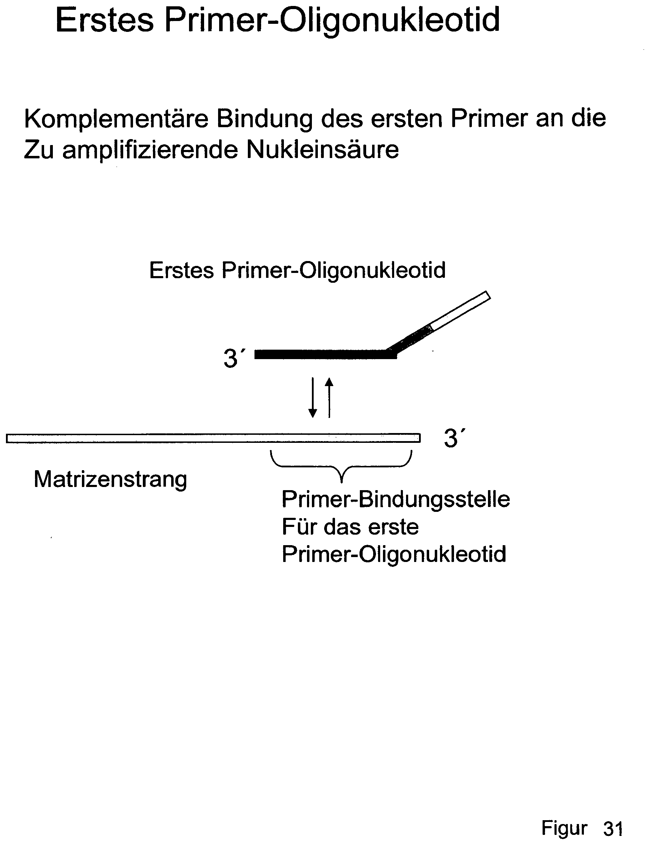

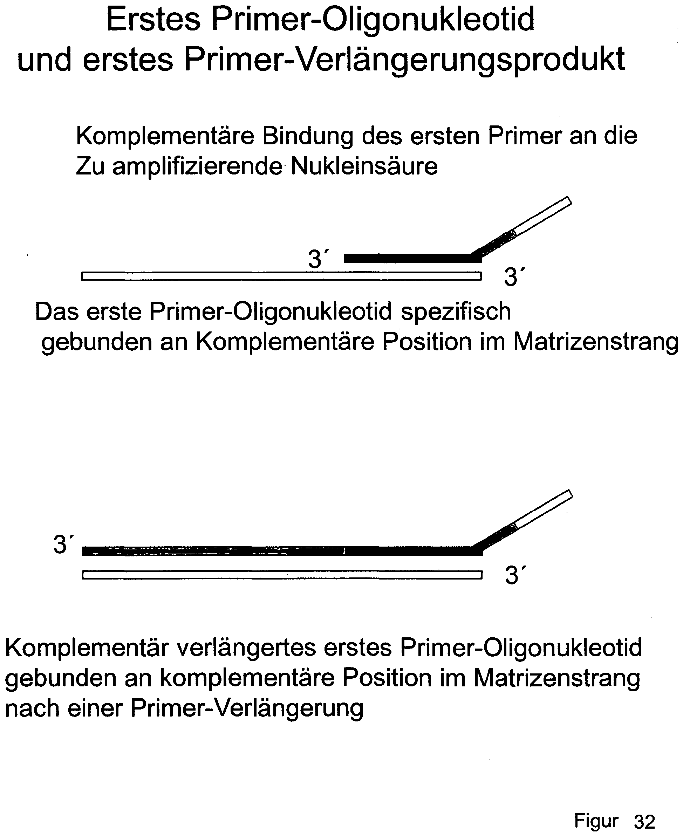

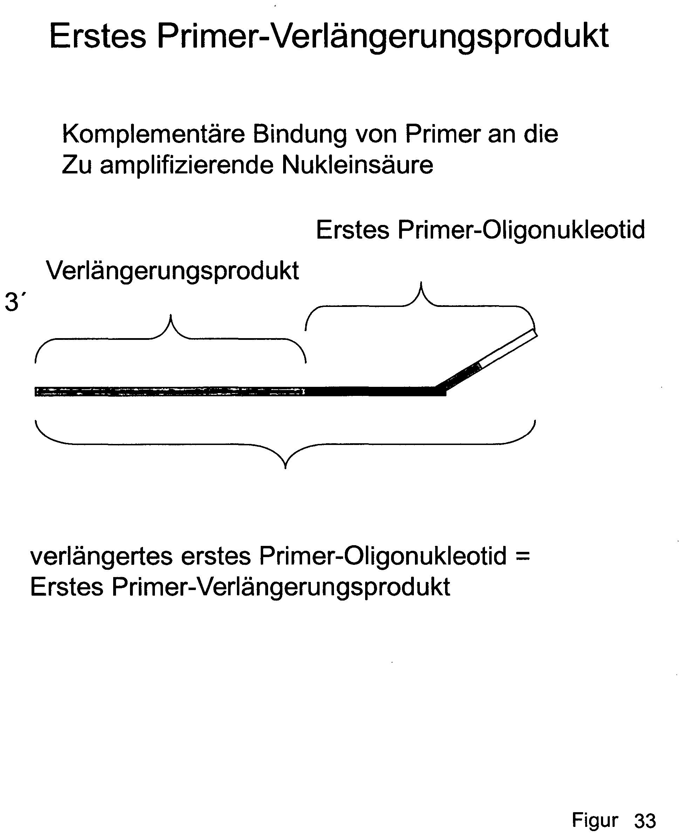

Figuren 29 - 34 zeigen schematisch die Struktur des ersten Primer-Oligonukleotids und des Aktivator-Oligonukleotides, sowie die Interaktion zwischen dem ersten Primer-Oligonukleotid und der Matrize, sowie die Synthese des ersten Primer-Verlängerungsprodukts.

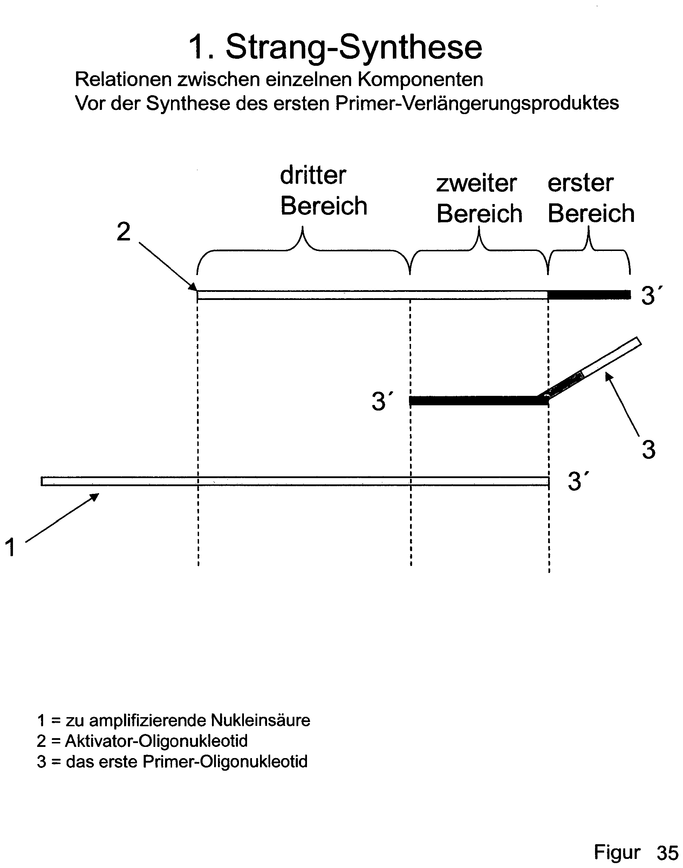

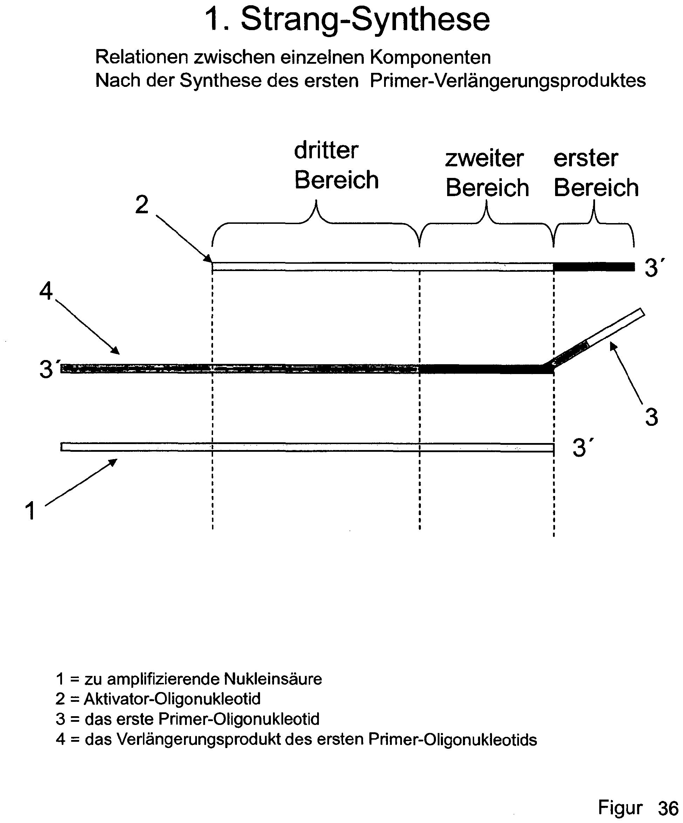

Figuren 35 -36 zeigen schematisch die Interaktion zwischen Strukturen während der Primer-Verlängung des ersten Primer-Oligonukleotids.

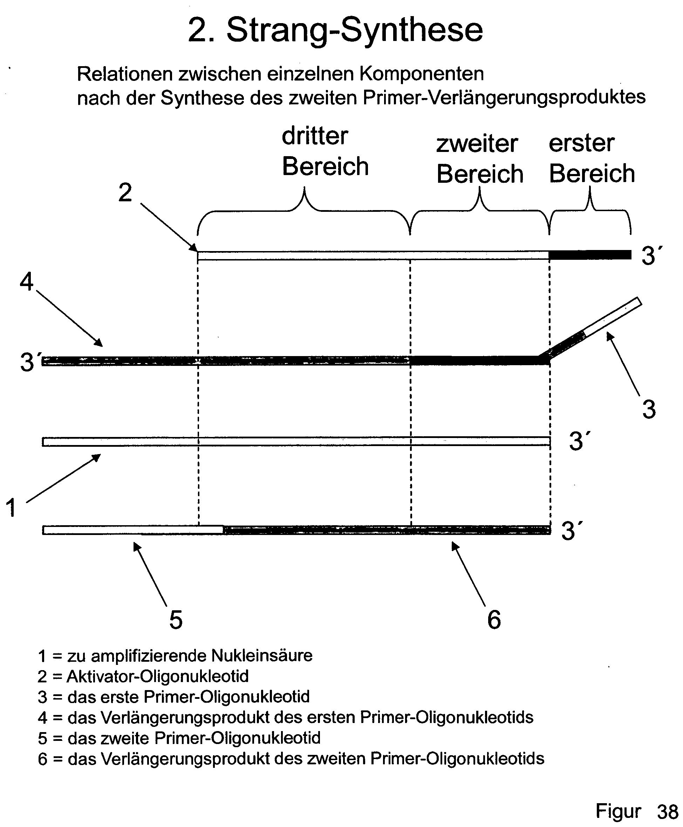

Figuren 37- 38 zeigen schematisch die Interaktion zwischen Strukturen während der Primer-Verlängerung des zweiten Primer-Oligonukleotids.

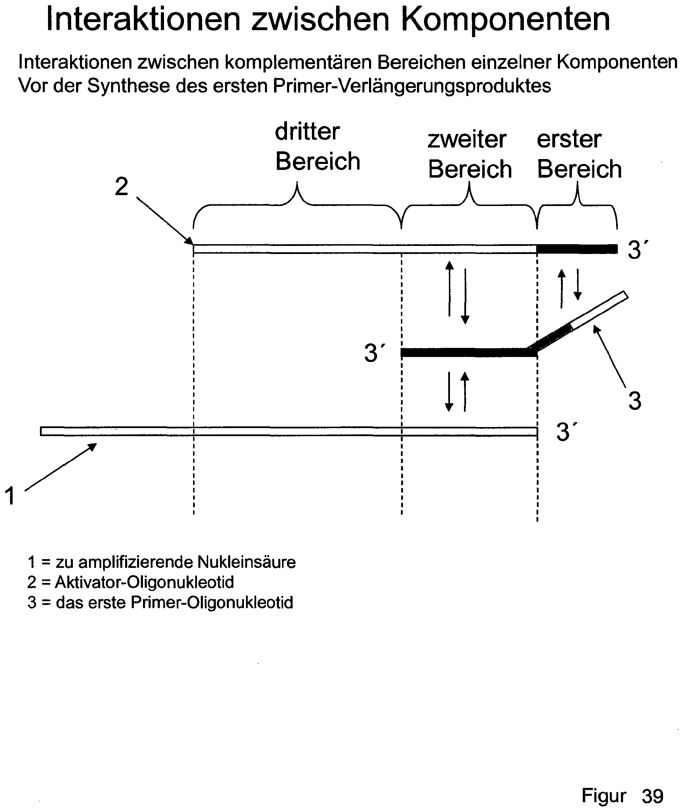

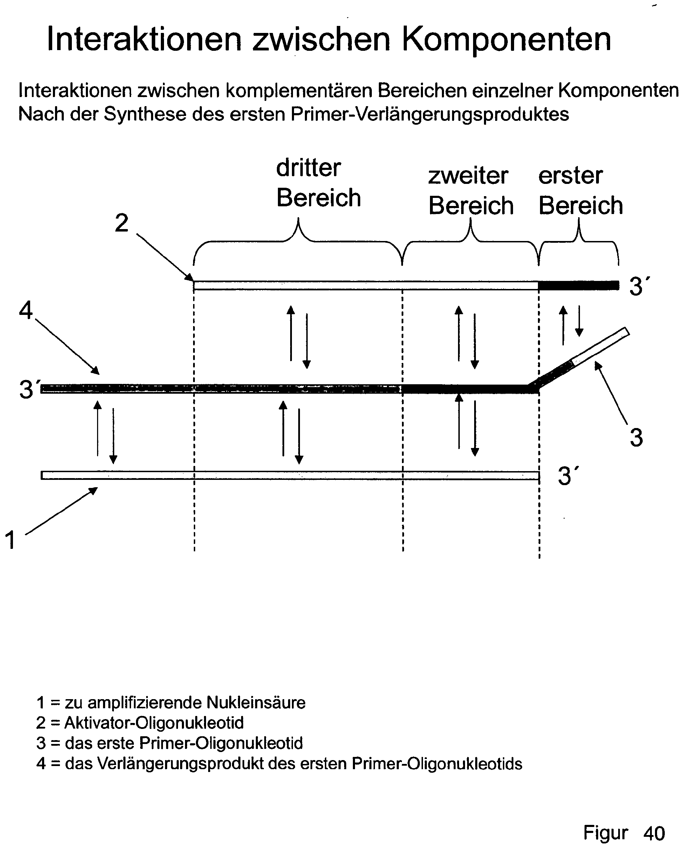

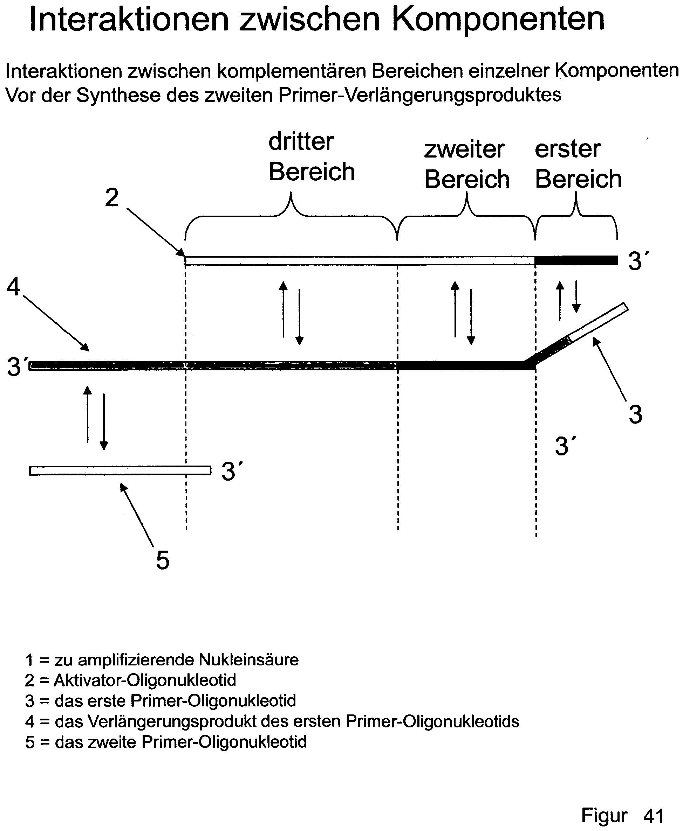

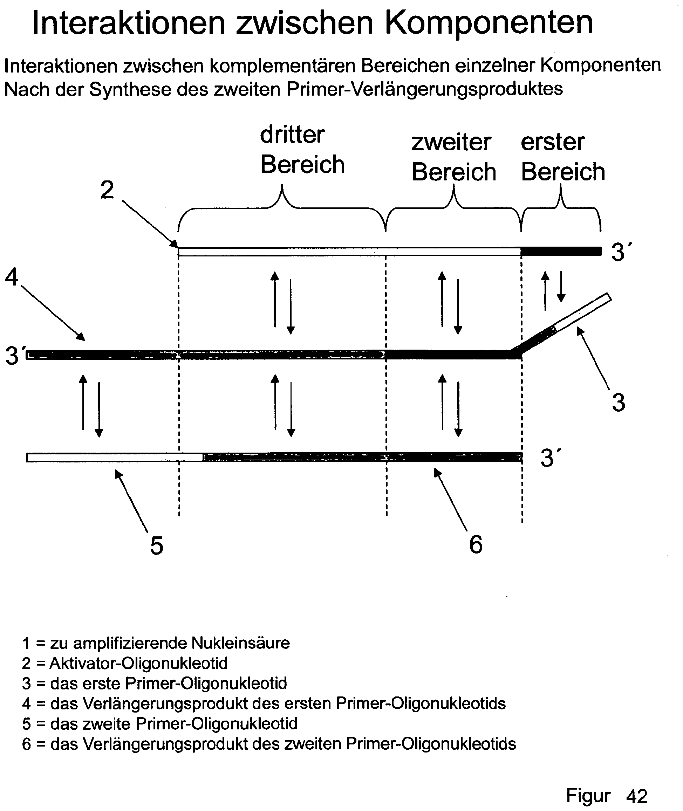

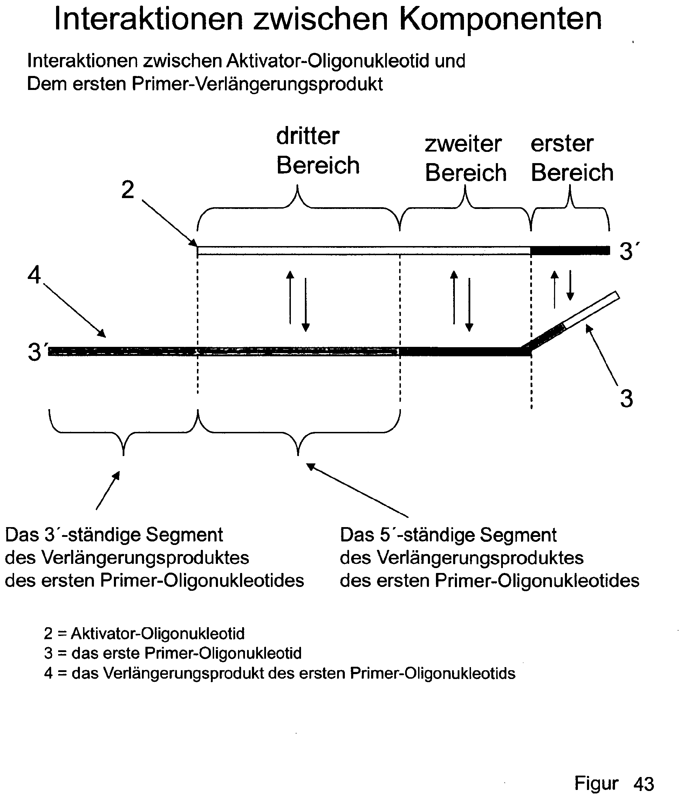

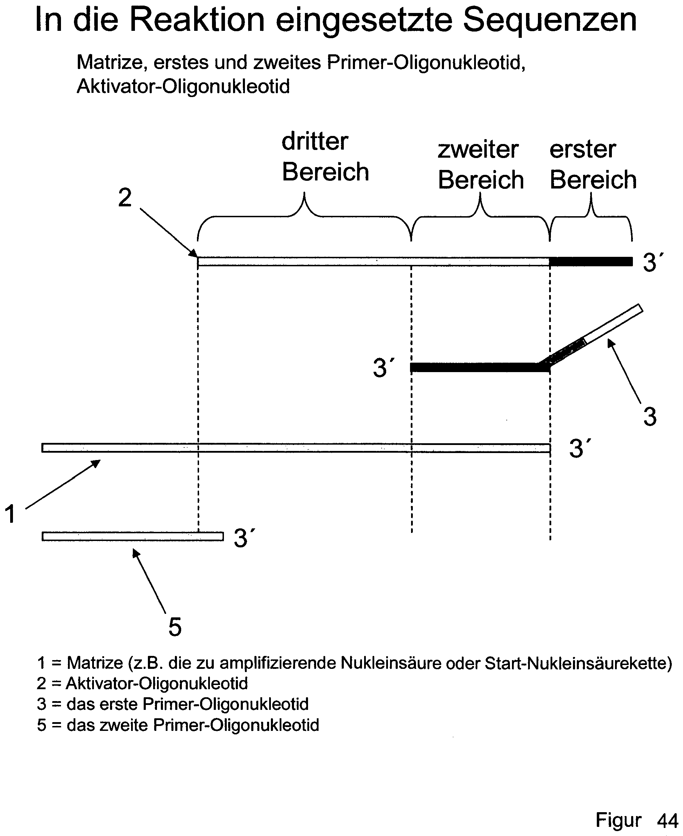

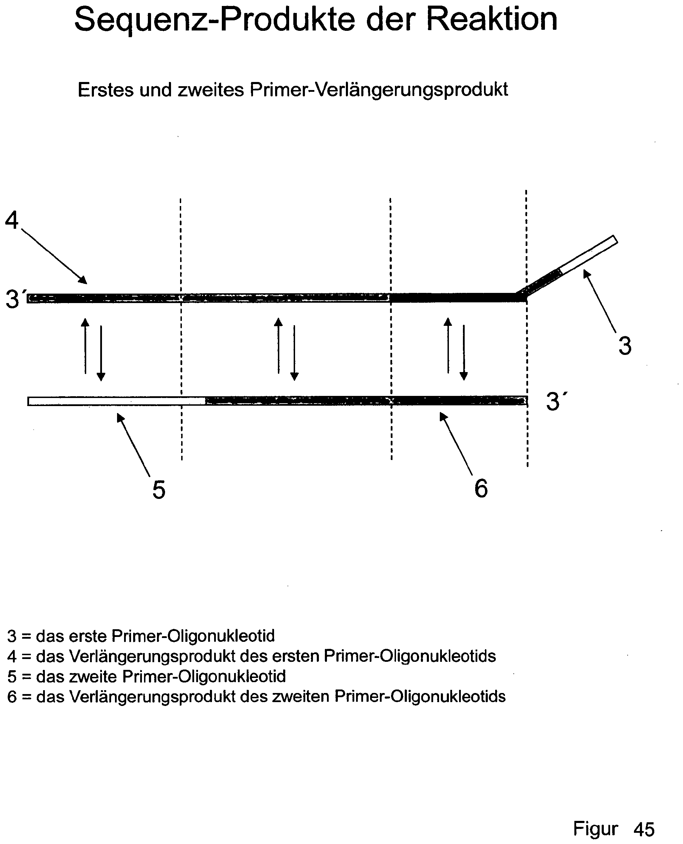

Figuren 39 - 45 zeigen schematisch die Interaktion zwischen einzelnen Bereichen von Komponenten während der Amplifikation.

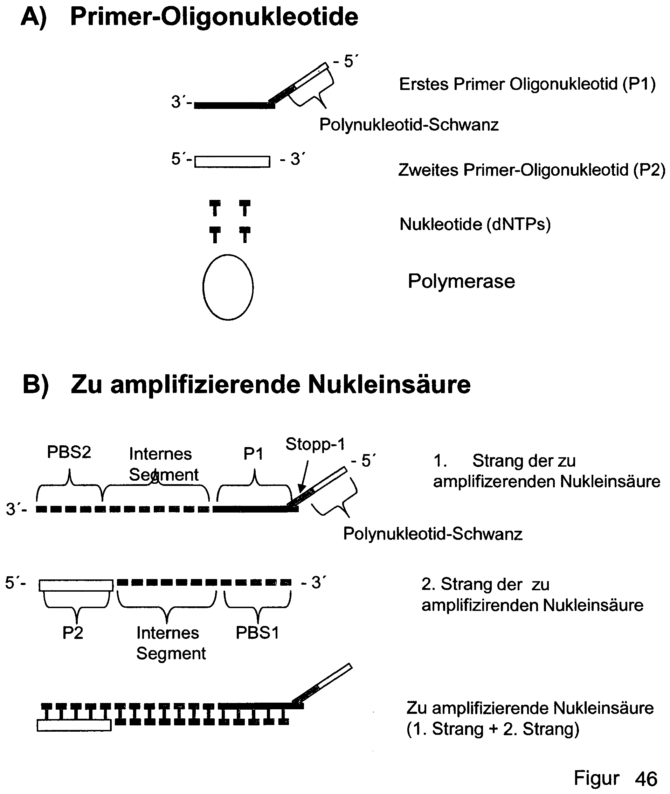

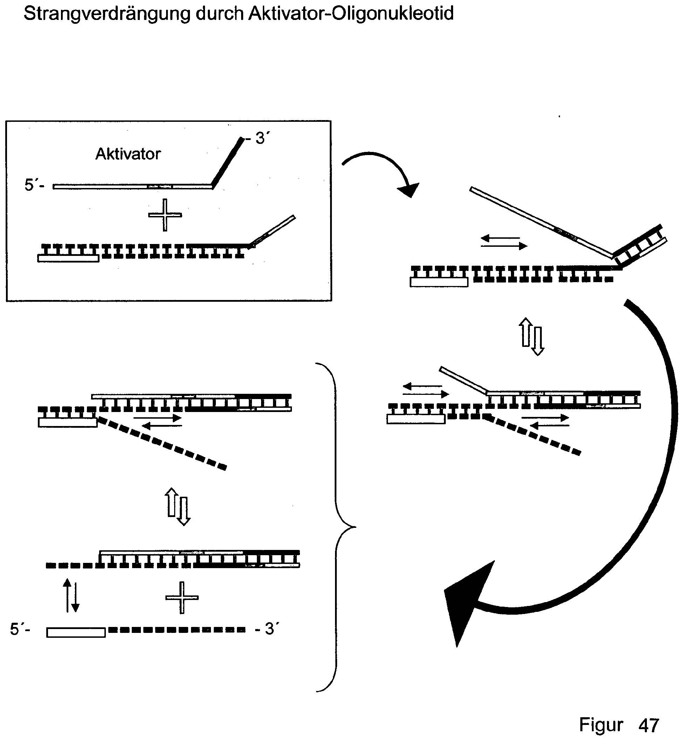

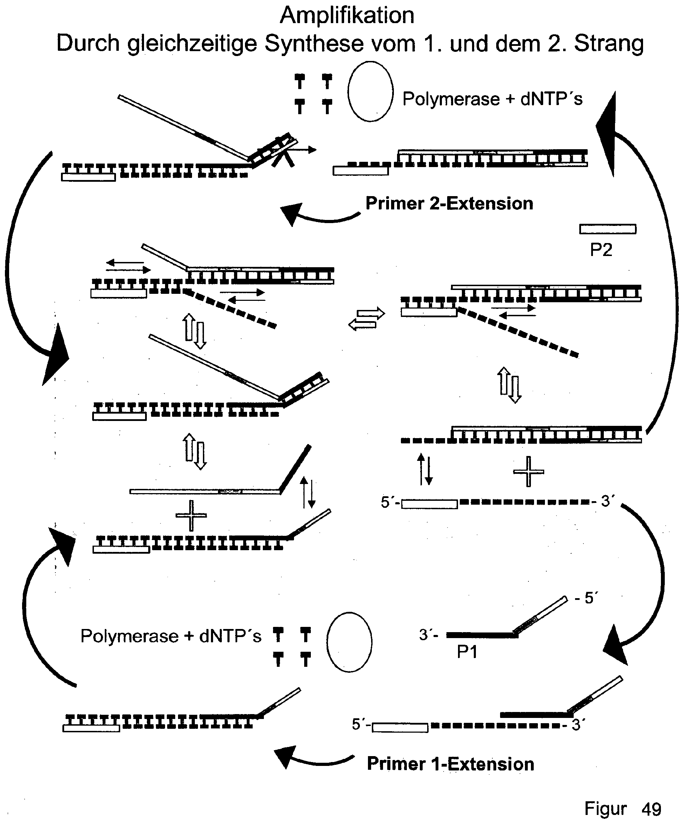

Figur 46 beschreibt mehr im Detail die Komponenten der in Fig. 47 - 51 dargestellten Strukturen.
Figur 47 zeigt schematisch den Strangverdrängungsmechanismus.
Figur 48 zeigt schematisch das Zusammenwirken der Strukturen bei Strangverdrängung.
Figur 49 zeigt schematisch das Zusammenwirken der Strukturen während der Nukleinsäureamplifikation durch einerseits die Amplifikation von erstem Primer-Oligonukleotid und zweitem Primer-Oligonukleotid und weiterhin die Einwirkung des Aktivator-Oligonukleotids und dabei resultierende Strangverdrängung.
Fig. 50 - 51 zeigen schematisch einige Ausführungsformen von Strukturen einer Start-Nukleinsäurekette und ihre Verwendung als Matrize zu Beginn der Reaktion.
Fig. 52 zeigt Ergebnisse aus Beispiel 1.
Fig. 53 zeigt Ergebnisse aus Beispiel 2.
Fig. 54 zeigt Ergebnisse aus Beispiel 3.

### Detaillierte Beschreibung der Erfindung

In dem erfindungsgemäßen Verfahren kann als Ausgangsmaterial eine einzelsträngige Nukleinsäurekette dienen oder eine doppelsträngige Nukleinsäure, welche in einzelsträngige Form überführt wurde. Die Amplifikation erfolgt bevorzugt sequenzspezifisch, d.h. vorzugsweise wird die zu amplifizierende Nukleinsäure vervielfältigt.

Als Komponenten des Amplifikationssystems dienen eine zu amplifizierende Nukleinsäurekette, ein erstes spezifisches Primer-Oligonukleotid, ein zweiter Primer und ein Aktivator-Oligonukleotid, welches bei der Trennung der neu synthetisierten Stränge mitwirkt, sowie eine geeignete Polymerase und Substrate wie dNTPs. Die Amplifikation verläuft in einer Puffer-Lösung unter Bedingungen, welche eine Primer-Verlängerungsreaktion von beiden Primern zulassen, sowie welche eine Strangverdrängung durch Aktivator-Oligonukleotid zur Trennung von beiden Primer-Verlängerungsprodukten unterstützen.

In einer Ausführungsform sollen alle Verfahrensschritte unter Bedingungen verlaufen, welche keine Trennung von synthetisierten Primer-Verlängerungsproduken in Abwesenheit eines geeigneten Aktivator-Oligonukleotides zulassen. Beispielsweise ist die Temperatur der Reaktionslösung dermaßen gewählt, dass die Tm eines Doppelstranges aus beiden Primer-Verlängerungsprodukten signifikant über der Reaktionstemperatur liegt.

Unter diesen Voraussetzungen erfolgt eine Trennung von beiden Primer-Verlängerungsprodukten in Abhängigkeit von der Wirkung des Aktivator-Oligonukleotides. Dieses Aktivator-Oligonukleotid ist in der Lage, komplementär an das erste Primer-Verlängerungsprodukt zu binden und dadurch das zweite Primer-Verlängerungprodukt aus seiner Bindung mit dem ersten Primerverlängerungsprodukt zu verdrängen. Um diese Strangverdrängungsreaktion zu initiieren, ist das erste Primer-Oligonukleotid mit einem Polynukleotid-Schwanz in seinem zweiten Bereich versehen, welcher in der Lage ist, das Aktivator-Oligonukleotid unter Reaktionsbedingungen vorübergehend zu binden und damit eine räumliche Nähe zu anderen Bereichen des ersten Primer-Verlängerungsnukleotides herbeizuführen. Nach der Initiierung der Strangverdrängung durch das Aktivator-Oligonukleotid wird das zweite Primer-Verlängerungsprodukt aus seiner Bindung mit dem ersten Primer-Verlängerungsprodukt verdrängt. Sein 3'-ständiges Segment wird damit frei und steht für weitere Bindung eines ersten Primer-Oligonukleotides zur Verfügung.

Der Polynukleotid-Schwanz des ersten Primer-Oligonukleotides ist vorzugsweise nicht von einer Polymerase kopierbar. Das kann entweder durch Verwendung entsprechender Modifikationen in diesem Bereich erreicht werden oder durch Einführung einer ersten Blockierungseinheit zwischen dem ersten Primer-Bereich und dem zweiten Primer-Bereich des ersten Primer-Oligonukleotides.

Die Synthese des zweiten Primer-Verlängerungsproduktes erfolgt nach der Bindung des zweiten Primer-Oligonukleotides an das erste Primer-Verlängerungsprodukt in seinem 3'-ständigen Segment. Dieses Segment geht vorzugsweise keine Bindung mit dem Aktivator-Oligonukleotid ein und ist hinreichend lang, um das zweite Primer-Oligonukleotid zu binden und eine erfolgreiche Primer-Verlängerungsreaktion zu unterstützen. Die Synthese des zweiten Primer-Verlängerungsproduktes erfolgt unter Verdrängung des Aktivator-Oligonukleotides aus der Bindung mit dem ersten Primer-Verlängerungsprodukt. Dies kann beispielsweise durch Polymerase-abhängige Strangverdrängung oder auch durch Strangverdrängung mittels des zweiten Primers erfolgen.

Beide Primer-Verlängerungsprodukte schließen kopierbare Bereiche ein und dienen gegenseitig als Matrize. Das Aktivator-Oligonukleotid dient nicht als Matrize. Dies kann bevorzugt durch Verwendung von Nukleotid-Modifikationen erreicht werden, welche zwar eine komplementäre Bindung mit dem ersten Primer-Verlängerungsprodukt eingehen können, nicht aber von der Polymerase als Matrize akzeptiert werden. Beispiele für solche Nukleotid-Modifikationen stellen Nukleotid-Verbindungen mit modifizierten Phosphat-Zucker-Rückgrad-Anteilen, z.B. 2'-O-Alkyl-RNA Modifikationen (z.B. 2'-OMe), LNA-Modifikationen oder Morpholino-Modifkationen dar. Im Allgemeinen hindert die Anwesenheit solcher Modifikationen in einem Strang eine DNA-Abhängige Polymerase beim Ablesen eines solchen Stranges. Die Anzahl von solchen Modifikationen kann unterschiedlich groß sein, in der Regel können wenige Modifikationen (zwischen 1 und 20) hinreichend sein, um eine Polymerase beim Ablesen eines solchen Stranges zu hindern. Solche Nukleotid-Modifikationen können beispielsweise an oder um die Bindungsstelle des ersten Primer-Oligonukleotides an das Aktivator-Oligonukleotid verwendet werden und/oder als Bestandteile des zweiten Bereichs des ersten Primer-Oligonukleotides.

Dank der Verwendung solcher Modifikationen kommt es zu einer lokalen Hinderung der Polymerase-Funktion, so dass bestimmte Segmente der verwendeten Strukturen nicht durch die Polymerase kopiert werden können und vorwiegend einzelsträngig bleiben. In dieser einzelsträngigen Form können sie weitere Reaktionskomponenten binden und somit ihre Funktion ausführen.

Unter Reaktionsbedingungen, welche einen Doppelstrang nicht denaturieren, führt die Verwendung der sequenzabhängigen nukleinsäurevermittelten Strangverdrängung zur sequenzspezifischen Trennung der beiden Primer-Verlängerungsprodukte während der Amplifikationsreaktion im beschriebenen Verfahren: eine hinreichende Komplementarität zwischen neusynthetisierten Verlängerungsfragmenten der Primer-Oligonukleotide mit der zu Beginn einer Amplifikation vorgegebenen Sequenz eines Aktivator-Oligonukleotides stellt eine Voraussetzung für eine erfolgreiche Strangverdrängung dar und kann somit Einfluss auf die Effizienz der Strangtrennung eines Doppelstranges (bestehend aus dem ersten und dem zweiten Primer-Verlängerungsprodukt) haben. Bei geringfügigen Abweichungen kommt es zu einer Verlangsamung der Strangverdrängung und somit auch zur Verlangsamung der Strangtrennung. Diese kann sich in einer Verlangsamung der Gesamtreaktion auswirken. Mit steigender Differenz in der Sequenz der neusynthetisierten Verlängerungsprodukte und der zu Beginn der Reaktion vorgegebenen Sequenz des Aktivator-Oligonukleotides kommt es zu einer immer höheren Hinderung der Strangverdrängung, welche im Endeffekt keine ausreichende Trennung von beiden Primer-Verlängerungsprodukten mehr leisten kann. Die beiden neu synthetisierten Stränge können nicht mehr ausreichend voneinander getrennt werden, so dass ihre Bindungsstellen für Primer-Oligonukleotide nicht mehr zugängig sind. Das führt im Allgemeinen zum Abbruch einer Amplifikation von Sequenzen mit Sequenzabweichungen.

Zusammengefasst können nicht nur die Spezifitäten der Bindung von beiden Primern mit ihren Matrizen, sondern auch die Beschaffenheit der Sequenzsegmente zwischen den Primern auf die Amplifikation Auswirkung haben, nämlich indem diese Abschnitte eine hinreichende Strangverdrängung zulassen oder nicht, je nach ihrer Übereinstimmung in der zu Beginn der Reaktion vorgegebenen Sequenz des Aktivator-Oligonukleotides. Das beschrieben Verfahren kann dadurch unter Umständen insgesamt eine höhere Spezifität vorweisen, als die herkömmlichen Amplifikations-Verfahren.

Die spezifische Amplifikation resultiert weiterhin durch Verwendung der Komponenten bei Reaktionsbedingungen, welche bevorzugt eine spontane Trennung von neu synthetisierten Primer-Verlängerungsprodukte nicht zulassen.

Das Verfahren umfasst mehrere Vorgänge, welche nachfolgend beschrieben sind. Diese Vorgänge können in einem Ansatz oder in getrennten Ansätzen ausgeführt werden. Wenn die Vorgänge in einem Ansatz durchgeführt werden sollen, so können diese unter gleichen Bedingungen, z.B. isothermal, oder unter unterschiedlichen Bedingungen, z.B. beim Thermocycling, ausgeführt werden. Vorzugsweise sind Primer-Oligonukleotide und Aktivator-Oligonukleotid zu Beginn der Reaktion präsent. Eine sequenzielle Zugabe einzelner Reagenzien ist allerdings auch möglich.

Ebenfalls sind Kombinationen mit anderen Amplifikationsverfahren möglich, z.B. mit PCR, wobei die PCR beispielsweise erst über 1 bis 10 Zyklen durchgeführt werden und anschließend wird beispielsweise unter isothermalen Bedingungen weitergearbeitet.

### Wesentliche Aspekte der Erfindung

### Ausführungsform 1

In einer vorteilhaften Form umfasst das Verfahren zu selektiven Amplifizierung von Nukleinsäureketten folgende Schritte:
1. Bereitstellung einer zu amplifizierenden Nukleinsäurekette (einer Start-Nukleinsäurekette), welche eine Zielsequenz umfasst, wobei diese Zielsequenz mindestens ein Sequenzsegment (genannt polymorpher Lokus) umfasst, welches mindestens zwei charakteristische und unterschiedliche Sequenzen umfassen kann (genannt Allel-Varianten), weiterhin umfasst die Zielsequenz mindestens ein weiteres Zielsequenzsegment, welches kein polymorpher Lokus ist und für diese Zielsequenz einheitlich und charakteristisch ist
2. Bereitstellung mindestens eines Amplifikations-Systems, jedes Amplifikations-System umfassend:
   a. mindestens ein erstes Primer-Oligonukleotid
   b. mindestens ein zweites Primer-Oligonukleotid
   c. mindestens eine Polymerase
   d. mindestes ein Aktivator-Oligonukleotid
   Wobei mindestens das Aktivator-Oligonukleotid eines Amplifikations-Systems eine für das jeweilige Amplifikations-System spezifische und charakteristische Sequenzzusammensetzung umfasst, welche die Amplifikation von einer charakteristischen und spezifischen Allel-Variante einer Zielsequenz ermöglicht,
3. Ausführung einer Amplifikation einer zu amplifizierenden Nukleinsäurekette, welche folgende Schritte umfasst:
   a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst, wobei das erste Primer-Oligonukleotid umfasst:
      - Einen ersten Primer-Bereich im 3'-Segment des ersten Primer-Oligonukleotides, der an einen Strang einer zu amplifizierenden Nukleinsäurekette sequenzspezifisch binden kann,
      - einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedingungen im Wesentlichen unkopiert bleibt.
   b) matrizenabhängige Verlängerung des ersten Primer-Oligonukleotids unter Verwendung eine Nukleinsäurekette als Matrizenstrang (umfassend entweder eine zu amplifizierende Nukleinsäurekette und / oder eine Start-Nukleinsäurekette und / oder ein zweites Primer-Verlängerungsprodukt) mit Hilfe einer matrizenabhängigen Polymerase zur Bildung eines ersten Primer-Verlängerungsproduktes, das zur Zielsequenz und/oder zu der zu amplifizierenden Nukleinsäurekette (a) komplementäre Sequenzabschnitte umfasst,
   c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
      - Einen ersten einzelsträngigen Bereich, welcher an den Polynukleotid-Schwanz des zweiten Bereichs des ersten Primer-Oligonukleotides binden kann,
      - einen zweiten einzelsträngigen Bereich, welcher zu dem ersten Bereich des ersten Primer-Oligonukleotides komplementär ist und an diesen binden kann,
      - einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
      wobei das Aktivator-Oligonukleotid nicht als Matrize für eine Primerverlängerung des ersten Primer-Oligonukleotid dient,
   d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
   e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zu amplifizierenden Nukleinsäurekette, wobei das 3'-Segment des ersten Primer-Verlängerungsproduktes einzelsträngig wird
   f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
   g) matrizenabhängige Verlängerung des zweiten Oligonukleotid-Primers unter Verwendung des ersten Primer-Verlängerungsproduktes als Matrize mit einer matrizenabhängigen Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
   h) Wiederholung der Schritte a)-g) bis das gewünschte Ausmaß der Amplifikation erreicht ist.

In einer Ausführungsform umfasst eine Zielsequenz einen polymorphen Lokus, welcher mindestens eine Nukleotid-Position umfasst, welche mindestens zwei charakteristische Sequenz-Varianten bei einer Zielsequenz umfasst.
In einer weiteren Ausführungsform umfasst eine Zielsequenz einen polymorphen Lokus, welcher mindestens zwei Nukleotid-Positionen umfasst, welche mindestens zwei charakteristische Sequenz-Varianten bei einer Zielsequenz umfassen.
In einer Ausführungsform umfasst eine Zielsequenz einen polymorphen Lokus, welcher eine Länge zwischen ein Nukleotid bis 100 Nukleotiden umfasst, wobei solcher Lokus mindestens zwei charakteristische Sequenz-Varianten einer Zielsequenz umfasst.
In einer Ausführungsform in Schritt (1) werden mindestens zwei Start-Nukleinsäureketten bereitgestellt, wobei jede Start-Nukleinsäurekette eine Variante einer Zielsequenz mit einer spezifischen und charakteristischen Sequenzvariante eines polymorphen Lokus umfasst.
In einer weiteren Ausführungsform in Schritt (1) werden mindestens zwei Start-Nukleinsäureketten bereitgestellt, wobei jede Start-Nukleinsäurekette eine eigne Zielsequenz umfasst und diese Zielsequenzen der ersten und einer weiteren Start-Nukleinsäurekette unterschiedlich sind.

### Ausführungsform 2

Verfahren nach Ausführungsform 1, welches im Schritt 2 mindestens ein Amplifikations-System umfasst, welches ein spezifisches Aktivator-Oligonukleotid umfasst, welches ein zum polymorphen Lokus korrespondirendes Sequenzsegment umfasst, welches mit einer charakteristischen und spezifischen Sequenzvariante des polymorphen Lokus einer im Schritt 1 bereitgestellen zu amplifizierenden Nukleinsäurekette umfassend eine Zielnukleinsäure oder ihrem komplementären Strang eine komplementäre perfekt-match Bindung eingehen kann

### Ausführungsform 3

Verfahren nach Ausführungsform 2, welches im Schritt 2 mindestens ein Amplifikations-System umfasst, welches ein spezifisches Aktivator-Oligonukleotid umfasst, welches ein zum polymorphen Lokus korrespondirendes charakteristisches und spezifisches komplementäres Sequenzsegment umfasst, und weiterhin mindestens ein weiteres Sequenzsegment umfasst, welches im Wesentlichen komplementär an mindestens ein spezifisches Segment einer Zielsequenz binden kann, wobei dieses Segment der Zielsequenz nicht polymorpher Lokus ist.

In einer Ausführungsform umfasst das in Schritt 2 bereitgestellte Amplifikations-System
- ein Aktivator-Oligonukleotid, wie in Ausführungsform 2 oder 3 spezifiziert und
- ein erstes Primer-Oligonukleotid, welches an das für die bereitgestelle Zielsequenz einheitliche Sequenzfragment im Wesentlichen komplementär binden kann und
- ein zweites Primer-Oligonukleotid, welches an das für die bereitgestelle Zielsequenz einheitliche Sequenzfragment im Wesentlichen komplementär binden kann
Wobei das erste Primer-Oligonukleotid und das zweite Primer-Oligonukleotid keine Sequenzsegmente umfassen, welche spezifisch für eine Sequenzvariante der Zielsequenz sind. Vielmehr kann das erste Primer-Oligonukleotid und das zweite Primer-Oligonukleotid vollständig komplementär oder im Wesenlichen komplementär an die Zielsequenz binden, ohne die Sequenzvarianten zu unterscheiden.
Ein solches Amplifikations-System ist somit dermassen konstruiert, dass der polymorpher Lokus im Sequenzsegment lokalisiert ist, welches zwischen beiden Primern liegt. Die Zielsequenz umfasst somit auf beiden Seiten eines polymorphen Lokus je ein Zielsequenz-spezifisches Sequenzsegment.
Das zum polymorphen Lokus korrespondierende Segment des Aktivator-Oligonukleotides liegt somit im dritten Bereich des Aktivator-Oligonukeotides.

In einer weiteren Ausführungsform umfasst das in Schritt 2 bereitgestellte Amplifikations-System
- ein Aktivator-Oligonukleotid, wie in Ausführungsform 2 oder 3 spezifiziert und
- ein erstes Sequenz-varianten spezifisches Primer-Oligonukleotid, welches zumindest teilweise (z.B. mit seinem 3'-terminalen Nukleotid) an das spezifische Sequenz-Segment eines polymorphen Lokus einer bereitgestellen Zielsequenz voll-komplementär binden kann
- ein zweites Primer-Oligonukleotid, welches an das für die bereitgestelle Zielsequenz einheitliche Sequenzfragment bindet und
Wobei das zweite Primer-Oligonukleotid keine Sequenzsegmente umfasst, welche charakteristisch und spezifisch für nur eine Sequenzvariante der Zielsequenz sind. Vielmehr kann das zweite Primer-Oligonukleotid vollständig komplementär oder im Wesenlichen komplementär an die Zielsequenz binden, ohne die Sequenzvarianten zu unterscheiden.
In einer Ausführungsform ist ein solches Amplifikations-System somit dermassen konstruiert, dass die Sequenz des ersten Primer-Oligonukleotides zumindest mit einer Nukleotid-Position mit einer charakteristischen Sequenz des polymorphen Lokus überlagert.
Das zum polymorphen Lokus korrespondierende Segment des Aktivator-Oligonukleotides liegt in einer Ausführungsform vollständig im zweiten Bereich des Aktivator-Oligonukeotides.
In einer weiteren Ausführungform liegt das zum polymorphen Lokus korrespondierende Segment des Aktivator-Oligonukleotides partiell im zweiten und partiell im dritten Bereich des Aktivator-Oligonukeotides.

In einer weiteren Ausführungsform umfasst das in Schritt 2 bereitgestellte Amplifikations-System
- ein Aktivator-Oligonukleotid, wie in Ausführungsform 2 oder 3 spezifiziert und
- ein erstes Primer-Oligonukleotid, welches an das für die bereitgestelle Zielsequenz einheitliche Sequenzfragment bindet und
- ein zweites Sequenz-varianten spezifisches Primer-Oligonukleotid, welches zumindest teilweise (z.B. mit seinem 3'-terminalen Nukleotid) an das spezifische Sequenz-Segment eines polymorphen Lokus einer bereitgestellen Zielsequenz voll-komplementär binden kann
Wobei das erste Primer-Oligonukleotid keine Sequenzsegmente umfasst, welche charakteristisch und spezifisch für nur eine Sequenzvariante der Zielsequenz sind. Vielmehr kann das erste Primer-Oligonukleotid vollständig komplementär oder im Wesenlichen komplementär an die Zielsequenz binden, ohne die Sequenzvarianten zu unterscheiden.
In einer Ausführungsform ist ein solches Amplifikations-System somit dermassen konstruiert, dass die Sequenz des zweiten Primer-Oligonukleotides zumindest mit einer Nukleotid-Position mit einer charakteristischen Sequenz des polymorphen Lokus überlagert.
Das zum polymorphen Lokus korrespondierende Segment des Aktivator-Oligonukleotides liegt in einer Ausführungsform vollständig im dritten Bereich des Aktivator-Oligonukeotides.

In einer weiteren Ausführungsform umfasst das in Schritt 2 bereitgestellte Amplifikations-System
- ein Aktivator-Oligonukleotid, wie in Ausführungsform 2 oder 3 spezifiziert und
- ein erstes Sequenz-varianten spezifisches Primer-Oligonukleotid, welches zumindest teilweise (z.B. mit seinem 3'-terminalen Nukleotid) an das spezifische Sequenz-Segment eines polymorphen Lokus einer bereitgestellen Zielsequenz voll-komplementär binden kann
- ein zweites Sequenz-varianten spezifisches Primer-Oligonukleotid, welches zumindest teilweise (z.B. mit seinem 3'-terminalen Nukleotid) an das spezifische Sequenz-Segment eines polymorphen Lokus einer bereitgestellen Zielsequenz voll-komplementär binden kann
In einer Ausführungsform ist ein solches Amplifikations-System somit dermassen konstruiert, dass die Sequenz des ersten und des zweiten Primer-Oligonukleotides zumindest mit einer Nukleotid-Position mit einer charakteristischen Sequenz des polymorphen Lokus überlagert.
Das zum polymorphen Lokus korrespondierende Segment des Aktivator-Oligonukleotides liegt in dieser Ausführungsform im zweiten und im dritten Bereich des Aktivator-Oligonukeotides. Die Länge des polymorphen Lokus umfasst dabei vorzugsweise Bereiche zwischen 5 und 100 Nukleotide.

### Ausführungsform 4

Verfahren nach Ausführungsform 1, bei welchen nur eine im Schritt 1 bereitgestellte Start-Nukleinsäurekette umfassend eine spezifsiche und charakteristische Sequenz-Variante eines polymorphen Lokus bevorzugt als eine zu amplifizierende Nukleinsäure amplifiziert werden soll, welche eine erste spezifische und charakteristische Sequenz im polymorphen Lokus umfasst.

Verfahren nach Ausführungsform 4, bei welchen nur eine im Schritt 1 bereitgestellte Start-Nukleinsäurekette umfassend eine spezifsiche und charakteristische Sequenz-Variante eines polymorphen Lokus bevorzugt als eine zu amplifizierende Nukleinsäure amplifiziert werden soll, welche eine erste spezifische und charakteristische Sequenz im polymorphen Lokus umfasst, wobei ggf. mindestens eine zweite im Schritt 1 bereitgestellte spezifische Start-Nukleinsäureketten umfassend eine zweite chakrateristische Sequenz im polymorphen Lokus in ihrer Amplifikation weniger bevorzugt amplifiziert werden soll bzw. ihre Amplifikation unterdrückt werden soll. Die erste und die zweite charakteristische Sequenz-Varaiten sind dabei nicht identisch.

### Ausführungsform 5

Verfahren nach Ausführungsform 4, welches im Schritt 1 mindestens zwei Start-Nukleinsäureketten umfasst, wobei jede Start-Nukleinsäurekette eine charakteristische und spezifische Sequenz-Variante einer Zielnukleinsäure umfasst und
im Schritt 2 nur ein spezifisches Amplifikations-System, umfassend ein für eine Sequenz-Variante der Zielsequenz spezifisches und charakteristisches Aktivator-Oligonukleotid umfasst, bereitgestellt wird, so dass im Schritt 3 nur eine charakteristische und spezifische zu amplifizierende Nukleinsäurekette bevorzugt amplifiziert wird, wobei die spezifisch amplifizierte Nukleinsäurekette mit dem zum polymorphen Lokus korrespondierenden Sequenzsegment eines verwendeten für diese zu amplifizierende Nukleinsäurekette charakteristischen Aktivator-Oligonukleotids eine perfekt-match Bindung eingehen kann.

### Ausführungsform 6

Verfahren nach Ausführungsform 4, welches im Schritt 1 mindestens zwei Start-Nukleinsäureketten umfasst, wobei jede Start-Nukleinsäurekette eine charakteristische und spezifische Sequenz-Variante einer Zielsequenz umfasst und
im Schritt 2 nur ein spezifisches Amplifikations-System, umfassend ein für eine Sequenz-Variante der Zielsequenz spezifisches und charakteristisches Aktivator-Oligonukleotid umfasst (wie in Ausführungsform 5 beschrieben) und weiterhin mindestens ein für eine Sequenz-Variante einer Zielsequenz spezifisches Primer-Oligonukleotid (das erste Primer-Oligonukleotid oder das zweite Primer-Oligonukleotid), bereitgestellt wird, so dass im Schritt 3 nur eine charakteristische und spezifische zu amplifizierende Nukleinsäurekette bevorzugt amplifiziert wird, wobei die spezifisch amplifizierte Nukleinsäurekette mit dem zum polymorphen Lokus korrespondierenden Sequenzsegment eines verwendeten für diese zu amplifizierende Nukleinsäurekette charakteristischen Aktivator-Oligonukleotids eine perfekt-match Bindung eingehen kann.

### Ausführungsform 7

Verfahren nach Ausführungsform 6, wobei das 3'-terminale Segment oder das 3'-terminale Nukleotid eines Primers (einen ersten Primer oder einen zweiten Primer) mit einer charakteristischen und spezifischen Sequenzvariante des polymorphen Lokus einer im Schritt 1 bereitgestellen Zielsequenz umfassenden Start-Nukleinsäurekette oder ihrem komplementären Strang eine komplementäre perfekt-match Bindung eingehen kann und von Polymerase unter Reaktionsbedingungen, wenn komplementär gebunden an Matrizenstrang umfassend diese Sequenz-Variante, verlängert werden kann, so dass dabei ein spezifisches und charakteristisches Primer-Verlängerungsprodukt resultiert.

### Ausführungsform 8

Verfahren nach einer von Ausführungsformen 1 bis 7, welches im Schritt 2 mindestens ein Amplifikations-System umfasst, welches zusätzlich mindestens ein Kompetitor-Primer-Oligonukleotid umfasst, welches nicht identisch ist mit dem ersten oder dem zweiten Primer eines Amplifikations-Systems und
dieses Kompetitor-Primer mindestens ein zum polymorphen Lokus korrespondierendes Sequenzsegment umfasst, welches mit einer charakteristischen und spezifischen Sequenzvariante des polymorphen Lokus einer im Schritt 1 bereitgestellen Zielnukleinsäure oder ihrem komplementären Strang eine komplementäre perfekt-match Bindung eingehen kann und von Polymerase unter Reaktionsbedingungen verlängert werden kann, so dass dabei ein spezifisches und chrakteristisches Kompetitor-Primer-Verlängerungsprodukt resultiert.

In einer Ausführungsform kann das verwendete Kompetitor-Oligonukleotid vollständig oder teilweise an das Segment der Zielsequenz binden, welches mit dem ersten Bereich des ersten Primer-Oligonukleotids komplementäre Bindung eingehen kann.

In einer Ausführungsform kann das verwendete Kompetitor-Oligonukleotid vollständig oder teilweise an das Segment der Zielsequenz binden, welches mit dem zweiten Bereich des ersten Primer-Oligonukleotids komplementäre Bindung eingehen kann.

In einer Ausführungsform ist dieses Kompetitor-Primer-Oligonukleotid voll-komplementär zu einer Sequenz-Variante einer Zielsequenz, welche nicht identisch ist mit der Sequenz-Variante der zu amplifizierenden Nukleinsäurekette.

In einer weiteren Ausführungsform wird die Amplifikation der Sequenz-Variante, welche zum Kompetitor-Primer-Oligonukleotid pefekt-match ausbilden kann, durch Verwendung des Kompetitor-Oligonukleotides selektiv unterdrückt.

In einer Ausführungsform des Verfahrens umfasst ein Kompetitor-Primer-Oligonukleotid keinen Sequenzsegment, welches mit dem ersten Bereich des Aktivator-Oligonukleotides im Wesentlichen komplementär binden kann.

### Ausführungform 9

Verfahren nach Ausführungsform 1, bei welchen mindestens zwei im Schritt 1 bereitgestellte zu amplifizierende Nukleinsäureketten amplifiziert werden sollen, wobei eine erste zu amplifizierende Nukleinsäurekette eine erste spezifische und charakteristische Sequenz im polymorphen Lokus umfasst, und jede weitere zu amplifizierende Nukleinsäurekette eine für sie spezifische und charakteristische Sequenz im polymorphen Lokus umfasst, und
im Schritt 2 wird für jede zu amplifizierende Nukleinsäurkette ein spezifisches Amplifikations-System bereitgestellt, bei welchem zumindest das Aktivator-Oligonukleoid eine für jede zu amplifizierende Nukleinsäurekette ein spezifisches zum polymorphen Lokus korrespondierendes Segment umfasst,
im Schritt 3 erfolgt eine spezifische Amplifikation einer jeden zu amplifizierenden Nukleinsäurekette, unter Mitwirkung eines entsprechenden spezifischen Aktivator-Oligonukleotides.

### Ausführungsform 10

Verfahren nach Ausführungsform 9, bei welchem für jede im Schritt 1 bereitgestellt spezifische und charakteristische Variante einer Zielsequenz im Schritt 2 mindestens zwei spezifische Amplifikations-Systeme bereitgestellt werden, wobei jedes spezifische Amplifikations-System umfasst
ein spezifisches Aktivator-Oligonukleotid für jeweils eine bereitgestelle zu amplifizierende Nukleinosäurekette umfassend eine spezifische und charakteristische Sequenz-Variante einer Zielnukleinsäurekette

### Ausführungsform 11

Verfahren nach Ausführungsform 1, bei welchem mindestens zwei unterschiedliche Zielsequenzen bereitgestellt werden und im Schritt 2 mindestens zwei jeweils für einzelne Zielsequenzen spezifische Amplifikations-Systeme bereitgestellt werden, wobei jedes spezifische Amplifikations-System umfasst
ein jeweils Zielsequenz-spezifisches Aktivator-Oligonukleotid für jeweils eine bereitgestelle zu amplifizierende Nukleinosäurekette und zwei Zielsequenz-spezifische Primer für jeweils eine bereitgestellte zu amplifizierende Nukleinosäurekette umfassend eine spezifische und charakteristische Zielsequenz. Im Ergebnis erfolgt eine Amplifikation von unterschiedlichen Zielsequenzen mit jeweils unterschiedlichen Amplifikations-Systemen.

In einer Ausführungsform wird das Verfahren unter Bedingungen ausgeführt, welche keine Trennung von komplementären Strängen der zu amplifizierenden Nukleinsäure in Abwesenheit von Aktivator-Oligonukleotid zulassen.

In einer Ausführungsform wird das Kopieren des Polynukleotid-Schwanzes im zweiten Primer-Bereich durch einen Stopp-Bereich für die Polymerase bewirkt, der zwischen dem ersten und dem zweiten Bereich angeordnet ist.

In einer Ausführungsform ist der dritte einzelsträngige Bereich des Aktivator-Oligonukleotid zum Segment des von Polymerase synthetisierten Verlängerungsprodukts des ersten Primer-Verlängerungsprodukts, welches unmittelbar an den ersten Primer-Bereich anschließt, im Wesentlichen komplementär, wobei:
In einer Ausführungsform ist der dritte einzelsträngige Bereich des Aktivator-Oligonukleotides zum genannten 5'-Segment des Verlängerungsprodukts des ersten Primer-Verlängerungsprodutkes vollständig komplementär, wobei die Länge dieses komplemetären Sequenzabschnittes folgende Bereiche umfasst: von mindestens 3 bis 70 Nukleotide, besser von mindestens 5 bis 50 Nukleotide, bevorzugt von 5 bis 40 Nukleotide, weiter bevorzugt von 5 bis 30 Nukleotide, besonders bevorzugt von 5 bis 20 Nukleotide. Besonders bevorzugt ist die Ausführungsform, in welcher vollständig komplementäres Segment korrespondierend zum polymophen Lokus liegt.

In einer weiteren Ausführungsform umfassen die Sequenzen des dritten einzelsträngigen Bereich des Aktivator-Oligonukleotides und die entsprechenden Sequenz des genannten 5'-Segments des Verlängerungsprodukts des ersten Primer-Verlängerungsproduktes komplementäre Sequenzen bis auf eine Sequenzposition (ein Nukleotid-Paar / ein Basenpaar) mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide. Die Bindung des Aktivator-Oligonukleotides erfolgt somit im Wesentlichen komplementär. Vorzugsweise liegt die Sequenzposition mit nicht komplementären Basenpaarung ausserhalb des Sequenz-Segments, welches dem polymophen Lokus entspricht / korrespondiert.

In einer weiteren Ausführungsform umfassen die Sequenzen des dritten einzelsträngigen Bereich des Aktivator-Oligonukleotides und die entsprechenden Sequenz des genannten 5'-Segments des Verlängerungsprodukts des ersten Primer-Verlängerungsproduktes komplementäre Sequenzen bis auf zwei Sequenzpositionen (ein Nukleotid-Paar / ein Basenpaar) mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide. Die Bindung des Aktivator-Oligonukleotides erfolgt somit im Wesentlichen komplementär. Vorzugsweise liegt die Sequenzposition mit nicht komplementären Basenpaarung ausserhalb des Sequenz-Segments, welches dem polymophen Lokus entspricht / korrespondiert.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsproduktes komplementäre Sequenzen (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide, weiterhin umfassen genannte Segmente nicht komplementäre Bereiche an mindestens drei Sequenzpositionen, wobei diese Positionen im 5'-Segment des dritten Abschnitt des Aktivator-Oligonukleotides liegen. Die Bindung des Aktivator-Oligonukleotides erfolgt somit im Wesentlichen komplementär. Vorzugsweise liegt die Sequenzposition mit nicht komplementären Basenpaarung ausserhalb des Sequenz-Segments, welches dem polymophen Lokus entspricht / korrespondiert.

In einer weiteren Ausführungsform umfassen die Sequenzen des dritten einzelsträngigen Bereichs des Aktivator-Oligonukleotides und die Sequenz des genannten 5'-Segments des Verlängerungsprodukts des ersten Primer-Verlängerungsprodutkes komplementäre Sequenzen bis auf mindestens eine und maximal zehn Sequenzpositionen mit nicht komplementärer Basenpaarung (im Sinne von Watson-Crick Basenpaarung) über eine Länge von mindestens 3 bis 70 Nukleotide, besser von mindestens 5 bis 60 Nukleotide, bevorzugt von 10 bis 40 Nukleotide, besonders bevorzugt von 10 bis 20 Nukleotide, wobei an Sequenzpositionen mit nicht komplementärer Basenpaarung (im Sinne von Watson-Crick Basenpaarung) mindestens ein modifiziertes Nukleotid mit modifizierten Nukleobasen beteiligt sind. Solche modifizierte Nukleobasen umfassen beispielsweise Nukleobasen mit verstärkter Bindung von natürlichen Nukleobasen (z.B. 2-Amino-Adenine), oder mit abgeschwächter Bindung, wie beispielsweise so genannte universale Basen, wie Inosine oder 5-Nitroindol. Die modifizierten Nukleobasen sind vorzugsweise im dritten Sequenzbereich des Aktivator-Oligonukleotides lokalisiert. Die Bindung des Aktivator-Oligonukleotides erfolgt somit im Wesentlichen komplementär. Vorzugsweise liegt die Sequenzposition mit nicht komplementären Basenpaarung ausserhalb des Sequenz-Segments, welches dem polymophen Lokus entspricht / korrespondiert.

In einer weiteren Ausführungsform des Verfahrens wird Schritt (e) des Verfahrens weiterhin modifiziert und umfasst:
Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, bis dieser komplementärer Strang der zu amplifizierenden Nukleinsäure vom ersten Primer-Verlängerungsprodukt abgelöst wird, wobei das 3'-Segment des ersten Primer-Verlängerungsproduktes einzelsträngig wird

In einer weiteren Ausführungsform des Verfahrens wird Schritt (f) des Verfahrens weiterhin modifiziert und umfasst:
Hybridisierung eines zweiten Oligonukleotid-Primers an das erste Primer-Verlängerungsprodukt, wobei gleichzeitige zumindest partielle Verdrängung des Aktivator-Oligonukleotides aus der Bindung mit dem ersten Verlängerungsprodukt durch Strangverdrängung erfolgt.

In einer weiteren Ausführungsform des Verfahrens wird Schritt (g) des Verfahrens weiterhin modifiziert und umfasst eine Verdrängung des Aktivator-Oligonukleotides aus der Bindung mit dem ersten Primer-Verlängerungsprodukt unter Mitwirkung der Polymerase.

In einer weiteren Ausführungsform des Verfahrens wird Schritt (h) des Verfahrens weiterhin modifiziert und umfasst: ggf. Bindung des Aktivator-Oligonukleotides an den unkopierten Polynukleotid-Schwanz des ersten verlängerten Primer-Oligonukleotid und Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oiigonukleotides.

In einer weiteren Ausführungsform des Verfahrens wird Verfahren weiterhin modifiziert und umfasst: h) Fortführung der Reaktion unter Bedingungen, welche Wiederholung von Schritten (a) bis (g) zulassen.

In einer weiteren Ausführungsform des Verfahrens wird des Verfahrens weiterhin modifiziert und umfasst: gleichzeitige Amplifizierung des ersten und zweiten Primer-Verlängerungsproduktes in einer exponentiellen Reaktion unter Verwendung der ersten und zweiten Primer-Oligonukleotid und des Aktivator-Oligonukleotides, wobei die gebildeten Primer-Verlängerungsprodukte als Matrizen für gegenseitige Synthese auftreten.

### Reaktionsbedingungen

Reaktionsbedingungen umfassen unter anderem Puffer-Bedingungen, TemperaturBedingungen, Zeitdauer der Reaktion und Konzentrationen von jeweiligen Reaktionskomponenten.

Im Verlauf der Reaktion akkumuliert die Menge der spezifischen produzierten zu amplifizierenden Nukleinsäure in einer exponentiellen Weise. Die Reaktion umfassend die Synthese der Verlängerungsprodukte kann zur Produktion der gewünschten Menge der spezifischen Nukleinsäuresequenz so lange wie nötig durchgeführt werden. Das erfindungsgemäße Verfahren wird bevorzugt kontinuierlich durchgeführt. In bevorzugter Ausführungsform läuft die Amplifikations-Reaktion bei gleicher Reaktionstemperatur ab, wobei die Temperatur bevorzugt zwischen 50°C und 70°C liegt. In einer anderen Ausführungsform kann die Reaktions-Temperatur auch variabel gesteuert werden, so dass einzelne Schritte der Amplifikation bei jeweils unterschiedlichen Temperaturen verlaufen.

Die für die exponentielle Amplifikation benötigten Reagenzien sind vorzugsweise bereits zu Beginn einer Reaktion im gleichen Ansatz vorhanden. In einer anderen Ausführungsform können Reagenzien auch in späteren Stadien des Verfahrens zugegeben werden.

Bevorzugt werden keine Helicasen oder Recombinasen in der Reaktionsmischung zur Trennung der neu synthetisieren Doppelstränge der zu amplifizierenden Nukleinsäure verwendet.

In einer bevorzugten Ausführungsform beinhaltet die Reaktionsmischung keine biochemischen Energie-spendenden Verbindungen wie ATP.

Die Menge der zu Beginn der Reaktion vorliegenden zu amplifizierenden Nukleinsäure kann zwischen wenigen Kopien und mehreren Milliarden Kopien in einem Ansatz vorliegen. Bei diagnostischen Einsätzen kann die Menge der zu amplifizierenden Nukleinsäurekette unbekannt sein.

In der Reaktion können auch weitere, nicht zu amplifizierende Nukleinsäuren vorliegen. Diese Nukleinsäuren können von natürlichen DNA oder RNA oder ihren Äquivalenten abstammen. In einer Ausführungsform liegen Kontroll-Sequenzen im gleichen Ansatz vor, welche parallel zu der zu amplifizierenden Nukleinsäure ebenfalls amplifiziert werden sollen.

Bevorzugt wird ein molarer Überschuss von ungefähr 10³:1 bis ungefähr 10¹⁵:1 (Verhältnis Primer:Matrize) der eingesetzter Primer und des Aktivator-Oligonukleotides zu der Reaktionsmischung gegeben, welche Matrizen-Stränge für die Synthese der zu amplifizierenden Nukleinsäurekette umfasst.

Es kann die Menge der Zielnukleinsäuren nicht bekannt sein, wenn das erfindungsgemäße Verfahren in diagnostischen Anwendungen benutzt wird, so dass die relative Menge des Primers und des Aktivator-Oligonukleotides bezüglich des komplementären Stranges nicht mit Sicherheit bestimmt werden kann. Die Menge des zugefügten Primers wird im Allgemeinen im molaren Überschuss bezüglich der Menge des komplementären Stranges (Matrize) vorhanden sein, wenn die zu amplifizierende Sequenz in einem Gemisch von komplizierten langkettigen Nukleinsäuresträngen enthalten ist. Ein großer molarer Überschuss wird bevorzugt, um die Effizienz des Verfahrens zu verbessern.

Die verwendeten Konzentrationen von Primer-1, Primer-2 und Aktivator-Oligonukleotide liegen beispielsweise in Bereichen zwischen 0,01 µmol/l und 100 µmol/l, bevorzugt zwischen 0,1 µmol/l und 100 µmol/l, bevorzugt zwischen 0,1 µmol/l und 50 µmol/l, besser zwischen 0,1 µmol/l und 20 µmol/l. Die hohe Konzentration von Komponenten kann die Geschwindigkeit der Amplifikation erhöhen. Die jeweiligen Konzentrationen einzelner Komponenten können unabhängig von einander variiert werden, um das gewünschte Reaktionsergebnis zu erzielen.

Die Konzentration von Polymerase liegt in Bereichen zwischen 0,001 µmol/l und 50 µmol/l, vorzugsweise zwischen 0,01 µmol/l und 20 µmol/l, besser zwischen 0,1 µmol/l und 10 µmol/l.

Die Konzentration von einzelnen dNTP-Substraten liegt in Bereichen zwischen 10 µmol/l und 10 mmol/l, vorzugsweise zwischen 50 µmol/l und 2 mmol/l, besser zwischen 100 µmol/l und 1 mmol/l. Die Konzentration von dNTP kann die Konzentration von divalenten Metal-Kationen beeinflußen. Gegebenenfalls wird diese entsprechend angepasst.

Als divalente Metal-Kationen werden beispielsweise Mg2+ verwendet. Als entsprechendes Anion können beispielsweise Cl, Acetat, Sulfat, Glutamat etc. verwendet werden.

Die Konzentration von divalenten Metal-Kationen wird beispielsweise an den für jeweilige Polymerase optimalen Bereich angepasst und umfasst Bereiche zwischen 0,1 mmol/l und 50 mmol/l, besser zwischen 0,5 mmol/l und 20 mmol/l, bevorzugter zwischen 1 mmol/l und 15 mmol/l.

Die enzymatische Synthese erfolgt im Allgemeinen in einer gepufferten wässrigen Lösung. Als Puffer-Lösungen können gelöste konventionelle Puffer-Substanzen, wie Tris-HCl, Tris-Acetat, Kalium-Glutamat, HEPES-Puffer, Natrium-Glutamat in gängigen Konzentrationen verwendet werden. Der pH-Wert dieser Lösungen liegt üblicherweise zwischen 7 und 9,5, bevorzugt etwa bei 8 bis 8,5. Die Puffer-Bedingungen können beispielsweise nach Empfehlung des Herstellers der verwendeten Polymerase angepasst werden.

Weitere Substanzen, wie sogenannte Tm-Depressoren (z.B. DMSO, Betaine, TPAC) etc. können zum Puffer zugegeben werden. Solche Substanzen verringern die Schmelztemperatur ("Tm-Depressoren") von Doppelsträngen und können somit einen positiven Einfluss auf die Öffnung von Doppelsträngen haben. Auch Polymerase-stabilisierende Komponenten, wie Tween 20 oder Triton 100 können in üblichen Mengen zum Puffer zugegeben werden. EDTA oder EGTA kann zu Komplexierung von Schwermetallen in konventionellen Mengen zugegeben werden. Polymerase-stabilisierende Substanzen, wie Trehalose oder PEG 6000 können ebenfalls zum Reaktionsgemisch zugegeben werden.

Vorzugsweise enthält die Reaktionsmischung keine Inhibitoren der Strangverdrängungsreaktion und keine Inhibitoren einer Polymerasenabhängigen Primer-Verlängerung.

In einer Ausführungsform enthält die Reaktionsmischung DNA-bindende Farbstoffe, bevorzugt interkalierenden Farbstoffe, wie z.B. EvaGreen oder SybrGreen. Solche Farbstoffe können ggf. bei Detektion der Neubildung von Nukleinsäureketten ermöglichen.

Die Reaktionsmischung kann weiterhin Proteine bzw. andere Substanzen enthalten, welche beispielsweise aus einem Original-Material stammen und welche die Amplifikation vorzugsweise nicht beeinflussen.

### Bevorzugte Ausführungsformen von Reaktionsbedingungen

Die Temperatur hat einen wesentlichen Einfluss auf die Stabilität der Doppelstränge.

In einer bevorzugten Ausführungsform werden während der Amplifikationsreaktion keine Temperaturbedingungen verwendet, welche im Wesentlichen zur Trennung von Doppelsträngen der zu amplifizierenden Nukleinsäure in Abwesenheit von Aktivator-Oligonukleotid führen. Damit soll sichergestellt werden, dass die Doppelstrang-Trennung von zu amplifizierenden Nukleinsäureketten von Anwesenheit des Aktivator-Oligonukleotides im gesamten Verlauf der Amplifikation abhängig ist.

Bei Temperatur etwa in Höhe von der gemessenen Schmelztemperatur (Tm) der zu amplifizierenden Nukleinsäure kommt es zu einer spontanen Trennung von beiden Strängen der zu amplifizierenden Nukleinsäure, so dass Einfluss des Aktivator-Oligonukleotids auf Trennung von synthetisierten Strängen und somit auf die Sequenzspezifität der Amplifikation minimal bis begrenzt ist.

Bei einer exponentiellen Amplifikation, welche weniger sequenzspezifisch (d.h. wenig Aktivator-Oligonukleotid-abhängig) ablaufen muss, kann die Reaktionstemperatur beispielsweise um die Schmelztemperatur (d.h. Tm plus/minus 3° bis 5°C) der zu amplifizierenden Nukleinsäure liegen. Bei einer solchen Temperatur können Sequenzunterschiede zwischen Aktivator-Oligonukleotid und dem synthetisierten Primer-Verlängerungsprodukt im Rahmen einer Strangverdrängungsreaktion im Allgemeinen gut toleriert werden.

Auch im Temperatur-Bereich von ca. (Tm minus 3° C) bis ca. (Tm minus 10°C) kann es immer noch zu einer spontanen Strangtrennung von synthetisierten Primer-Verlängerungsprodukten kommen, obwohl mit geringerer Effizienz. Der Einfluss von Aktivator-Oligonukleotid auf die Sequenzspezifität der zu amplifizierenden Nukleinsäure ist höher als bei Temperaturbedingungen um die Schmelztemperatur (Tm) der zu amplifizierenden Nukleinsäurekette.

Zwar findet die Strangtrennung mit sinkender Reaktionstemperatur im Wesentlichen dank Interaktion des neusynthetisierten Doppelstranges mit Aktivator-Oligonukleotid statt, Allerdings können Duplexe aus Primer bei Verlängerungstemperatur unter den genannten Bedingungen auch spontan zerfallen, d.h. ohne sequenzabhängige Strangverdrängung durch Aktivator-Oligonukleotid. Beispielsweise liegt die Reaktionstemperatur bei einer vermindert sequenzspezifischen Amplifikation in Bereichen zwischen ca. (Tm minus 3°C) und ca. (Tm minus 10°C), bevorzugt zwischen ca. (Tm minus 5°C) und ca. (Tm minus 10°C). Bei einer solchen Temperatur werden Sequenzunterschiede zwischen Aktivator-Oligonukleotid und dem synthetisierten Primer-Verlängerungsprodukt im Rahmen einer Strangverdrängungsreaktion schlechter toleriert.
Eine hohe Sequenzspezifität der Amplifikation des Verfahrens wird vor allem dann erreicht, wenn die neu synthetisierten Stränge der zu amplifizierenden Nukleinsäure unter Reaktionsbedingungen nicht spontan in Einzelstränge dissoziieren können. In einem solchen Fall spielt die sequenzspezifische Strangverdrängung durch Aktivator-Oligonukleoid für eine sequenzspezifische Strangtrennung eine entscheidende Rolle und ist für die Sequenzspezifität der Amplifikatonsreaktion maßgeblich verantwortlich. Dies kann im allgemeinen erreicht werden, wenn die Reaktionstemperatur deutlich unter der Schmelztemperatur von beiden Strängen der zu amplifizierenden Nukleinsäure liegt und keine weiteren Komponenten für eine Strangtrennung verwendet werden, beispielsweise keine Helicasen oder Recombinasen. Beispielsweise liegt die Reaktionstemperatur bei einer sequenzspezifischen Amplifikation in Bereichen zwischen ca. (Tm minus 10°C) und ca. (Tm minus 50°C), bevorzugt zwischen ca. (Tm minus 15°C) und ca. (Tm minus 40°C), besser zwischen ca. (Tm minus 15°C) und ca. (Tm minus 30°C).

In einer bevorzugten Ausführungsform der Amplifikation wird die maximale Reaktions-Temperatur im Verlauf der gesamten Amplifikationsreaktion nicht über die Schmelztemperatur der zu amplifizierenden Nukleinsäurekette erhöht.

In einer weiteren Ausführungsform der Amplifikation kann die Reaktionstemperatur über die Schmelztemperatur der zu amplifizierenden Nukleinsäurketten mindestens einmal erhöht werden. Die Erhöhung der Temperatur kann beispielsweise zu Beginn der Amplifikationsreaktion erfolgen und zu Denaturierung von Doppelsträngen einer genomischen DNA führen. Dabei muss beachet werden, dass während eines solchen Schrittes die Abhängigkeit der Doppelstrang-Trennung von der Wirkung des Aktivator-Oligonukleotides aufgehoben ist oder zumindest signifikant reduziert ist.

Die Reaktionstemperaturen der einzelnen Schritte der Amplifikationsreaktion können im Bereich von ca. 15°C bis ca. 85°C, besser im Bereich von ca. 15°C bis ca. 75°C, bevorzugt im Bereich von ca. 25°C bis ca. 70°C liegen.

Im Allgemeinen kann für die Reaktionstemperatur für jeden einzelnen Reaktionsschritt optimal eingestellt werden, so dass für jeden Reaktionsschritt eine solche Temperatur herbeigeführt wird. Die Amplifikationsreaktion umfasst somit eine sich wiederholende Änderung von Temperaturen, welche zyklisch wiederholt werden. In einer vorteilhaften Ausführungsform des Verfahrens werden Reaktionsbedingungen für mehrere Reaktionsschritte vereinheitlicht, so dass die Anzahl von Temperatur-Schritten geringer ist als die Anzahl von Reaktionsschritten. In einer solchen bevorzugten Ausführungsform der Erfindung erfolgt mindestens einer der Schritte der Amplifikation bei einer Reaktionstemperatur, welche sich von der Reaktionstemperatur anderer Schritte der Amplifikation unterscheidet. Die Reaktion verläuft somit nicht isothermal, sondern die Reaktionstemperatur wird zyklisch geändert.

Es werden beispielsweise während Amplifikation mindestens zwei Temperatur-Bereiche verwendet, welche abwechselnd herbeigeführt werden (zyklische Änderung von Temperaturen zwischen einzelnen Temperatur-Bereichen). In einer Ausführungsform umfasst der untere Temperatur-Bereich beispielsweise Temperaturen zwischen 25°C und 60°C, besser zwischen 35°C und 60°C, bevorzugt zwischen 50°C und 60°C und der obere Temperatur-Bereich umfasst beispielsweise Temperaturen zwischen 60°C und 75°C, besser zwischen 60°C und 70°C.

In einer weiteren Ausführungsform umfasst der untere Temperatur-Bereich beispielsweise Temperaturen zwischen 15°C und 50°C, besser zwischen 25°C und 50°C, bevorzugt zwischen 30°C und 50°C und der obere Temperatur-Bereich umfasst beispielsweise Temperaturen zwischen 50°C und 75°C, besser zwischen 50°C und 65°C.

In einer weiteren Ausführungsform umfasst der untere Temperatur-Bereich beispielsweise Temperaturen zwischen 15°C und 40°C, besser zwischen 25°C und 40°C, bevorzugt zwischen 30°C und 40°C und der obere Temperatur-Bereich umfasst beispielsweise Temperaturen zwischen 40°C und 75°C, besser zwischen 40°C und 65°C.

Die Temperatur kann im jeweiligen Bereich konstant gehalten werden oder als TemperaturGradient (abfallend oder aufsteigend) geändert werden.

Weitere Erläuterungen zu Temperatur-Einstellungen werden in folgenden Abschnitten bei Ausführungsformen im Einzelnen angeführt.

Jede herbeigeführte Temperatur kann eine gewisse Zeit aufrechterhalten werden, so dass dabei ein Inkubations-Schritt resultiert. Das Reaktionsgemisch kann somit während einer Amplifikation bei einer ausgewählten Temperatur eine gewisse Zeit inkubiert werden. Diese Zeit kann unterschiedlich lange für jeweiligen Inkubations-Schritt sein und kann sich an den Fortschritt der jeweiligen Reaktion bei gegebener Temperatur richten (z.B. Primer-Verlängerung oder Strangverdrängung usw.). Die Zeit eines Inkubationsschrittes kann folgende Bereiche umfassen: zwischen 0,1 sec und 10.000 sec, besser zwischen 0,1 sec und 1000 sec, bevorzugt zwischen 1 sec und 300 sec, noch bevorzugter zwischen 1 sec und 100 sec.

Durch eine solche Temperatur-Änderung können einzelne Reaktionsschritte bevorzugt bei einer ausgewählten Temperatur ausgeführt werden. Dadurch können Ausbeuten von einem jeweiligen Reaktionsschritt verbessert werden. Innerhalb eines Synthese-Zyklus kann Temperatur-Änderung bzw. Temperatur-Wechsel zwischen einzelnen Temperatur-Bereichen ggf. mehrmals herbeigeführt werden. Synthese-Zyklus kann somit mindestens ein Temperatur-Wechsel umfassen. Ein solcher Temperatur-Wechsel kann beispielsweise in einem PCR-Gerät / Thermocycler routinemäßig als zeitliches Programm ausgeführt werden.

In einer Ausführungsform wird ein Amplifikationsverfahren bevorzugt, bei welchem mindestens eines der Schritte, welche Strangverdrängung umfassen, und mindestens eines der Schritte, welche Primer-Verlängerungsreaktionen umfassen, gleichzeitig bzw. parallel stattfinden und unter gleichen Reaktionsbedingungen erfolgen. In einer solchen Ausführungsform kann beispielsweise eine Primer-Verlängerungsreaktion von mindestens einem Primer-Oligonukleotid (z.B. vom ersten Primer-Oligonukleotid) bevorzugt bei Temperaturbedingungen im unteren Temperatur-Bereich erfolgen. Hingegen erfolgt die Strangverdrängung unter Mitwirkung von Aktivator-Oligonukletid und die eine weitere Primer-Verlängerungsreaktion (z.B. vom zweiten Primer-Oligonukleotid) bevorzugt im Reaktionsschritt im oberen Temperatur-Bereich.

In einer weiteren Ausführungsform wird ein Amplifikationsverfahren bevorzugt, bei welchem mindestes eines der Schritte, welche Strangverdrängung durch das Aktivator-Oligonukleotid umfassen und mindestens eines der Schritte, welche Primer-Verlängerungsreaktion umfassen, bei unterschiedlichen Temperaturen durchgeführt. In einer solchen Ausführungsform können beispielsweise Primer-Verlängerungsreaktionen von mindestens einem Primer-Oligonukleotid (z.B. vom ersten Primer-Oligonukleotid und/oder von zweiten Primer-Oligonukleotid) bevorzugt bei Temperaturbedingungen im unteren Temperatur-Bereich erfolgen. Hingegen erfolgt die Strangverdrängung unter Mitwirkung von Aktivator-Oligonukletid bevorzugt im Reaktionsschritt im oberen Temperatur-Bereich.
In einer weiteren bevorzugten Ausführungsform verlaufen sämltliche Schritte einer Amplifikatons-Reaktion unter gleichen Reaktionsbedingungen.

In einer solchen Ausführungsform kann das Amplifikationsverfahren unter isothermalen Bedingungen durchgeführt werden, d.h. dass keine Temperaturänderungen für die Ausführung des Verfahrens erforderlich sind. In einer solchen bevorzugten Ausführungsform der Erfindung erfolgt die gesamte Amplifikationsreaktion unter konstanter Temperatur, d.h. die Reaktion ist isothermal. Die Zeit einer solchen Reaktion umfasst beispielsweise folgende Bereiche: zwischen 100 sec und 30.000 sec, besser zwischen 100 sec und 10.000 sec, noch besser zwischen 100 sec und 1000 sec.

Im Abschnitt "Beispiele" wird gezeigt, dass es möglich ist, Strukturen einzelner Reaktionskomponenten und entsprechende Reaktionsschritte dermaßen aneinander abzustimmen, dass eine isothermale Reaktion möglich ist.

Die Summe aller Verfahrens-Schritte, welche zu einer Verdoppelung der Menge einer zu amplifizierenden Nukleinsäurekette führt, kann als Synthese-Zyklus bezeichnet werden. Ein solcher Zyklus kann entsprechend isothermal ablaufen oder auch durch Änderungen der Temperatur in seinem Verlauf gekennzeichnet sein. Die Temperatur-Änderungen können sich von Zyklus zu Zyklus wiederholen und identisch gestaltet werden.

Besonders vorteilhaft sind Amplifikationsverfahren, bei welchen die maximal erreichbare Temperatur eine Strang-Trennung unter Mitwirkung von Aktivator-Oligonukleotid nur dann im Wesentlichen zulässt, wenn mehr als 5 Nukleotide des dritten Bereichs des Aktivator-Oligonukleotides eine komplementäre Bindung mit dem ersten Primer-Verlängerungsprodukt eingehen können, vorteilhafter ist es, wenn mehr als 10, noch vorteilhafter, wenn mehr als 20 Nukleotide des Aktivator-Oligonukleotides mit dem ersten Primer-Verlängerungsprodukt eine Bindung eingehen. Im Allgemeinen gilt, je länger die erforderliche Bindung zwischen Aktivator-Oligonukleotid und dem komplementären Strang des ersten Primer-Verlängerungsproduktes, bevor die synthetisierten Stränge unter Reaktionsbedingungen dissoziieren, desto spezifischer ist die Amplifikations-Reaktion. Im Einzelnen, kann durch Verlängerung bzw. Verkürzung des dritten Abschnittes des Aktivator-Oligonukleotides das gewünschte Maß an Spezifität ermittelt werden.

Ein Verfahrensschritt kann bei seiner Wiederholung bei konstanter Temperatur erfolgen über die Gesamt-Dauer des Verfahrens oder auch bei unterschiedlichen Temperaturen.

Einzelne Verfahrensschritte können jeweils hintereinander durch Zugabe von einzelnen Komponenten ausgeführt werden. Bei einer vorteilhaften Ausführungsform liegen sämtliche für die Ausführung einer Amplifikation notwendige Reaktions-Komponenten zu Beginn einer Amplifikation in einem Reaktionsgemisch vor.

Der Start einer Amplifikations-Reaktion kann durch Zugabe einer Komponente erfolgen, z.B. durch Zugabe einer Nukleinsäurekette umfassend eine Zielsequenz (z.B. eine Start-Nukleinsäurekette), oder einer Polymerase oder divalenten Metal-lonen, oder auch durch Herbeiführung von Reaktions-Bedingungen, welche für Amplifikation notwendig sind, z.B. Einstellung einer erforderlichen Reaktionstemperatur für einen oder mehrere Verfahrenschritte.

Die Amplifikation kann so lange ausgeführt werden, bis die gewünschte Menge an zu amplifizierenden Nukleinsäure erreicht ist. In einer anderen Ausführungsform wird die Amplifikationsreaktion über eine Zeit ausgeführt, welche bei Vorliegen einer zu amplifizierenden Nukleinsäure ausgereicht hätte, um eine ausreichende Menge zu bekommen. In einer anderen Ausführungsform wird die Amplifikationsreaktion über eine hinreichede Anzahl von Synthese-Zyklen (Verdoppelungszeiten) ausgeführt, welche bei Vorliegen einer zu amplifizierenden Nukleinsäure ausgereicht hätte, um eine ausreichende Menge zu bekommen.

Die Reaktion kann durch verschiedene Eingriffe gestoppt werden. Beispielsweise durch Änderung der Temperatur (z.B. Abkühlen oder Erhitzen, wobei beispielsweise Polymerase in ihrer Funktion gestört wird) oder durch Zugabe einer Substanz, welche eine Polymerase-Reaktion stoppt, z.B. EDTA oder Formamid.

Im Anschluss an die Amplifikation kann die amplifizierte Nukleinsäurekette für weitere Analysen verwendet werden. Dabei können synthetisierte Nukleinsäureketten durch verschiedene Detektionsverfahren analysiert werden. Beispielsweise können fluoreszenzmarkierte Oligonukleotid-Sonden verwendet werden oder Sequenzierungsverfahren (Sanger-Sequenzierung oder Next-Generation Sequenzierung), Festphasen-Analysen wie Microarray oder Bead-Array-Analysen usw. Die synthetisierte Nukleinsäurekette kann als Substrat / Matrize in weiteren Primer-Verlängerungsreaktionen verwendet werden.

In einer vorteilhaften Ausführungsform wird der Fortschritt der Synthese-Reaktion während der Reaktion überwacht. Das kann beispielsweise durch Einsatz von interkalierenden Farbstoffen erfolgen, z.B. Sybrgreen oder Evagreen, oder unter Einsatz von markierten Primern (z.B. Lux-Primer oder Scorpion-Primer) oder unter Einsatz von fluoreszenzmarkierten Oligonukleotid-Sonden.

Die Detektion der Veränderung der Fluoreszenz während der Amplifikation wird in einem Detektions-Schritt des Verfahrens umgesetzt. Dabei kann die Temperatur und die Dauer dieses Schrittes an die jeweiligen Erfordernisse der einer Oligonukleotid-Sonde angepasst werden. Die Temperaturen des Detektions-Schrittes umfassen beispielsweise Bereiche zwischen 20°C und 75°C, besser zwischen 40 und 70°C, bevorzugt zwischen 55 und 70°C.

Während des Detektionschrittes erfolgt Beleuchtung der Reaktion mit Licht einer Wellenlänge, welche in der Lage in ein verwendetes Fluorophor des Detektions-Systems (ein Donor oder ein Fluoreszenzreporter) anzuregen. Die Signal-Erfassung erfolgt in der Regel parallel zu Anregung, wobei das spezifische Fluoreszenzsignal detektiert wird und seine Intensität quantifiziert wird.

Das Amplifikationsverfahren kann zur Überprüfung der Anwesenheit einer Zielnukleinsäurekette in einem biologischen Material oder einem diagnostischen Material im Rahmen eines diagnostischen Verfahrens eingesetzt werden.

In einer Ausführungsform werden Reaktionsbedingungen von mindestens einem Reaktionsschritt, bei welchem mindestens ein allel-spezifischer Primer an eine allel-spezifische Sequenzvariante hybridisieren soll und eine Primer-Verlängerungsreaktion durch eine Polymerase stattfinden soll, dermaßen gewählt, dass eine vorwiegend spezifische Hybridisierung eines solchen Primers an seine Primer-Bindungsstelle stattfinden kann. Solche Bedingungen können auch als stringente Bedingungen bezeichnet werden. Beispielsweise liegt die Temperatur in einem solchen Schritt etwa bei Tm der respektiven Primer/Primerbindungsstelle oder die Temperatur liegt über einer solchen Tm. Beispielsweise kann die Temperatur bei etwa Tm +5 bis etwa Tm + 15°C liegen.

### Bevorzugte Ausführungsformen einer Start-Nukleinsäurekette:

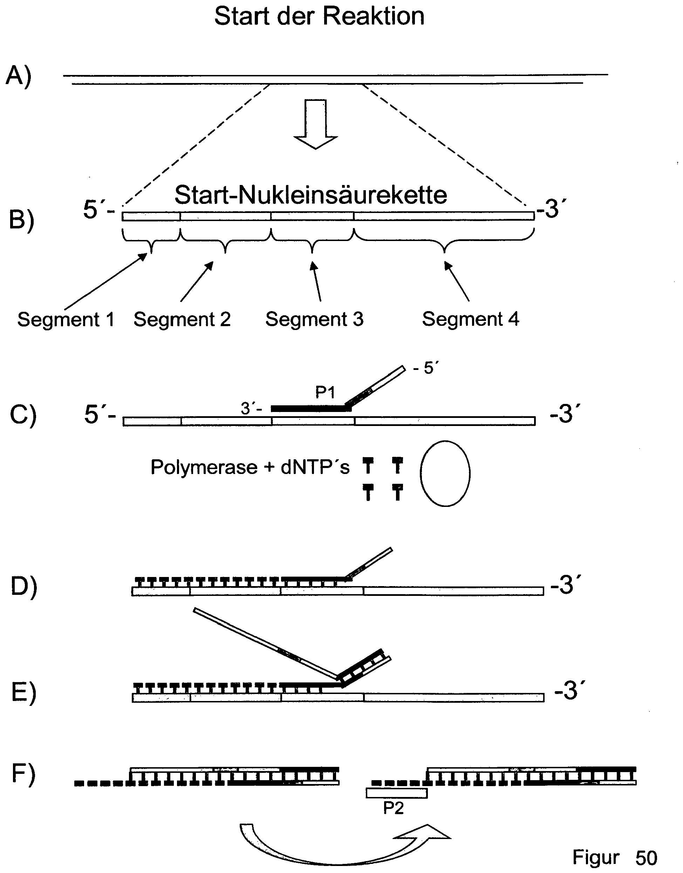

Die zu Beginn der Amplifikations-Reaktion eingesetzte bzw. einzusetzende Nukleinsäurekette kann als Start-Nukleinsäurekette bezeichnet werden (Fig. 50 - 51).

Ihre Funktion kann dahingehend gesehen werden, dass sie die anfängliche Matrize darstellt, welche eine korrekte Positionierung von Primern, der Synthese-Abschnitte zwischen beiden Primern, sowie die Initiierung von Bindungs- und Verlängerungsvorgängen ermöglicht. In einer bevorzugten Ausführungsform umfasst eine Start-Nukleinsäurekette eine Zielsequenz.

Durch Bindung von Primern an ihre entsprechenden Primer-Bindungsstellen (PBS 1 und PBS 2) und Initiierung von entsprechenden Primer-Verlängerungsreaktionen erfolgt durch Generierung von ersten Primer-Verlängerungsprodukten. Diese werden als spezifische Kopien der zu Begin der Reaktion vorliegenden Nukleinsäurekette synthetisiert.

In einer Ausführungsform kann diese in das Reaktionsgemisch vor Beginn der Amplifikatons-Reaktion einzusetzende Nukleinsäurekette (Start-Nukleinsäurekette) mit der zu amplifizierenden Nukleinsäurekette identisch sein. Durch die Amplifikations-Reaktion wird lediglich die Menge einer solchen Nukleinsäurekette vermehrt.

In einer weiteren Ausführungsform unterscheiden sich die zu amplifizierende Nukleinsäure und die Start-Nukleinsäurekette dahingehend, dass die Start-Nukleinsäurekette zwar die Anordnung einzelner Sequenzelemente der zu amplifizierenden Nukleinsäurekette vorgibt, doch kann die Sequenzzusammensetzung der Start-Nukleinsäurekette von der Sequenz der zu amplifizierenden Nukleinsäurekette abweichen. Beispielsweise können im Rahmen der Primer-Bindung und Verlängerung während einer Amplifikation neue Sequenzinhalte (bezogen auf Start-Nukleinsäurekette) in die zu amplifizierende Nukleinsäurekette integriert werden. Weiterhin können sich Sequenzelemente einer zu amplifizierenden Nukleinsäurekette von solchen Sequenzelementen einer Start-Nukleinsäurekette in ihrer Sequenzzusammensetzung unterscheiden (z.B. Primer-Bindungsstellen oder Primer-Sequenzen). Die Start-Nukleinsäure dient nur als initiale Matrize für die spezifische Synthese der zu amplifizierenen Nukleinsäurekette. Diese initiale Matrize kann im Reaktionsgemisch bis zum Ende der Amplifikation verbleiben. Durch den exponentiellen Charakter der Amplifikation überwiegt allerdings die Menge der zu amplifizierenden Nukleinsäurekette am Ende einer Amplifikationsreaktion die Menge einer in die Reaktion zugegebenen Start-Nukleinsäurekette.

Die Start-Nukleinsäurekette umfasst in einer Ausführungsform eine Zielsequenz oder ihre Teil-Segmente, welche zumindest eine zu erwartende Sequenz-Variante eines polymorphen Lokus der Zielsequenz umfassen.

Eine Start-Nukleinsäure umfasst weiterhin zumindest ein vorwiegend einzelsträngiges Sequenzsegment, zu welchem mindestens eines der Primer des Amplifikations-Systems mit seinem 3'-Segment vorwiegend komplementär binden kann, so dass verwendete Polymerase einen solchen Primer, wenn hybridisiert an die Start-Nukleinsäurekette, matrizenspezifische unter Einbau von dNTPs verlängern kann.

Eine Start-Nukleinsäure, welche mehrere Sequenzvarianten in einem polymorphen Lokus einer Zielsequenz umfassen kann, umfasst vorzugsweise weiterhin mindestens ein erstes Zielsequenzsegment, welches für alle Zielsequenzvarianten charakteristisch und einheitlich ist. In einer weiteren Ausführungsform umfasst eine Start-Nukleinsäure mindestens zwei Zielsequenzsegmente (ein erstes Zielsequenzsegment und ein zweites Zielsequenzsegment), welche auf beiden Seiten eines polymorphen Lokus einer Zielsequenz lokalisiert sind und somit die Sequenzvarianten einer Zielsequenz von beiden Seiten flankieren.

Die Länge eines polymorphen Lokus einer Start-Nukleinsäureke kann Bereiche von einem Nukleotid bis zu 200 Nukleotiden, besser von einem Nukleotid bis zu 50 Nukleotiden, bevorzugt von einem Nukleotid bis zu 20 Nukleotiden umfassen. Die Längen von einheitlichen Sequenzsegmenten (erstes und zweite einheitliches Sequenzsegment einer Start-Nukleinsäure) kann zumindest für eines der beiden einheitlichen Sequenz-Segmente folgende Bereiche umfassen: von 4 bis 200 Nukleotide, besser von 6 bis 100 Nukleotide, bevorzugt von 8 bis 50 Nukleotide.

In einer weiteren Ausführungsform kann die Start-Nukleinsäurekette mindestens einen Sequenzabschnitt umfassen, welcher nicht amplifiziert wird. Eine solche Start-Nukleinsäurekette ist somit nicht mit der zu amplifizierenden Sequenz identisch. Solche nicht zu amplifizierenden Abschnitte können beispielsweise als Folge von Sequenzvorbereitungsschritten bzw. als Folge von vorangegangenen Sequenz-Manipulations-Schritten einen Sequenzabschnitt einer Start-Nukleinsäurekette repräsentieren.

In einer bevorzugten Ausführungsform schließt die in das Reaktionsgemisch vor Beginn der Reaktion einzusetzende Start-Nukleinsäurekette mindestens eine Zielsequenz ein.

In einer weiteren Ausführungsform schließt eine solche Start-Nukleinsäurekette mindestens eine Zielsequenz (engl. Target-Sequence) und noch weitere Sequenzen ein, welche nicht Zielsequenz (engl. Non-target Sequences) sind. Während der Amplifikation werden Sequenzsegmente umfassend die Zielsequenz exponentiell vermehrt und andere Sequenzsegmente werden dabei entweder gar nicht oder nur teilweise exponentiell vermehrt.

### Aufbau einer Start-Nukleinsäurekette

Beispiel für eine solche Start-Nukleinsäurekette stellt eine Nukleinsäurekette dar, welche eine Zielsequenz einschließt und welche ein Sequenzfragment-A umfasst und ein Sequenzfragment-B umfasst.

Das Sequenzfragment-A der Start-Nukleinsäurekette umfasst eine Sequenz, die mit der Sequenz eines der beiden in der Amplifikation verwendeten Primer eine signifikante Homologie aufweist oder im Wesentlichen mit dem kopierbaren Anteil des 3'-Segments des einen Primers identisch ist. Bei Synthese eines komplementären Stranges zu diesem Segment wird eine komplementäre Sequenz generiert, welche eine entsprechende Primer-Bindungs-Stelle darstellt.

Das Sequenzfragment-B der Start-Nukleinsäurekette umfasst eine Sequenz, welche zur komplementären Bindung eines entsprechenden weiteren Primers oder seines 3'-Segmentes unter Ausbildung eines extensionsfähigen Primer-Matrizen-Komplexes geeignet ist, wobei Sequenzfragment-A und Sequenzfragment-B im Verhältnis zueinander vorwiegend/bevorzugt nicht komplementär sind.

In einer bevorzugten Ausführungsform wird zum Reaktionsgemisch eines Amplifikationsverfahrens eine Start-Nukleinsäurekette gegeben, welche folgende Eigenschaften aufweist:
Vorzugsweise liegt das Sequenzfragment-A im 5'-Segment der Start-Nukleinsäurekette. Vorzugsweise bildet dieses Sequenzfragment-A eine Begrenzung des Nukleinsäurekette-Stranges in 5'-Richtung.

Vorzugsweise liegt das Sequenzfragment-B stromabwärts vom Sequenzfragment-A.

In einer bevorzugten Ausführungsform bildet dieses Sequenzfragment-B eine Begrenzung des Nukleinsäurekette-Stranges in 3'-Richtung. In einer weiteren Ausführungsform stellt dieses Sequenzfragment-B keine Begrenzung des Nukleinsäurekette-Stranges in 3'-Richtung dar, sondern wird von weiteren Sequenzen von der 3'-Seite flankiert. Vorzugsweise sind diese Sequenzen keine Zielsequenzen und nehmen an der exponentiellen Amplifikation nicht teil.

In einer Ausführungsform umfasst die Zielsequenz mindestens eines von beiden Sequenzfragment-A oder Sequenzfragment-B. In einer weiteren Ausführungsform liegt die Zielsequenz zwischen Sequenzfragment-A und Sequenzfragment-B.

In einer Ausführungsform kann eine solche Start-Nukleinsäurekette als Matrize für die Synthese eines ersten Primer-Verlängerungsproduktes dienen (Fig. 50B). Die Start-Nukleinsäurekette kann dabei beispielsweise im Rahmen einer Primer-Verlängerungsreaktion unter Verwendung des zweiten Primer-Oligonukleotides als ein Sequenzsegment einer längeren Ausgangs-Nukleinsäurekette während eines vorbereitenden Schrittes vor der exponentiellen Amplifikation bereitgestellt werden und in einer einzelsträngige Form überführt werden. Die Ausgangsnukleinsäurekette kann beispielsweise eine genomische DNA oder RNA sein und dient als Quelle der Zielsequenz (schematisch dargestellt in Fig. 50A als Doppelstrang). Die Start-Nukleinsäurekette umfasst (Fig. 50 B) im 5'-Segment der Nukleinsäurekette ein Segment 1, was Sequenzabschnitte des zweiten Primer-Oligonukleotides umfasst und Begrenzung in 5'-Richtung darstellt, ein Segment 2, was eine Zielsequenz oder ihre Anteile umfasst, ein Segment 3, was eine Primerbindungsstelle für das erste Primer-Oligonukleotid umfasst und ein Segment 4 was eine nicht-Zielsequenz im 3'-Segment der Start-Nukleinsäurekette lokalisiert ist und somit das Segment 3 auf der 3'-Seite flankiert.

Im Rahmen der Initiierung der Amplifikation kann der erste Primer zumindest mit 3'-Segment seines ersten Bereichs an eine solche Start-Nukleinsäurekette im Segment 3 binden (Fig. 50 C) und in Anwesenheit einer Polymerase und Nukleotide entsprechend verlängert werden. Dabei entsteht ein erstes Primer-Verlängerungsprodukt, welches dem Matrizenstrang der Start-Nukleinsäurekette komplementär ist und in seiner Länge begrenzt ist (Fig. 50 D). Im Rahmen der Amplifikationsreaktion kann ein solches Primer-Verlängerungsprodukt via Aktivator-Oligonukleotid von seinem Matrizenstrang sequenzspezifisch abgelöst werden (Fig. 50 E-F), so dass entsprechende Sequenzabschnitte im 3'-Segment des nun synthetisierten ersten Primer-Verlängerungsproduktes als Bindungsstelle für das zweite Primer-Oligonukleoid zur Verfügung stehen (Fig. 50 F).

In einer bevorzugten Ausführungsform umfasst eine Start-Nukleinsäurekette somit folgende Sequenzfragmente (Fig. 50):
- Sequenzsegment-1 (in Fig. 50 als Segment 1 bezeichnet), welches eine Sequenz umfasst, die mit der Sequenz des zweiten Primers eine signifikante Homologie aufweist oder im Wesentlichen mit dem kopierbaren Anteil des 3'-Segments des zweiten Primers identisch ist. Dieses Sequenzsegment-1 liegt im 5'-Segment der Start-Nukleinsäurekette, vorzugsweise bildet dieses Sequenzsegment-1 eine Begrenzung des Nukleinsäurekette-Stranges in 5'-Richtung.
- Das Sequenzsegment-3 (in Fig. 50 als Segment 3 bezeichnet)" welches eine Sequenz umfasst, welche zu komplementären Bindung des ersten Bereichs des ersten Primers oder seines 3'-Segmentes unter Ausbildung eines extensionsfähigen Primer-Matrizen-Komplexes geeignet ist. Vorzugsweise liegt das Sequenzsegment-3 stromabwärts vom Sequenzsegment-1.
- Zielsequenz , welche teilweise oder ganz zwischen Segment 1 und Segment 3 liegt (in Fig. 50 dieser Anteil der Zielsequenz wird als Segment 2 bezeichnet). In einer bevorzugten Ausführungsform umfasst die Zielsequenz mindestens eines von Segment 1 und / oder Segment 3.
- Optional umfasst die Start-Nukleinsäurekette im 3'-Segment flankierende Sequenzabschnitte (in Fig. 50 als Segment 4 bezeichnet), welche nicht amplifiziert werden.

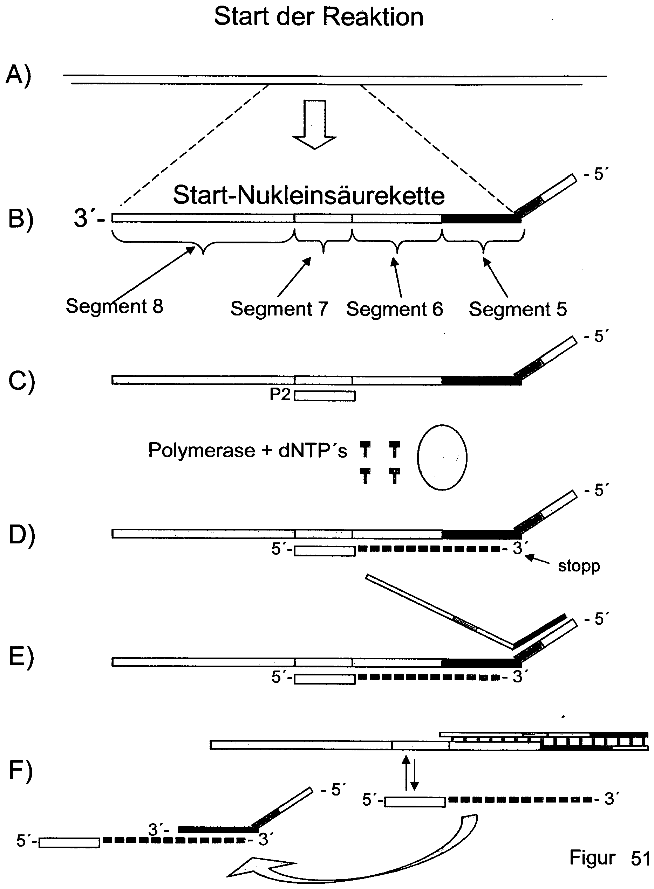

In einer weiteren Ausführungsform kann eine solche Start-Nukleinsäurekette als Matrize für die Synthese eines zweiten Primer-Verlängerungsproduktes dienen (Fig. 51).

Die Start-Nukleinsäurekette kann dabei beispielsweise im Rahmen einer Primer-Verlängerungsreaktion unter Verwendung des ersten Primer-Oligonukleotides als ein Sequenzsegment einer längeren Ausgangs-Nukleinsäurekette (Fig. 51 A) während eines vorbereitenden Schrittes vor der exponentiellen Amplifikation bereitgestellt werden und in einer einzelsträngige Form überführt werden. Die Ausgangsnukleinsäurekette kann beispielsweise eine genomische DNA oder RNA sein und dient als Quelle der Zielsequenz (schematisch dargestellt in Fig. 51A als Doppelstrang). Die Start-Nukleinsäurekette umfasst (Fig. 51 B) im 5'-Segment der Nukleinsäurekette ein Segment 5, was Sequenzabschnitte des ersten Primer-Oligonukleotides umfasst und Begrenzung in 5'-Richtung darstellt, ein Segment 6, was eine Zielsequenz oder ihre Anteile umfasst, ein Segment 7, was eine Primerbindungsstelle für das zweite Primer-Oligonukleotid umfasst und ein Segment 8 was eine nicht-Zielsequenz im 3'-Segment der Start-Nukleinsäurekette lokalisiert ist und somit das Segment 7 auf der 3'-Seite flankiert.

Im Rahmen der Initiierung der Amplifikation kann der zweite Primer zumindest mit 3'-Segment seines ersten Bereichs an eine solche Start-Nukleinsäurekette im Segment 7 binden (Fig. 51 C) und in Anwesenheit einer Polymerase und Nukleotide entsprechend bis zu hin zum Stopp-Abschnitt des ersten Primer-Oligonukleotides verlängert werden. Dabei entsteht ein zweites Primer-Verlängerungsprodukt, welches dem Matrizenstrang der Start-Nukleinsäurekette komplementär ist und in seiner Länge begrenzt ist (Fig. 51 D). Im Rahmen der Amplifikationsreaktion kann ein solches Primer-Verlängerungsprodukt via Aktivator-Oligonukleotid von seinem Matrizenstrang sequenzspezifisch abgelöst werden (Fig. 51 E-F), so dass entsprechende Sequenzabschnitte im 3'-Segment des nun synthetisierten zweiten Primer-Verlängerungsproduktes als Bindungsstelle für das erste Primer-Oligonukleoid zur Verfügung stehen (Fig. 51 F).

In dieser bevorzugten Ausführungsform umfasst eine Start-Nukleinsäurekette folgende Sequenzfragmente (Fig. 51):
- Sequenzsegment-5 (genannt Segment 5), welches eine Sequenz umfasst, die mit der Sequenz des ersten Bereichs des ersten Primers eine signifikante Homologie aufweist oder im Wesentlichen mit dem kopierbaren Anteil des ersten Bereichs des ersten Primers identisch ist. Dieses Segment-7 liegt im 5'-Segment der Start-Nukleinsäurekette und ist von einem nicht kopierbaren Oligonukleotid-Schwanz flankiert (analog zum ersten Primer-Oligonukleotid), vorzugsweise bildet dieses Segment - 7 eine Begrenzung des kopierbaren Nukleinsäurekette-Stranges in 5'-Richtung.
- Das Sequenzfragment-7 (genannt Segment 7), welches eine Sequenz umfasst, welche zur komplementären Bindung eines zweiten Primers oder seines 3'-Segmentes unter Ausbildung eines extensionsfähigen Primer-Matrizen-Komplexes geeignet ist. Vorzugsweise liegt das Segment 7 stromabwärts vom Segment 5.
- Zielsequenz , welche teilweise oder ganz zwischen Segment 5 und Segment 7 liegt (in Fig. 51 dieser Anteil der Zielsequenz wird als Segment 6 bezeichnet). In einer bevorzugten Ausführungsform umfasst die Zielsequenz mindestens eines von Segment 5 und/oder Segment 7.
- Optional umfasst die Start-Nukleinsäurekette im 3'-Segment flankierende Sequenzabschnitte (in Fig. 51 als Segment 8 bezeichnet), welche nicht amplifiziert werden.

### Funktionsweise der Start-Nukleinsäurekette

Zu Beginn der Amplifikations-Reaktion dient die Start-Nukleinsäurekette als Matrize für initiale Entstehung von jeweiligen Primer-Verlängerungsprodukten. Sie stellt somit die Ausgangs-Matrize für die zu amplifizierende Nukleinsäurekette dar. Die Start-Nukleinsäurekette braucht nicht zwingend mit der zu amplifizierenden Nukleinsäurekette identisch zu sein. Durch Bindung und Verlängerung von beiden Primern während der Amplifikations-Reaktion bestimmen im Wesentlichen die beiden Primer, welche Sequenzen an beiden endständigen Segmenten der zu amplifizierenden Nukleinsäurekette im Rahmen der Amplifikation generiert werden.

In einer bevorzugten Ausführungsform des Verfahrens werden für einen Doppelstrang nicht-denaturierenden Reaktions-Bedingungen während des exponentiellen Amplifikationsvorgangs aufrechterhalten. Daher ist es vorteilhaft, wenn Start-Nukleinsäurekette eine Begrenzung in ihrem durch eine Polymerase verlängerbaren 5'-Sequenzsegment aufweist, was zu einem Stopp in der enzymatischen Verlängerung eines entsprechenden Primers führt. Damit wird die Länge der unter Reaktionsbedingungen generierten Primer-Verlängerungsfragmente begrenzt. Das kann sich vorteilhaft auf Strangverdrängung durch Aktivator-Oligonukleotid auswirken und zu einer Dissoziation des jeweiligen Stranges führen, so dass Primer-Bindungsstellen in einzelsträngigen Zustand überführt werden und somit für eine erneute Bindung von Primern zugängig werden.

In einer weiteren bevorzugten Ausführungsform des Verfahrens werden während initialen Synthese-Schritte (beispielsweise initiale eine bis zehn Synthese-Wiederholungen) für einen Doppelstrang denaturierenden Reaktions-Bedingungen verwendet (z.B. TemperaturErhöhung bis auf über 90°C), allerdings in darauffolgenden Wiederholungen der Synthese-Schritte werden nicht-denaturierende Bedingungen während des exponentiellen Amplifikationsvorgangs aufrechterhalten. Für eine solche Kombination von TemperaturBedingungen spielt es keine Rolle, ob Start-Nukleinsäurekette eine Begrenzung in ihrem 5'-Sequenzsegment aufweist oder nicht. Durch initiale Denaturierung erfolgt Trennung von synthetisierten Strängen unabhängig von ihrer Länge in Analogie zu PCR.

### Bevorzugte Ausführungsformen des ersten Primer-Oligonukleotids (Primer-1)

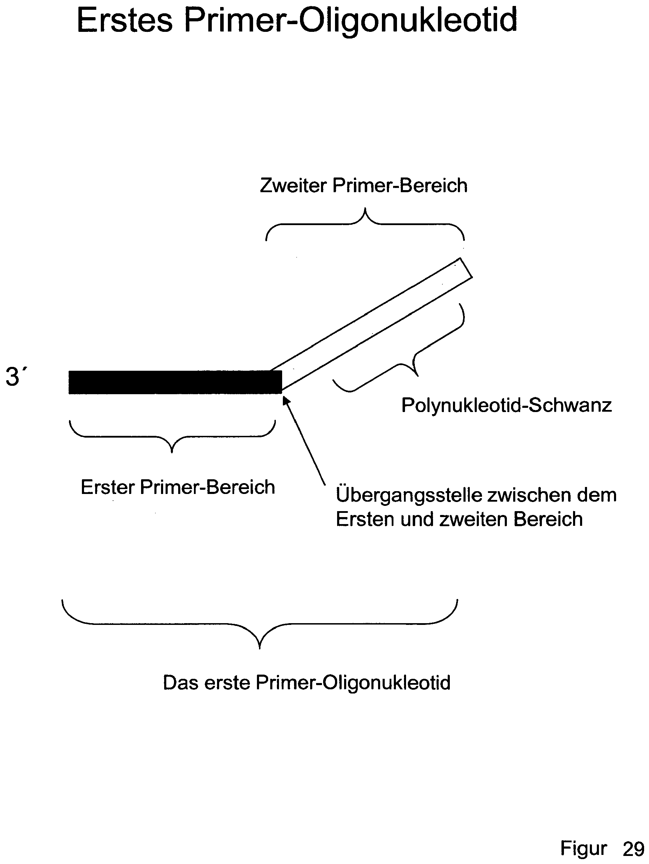

**Das erste Primer-Oligonukleotid** (Primer-1) ist eine Nukleinsäurekette welche mindestens folgende Bereiche einschließt (Fig. 29):
- Einen ersten Primer-Bereich im 3'-Segment des ersten Primer-Oligonukleotides, der an einen Strang einer zu amplifizierenden Nukleinsäurekette im Wesentlichen sequenzspezifisch binden kann
- Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz unter Reaktionsbedingungen im wesentlichen einzelsträngig bleibt, d.h. keine stabile Hairpin-Struktur oder ds-Strukturen ausbildet, und vorzugsweise nicht von Polymerase kopiert wird.

Die Gesamtlänge des ersten Primer-Oligonukleotides liegt zwischen 10 und 80, vorzugsweise zwischen 15 und 50, besser zwischen 20 und 30 Nukleotiden oder ihren Äquivalenten (z.B. Nukleotid-Modifikationen). Die Struktur des ersten Primer-Oligonukleotids ist dermaßen angepasst, dass es unter gewählten Reaktionsbedingungen eine reversible Bindung an Aktivator-Oligonukleotid eingehen kann. Weiterhin ist die Struktur des ersten Primer-Oligonukletides an seine Primer-Funktion angepasst. Weiterhin ist die Struktur so angepasst, dass eine Strangverdrängung mittels Aktivator-Oligonukleotid ausgeführt werden kann. Insgesamt sind Strukturen des ersten und des zweiten Bereichs aneinander angepasst, so dass eine exponentielle Amplifikation ausgeführt werden kann.

In einer vorteilhaften Ausführungsform der Erfindung sind der erste und der zweite Bereich des Primers in einer konventionellen 5'-3' Anordnung gekoppelt. In einer weiteren Ausführungsform der Erfindung erfolgt die Kopplung von beiden Abschnitten über eine 5'-5'-Bindung, so dass der zweite Bereich eine umgekehrte Richtung hat als der erste Bereich.

Die Kopplung von Bereichen zwischeneinander/untereinander erfolgt vorzugsweise kovalent. In einer Ausführungsform ist die Kopplung zwischen dem ersten und zweiten Bereich eine für DNA konventionelle 5'-3'-Phosphodiester-Kopplung. In einer weiteren Ausführungsform ist es eine 5'-5'-Phosphodiester-Kopplung. In einer weiteren Ausführungsform ist es eine 5'-3'-Phosphodiester-Kopplung, wobei zwischen benachbarten endständigen Nukleotiden bzw. Nukleotid-Modifikationen der beiden Bereiche mindestens ein Linker (z.B. ein C3, C6, C12 oder ein HEG-Linker oder eine abasische Modifikation) positioniert ist.

Einzelne Bereiche können unterschiedliche Nukleotid-Modifikationen einschließen. Dabei können individuelle Elemente von Nukleotiden modifiziert sein: Nukleobase und Rückgrad (Zucker-Anteil und / oder Phosphat-Anteil). Weiterhin können Modifikationen verwendet werden, welchen mindestens eine Komponente der Standard-Nukleotid-Bausteine fehlt oder modifiziert ist, z.B. PNA.

In einer weiteren Ausführungsform umfasst ein zweiter Bereich des ersten Primer-Oligonukleotides weitere Sequenzen, die nicht an das Aktivator-Oligonukleotid binden. Diese Sequenzen können zu anderen Zwecken verwendet werden, z.B. zu Bindung an die feste Phase. Diese Sequenzen sind vorzugsweise am 5'-Ende des Polynukleotid-Schwanzes lokalisiert.

In einer weiteren Ausführungsform kann ein erstes Primer-Oligonukleotid charakteristische Markierung umfassen. Beispiele für eine solche Markierung stellen Farbstoffe dar (z.B. FAM, TAMRA, Cy3, Alexa 488 etc.) oder Biotin bzw. andere Gruppen, die spezifisch gebunden werden können, z.B. Digoxigenin.

### Der erste Primer-Bereich des ersten Primer-Oligonukleotides

Die Sequenz-Länge liegt zwischen ca. 3-30 Nukleotiden, vorzugsweise zwischen 5 und 20 Nukleotiden, wobei die Sequenz vorwiegend komplementär zum 3'-Segment eines Stranges der zu amplifizierenden Nukleinsäurekette ist. Im Einzelnen, muss dieser Primer-Bereich in der Lage sein, an das komplementäre 3'-Segment eines zweiten Primer-Verlängerungsproduktes spezifisch zu binden. Dieser erste Bereich soll bei Rücksynthese kopiert werden können und dient auch als Matrize für 2. Strang. Die Nukleotid-Bausteine sind vorzugsweise untereinander via übliche 5'-3' Phosphodiester-Bindung oder Phosphothioester-Bindung verknüpft.

Der erste Primer-Bereich schließt bevorzugt Nukleotid-Monomere ein, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung.

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt werden kann. Der erste Primer-Bereich dient als Initiator der Synthese des ersten Primer-Verlängerungsproduktes in der Amplifikation. In einer weiteren bevorzugten

Ausführungsform umfasst der erste Bereich mindestens eine Phosphorothioat-Verbindung, so dass kein Abbau von 3'-Ende des Primern durch 3'-Exonuclease Aktivität von Polymerasen erfolgen kann.

Die Sequenz des ersten Bereichs des ersten Primer-Oligonukleotids und die Sequenz des zweiten Bereichs des Aktivator-Oligonukleotides sind vorzugsweise einander komplementär.

In einer Ausführungsform kann der erste Primer-Bereich oder sein 3'-Segment an Sequenzsegmente einer Zielsequenz binden.

**In einer Ausführungsform wird ein Allel-spezifischer erster Primer in Kombination mit einem allel-spezifischen Aktivator-Oligonukleotid verwendet.**

Der erste Primer-Bereich des ersten Primers kann an die ensprechende komplementäre Position einer Start-Nukleinsäure oder einer zu amplifizierenden Nukleinsäurekette binden. Bevozugt umfasst ein erster Primer Bereich zumindest eine Sequenzsegment, welches bevorzugt spezifisch unter verwendeten Reaktionsbedingungen an eine allel-spezifische Sequenzvariante der Zielnukleinsäure binden kann, wobei Polymerase in der Lage ist, einen dabei gebildeten perfekt-match Komplex zu verlängert, so dass dabei ein erster Primer-Verlängerungsprodukt resultiert.

Die Position einer allel-spezifischen Sequenz im Primer umfasst in einer Ausführungsform das 3'-Terminalen Nukleotid. In einer weiteren Ausführungsfrom umfasst die Position einer allel-spezifischen Sequenz im Primer die mindestens eine der Positionen -1 bis -6 Nukleotide im 3'-terminalen Segment des ersten Bereichs des ersten Primers. In einer weiteren Ausführungsfrom umfasst die Position einer allel-spezifischen Sequenz im Primer mindestens eine der Positionen von -6 bis mindestens -15 im 3'-terminalen Segment des ersten Bereichs des ersten Primers.

Ein solcher Primer umfasst weiterhin Sequenzsegmente, welche komplementär an alle Allel-Varianten einer Zielsequenz einheitlich binden können. Somit umfasst der erste Bereich eines ersten allel-spezifischen Primers Sequenzsegmente, welche sowohl zielsequenzspezifisch sind als auch solche, welche allel-spezifisch sind.

Bevorzugt wird eine Kombination eines allel-spezifischen Primers und eines allel-spezifischen Aktivator-Oligonukleotides verwendet, wobei der erste Bereich des ersten Primer-Oligonukleotide vollständig komplementär ist zum zweiten Bereich des Aktivator-Oligonukleotides.

Einzelne allel-spezifische Primer können zu einer Gruppe zusammengefasst werden, welche sämtliche Varianten einer Zielsequenz abdecken. Eine solche Gruppe von allel-spezifischen Primern umfasst zumindest zwei unterschiedliche allel-spezifische Primer, da ein polymorpher Lokus an einer vorgegeben Position in der Zielsequenz mindestens zwei Sequenzvarianten umfasst. Die allel-spezifischen Primer sind dahingehend konstruiert, dass sie unter stringenten Reaktionsbedingungen vorzugsweise mit ihrer jeweils spezifischen Matrize eine perfekt-match Bindung eingehen und somit diese spezifische perfekt-Match Matrize zur Ausbildung der jeweiligen Primer-Verlängerungsprodukte unter katalytischer Wirkung der Polymerase verwenden. Vorzugsweise können 3'-terminale Nukleotide und / oder 3'-Terminale Segmente von allel-spezifischen Primern zur Diskriminierung von Varianten von Zielsequenzen verwendet werden und dabei dermaßen in ihrer Sequenzzusammensetzung an die jeweiligen Varianten angepasst, dass solche Primer einen perfekt-match Doppelstrang unter stringenten Bedingungen mit der jeweiligen Variante ausbilden. Solche perfekt-match Doppelstränge können in der Regel von einer Polymerase gut erkannt werden und unter geeigneten Reaktionsbedingungen kommt es zu einer Primer-Verlängerung. Bei der Interaktion eines allel-spezifischen Primers mit einer anderen Variante einer Zielsequenz entsteht somit ein Mismatch- Doppelstrang. Solche Mismatches führen in der Regel zu einer Verzögerung der Verlängerung durch eine Polymerase bzw. zu einer Verlangsamung der Gesamtreaktion. In einer Ausführungsform können allel-spezifischen Primer im 3'-Segment mindestens eine phosphoro-thioat-Bindung umfassen, welche allel-spezifische Primer vor 3'-5'-Nuklease-Abbau durch eine Polymerase schützt.

Mehrere allel-spezifische Primer umfassen somit Sequenzsegmente, welche für eine Gruppe von allel-spezifischen Primern im Wesentlichen identisch bzw. einheitlich sind, sowie Sequenzsegmente, welche unter den Primern einer Gruppe unterschiedlich sind und charakteristisch für jeweilige Sequenzvariante einer Zielsequenz. Unter Einbeziehung von einheitlichen Sequenzsegmenten können solche Primer an die jeweilige Zielsequenz unter Reaktionsbedingungen hybridisieren. Unter Einbeziehung von charakteristischen Sequenzsegmenten kann ein jeweiliger Primer spezifisch an eine Sequenzvariante der Zielsequenz unter Ausbildung eines perfekt-match Doppelstranges binden. Vorzugsweise sind die Primer dermaßen konstruiert, dass unter verwendeten Reaktionsbedingungen die Bindung an eine Zielsequenz unter Ausbildung eines perfekt-match Doppelstranges bevorzugt wird und die Bindung an einer Zielsequenz unter Ausbildung eines Mismatch-Doppelstranges weniger bevorzugt wird.

**In einer weiteren Ausführungsform wird ein zielsequenz spezifischer erster Primer, (aber kein allel-spezifischer erster Primer) in Kombination mit einem allel-spezifischen Aktivator-Oligonukleotid verwendet.**

Der erste Primer-Bereich des ersten Primers kann an die ensprechende komplementäre Position einer Start-Nukleinsäure oder einer zu amplifizierenden Nukleinsäurekette binden. Bevozugt umfasst ein erster Primer Bereich zumindest eine Sequenzsegment, welches bevorzugt sequenz-spezifisch unter verwendeten Reaktionsbedingungen an Sequenzsegmente einer Zielnukleinsäurekette (beispielsweise umfassend eine Start-Nukleinsäure und/oder die zu amplifizierende Nukleinsäurekette) binden kann, wobei diese Bindung im wesentlichen unabhängig von potenziell vorhandenen Sequenzunterschieden im polymorphen Lokus erfolgt, wobei Polymerase in der Lage ist, einen dabei gebildeten perfekt-match Komplex zu verlängert, so dass dabei ein erstes Primer-Verlängerungsprodukt resultiert.

Die Bindung eines solchen Primers erfolgt im Wesentlichen im Sequenzsegment der Zielnukleinsäure, welches für mindestens zwei Allel-Varianten dieser Zielnukleinsäureketten einheitlich ist. Bevorzugt erfolgt die Primer-Bindung im Sequenzsegment der Zielnukleinsäure, welches für alle Allel-Varianten dieser Zielnukleinsäure einheitlich ist.

Die Bindung erfolgt dabei dermaßen, dass der polymorphe Lokus der Zielsequenz in 3'-Richtung vom ersten Bereich des ersten Primers liegt, so dass bei einer Primer-Verlängerungsreaktion dieser polymorphe Lokus von Polymerase kopiert wird. Ein resultierendes erstes Primer-Verlängerungsprodukt umfasst somit eine komplementäre Sequenz zum polymorphen Lokus. Diese Sequenz liegt in 3'-Richtung vom ersten Primer.

Ein solcher Primer umfasst somit Sequenzsegmente, welche bevorzugt komplementär an alle Allel-Varianten einer Zielsequenz einheitlich binden können. Damit eine Differenzierung von allel-Varianten stattfinden kann, muss ein solcher Primer mit mindestens mit einem allel-spezifischen Aktivator-Oligonukleotid kombiniert werden. Die Allel-Diskriminierung erfolgt somit durch Wirkung von Aktivator-Oligonukleotid. Die Positionierung des polymorphen Lokus in 3'-Richtung vom Primer lokalisiert ist, bedingt seine Lokalisation im dritten Bereich des Aktivator-Oligonukleotides.

### Der zweite Bereich des ersten Primer-Oligonukleotides

Der zweite Bereich des ersten Primer-Oligonukleotides ist vorzugsweise eine Nukleinsäuresequenz, welche mindestens einen Polynukleotid-Schwanz umfasst, welcher vorzugsweise während der Synthesereaktion von Polymerase unkopiert bleibt und welcher zur Bindung mit dem ersten Bereich des Aktivator-Oligonukleotides fähig ist. Das Segment des zweiten Bereichs, welches überwiegend diese Bindung mit dem Aktivator-Oligonukleotid eingeht, kann als Polynukleotid-Schwanz bezeichnet werden.

Weiterhin muss der zweite Bereich des ersten Primer-Oligonukleotids nicht nur das Aktivator-Oligonukleotides unter Reaktionsbedingungen spezifisch binden, sondern auch beim Vorgang der Strangverdrängung mittels Aktivator-Oligonukleotid mitwirken. Die Struktur des zweiten Bereichs muß folglich für die Herbeiführung einer räumlichen Nähe zwischen dem Aktivator-Oligonukleotid und dem entsprechenden Doppelstrang-Ende (im Einzelnen, dem 3'-Ende des zweiten Primer-Verlängerungsproduktes) geeignet sein.

Die Gestaltung der Struktur des zweiten Bereichs des ersten Primer-Oligonukleotids wird in mehreren Ausführungsformen genauer dargestellt. Dabei werden Anordnung der Oligonukleotid-Segmente und verwendete Modifikationen berücksichtigt, welche zu einem Stopp in der Polymerase-katalysierten Synthese führen.

Die Länge des zweiten Bereichs liegt zwischen 3 und 60, vorzugsweise zwischen 5 und 40, bevorzugt zwischen 6 und 15 Nukleotiden oder ihren Äquivalenten.

Die Sequenz des zweiten Bereichs kann willkürlich gewählt sein. Vorzugsweise ist sie nicht komplementär mit der zu amplifizierenden Nukleinsäure und/oder mit dem zweiten Primer-Oligonukleotid und/oder mit dem ersten Bereich des ersten Primer-Oligonukleotid. Weiterhin enthält sie vorzugsweise keine selbstkomplementären Segmente, wie Hairpins oder Stemmloops.

Die Sequenz des zweiten Bereichs ist vorzugsweise auf die Sequenz des ersten Bereichs des Aktivator-Oligonukleotids abgestimmt, so dass beide Sequenzen unter Reaktionsbedingungen eine Bindung eingehen können. In einer bevorzugten Ausführungsform ist diese Bindung unter Reaktionsbedingungen reversibel: es besteht somit ein Gleichgewicht zwischen aneinander gebundenen Komponenten und nichtgebundenen Komponenten.

Die Sequenz des zweiten Bereichs des ersten Primer-Oligonukleotides ist vorzugsweise dermaßen gewählt, dass die Anzahl von komplementären Basen, welche mit dem ersten Bereich des Aktivator-Oligonukleotid binden können, zwischen 1 und 40, besser zwischen 3 und 20, bevorzugt zwischen 6 und 15 liegt.

Die Funktion des zweiten Bereichs besteht unter anderem in Bindung des Aktivator-Oligonukleotides. In einer Ausführungsform ist diese Bindung vorzugsweise spezifisch, so dass ein zweiter Bereich eines ersten Primer-Oligonukleotides ein spezifisches Aktivator-Oligonukleotid binden kann. In einer anderen Ausführungsform kann ein zweiter Bereich mehr als nur ein Aktivator-Oligonukleotid unter Reaktionsbedingungen binden.

Es besteht im Allgemeinen keine Notwendigkeit in einer perfekten Übereinstimmung in der Sequenz zwischen dem zweiten Bereich des ersten Primer-Oligonukleotides und dem ersten Bereich des Aktivator-Oligonukleotides. Das Maß der Komplementarität zwischen dem zweiten Bereich des ersten Primer-Oligonukleotides und dem ersten Bereich des Aktivator-Oligonukleotides kann zwischen 20 % und 100 %, besser zwischen 50 % und 100 %, bevorzugt zwischen 80 % und 100 % liegen. Die jeweils komplementären Bereiche können unmittelbar aneinander anschließend positioniert sein oder auch nichtkomplementäre Sequenz-Segmente dazwischen umfassen.

In einer Ausführungsform werden vorzugsweise spezifische Sequenzsegmente im zweiten Bereich des ersten Primers verwendet. Diese sequenzspezifische und damit charakteristische Sequenzsegmente sind vorzugsweise nicht komplementär zu Zielnukleinsäurekette uund werden dermaßen an die Sequenzsegmente des ersten Bereichs eines allel-spezifischen Aktivator-Oligonukleotides angepasst, dass dabei vorwiegend spezifische komplementäre Duplexe zwischen dem ersten Bereich des Aktivator-Oligonukleotides und dem zweiten Bereich des ersten Primer-Oligonukleotdies gebildet werden können. Dadurch kann beispielsweise bei Anwesenheit von mehreren Aktivator-Oligonukleotiden (z.B. welche allel-spezifisch sind), eine Paar-Bildung aus einem bestimmten ersten Primer und einem bestimmten Aktivator-Oligonukleotid erfolgen. Der zweite Bereich des ersten Primer-Oligonukleotides kann somit zu einer charakteristischen Kodierung besonders bei multiplexen Analysen verwendet werden.

Durch Verwendung von vorwiegend sequenzspezifischen Sequenzsegmenten im zweiten Primer-Bereich und einer gleichzeitigen Verwendung von entsprechend komplementären Sequenzsegmenten im ersten Bereich des Aktivator-Oligonukleotides, kann somit eine verbesserte Zuordnung von potenziell resultierenden Primer-Verlängerungsprodukten und charakteristischen Aktivator-Oligonukleotiden erfolgen.

Der zweite Bereich des ersten Primer-Oligonukleotides kann in einer Ausführungsform mindestens eine Tm-modifizierende Modifikation einschließen. Durch Einführung solcher Modifikationen kann die Stabilität der Bindung zwischen dem zweiten Bereich des ersten Primer-Oligonukleotides und dem ersten Bereich des Aktivator-Oligonukleotides modifiziert werden. Beispielsweise können Tm-erhöhende Modifikationen (Nukleotid-Modifikationen oder nicht-Nukleotid-Modifikationen) verwendet werden, wie LNA-Nukleotide, 2-Amino-Adenosine oder MGB-Modifikationen. Andererseits können auch Tm-Senkende Modifikationen verwendet werden, wie beispielsweise Inosine-Nukleotid. In der Struktur des zweiten Bereichs können auch Linker (z.B. C3, C6, HEG-Linker) intergriert werden.

Für die Strangverdrängung muss das Aktivator-Oligonukleotid in räumliche Nähe des Doppelstrang-Endes der zu amplifizierenden Nukleinsäure gebracht werden. Dieses Doppelstrang-Ende besteht aus Segmenten des ersten Primer-Bereichs des ersten Primer-Verlängerungsproduktes und einem entsprechend komplementären dazu 3'-Segment des zweiten Primer-Verlängerungsproduktes.

Der Polynukleotid-Schwanz bindet vorwiegend komplementär das Aktivator-Oligonukleotides unter Reaktionsbedingungen und bewirkt damit eine vorübergehende Annäherung des zweiten Bereichs des Aktivator-Oligonukleotides und des ersten Bereichs eines verlängerten Primer-Verlängerungsproduktes, so dass eine komplementäre Bindung zwischen diesen Elementen im Rahmen eines Strangverdrängungsvorgangs initiiert werden kann.

In einer Ausführungsform führt die Bindung des Aktivator-Oligonukleotides an den Polynukleotid-Schwanz des ersten Primer-Oligonukleotides unmittelbar zu einem solchen Kontakt. Das bedeutet, dass der Polynukleotid-Schwanz und der erste Primer-Bereich des ersten Primer-Oligonukleotides unmittelbar aneinander gekoppelt sein müssen. Dank einer solchen Anordnung kann nach einer Bindung eines Aktivator-Oligonukleotids im seinem ersten Bereich unmittelbar zu einem Kontakt zwischen komplementären Basen des zweiten Bereichs des Aktivator-Oligonukleotides und zu entsprechenden Basen des ersten Primer-Bereichs kommen, so dass eine Strangverdrängung initiiert werden kann.

In einer weiteren Ausführungsform liegen zwischen Strukturen des Polynukleotid-Schwanzes und dem ersten Primer-Bereich andere Strukturen des zweiten Bereichs des ersten Primer-Oligonukleotides. Nach einer Bindung eines Aktivator-Oligonukleotids an den Polynukleotid-Schwanz ist dieser somit nicht unmittelbar an den ersten Primer-Bereich positioniert, sondern in einer gewissen Distanz dazu. Die Strukturen zwischen dem unkopierbarem Polynukleotid-Schwanz und dem kopierbaren ersten Primer-Bereich des Primer-Oligonukleotides können einen solchen Abstand generieren. Dieser Abstand hat einen Wert, welcher zwischen 0,1 und 20 nm, vorzugsweise zwischen 0,1 und 5 nm, besser zwischen 0,1 und 1 nm liegt.

Solche Strukturen stellen beispielsweise Linker (z.B. C3 oder C6 oder HEG-Linker) oder nicht zu Aktivator-Oligonukleotid komplementäre Segmente dar (z.B. in Form von nichtkomplementären, nicht-kopierbaren Nukleotid-Modifikationen). Die Länge dieser Strukturen kann im Allgemeinen in Ketten-Atomen ausgemessen werden. Diese Länge beträgt zwischen 1 und 200 Atomen, vorzugsweise zwischen 1 und 50 Kettenatomen, bevorzugt zwischen 1 und 10 Kettenatomen.

Damit der Polynukleotid-Schwanz von Polymerase unter Amplifikationsbedingungen unkopierbar bleibt, umfasst der zweite Bereich des ersten Primer-Oligonukleotides im allgemeinen Sequenz-Anordnungen bzw. Strukturen, welche zu einem Stopp der Polymerase in der Synthese des zweiten Primer-Verlängerungsproduktes bewirken, nachdem die Polymerase den ersten Primer-Bereich erfolgreich kopiert hat. Diese Strukturen sollen das Kopieren des Polynukleotid-Schwanzes des zweiten Bereichs verhindern. Der Polynukleotid-Schwanz bleibt somit vorzugsweise von der Polymerase unkopiert.

In einer Ausführungsform liegen solche Strukturen zwischen dem ersten Primer-Bereich und dem Polynukleotid-Schwanz.

In einer weiteren Ausführungsform kann die Sequenz des Polynukleotid-Schwanzes Nukleotid-Modifikationen einschließen, welche zum Stopp der Polymerase führen. Dadurch kann ein Sequenzsegment des zweiten Bereichs des ersten Primer-Oligonukleotids beide Funktionen umfassen: es ist sowohl ein Polynukleotid-Schwanz als auch eine zum Stopp der Polymerase führende Sequenz aus Nukleotid-Modifikationen.

Modifikationen im zweiten Bereich des ersten Primer-Oligonukleotid, welche zu einem Synthese-Stopp führen und somit den Polynukleotid-Schwanz unkopierbar lassen, werden in dieser Anmeldung unter dem Begriff "erste Blockierungs-Einheit oder einen ersten Stopp-Bereich" zusammengefasst.

**Nachfolgend sind weitere Ausführungsformen von Strukturen angeführt, welche zum Stopp in der Synthese des zweiten Stranges führen können.**

Mehrere Bausteine bei der Oligonukleotid-Synthese sind bekannt, welche die Polymerase beim Ablesen der Matrize hindern und zur Termination von Polymerase-Synthese führen. Beispielsweise sind nicht-kopierbare Nukleotid-Modifikationen oder Nicht-Nukleotid-Modifikationen bekannt. Es gibt auch Synthese-Arten/Anordnungen von Nukleotid-Monomeren innerhalb eines Oligonukleotides, welche zum Stopp der Polymerase führen (z.B. 5'-5-Anordnung oder 3'-3'Anordnung). Primer-Oligonukleotide mit einem nicht kopierbaren Polynukleotid-Schwanz sind ebenfalls im Stand der Technik bekannt (z.B. Scorpion-Primer-Strukturen bzw. Primer für Bindung an die feste Phase). Beide Primer-Varianten beschreiben Primer-Oligonukleotid-Strukturen, welche in der Lage sind, einerseits die Synthese eines Stranges zu initiieren, so dass eine Primer-Verlängerungsreaktion stattfinden kann. Es resultiert ein erster Strang, welcher im Primer-Verlängerungs-Produkt auch die Primer-Struktur mit Schwanz integriert. Bei der Synthese eines komplementären Stranges zum ersten Primer-Verlängerungs-Produkt, z.B. im Rahmen einer PCR-Reaktion, wird der zweite Strang bis zur "Blockierungs-Einheit/Stopp-Struktur" der Primer-Struktur verlängert. Beide beschriebene Primer-Strukturen sind dermaßen konzipiert, dass der 5'-Anteil des Primer-Oligonukleotides einzelsträngig bleibt und nicht von der Polymerase kopiert wird.

In einer weiteren Ausführungsform umfasst der zweite Bereich des Primer-Oligonukleotides einen Polynukleotid-Schwanz, welche in seiner gesamten Länge eine konventionelle Anordnung von 5'- nach 3'- aufweist und nicht-kopierbare Nukleotid-Modifikationen einschließt. Zu solchen nicht-kopierbaren Nukleotid-Modifikationen zählen beispielsweise 2'-O-Alkyl-RNA Modifikationen, PNA, Morpholino. Diese Modifikationen können unterschiedlich im zweiten Primer-Bereich verteilt sein.

Der Anteil von nicht-kopierbaren Nukleotid-Modifikationen kann im Polynukleotid-Schwanz zwischen 20 % und 100 % liegen, vorzugsweise beträgt er mehr als 50% der Nukleotid-Bausteine. Vorzugsweise liegen diese Nukleotid-Modifikationen im 3'-Segment des zweiten Bereichs und grenzen somit an den ersten Bereich des ersten Primer-Oligonukleotides.

In einer Ausführungsform ist die Sequenz der nicht-kopierbaren Nukleotid-Modifikationen zumindest teilweise komplementär zu der Sequenz im Matrizenstrang, so dass die Primer-Bindung an die Matrize unter Einbeziehung von zumindest einem Teil dieser Nukleotid-Modifikationen stattfindet. In einer weiteren Ausführungsform ist die Sequenz der nicht-kopierbaren Nukleotid-Modifikationen nicht-komplementär zu der Sequenz im Matrizenstrang.

Die nicht-kopierbaren Nukleotid-Modifikationen werden vorzugsweise aneinander kovalent gekoppelt und stellen somit ein Sequenz-Segment im zweiten Bereich dar. Die Länge dieses Segments umfasst zwischen 1 und 40, bevorzugt zwischen 1 und 20 Nukleotid-Modifikationen, bevorzugter zwischen 3 und 10 Nukleotid-Modifikationen.

In einer weiteren Ausführungsform umfasst der zweite Bereich des ersten Primer-Oligonukleotides einen Polynukleotid-Schwanz, welcher in seiner gesamten Länge eine konventionelle Anordnung von 5'-3' aufweist und nicht-kopierbare Nukleotid-Modifikationen einschließt (z.B. 2'-O-Alkyl-Modifikationen) und mindestens einen Nicht-Nukleotid-Linker (z.B. C3, C6, HEG-Linker) einschließt. Ein Nicht-Nukleotid-Linker hat die Funktion, benachbarte Nukleotide oder Nukleotid-Modifikationen kovalent zu verbinden und gleichzeitig die Synthese-Funktion der Polymerase ortspezifisch zu unterbrechen.

Ein solcher Nicht-Nukleotid-Linker soll die Strukturen des Polynukleotid-Schwanzes und des ersten Primer-Bereichs nicht zu weit voneinander entfernen. Vielmehr sollte der Polynukleotid-Schwanz in einer räumlichen Nähe vom ersten Primer-Bereich sein. Unter einem Nicht-Nukleotid-Linker werden Modifikationen zusammengefasst, welche in ihrer Länge nicht länger sind als 200 Ketten-Atome, noch vorteilhafter nicht länger als 50 Ketten-Atome, besonders bevorzugt nicht länger als 10 Ketten-Atome umfassen. Die Minimal-Länge eines solchen Linkers kann ein Atom betragen. Ein Beispiel für solche Nicht-Nukleotid-Linker stellen gerade oder verzweigte Alkyl-Linker mit einer Alkyl-Kette dar, welche mindestens ein Kohlenstoff-Atom einschließt, vorteilhafter mindestens 2 bis 30, bevorzugter 4 bis 18. Solche Linker sind in der Oligonukleotid-Chemie hinreichend bekannt (z.B. C3, C6 oder C12 Linker) und können während einer Festphasen-Synthese von Oligonukleotiden zwischen der Sequenz des Polynukleotid-Schwanzes und der Sequenz des ersten Bereichs des ersten Primer-Oligonukleotides eingeführt werden. Ein anderes Beispiel für solche Nicht-Nukleotid-Linker stellen Lineare oder verzweigte PolyethylenGlykol-Derivate dar. Ein bekanntes Beispiel in der Oligonukleotid-Chemie stellt Hexaethylenglycol (HEG) dar. Ein weiteres Beispiel für solche Nicht-Nukleotid-Linker stellen abasische Modifikationen dar (z.B. THF-Modifikation, als Analog von dRibose).

Wenn eine oder mehrere solche Modifikationen in einen zweiten Bereich integriert sind, können sie eine Polymerase in ihrer Kopier-Funktion während ihrer Synthese des zweiten Primer-Verlängerungsprodukts effektiv stören, so dass stromabwärts gelegene Segmente nach einer solchen Modifikation unkopiert bleiben. Die Anzahl von solchen Modifikationen im zweiten Bereich kann zwischen 1 und 100 liegen, vorzugsweise zwischen 1 und 10, bevorzugt zwischen 1 und 3.

Die Position eines solchen Nicht-Nukleotid-Linker kann am 3'-Ende des zweiten Bereichs liegen und somit den Übergang zum ersten Bereich und dem zweiten Bereich des Primer-Oligonukleotid darstellen.

Die Lage des Nicht-Nukleotid-Linkers im mittleren Segment des zweiten Bereichs kann ebenfalls verwendet werden. Damit wird ein Polynukleotid-Schwanz in mindestens zwei Segmente geteilt. In dieser Ausführungsform schließt das 3'-Segment des Polynukleotid-Schwanzes mindestens eine, besser mehrere, z.B. zwischen 2 und 20, vorzugsweise zwischen 2 und 10 nicht-kopierbaren Nukleotid-Modifikationen ein. Diese nicht-kopierbaren Nukleotid-Modifikationen liegen vorzugsweise am Übergang zwischen dem ersten und dem zweiten Bereich des Primer-Oligonukleotides.

In einer weiteren Ausführungsform umfasst der zweite Bereich des Primer-Oligonukleotides einen Polynukleotid-Schwanz, welcher in seiner gesamten Länge eine Anordnung von 5'-nach 3'- aufweist und zumindest ein Nukleotid-Monomer in einer "umgekehrten" Anordnung von 3'-nach 5'- einschließt und welche am Übergang zwischen dem ersten und dem zweiten Bereich des ersten Primer-Oligonukleotides positioniert sind.

In einer weiteren Ausführungsform umfasst der zweite Bereich des Primer-Oligonukleotides einen Polynukleotid-Schwanz, wobei ein solcher Polynukleotid-Schwanz vollständig aus Nukleotiden besteht, welche in umgekehrten Anordnung direkt an den ersten Bereich des ersten Primer-Oligonukleotides angrenzen, so dass die Kopplung des ersten und des zweiten Bereichs durch 5'-5' Position erfolgt. Ein Vorteil einer solchen Anordnung ist, dass die Polymerase nach einer Kopierung des ersten Bereichs an eine "umgekehrte" Anordnung von Nukleotiden trifft, was typischerweise zu Termination der Synthese an dieser Stelle führt.

Bei einer "umgekehrten" Anordnung von Nukleotiden in der Gesamtlänge des Polynukleotid-Schwanzes soll vorzugsweise das 3'-terminales Nukleotid des Polynukleotid-Schwanzes an seinem 3'-OH-Ende blockiert sein, um Nebenreaktionen vorzubeugen. Alternativ kann auch ein terminales Nukleotid verwendet werden, welches gar keine 3'-OH Gruppe aufweist, z.B. ein Dideoxy-Nukleotid.

In einer solchen Ausführungsform soll natürlich auch die entsprechende Nukleotid-Anordnung im Aktivator-Oligonukleotid angepasst werden. In einem solchen Fall müssen der erste und der zweite Bereich des Aktivator-Oligonukleotids in 3'-3' Anordnung verknüpft werden.

In einer weiteren Ausführungsform umfasst der zweite Bereich des Primer-Oligonukleotides einen Polynukleotid-Schwanz, welche in seiner gesamten Länge eine konventionelle Anordnung von 5'-nach 3'- aufweist und mindestens eine Nukleotid-Modifikation einschließt, welche für die Polymerase keine komplementäre Nukleobase darstellt, wenn die Synthese mit ausschließlich natürlichen dNTP (dATP, dCTP, dGTP, dTTP oder dUTP) durchgeführt wird.

Beispielsweise können Iso-dG bzw. Iso-dC Nukleotid-Modifikationen als einzelne, vorzugsweise aber mehrere (mindestens 2 bis 20) Nukleotid-Modifikationen im zweiten Bereich des ersten Primer-Oligonukleotids integriert sein. Weitere Beispiele für Nukleobasen-Modifikationen stellen verschiedene Modifikationen des erweiterten genetischen Alphabets dar. Solche Nukleotid-Modifikationen unterstützen vorzugsweise keine komplementäre Basen-Paarung mit natürlichen Nukleotiden, so dass eine Polymerase (zumindest theoretisch) kein Nukleotid aus der Reihe (dATP, dCTP, dGTP, dTTP oder dUTP) einbaut. In der Realität kann es dennoch zum rudimentären Einbau kommen, insbesondere bei höheren Konzentrationen von dNTP-Substraten und prolongierten Inkubationszeiten (z.B. 60 min oder länger). Daher sollen bevorzugt mehrere solche Nukleotid-Modifikationen positioniert an benachbarten Stellen zum Einsatz kommen. Der Stopp der Polymerase-Synthese wird durch Fehlen von passenden komplementären Substraten für-diese Modifikationen bewirkt. Oligonukleotide mit Iso-dC bzw. Iso-dG können mit Standardverfahren synthetisiert werden und sind von mehreren kommerziellen Anbietern erhältlich (z.B. Trilink-Technologies, Eurogentec, Biomers GmbH). Wahlweise kann auch die Sequenz des ersten Bereichs des Aktivator-Oligonukleotids an die Sequenz eines solchen zweiten Primer-Bereichs angepasst werden. Dabei können komplementäre Nukleobasen des erweiterten genetischen Alphabets in den ersten Bereich des Aktivator-Oligonukleotides entsprechend während der chemischen Synthese intergriert werden. Beispielsweise kann Iso-dG im zweiten Bereich des ersten Primer-Nukleotids integriert sein, sein komplementäres Nukleotid (Iso-dC-5-Me) kann an der passenden Stelle im ersten Bereich des Aktivator-Oligonukleotids platziert werden.

Zusammenfassend, kann die Termination der Synthese von Polymerase im zweiten Bereich auf verschiedene Art und Weise erreicht werden. Diese Blockade erfolgt bevorzugt allerdings erst, wenn Polymerase den ersten Bereich des ersten Primer-Oligonukleotides kopiert hat. Damit wird sichergestellt, dass ein zweites Primer-Verlängerungsprodukt eine passende Primer-Bindungsstelle in seinem 3'-Segment besitzt. Diese Primer-Bindungsstelle wird im Rahmen der Strangverdrängung freigelegt und steht somit für eine erneute Bindung eines weiteren ersten Primer-Oligonukleotids zur Verfügung.

Bei der Synthese des komplementären Stranges zum ersten Primer-Verlängerungsprodukt bleibt die Primer-Verlängerungsreaktion vor dem Polynukleotid-Schwanz stehen. Da dieser Polynukleotid-Schwanz für die Interaktion mit dem Aktivator-Oligonukleotid dadurch einzelsträngig bleibt und somit für Bindung zur Verfügung steht, unterstützt dieser die Initiierung der Strangverdrängungs-Reaktion durch das Aktivator-Oligonukleotid, indem er den entsprechenden komplementäre Segmente des Aktivator-Oligonukleotids in unmittelbare Nähe des passenden Duplex-Endes bringt. Der Abstand zwischen dem komplementären Teil des Aktivator-Oligonukleotides (zweiter Bereich) und dem komplementären Teil des verlängerten Primer-Oligonukleotid (erster Bereich) wird dadurch auf ein Minimum reduziert. Solche räumliche Nähe erleichtert die Initiierung der Strangverdrängung.

Im Rahmen einer schematischen Darstellung einer nukleinsäurevermittelten Strangverdrängungs-Reaktion liegt nun eine komplementäre Sequenz von Aktivator-Oligonukleotid unmittelbar in der Nähe des passenden Duplex-Endes. Dabei kommt es zu Konkurrenz um die Bindung an den ersten Bereich des ersten Primer-Oligonukleotids zwischen dem Strang des Aktivator-Oligonukleotides und dem zum Primer komplementären Matrizenstrang. Durch repetetive Schließung und Formierung von Basenpaarung zwischen dem Primer-Bereich und dem komplementären Segment des Aktivator-Oligonukleotids (zweiter Bereich des Aktivator-Nukleotides) bzw. dem komplementären Segment des Matrizenstranges kommt es zur Initiierung des nukleinsäurevermittelten Strangverdrängungs-Vorgangs.

Im Allgemeinen ist die Ausbeute der Initiierung der Stang-Verdrängung ist umso höher, je näher der entsprechende komplementäre Sequenzabschnitt des Aktivator-Oligonukleotides zum komplementären Segment des Primer-Bereichs liegt. Bei Vergrößerung dieses Abstandes sinkt dagegen die Ausbeute der Initiierung der Strangverdrängung.

Im Rahmen der vorliegenden Erfindung ist es nicht zwingend notwendig, dass die Initiierung der Strangverdrängung mit maximaler Ausbeute funktioniert. Daher können Abstände zwischen dem 5'-Segment des ersten Primer-Bereichs des ersten Primer-Oligonukleotid, welcher eine Bindung mit einem komplementären Strang der Matrize eingeht und eine komplementäre Duplex bildet, und einem entsprechend komplementären Sequenzabschnitt im Aktivator-Oligonukleotides, wenn gebunden an Polynukleotid-Schwanz des zweiten Bereichs des ersten Primer-Oligonukleotides in folgenden Bereichen liegen: zwischen 0,1 und 20 nm, besser zwischen 0,1 und 5 nm, noch besser zwischen 0,1 und 1 nm. Im bevorzugten Fall beträgt diese Distanz weniger als 1 nm. In anderen Einheiten ausgedruckt entspricht dieser Abstand einer Strecke von weniger als 200 Atomen, noch besser weniger als 50 Atomen, noch besser weniger als 10 Atomen. Im bevorzugten Fall ist diese Distanz ein Atom. Die Angaben zur Distanz sollen lediglich zur Orientierung dienen und veranschaulichen, dass kürzere Distanzen zwischen diesen Strukturen bevorzugt werden. Die Messung dieser Distanz ist in vielen Fällen nur durch Analyse der genauen Strukturen von Oligonukleotiden und Ausmessung der Messung von Sequenzabständen bzw. von Linker-Längen möglich.

Der erste Primer kann auch noch weitere Sequenzabschnitte umfassen, welche nicht zu Interaktion mit Aktivator-Oligonukleotid oder Matrizen Strang benötigt werden. Solche Sequenzabschnitte können beispielsweise weitere Oligonukleotide binden, welche als Detektionssonden oder Immobilisierungspartner bei einer Bindung an die feste Phase verwendet werden.

### Primer-Funktion des ersten Primer-Oligonukleotides

Das erste Primer-Oligonukleotid kann in mehreren Teil-Schritten verwendet werden. In erster Linie übernimmt es eine Primer-Funktion in der Amplifikation. Dabei wird Primer-Verlängerungsreaktion unter Verwendung des zweiten Primer-Verlängerungsproduktes als Matrize ausgeführt. In einer weiteren Ausführungsform kann das erste Primer-Oligonukleotid zu Beginn der Amplifikationsreaktion die Start-Nukleinsäurekette als Matrize verwenden. In einer weiteren Ausführungsform kann das erste Primer-Oligonukleotid bei der Erstellung/Bereitstellung einer Start-Nukleinsäurekette verwendet werden.

Im Rahmen der Amplifikation dient der erste Primer als Initiator der Synthese des ersten Primer-Verlängerungsproduktes unter Verwendung des zweiten Primer-Verlängerungsproduktes als Matrize. Das 3'-Segment des ersten Primers umfasst eine Sequenz, welche vorwiegend komplementär an das zweite Primer-Verlängerungsprodukt binden kann. Die enzymatische Verlängerung des ersten Primer-Oligonukleotid unter Verwendung des zweiten Primer-Verlängerungsproduktes als Matrize führt zu Ausbildung des ersten Primer-Verlängerungsproduktes. Ein solches erstes Primer-Verlängerungsprodukt umfasst die Zielsequenz bzw. ihre Sequenz-Anteile. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird, so dass eine entsprechende Primer-Bindungsstelle für das erste Primer-Oligonukleotid resultiert. Die Synthese des ersten Primer-Verlängerungsproduktes erfolgt bis einschließlich 5'-Segment des zweiten Primer-Oligonukleotids. Unmittelbar nach der Synthese des ersten Primer-Verlängerungsproduktes ist dieses Produkt an das zweite Primer-Verlängerungsprodukt gebunden und bildet doppelsträngigen Komplex. Das zweite Primer-Verlängerungsprodukt wird aus diesem Komplex sequenzspezifisch durch das Aktivator-Oligonukleotid verdrängt. Dabei bindet das Aktivator-Oligonukleotid an das erste Primer-Verlängerungsprodukt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen. Das nun frei gewordene 3'-Segment des ersten Primer-Verlängerungsproduktes kann ein weiteres zweites Primer-Oligonukleotid binden, so dass eine neue Synthese des zweiten Primer-Verlängerungsproduktes initiiert werden kann.

Weiterhin kann das erste Primer-Oligonukleotid als Initiator der Synthese des ersten Primer-Verlängerungsproduktes ausgehend von Start-Nukleinsäurekette zu Begin der Amplifikation dienen. In einer Ausführungsform ist die Sequenz des ersten Primers vollständig komplementär zum entsprechenden Sequenzsegment einer Start-Nukleinsäurekette. In einer weiteren Ausführungsform ist die Sequenz des ersten Primer-Oligonukleotides nur teilweise komplementär zum entsprechenden Sequenzsegment einer Start-Nukleinsäurekette. Diese abweichende Komplementarität soll das erste Primer-Oligonukleotid allerdings nicht daran hindern, eine vorwiegend sequenzspezifische Primer-Verlängerungsreaktion zu starten. Die jeweiligen Unterschiede in Komplementarität des ersten Primer-Oligonukleotides zur jeweiligen Position in der Start-Nukleinsäurekette liegen vorzugsweise im 5'-Segment des ersten Bereichs des ersten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4-10 Positionen im 3'-Segment vollkomplementär zu Matrize (Start-Nukleinsäurekette) sein. Die restlichen Nukleotidpositionen können von perfekten Komplementarität abweichen. Das Ausmaß einer perfekten Komplementarität im restlichen 5'-Segment des ersten Bereichs des ersten Primer-Oligonukleotides kann somit Bereiche umfassen zwischen 50 % bis 100 %, besser zwischen 80 % und 100 % der Basenzzusammensetzung. Je nach Länge des ersten Bereichs des ersten Primer-Oligonukleotides, umfassend die Sequenz-Abweichungen von 1 bis maximal 15 Positionen, besser 1 bis maximal 5 Positionen. Das erste Primer-Oligonukleotid kann somit eine Synthese von einer Start-Nukleinsäukette initiieren. Bei einer darauffolgenden Synthese des zweiten Primer-Verlängerungsproduktes werden kopierbare Sequenzabschnitte des ersten Primer-Oligonukleotides von Polymerase kopiert, so dass wiederum in darauf folgenden Synthese-Zyklen eine vollkomplementäre Primer-Bindungsstelle innerhalb des zweiten Primer-Verlängerungsprodukte für die Bindung des ersten Primer-Oligonukleotides ausgebildet wird und in darauf folgenden Synthese-Zyklen zur Verfügung steht.

In einer weiteren Ausführungsform kann das erste Primer-Oligonukleotid im Rahmen der Vorbereitung einer Start-Nukleinsäurekette verwendet werden. Dabei kann ein solches erste Primer-Oligonukleotid an eine Nukleinsäure (z.B. eine einzelsträngige genomische DNA oder RNA oder ihre Äquivalente umfassend eine Zielsequenz) vorwiegend/bevorzugt sequenzspezifisch binden und in Anwesenheit einer Polymerase eine matrizenabhängige Primer-Verlängerungsreaktion initiieren. Die Bindungsposition ist dermaßen gewählt, dass das Primer-Verlängerungsprodukt eine gewünschte Zielsequenz umfasst. Durch die Verlängerung des ersten Primer-Oligonukleotides resultiert ein Nukleinsäure-Strang, welcher zu Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des ersten Primer-Oligonukleotides, weiterhin umfasst sie eine Zielsequenz oder ihre Äquivalente und eine Primer-Bindungsstelle für das zweite Primer-Oligonukleotid. Weitere Schritte sind im Abschnitt "Start-Nukleinsäurekette" erläutert.

Die Synthese des ersten Primer-Verlängerungsproduktes ist eine Primer-Verlängerungsreaktion und bildet einen Teilschritt in der Amplifikation. Die Reaktionsbedingungen während dieses Schrittes sind entsprechend angepasst. Die Reaktions-Temperatur und die Reaktions-Zeit sind dermaßen gewählt, dass die Reaktion erfolgreich stattfinden kann. Die jeweils bevorzugte Temperatur in diesem Schritt hängt von der verwendeten Polymerase ab und der Bindungsstärke des jeweiligen ersten Primer-Oligonukleotides an seine Primer-Bindungsstelle und umfasst beispielsweise Bereiche von 15°C bis 75°C, besser von 20 bis 65°C, bevorzugt von 25°C bis 65°C. Die Konzentration des ersten Primer-Oligonukleotides umfasst Bereiche von 0,01 µmol/l bis 50 µmol/l, besser von 0,1 mol/l bis 20 µmol/l, bevorzugt von 0,1 mol/l bis 10 µmol/l.

In einer Ausführungsform verlaufen alle Schritte der Amplifikation unter stringenten Bedingungen, welche Ausbildung von unspezifischen Produkten / Nebenprodukten verhindern bzw. verlangsamen. Zu solchen Bedingungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

Vorzugsweise sind Sequenzen des ersten, des zweiten Primer-Oligonukleotides und des Aktivator-Oligonukleotides dermaßen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander demassen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten bzw. zu unterstützen. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedehnte eigenkomplementäre Strukturen (Self-complement) umfassen.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des ersten Primer-Verlängerungsproduktes und Strangverdrängung durch Aktivator-Oligonukleotid bei unterschiedlichen Temperaturen.

Verwendung eines ersten Primer-Oligonukleotides in Kombination mit einem ersten Kompetitor-Pimer-Oligonukleotides:

### Bevorzugte Ausführungsformen des ersten Kompetitor-Primer-Oligonukleotids (Primer-1)

Ein erstes Kompetitor-primer-Oligonukleotid konkurriert mit dem ersten Primer-Oligonukleotid um die Bindung an ein Sequenzsegment einer Zielsequenz umfassenden Nukleinsäurekette, welche zumindest zwei Varianten einer Zielnukleinsäurekette umfasst.

Das erste Primer-Oligonukleotid bindet dabei an die gewünschte bzw. zu erwartende Sequenzvariante und wird bevorzugt unter stringenten Reaktionsbedingungen durch eine Polymerase verlängert. Das erste Kompetitor-Oligonukleotid bindet an die zweite potenzielle bzw. erwartende Sequenzvariante und wird bevorzugt unter stringenten Reaktionsbedingungen durch eine Polymerase verlängert. Es entstehen somit zwei Primer-Verlängerungsprodukte: das eine vom ersten Primer-Oligonukleotid, welches weiter in der Amplifikation vermehr werden soll und das andere vom ersten Kompetitor-Primer-Oligonukleotid, welches in einer Amplifikation nicht vermehrt werden soll.

Durch die Konkurrenz von zwei Primern um eine Primerbindungssequenz in der Zielnukleinsäurekette kann Spezifität der Analyse verbessert werden.

Die beiden Primer (der erste Primer und der erste Kompetitor-Primer) stellen somit allel-spezifische Primer in Bezug auf potenzielle bzw. zu erwartende Sequenzvarianten dar. Der wesentliche Unterschied besteht vor allem darin, dass der Kompetitor-Primer nicht in der Lage ist, unter Mitwirkung eines allel-spezifischen Aktivator-Oligonukleotdies eine Strang-Trennung zu initiieren.

**In einer Ausführungsform umfasst ein erstes Kompetitor-Primer-Oligonukleotid (Kompetitor-Primer-1)** eine Nukleinsäurekette welche mindestens folgende Eigenschaften einschließt:
- Einen ersten Kompetitor-Primer-Bereich im 3'-Segment des ersten Kompetitor-Primer-Oligonukleotides, der an einen Strang einer zu amplifizierenden Nukleinsäurekette im Wesentlichen sequenzspezifisch binden kann und somit konkurrierend mit dem ersten Primer-Oligonukleotid um seine Primer-Bindungsstelle wirken kann.
- 3'-Ende des Kompetitor-Primers, welches nach einer Bindung an die Primer-Bindungsstelle in der Zielsequenz sequenzspezifisch von Polymerase verlängert werden kann unter Ausbildung eines Kompetitor-Primer-Verlängerungsproduktes
- 3'-Ende des Kompetitor, welches von der "zweiten Blockierungs-Einheit" und/oder von der "vierten Blockierungs-Einheit" des Aktivator-Oligonukleotides am Verlängern durch eine Polymerase verhindert wird, wenn das erste Kompetitor-Primer Oligonukleotid an Aktivator-Oligonukleotid komplementär gebunden ist

Die Gesamtlänge des ersten Kompetitor-Primer-Oligonukleotides liegt zwischen 10 und 80, vorzugsweise zwischen 15 und 50, besser zwischen 20 und 30 Nukleotiden oder ihren Äquivalenten (z.B. Nukleotid-Modifikationen). Die Länge des 3'-Segments, welches eine komplementäre Bindung an eine zu erwartende Sequenzvariante einer Zielsequenz eingehen kann, umfasst im Wesentlichen die gleichen Bereiche, wie der erste Bereich des ersten Primers. Damit wird sichergestellt, dass beide Primer konkurrierend an die Zielsequenz binden können und diese Bindung etwa im gleichen Temperatur-Bereich erfolgt. Die konkurrierende Bindung und vor allem konkurrierende Primer-Verlängerung unter Verwendung von allel-spezifischen Sequenzsegmente von Zielnukleinsäure führt in der Regel zu einer weiteren Steigerung der Spezifität.

Die Struktur des ersten Kompetitor-Primer-Oligonukleotids ist dermaßen angepasst, dass es unter gewählten Reaktionsbedingungen eine reversible Bindung an Aktivator-Oligonukleotid eingehen kann. In einer Ausführungsform umfasst der erste Kompetitor-Primer Sequenzsegmente, welche mit dem ersten Bereich des Aktivator-Oligonukleotides keine komplementäre Bindung eingehen sollen. Somit kann dieses erste Kompetitor-Oligonukleotid nicht als Initiatiator eine Strang-Verdrängung durch ein Aktivator-Oligonukleotide auftreten.

In einer vorteilhaften Ausführungsform der Erfindung ist der erste Kompetitor-Primer-Oligonukleotid ein DNA-Oligonukleotid mit einer konventionellen 5'-3' Anordnung von Nukleotiden.

In einer weiteren Ausführungsform umfasst ein Kompetitor-Primer-Oligonukleotid weitere Sequenzen, die nicht an das Aktivator-Oligonukleotid binden. Diese Sequenzen können zu anderen Zwecken verwendet werden, z.B. zu Bindung an die feste Phase. Diese Sequenzen sind vorzugsweise am 5'-Ende des 3'-Segmentes lokalisiert.

### Der erste Primer-Bereich des ersten Kompetitor-Primer-Oligonukleotides

Die Sequenz-Länge liegt zwischen ca. 15-30 Nukleotiden, vorzugsweise zwischen 5 und 20 Nukleotiden, wobei die Sequenz vorwiegend komplementär zum 3'-Segment eines Stranges der in der Amplifikation zu unterdrückenden Allel-Variante einer Zielnukleinsäure ist. Der erste Primer-Bereich kann Nukleotid-Monomere einschließen, welche Funktion der Polymerase nicht oder nur unwesentlich beeinflussen, dazu gehören beispielsweise:
- natürliche Nukleotide (dA, dT, dC, dG etc.) oder deren Modifikationen ohne veränderte Basen-Paarung
- Modifizierte Nukleotide, 2-Amino-dA, 2-thio-dT oder andere Nukleotid-Modifikationen mit abweichender Basen-Paarung.

In einer Ausführungsform wird ein Allel-spezifischer erster Primer in Kombination mit einem allel-spezifischen Aktivator-Oligonukleotid und einem allel-spezifischen Kompetitor-Primer-Oligonukleotid verwendet.

### Bevorzugte Ausführungsformen des Aktivator-Oligonukleotids

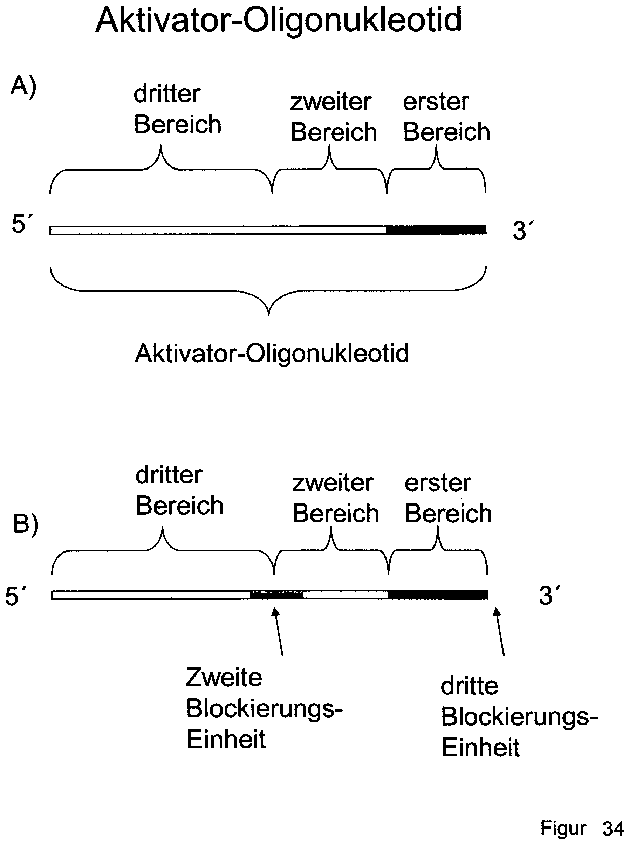

Ein Aktivator-Oligonukleotid (Fig. 34) umfasst:
- Einen ersten einzelsträngigen Bereich, welcher an den Polynukleotid-Schwanz des zweiten Bereichs des ersten Primer-Oligonukleotides binden kann,
- einen zweiten einzelsträngigen Bereich, welcher an den ersten Bereich des ersten Primer-Oligonukleotides im wesentlich komplementär binden kann,
- einen dritten einzelsträngigen Bereich, welcher zumindest zu einem Segment des Verlängerungsprodukts des ersten Primer-Verlängerungsprodukts im Wesentlichen komplementär ist,
und das Aktivator-Oligonukleotid dient nicht als Matrize für Primerverlängerung des ersten oder des zweiten Primer-Oligonukleotids.

Im Allgemeinen wird die Sequenz des dritten Bereichs des Aktivator-Oligonukleotides an die Sequenz der zu amplifizierenden Nukleinsäure angepasst, da diese als Matrize für die Reihenfolge der Nukleotide im Verlängerungsprodukt eines ersten Primers maßgeblich ist. Die Sequenz des zweiten Bereichs von Aktivator-Oligonukleotid wird an die Sequenz des ersten Primer-Bereiches angepasst. Die Struktur des ersten Bereichs des Aktivator-Oligonukleotides wird an die Sequenz des zweiten Bereichs des ersten Primer-Oligonukleotides angepasst, vor allem an die Beschaffenheit des Polynukleotid-Schwanzes.

Ein Aktivator-Oligonukleotid kann auch weitere Sequenz-Segmente einschließen, welche nicht zum ersten, zweiten oder dritten Bereich gehören. Diese Sequenzen können beispielsweise als flankierende Sequenzen am 3'- und am 5'-Ende angebracht werden. Vorzugsweise stören diese Sequenzsegmente die Funktion des Aktivator-Oligonukleotids nicht.

Die Struktur des Aktivator-Oligonukleotids weist bevorzugt folgende Eigenschaften auf:
Die einzelnen Bereiche sind untereinander kovalent gebunden. Die Bindung kann beispielsweise via konventionelle 5'-3' Bindung erfolgen. Es kann beispielsweise eine Phospho-Diesterbindung oder eine Nuklease-resistente Phospho-Thioester Bindung verwendet werden.

Ein Aktivator-Oligonukleotid kann mittels seines ersten Bereichs an den Polynukleotid-Schwanz des ersten Primer-Oligonukleotides binden, wobei die Bindung vorwiegend durch Hybridisierung komplementärer Basen vermittelt wird. Die Länge dieses ersten Bereichs beträgt 3 - 80 Nukleotide, vorzugsweise 4 - 40 Nukleotide, besonders bevorzugt 6 - 20 Nukleotide. Das Ausmaß an Sequenzübereinstimmung zwischen der Sequenz des ersten Bereichs des Aktivator-Oligonukleotids und der Sequenz des zweiten Bereichs des ersten Primer-Oligonukleotide kann zwischen 20 % und 100 %, vorzugsweise zwischen 50 % und 100 %, besonders bevorzugt zwischen 80 % und 100 % liegen. Die Bindung des ersten Bereichs des Aktivator-Oligonukleotids sollte vorzugsweise spezifisch an den zweiten Bereich des ersten Primer-Oligonukleotid unter Reaktionsbedingungen erfolgen.

Die Sequenz des ersten Bereichs des Aktivator-Oligonukleotids ist vorzugsweise dermaßen gewählt, dass die Anzahl von komplementären Basen, welche mit dem zweiten Bereich des ersten Primer-Oligonukleotids komplementär binden können, zwischen 1 und 40, besser zwischen 3 und 20, bevorzugt zwischen 6 und 15 liegt.

Da das Aktivator-Oligonukleotid keine Matrize für Polymerase darstellt, kann es Nukleotid-Modifikationen einschließen, welche die Polymerase-Funktion nicht unterstützen, welche sowohl Basenmodifikationen und/oder Zucker-Phosphat-Rückgrat-Modifikationen sein können. Das Aktivator-Oligonukleotid kann beispielsweise in seinem ersten Bereich Nukleotide und/oder Nukleotid-Modifikationen einschließen, welche aus der folgenden Liste ausgewählt sind: DNA, RNA, LNA ("locked nucleic acids" Analoga mit 2'-4' Brückenbindung im Zuckerrest), UNA ("unlocked nucleid acids" ohne Bindung zwischen 2'-3'-Atomen des Zuckerrestes), PNA ("peptide nucleic acids" Analoga), PTO (phosphorothioate), Morpholino-Analoga, 2'-O-Alkyl-RNA Modifikationen (wie 2'-OMe, 2'-O Propargyl, 2'-O-(2-Methoxyethyl), 2'-O-Propyl-Amin), 2'-Halogen-RNA, 2'-Amino-RNA etc. Diese Nukleotide oder Nukleotid-Modifikationen sind untereinander beispielsweise durch konventionelle 5'-3'-Bindung oder 5'-2' Bindung verknüpft. Es kann beispielsweise eine Phospho-Diesterbindung oder eine nuklease resistente Phospho-Thioester Bindung verwendet werden.

Das Aktivator-Oligonukleotid kann in seinem ersten Bereich Nukleotide und/oder Nukleotid-Modifikationen einschließen, wobei die Nukleobasen aus der folgenden Liste ausgewählt sind: Adenine und seine Analoga, Guanine und seine Analoga, Cytosine und seine Analoga, Uracil und seine Analoga, Thymine und seine Analoga, Inosine oder andere Universalbasen (z.B. Nitroindol), 2-Amino-Adenin und seine Analoga, Iso-Cytosine und seine Analoga, Iso-Guanine und seine Analoga.

Das Aktivator-Oligonukleotid kann in seinem ersten Bereich Nicht-Nukleotid-Verbindungen einschließen, welche aus der folgenden Liste ausgewählt sind: Interkallierende Stoffe, welche Bindungsstärke zwischen dem Aktivator-Oligonukleotid und dem ersten Primer-Oligonukleotid beeinflussen können, z.B. MGB, Naphthalene etc. Gleiche Elemente können auch im zweiten Bereich des ersten Primers verwendet werden.

Das Aktivator-Oligonukleotid kann in seinem ersten Bereich Nicht-Nukleotid-Verbindungen einschließen, z.B. Linker wie C3, C6, HEG Linker, welche einzelne Segmente des ersten Bereichs untereinander verknüpfen.

Das Aktivator-Oligonukleotid kann mittels seines zweiten Bereichs an den ersten Primer-Bereich des ersten Primer-Oligonukleotids binden, wobei die Bindung im Wesentlichen durch Hybridisierung komplementärer Basen vermittelt wird.
Die Länge des zweiten Bereichs des Aktivator-Oligonukleotids ist an die Länge des ersten Bereichs des ersten Primer-Oligonukleotid angepasst und stimmt mit dieser vorzugsweise überein. Sie liegt zwischen ca. 3-30 Nukleotiden, vorzugsweise zwischen 5 und 20 Nukleotiden. Die Sequenz des zweiten Bereichs von Aktivator-Oligonukleotid ist vorzugsweise komplementär zum ersten Bereich des ersten Primer-Oligonukleotid. Das Maß der Übereinstimmung in Komplementarität liegt zwischen 80 % und 100 %, vorzugsweise zwischen 95 % und 100 %, bevorzugt bei 100 %. Der zweite Bereich von Aktivator-Oligonukleotid schließt vorzugsweise Nukleotid-Modifikationen ein, welche Polymerase bei Verlängerung des ersten Primer-Oligonukleotides verhindern allerdings Ausbildung von komplementären Doppelsträngen nicht blockieren oder nicht wesentlich verhindern, beispielsweise 2'-O-Alkyl RNA-Analoga (z.B. 2'-O-Me, 2'-O-(2-Methoxyethyl), 2'-O-Propyl, 2'-O-Propargyl Nukleotid-Modifikationen), LNA, PNA oder Morpholino Nukleotid-Modifikationen. Einzelne Nukleotid-Monomere sind vorzugsweise via 5'-3'-Bindung verknüpft, aber auch alternative 5'-2'-Bindung zwischen Nukleotid-Monomeren kann verwendet werden.

Die Sequenz-Länge und ihre Beschaffenheit des ersten und des zweiten Bereichs des Aktivator-Oligonukleotides sind vorzugsweise dermaßen gewählt, dass die Bindung dieser Bereiche an das erste Primer-Oligonukleotid unter Reaktionsbedingungen zumindestens in einem Reaktions-Schritt des Verfahrens reversibel ist. Das bedeutet, dass Aktivator-Oligonukleotid und das erste Primer-Oligonukleotid zwar spezifisch an einander binden können, diese Bindung soll allerdings nicht zur Ausbildung eines unter Reaktionsbedingungen dauerhaft stabilen Komplexes aus beiden Elementen führen.

Vielmehr soll ein Gleichgewicht zwischen einer gebunden Komplex-Form aus Aktivator-Oligonukleotid und dem ersten Primer-Oligonukleotid und einer freien Form von einzelnen Komponenten unter Reaktionsbedingungen zumindest in einem Reaktions-Schritt angestrebt bzw. ermöglicht werden. Damit wird sichergestellt, dass zumindest ein Anteil der ersten Primer-Oligonukleotide unter Reaktionsbedingungen in freier Form vorliegt und mit der Matrize interagieren kann, um eine Primer-Verlängerungsreaktion zu initiieren. Andererseits, wird damit sichergestellt werden, dass entsprechende Sequenzbereiche des Aktivator-Oligonukleotides zu Bindung mit einem verlängerten Primer-Oligonukleotid zur Verfügung stehen.

Durch Temperatur-Wahl während der Reaktion kann der Anteil von freien, einzelsträngigen und somit reaktionsfähigen Komponenten beeinflusst werden: durch Absenkung der Temperatur binden erste Primer-Oligonukleotide an die Aktivator-Oligonukleotide, so dass beide Teilnehmer einen komplementären doppelsträngigen Komplex binden. Damit kann Konzentration einzelsträngiger Formen einzelner Komponenten abgesenkt werden. Eine Erhöhung der Temperatur kann zu Dissoziation beider Komponenten in einzelsträngige Form führen. Im Bereich der Schmelz-Temperatur des Komplexes (Aktivator-Oligonukleotid / erstes Primer-Oligonukleotid) liegen ca. 50 % der Komponenten in einzelsträngiger Form und ca. 50 % als doppelsträngiger Komplex vor. Durch Verwendung entsprechender Temperaturen kann somit die Konzentration einzelsträngiger Formen im Reaktionsgemisch beeinflusst werden.

Bei Ausführungsformen des Amplifikationsverfahrens, welche Temperatur-Wechsel zwischen einzelnen Reaktions-Schritten umfassen, können gewünschte Reaktionsbedingungen während entsprechender Reaktions-Schritte herbeigeführt werden. Beispielsweise durch Verwendung von Temperatur-Bereichen etwa in Höhe von Schmelztemperatur von Komplexen aus Aktivator-Oligonukleotid / erstes Primer-Oligonukleotid können Anteile von jeweils freien Formen einzelner Komponenten beeinflusst werden. Dabei führt die verwendete Temperatur zu einer Destabilisierung von Komplexen umfassend Aktivator-Oligonukleotid / erster Primer-Oligonukleotid, so dass während dieses Reaktionsschrittes einzelne Komplex-Komponente zumindest vorübergehend einzelsträngig werden und damit in die Lage versetzt werden, mit anderen Reaktionspartnern zu interagieren. Beispielsweise kann erster Sequenzbereich des Aktivator-Oligonukleotides aus dem doppelsträngigen Komplex mit einem nichtverlängerten ersten Primer freigesetzt werden und kann somit mit dem zweiten Sequenzbereich eines verlängerten ersten Primer-Oligonukleotides interagieren und damit eine Strangverdrängung initiieren. Andererseits, führt Freisetzung eines ersten, nicht verlängerten Primer-Oligonukleotides aus einem Komplex umfassend Aktivator-Oligonukleotid / erster Primer-Oligonukleotid dazu, dass der erster Primer-Bereich einzelsträngig wird und somit mit der Matrize interagieren kann, so dass eine Primer-Verlängerung durch eine Polymerase initiiert werden kann.

Die verwendete Temperatur muss dabei nicht exakt der Schmelztemperatur des Komplexes aus Aktivator-Oligonukleotid / erstes Primer-Oligonukleotid entsprechen. Es genügt, wenn Temperatur in einem Reaktionsschritt etwa im Bereich der Schmelztemperatur verwendet wird. Beispielsweise umfasst die Temperatur in einem der Reaktionsschritte Bereiche von Tm +/- 10°C, besser Tm +/- 5°C, bevorzugt Tm +/- 3°C des Komplexes aus Aktivator-Oligonukleotid / erstes Primer-Oligonukleotid.

Eine solche Temperatur kann beispielsweise im Rahmen des Reaktions-Schrittes eingestellt werden, welcher eine sequenzspezifische Strandverdrängung durch das Aktivator-Oligonukleotid umfasst.

Bei Ausführungsformen des Amplifikationsverfahrens, welche keinen Temperatur-Wechsel zwischen einzelnen Reaktions-Schritten umfassen und Amplifikation unter isothermalen Bedingungen verläuft, werden über die Gesamtdauer der Amplifikationsreaktion Reaktionsbedingungen aufrechterhalten, bei welchen ein Gleichgewicht zwischen einer Komplex-Form aus Aktivator-Oligonukleotid und dem ersten Primer-Oligonukleotid und einer freien Form von einzelnen Komponenten möglich ist.

Das Verhältnis zwischen einer Komplex-Form aus Aktivator-Oligonukleotid und dem ersten Primer-Oligonukleotid und freien Formen einzelner Komponenten kann sowohl durch Reaktionsbedingungen (z.B. Temperatur und Mg2+ Konzentration) als auch durch Strukturen und Konzentrationen einzelner Komponenten beeinflusst werden.

Die Sequenz-Länge und ihre Beschaffenheit des ersten und des zweiten Bereichs des Aktivator-Oligonukleotides sind in einer Ausführungsform so gewählt, dass unter gegebenen Reaktionsbedingungen (z.B. im Reaktionsschritt einer Strangverdängung durch das Aktivator-Oligonukleotid) das Verhältnis zwischen einem Anteil eines freien Aktivator-Oligonukleotids und einem Anteil eines Aktivator-Oligonukleotid im Komplex mit einem ersten Primer-Oligonukleotid folgende Bereiche umfasst: von 1:100 bis 100:1, vorzugsweise von 1:30 bis 30:1, besonders bevorzugt von 1:10 bis 10:1. Das Verhältnis zwischen einem Anteil eines freien ersten Primer-Oligonukleotid und einem Anteil eines ersten Primer-Oligonukleotids im Komplex mit einem Aktivator-Oligonukleotid umfasst Bereiche von 1:100 bis 100:1, vorzugsweise von 1:30 bis 30:1, besonders bevorzugt von 1:10 bis 10:1.

In einer Ausführungsform ist die Konzentration des ersten Primer-Oligonukleotides höher als die Konzentration des Aktivator-Oligonukleotides. Dadurch liegt ein Überschuß des ersten Primers in der Reaktion vor und Aktivator-Oligonukleotid muss für seine Wirkung aus der Bindung mit dem ersten Primer durch Wahl entsprechender Reaktionstemperatur freigesetzt werden. Im Allgemeinen erfolgt das durch eine Temperaturerhöhung, bis hinreichende Konzentrationen von freien Formen des Aktivator-Oligonukleotids vorliegen.

In einer weiteren Ausführungsform ist die Konzentration des ersten Primer-Oligonukleotides niedriger als die Konzentration des Aktivator-Oligonukleotides. Dadurch liegt ein Überschuss des Aktivator-Oligonukleotids vor und das erste Primer-Oligonukleotid muss für seine Wirkung aus der Bindung mit dem Aktivator-Oligonukleotid durch Wahl entsprechender Reaktionstemperatur freigesetzt werden. Im Allgemeinen erfolgt das durch eine Temperaturerhöhung, bis hinreichende Konzentrationen von freien Formen des ersten Primer-Oligonukleotids vorliegen.

Bei isothermalen Bedingungen liegt ein Gleichgewicht vor: gewisse Anteile des ersten Primer-Oligonukleotides und Aktivator-Oligonukleotides sind aneinander gebunden, andere dagegen sind als einzelsträngige Form in der Reaktion vorhanden.

Das Aktivator-Oligonukleotid kann mittels seines dritten Bereichs an mindestens ein Segment des spezifisch synthetisierten Verlängerungsprodukts des ersten Primer-Oligonukleotids binden. Die Bindung erfolgt bevorzugt durch Hybridisierung komplementärer Basen zwischen dem Aktivator-Oligonukleotid und dem von Polymerase synthetisierten Verlängerungsprodukt.

Zu Unterstützung der Strangverdrängungsreaktion soll die Sequenz des dritten Bereichs vorzugsweise eine hohe Komplementarität zum Verlängerungsprodukt aufweisen. In einer Ausführungsform ist die Sequenz des dritten Bereichs zu 100 % dem Verlängerungsprodukt komplementär.

Die Bindung des dritten Bereichs erfolgt vorzugsweise an das Segment des Verlängerungsproduktes, welches unmittelbar an den ersten Bereich des ersten Primer-Oligonukleotides anschließt. Somit liegt das Segment des Verlängerungsprodukts vorzugsweise im 5'-Segment des gesamten Verlängerungsprodukts des ersten Primer-Oligonukleotids.

Die Bindung des dritten Bereichs des Aktivator-Oligonukleotids erfolgt vorzugsweise nicht über die gesamte Länge des Verlängerungsprodukts des ersten Primer-Oligonukleotides. Vorzugsweise bleibt ein Segment am 3'-Ende des Verlängerungsproduktes ungebunden. Dieses 3'-ständige Segment ist für die Bindung des zweiten Primer-Oligonukleotides notwendig.

Die Länge des dritten Bereichs ist entsprechend dermaßen angepasst, dass der dritte Bereich zum einen an das 5'-ständige Segment des Verlängerungsproduktes bindet, andererseits das 3'-ständige Segment des Verlängerungsproduktes nicht bindet.

Die Gesamtlänge des dritten Bereichs des Aktivator-Oligonukleotids beträgt von 2 bis 100, vorzugsweise von 6 bis 60, besonders bevorzugt von 10 bis 40 Nukleotide oder ihrer Äquivalente. Das Aktivator-Oligonukleotid kann mit dem Segment des Verlängerungsproduktes über diese Länge komplementäre Bindung eingehen und damit dieses 5'-ständige Segment des Verlängerungsprodukts aus der Bindung mit seinem komplementären Matrizen-Strang verdrängen.

Die Länge des 3'-ständigen Segments des Verlängerungsproduktes, welches nicht vom Aktivator-Oligonukleotid gebunden wird, umfasst beispielsweise Bereiche zwischen 5 und 200, besser zwischen 5 und 100, noch besser zwischen 5 und 60 Nukleotide, vorzugsweise zwischen 10 und 40, bevorzugt zwischen 15 und 30 Nukleotide.

Dieses 3'-ständige Segment des Verlängerungsprodukts wird nicht vom Aktivator-Oligonukleotid aus der Bindung mit dem Matrizenstrang verdrängt. Auch bei vollständig gebundenem dritten Bereich des Aktivator-Oligonukleotids an sein komplementäres Segment des Verlängerungsproduktes kann das erste Primer-Verlängerungsprodukt via sein 3'-ständiges Segment mit dem Matrizenstrang gebunden bleiben. Die Bindungsstärke dieses Komplexes ist vorzugsweise so gewählt, dass er unter Reaktionsbedingungen (Schritt e)) beispielsweise spontan dissoziieren kann. Dies kann bespielsweise dadurch erreicht werden, dass die Schmelztemperatur dieses Komplexes aus dem 3'-ständigen Segment des Verlängerungsprodukts des ersten Primer-Oligonukleotid und seinem Matrizenstrang etwa im Bereich der Reaktionstemperatur liegt oder unterhalb der Reaktionstemperatur bei einem entsprechenden Reaktionsschritt (Reaktionsschritt e). Bei geringer Stabilität dieses Komplexes im 3'-Segment des Verlängerungsprodutkes führt eine vollständige Bindung des dritten Bereichs des Aktivator-Oligonukleotid an das 5'-ständige Segment des Verlängerungsproduktes zu einer schnellen Dissoziation der ersten Primer-Verlängerungsprodukts von seinem Matrizenstrang.

Das Aktivator-Oligonukleotid hat insgesamt eine passende Struktur, um seine Funktion auszuführen: unter entsprechenden Reaktionsbedingungen ist es in der Lage, das verlängerte erste Primer-Oligonukleotid aus der Bindung mit dem Matrizenstrang sequenzspezifisch zu verdrängen, wodurch der Matrizenstrang in einzelsträngige Form überführt wird und somit für weitere Bindungen mit einem neuen ersten Primer-Oligonukleotid und deren zielsequenzspezifische Verlängerung durch Polymerase zur Verfügung steht.

Um die Funktion der Strangverdrängung zu erfüllen, sollten Bereiche ein, zwei und drei des Aktivator-Oligonukleotids vorwiegend in einzelsträngiger Form unter Reaktionsbedingungen vorliegen. Daher sollten doppelsträngige selbstkomplementäre Strukturen (z.B. Hairpins) in diesen Bereichen nach Möglichkeit vermieden werden, da sie die Funktionalität des Aktivator-Oligonukleotides herabsetzen können.

Das Aktivator-Oligonukleotid soll im erfindungsgemäßen Verfahren nicht als Matrize auftreten, daher soll das erste Primer-Oligonukleotid, wenn angelagert an das Aktivator-Oligonukleotid unter Reaktionsbedingungen, nicht von Polymerase verlängert werden.

Dies wird vorzugsweise durch Verwendung von Nukleotid-Modifikationen erreicht, welche die Polymerase am Kopieren des Stranges hindern. Vorzugsweise bleibt das 3'-Ende des ersten Primer-Oligonukleotid nicht verlängert, wenn das erste Primer-Oligonukleotid an das Aktivator-Oligonukleotid unter Reaktionsbedingungen bindet.

Das Ausmaß der Blockade/Hinderung/Verlangsamung/Erschwerung der Reaktion kann zwischen vollständiger Ausprägung dieser Eigenschaft (z.B. 100 % Blockade unter gegebenen Reaktionsbedingungen) und einer partiellen Ausprägung dieser Eigenschaft liegen (z.B. 30-90 % Blockade unter gegebenen Reaktionsbedingungen). Bevorzugt werden Nukleotid-Modifikationen, welche einzeln oder in einer Reihe aneinander gekoppelt (z.B. als Sequenzfragment bestehend aus modifizierten Nukleotiden)mehr als 70 %, bevorzugt mehr als 90 %, bevorzugter mehr als 95 %, und besonders bevorzugt 100 % die Verlängerung eines ersten Primers verhindern können.
Die Nukleotid-Modifikationen können Basen-Modifikationen und/oder Zucker-Phosphat-Rest Modifikationen umfassen. Die Zucker-Phosphat-Modifikationen sind bevorzugt, da durch Kombination mit konventionellen Nukleobasen eine beliebige komplementäre Sequenz eines Aktivator-Oligonukleotides zusammengestellt werden kann. Die Nukleotide mit Modifikationen im Zucker-Phosphat-Rest, welche zu Hinderung bzw. Blockade der Synthese der Polymerase führen können, schließen beispielsweise ein: 2'-O-Alkyl-Modifikationen (z.B. 2'-O-Methyl, 2'-O-(2-Methoxyethyl), 2'-O-Propyl, 2'-O-Propargyl Nukleotid-Modifikationen), 2'-Amino-2'-Deoxy-Nukleotid-Modifikationen, 2'-Amino-Alkyl-2'-Deoxy-Nukleotid-Modifikationen, PNA, Morpholino-Modifikationen usw.

Die Blockade kann sowohl durch eine einzelne Nukleotid-Modifikation erfolgen oder erst durch Kopplung von mehreren Nukleotid-Modifikationen in Reihe erfolgen (z.B. als Sequenzfragment bestehend aus modifizierten Nukleotiden). Beispielsweise können mindestens 2, vorzugsweise mindestens 5, besonders bevorzugt mindestens 10 solcher Nukleotid-Modifikationen nebeneinander im Aktivator-Oligonukleotid gekoppelt sein.

Ein Aktivator-Oligonukleotid kann eine einheitliche Art von Nukleotid-Modifikationen aufweisen oder auch mindestens zwei verschiedene Arten der Nukleotid-Modifizierung umfassen.

Die Position solcher Nukleotid-Modifikationen im Aktivator-Oligonukleotid soll vorzugsweise die Polymerase daran hindern, das 3'-Ende eines am Aktivator-Oligonukleotid gebundenen ersten Primer-Oligonukleotids zu verlängern.

In einer Ausführungsform sind solche Nukleotid-Modifikationen im zweiten Bereich des Aktivator-Oligonukleotids lokalisiert. In einer weiteren Ausführungsform sind solche Nukleotid-Modifikationen im dritten Bereich des Aktivator-Oligonukleotids lokalisiert. In einer weiteren Ausführungsform sind solche Nukleotid-Modifikationen im zweiten und im dritten Bereich des Aktivator-Oligonukleotids lokalisiert.

Beispielsweise besteht der zweite Bereich des Aktivator-Oligonukleotids zu mindestens 20 % seiner Positionen aus solchen Nukleotid-Modifikationen, vorzugsweise zu mindestens 50%.

Beispielsweise besteht der dritte Bereich des Aktivator-Oligonukleotids zu mindestens 20 % seiner Positionen aus solchen Nukleotid-Modifikationen, vorzugsweise zu mindestens 50 %, besonders bevorzugt zu mindestens 90%. In einer Ausführungsform umfasst der gesamte dritte Bereich Nukleotid-Modifikationen, welche eine Polymerase daran hindern, einen an einen solchen Bereichen gebundenen Primer unter Verwendung des Aktivator-Oligonukleotides als Matrize zu verlängern. In einer weiteren Ausführungsform umfasst der gesamte dritte und zweite Bereich solche Nukleotid-Modifikationen. In einer weiteren Ausführungsform umfasst der gesamte erste, zweite und dritte Bereich solche Nukleotid-Modifikationen. Somit kann das Aktivator-Oligonukleotid vollständig aus solchen Nukleotid-Modifikationen bestehen. Solch modifizierten Aktivator-Oligonukleotide können beispielsweise bei Multiplex-Analysen verwendet werden, bei welchen weitere Primer verwendet werden. Dadurch soll verhindert werden, dass es zu unbeabsichtigten Primer-Verlängerungs-Reaktionen an einem oder mehreren Aktivator-Oligonukleotiden kommt.

Die Sequenz der Nukleobasen von diesen Nukleotid-Modifikationen ist den Anforderungen an die Sequenz im jeweiligen Bereich angepasst.

Den restlichen Anteil können beispielsweise natürliche Nukleotide ausmachen, oder Nukleotid-Modifikationen, welche die Polymerasen-Funktion gar nicht oder nur unwesentlich hindern z.B. DNA-Nukleotide, PTO-Nukleotide, LNA-Nukleotide, RNA-Nukleotide. Dabei können weitere Modifikationen, beispielsweise Basenmodifikationen wie 2-Amino-Adenosin, 2-Aminopurine, 5-Methyl-Cytosine, Inosine, 5-Nitroindole, 7-Deaza-Adenosin, 7-Deaza-Guanosin, 5-Propyl-Cytosin, 5-Propyl-Uridin oder Nicht-Nukleotid-Modifikationen wie Farbstoffe, oder MGB-Modifikationen etc. z.B. zur Anpassung von Bindungsstärken von einzelnen Bereichen der Aktivator-Oligonukleotide verwendet werden. Die einzelnen Nukleotid-Monomere können via konventionelle 5'-3'-Bindung untereinander gekoppelt werden oder auch abweichend via 5'-2'-Bindung.

Ein Segment des Aktivator-Oligonikleotids mit Nukleotid-Modifikationen, welche Verlängerung von 3'-Ende eines an Aktivator-Oligonukleotid gebundenen ersten Primer-Oligonukleotids durch Polymerase verhindern, wird als "zweite Blockierungs-Einheit" bezeichnet. Die Länge dieses Segments kann zwischen 1 bis 50 Nukletid-Modifikationen einschließen, vorzugsweise zwischen 4 und 30. Dieses Segment kann beispielsweise dermaßen im Aktivator-Oligonukleotid lokalisiert sein, dass das 3'-Ende des gebundenen ersten Primer-Oligonukleotids in diesem Segment liegt. Somit kann dieses Segment die Bereiche zwei und drei überspannen. Bei Verwendung weiterer Primer (z.B. Kompetitor-Primer), welche ebenfalls an das Aktivator-Oligonukleotid komplementär binden können, können weitere Blockierungs-Einheiten eingeführt werden, beispielsweise eine vierte Blockierungs-Einheit, welche an der gleichen Position im Aktivator-Oligonukleotid liegen kann wie die zweite Blockierungs-Einheit oder eine abweichende Position umfasst. Beispielsweise kann die vierte Blockierungs-Einheit in 5'-Richtung von der zweiten Blockierungs-Einheit liegen. Die Primer-Blockade durch eine solche vierte Blockierungs-Einheit erfolgt nach dem gleichen Prinzip wie bei der zweiten Blockierungs-Einheit: ein Primer, welcher zwar in der Lage ist, an das Aktivator-Oligonukleotid komplementär zu binden, wird von Polymerase nicht verlängert. Somit wird verhindert, dass Aktivator-Oligonukleotid für weitere Primer, z.B. einem Kompetitor-Primer, als Matrize dient.
In einer Ausführungsform werden vorzugsweise keine Linker-Strukturen oder Spacer-Strukturen, wie C3, C6, HEG-Linker zur Verhinderung der Verlängerung des 3'-Ende eines an das Aktivator-Oligonukleotid gebundenen ersten Primer-Oligonukleotids verwendet.

Das Aktivator-Oligonukleotid kann außer Bereichen eins, zwei und drei noch weitere Sequenzsegmente umfassen, welche beispielsweise im 5'-Segment oder 3'-Segment des Aktivator-Oligonukleotid die oben genannten Bereiche flankieren. Solche Sequenzelemente können beispielsweise für weitere Funktionen verwendet werden, wie beispielsweise Interaktion mit Sonden, Bindung an feste Phase etc. Solche Bereiche stören vorzugsweise die Funktion der Bereiche eins bis drei nicht. Die Länge dieser flankierenden Sequenzen kann beispielsweise zwischen 1 bis 50 Nukleotide betragen. Weiterhin kann ein Aktivator-Oligonukleotid mindestes ein Element zu Immobilisierung an einer festen Phase umfassen, z.B. ein Biotin-Rest. Weiterhin kann ein Aktivator-Oligonukleotid mindestes ein Element zu Detektion umfassen, z.B. ein Fluoreszenz-Farbstoff.

In Anwesenheit von einem Aktivator-Oligonukleotid werden neusynthetisierte Sequenzen auf ihre Sequenz-Inhalte durch Interaktion mit dem Aktivator-Oligonukleotid überprüft.
- Bei korrekter Übereinstimmung mit vorgegebenen Sequenzinhalten des Aktivator-Oligonukleotides erfolgt eine Strangverdrängung, wobei die Matrizen-Stränge von neusynthetisierten Strängen abgelöst werden. Dabei werden Primer-Bindungsstellen in einzelsträngigen Zustand überführt und stehen für neue Interaktion mit Primern und somit für eine weitere Synthese zur Verfügung. Somit wird das System aus beiden Primer-Verlängerungsprodukten in einen aktiven Zustand versetzt. Das Aktivator-Oligonukleotid hat somit eine aktivierende Wirkung auf das System.
- Bei fehlender Übereinstimmung mit vorgegebenen Sequenzinhalten des Aktivator-Oligonukleotides wird Strangverdrängung der Matrizen-Stränge von neusynthetisierten Strängen beieinflusst. Die Strangverdrängung und /oder Ablösung wird entweder quantitativ verlangsamt oder komplett aufgehoben. Es kommt somit gar nicht oder seltener zu Überführung von Primer-Bindungsstellen in einzelsträngigen Zustand. Somit stehen für neue Interaktion mit Primern gar keine oder quantitativ gesehen wenige Primer-Bindungsstellen zur Verfügung. Somit wird das System aus beiden Primer-Verlängerungsprodukten seltener in einen aktiven Zustand versetzt bzw. aktiver Zustand wird gar nicht erreicht.

Die Effizienz der Doppelstrang-Öffnung der neusynthetisierten Primer-Verlängerungsprodukte nach jedem einzelnen Synthese-Schritt wirkt sich auf die potenziell erreichbare Ausbeuten in darauf folgenden Zyklen aus: je weniger freier/einzelsträngiger Primer-Bindungsstellen einer zu amplifizierenden Nukleinsäurekette zu Beginn eines Synthese-Schrittes zu Verfügung gestellt werden, um so geringer ist die Anzahl von neusynthetisierten Stränge der zu amplifizierenden Nukleinsäurekette in diesem Schritt. Anders ausgedrückt: Die Ausbeute eines Synthese-Zyklus ist proportional der Menge von Primer-Bindungsstellen, welche für Interaktion mit entsprechenden komplementären Primern zur Verfügung stehen. Insgesamt kann dadurch ein Regelungs-Kreis realisiert werden.

Dieser Regelungs-Kreis entspricht einer Echtzeit-Kontrolle (real-time / on-line control) von synthetisierten Fragmenten: Sequenz-Kontrolle erfolgt im Reaktionsansatz während Amplifikation stattfindet. Diese Sequenzkontrolle erfolgt nach einem vorgegebenen Muster und das Oligonukleotid-System (durch Strang-Öffnende Wirkung von Aktivator-Oligonukleotid) kann ohne äußere Einmischung zwischen "korrekten" und "nicht-korrekten" Zuständen entscheiden. Im korrekten Zustand wird die Synthese von Sequenzen fortgesetzt, im nicht-korrekten Zustand wird die Synthese entweder verlangsamt oder vollständig verhindert. Die resultierenden Unterschiede in Ausbeuten an "korrekten" und "nicht-Korrekten" Sequenzen nach jedem Schritt wirken sich auf die gesamte Amplifikation aus, welche eine Vielzahl solcher Schritte umfasst.

Bei einer exponentiellen Amplifikation ist diese Abhängigkeit exponentiell, so dass sogar geringfügige Unterschiede in der Effizienz im Rahmen eines einzelnen Synthese-Zyklus aufgrund Sequenzunterschiede eine signifikante zeitliche Verzögerung der Gesamtamplifikation bedeuten können, bzw. das vollständige Ausbleiben einer detektierbaren Amplifikation in einem vorgegebenen Zeitrahmen bewirken.

Dieser Effekt der Echtzeit-Kontrolle der neusynthetisierten Nukleinsäureketten ist verbunden mit dem Einsatz des Aktivator-Oligonukleotides und der Einfluss von Aktivator-Oligonukleotid im Rahmen einer Amplifikation geht somit über die Länge von Primer-Oligonukleotide deutlich hinaus.

### Sequenz-Varianten-spezifische Aktivator-Oligonukleotide:

Zielsequenz-spezifisches Aktivator-Oligonukleotid wird vorzugsweise dermaßen in seinen Bereichen konstruiert, dass es in der Lage ist, bei Amplifikation von zumindest einer vordefinierten Sequenz-Variante einer Zielsequenz mitzuwirken.

In einer bevorzugten Form wird ein Amplifikations-System umfassend mindestens ein Aktivator-Oligonukleotid dermaßen gestaltet, dass die zu erwartende Position einer Allel-Variante einer Zielsequenz einem korrespondierendes Segment bzw. einer korrespondierende Position im Aktivator-Oligonukleotid entspricht.

Allel-spezifisches Aktivator-Oligonukleotid wird vorzugsweise dermaßen konstruiert, dass es in der Lage ist, die Amplifikation einer Sequenz-Varianten einer Zielsequenz (z.B. Allel 1) bevorzugt zu bewirken, wobei bei einer anderen Sequenz-Variante dergleichen Zielsequenz (z.B. Allel 2) verläuft Amplifikation gar nicht bzw. nur mit verminderter Effizient bzw. findet in der vorgegebener Zeit gar nicht statt, bzw. resultiert in einer nicht für eine Detektion hinreichenden Ausbeute an Amplifikationsprodukten.

Ein Allel-spezifisches Aktivator-Oligonukleotid wird somit nicht nur Zielsequenzspezifisch, sonder auch allel-spezifisch bereitgestellt. Ein solches Aktivator-Oligonukleotid umfasst somit Zielsequenzspezifische Sequenz-Segmente als auch allel-spezifische Sequenzsegmente. Je nach Komplexität eines polymorphen Lokus einer Zielsequenz können somit mehrere, für jeweilige allel-Variante einer Zielsequenz spezifische Aktivator-Oligonukleotide konstruiert werden.

Vorzugsweise umfasst ein Aktivator-Oligonukleotid mindestens ein Sequenzsegment, welches zu einer spezifischen Allel-Variante einer Zielsequenz komplementäre Sequenzzusammensetzung aufweist. Weiterhin umfasst ein Aktivator-Oligonukleotid vorzugsweise mindestens ein Sequenzsegment, welches für alle Sequenzvarianten einer Zielsequenz einheitliche komplementäre Sequenz umfasst. In einer Ausführungsform umfasst ein Aktivator-Oligonukleotid, welches mindestens ein Sequenzsegment umfasst, welches komplementär an eine Variante eines polymorphen Lokus einer Zielsequenz komplementär binden kann.

In einer Ausführungsform werden mehrere Aktivator-Oligonukleotide bereitgestellt, welche jeweils allel-spezifische Sequenzsegmente umfassen.

Die Länge eines zum polymorphen Lokus einer Start-Nukleinsäureke vollständig komplementären Sequenzsegmentes eines Aktivator-Oligonukleotides kann Bereiche von einem Nukleotid bis zu 100 Nukleotiden, besser von einem Nukleotid bis zu 50 Nukleotiden, bevorzugt von einem Nukleotid bis zu 20 Nukleotiden umfassen. Die Länge von mindestens einem Sequenzsegment eines Aktivator-Oligonukleotides, welches zu einheitlichen Sequenzsegmenten einer Zielsequenz (erstes und zweites einheitliches Sequenzsegment einer Start-Nukleinsäure) komplementär ist, kann folgende Bereiche umfassen: von 4 bis 100 Nukleotide, besser von 6 bis 100 Nukleotide, bevorzugt von 8 bis 50 Nukleotide.

In einer Ausführungsform wird ein Sequenzsegment eines Aktivator-Oligonukleotide, welches zu mindestens einer Variante eines polymophen Lokus einer Zielsequenz komplementär ist, im dritten Bereich des Aktivator-Oligonukleotides lokalisiert.

In einer weiteren Ausführungsform wird ein Sequenzsegment eines Aktivator-Oligonukleotide, welches zu mindestens einer Variante eines polymophen Lokus einer Zielsequenz komplementär ist, im zweiten Bereich des Aktivator-Oligonukleotides lokalisiert.

In einer weiteren Ausführungsform wird ein Sequenzsegment eines Aktivator-Oligonukleotides, welches zu mindestens einer Variante eines polymophen Lokus einer Zielsequenz komplementär ist, teilweise und/oder vollständig im zweiten und im dritten Bereich des Aktivator-Oligonukleotides lokalisiert.

Die Position von Allel-spezifischen Sequenzsegmenten eines Aktivator-Oligonukleotides kann somit in drei Gruppen aufgeteilt werden.

Die erste Gruppe umfasst allel-spezifische Sequenzsegmente, welche sowohl im ersten Primer-Oligonukleotid als auch im Aktivator-Oligonukleotid erfasst werden. Diese Position umfasst somit zumindest partiell den zweiten Bereich des Aktivator-Oligonukleotides und den ersten Bereich des ersten Primer-Oligonukleotides.

Die zweite Gruppe umfasst allel-spezifische korrespondierende Sequenzsegmente, welche nur vom Aktivator-Oligonukleotid abgedecket sind, beide Primer (der erste Primer und der zweite Primer) sind lediglich zielsequenzspezifisch, aber nicht allel-spezifisch. Die Position umfasst somit zumindest zum Teil den dritten Bereich des Aktivator-Oligonukleotides, welches mit dem 5'-Segment des von Polymerase synthetisierten Anteils des ersten Primer-Verlängerungspdoruktes komplementär binden soll.

Die dritte Gruppe umfasst allel-spezifische Sequenzsegmente, welche sowohl im zweiten Primer-Oligonukleotid als auch im Aktivator-Oligonukleotid erfasst werden. Diese Position umfasst somit zumindest partiell den dritten Bereich des Aktivator-Oligonukleotides vorwiegend im 5'-Segment des Aktivator-Oligonukleotides.

Diskriminierung zwischen Allel-Varianten einer Zielsequenz umfasst in diesen Gruppen unterschiedliche Mechanismen, welche beispielsweise bei der Wahl von Reaktionsbedingungen zu berücksichtigen sind.

Bei der ersten und der dritten Gruppe spielen Primer eine wesentliche Rolle bei der Diskriminierung. Die komplementäre Primer-Bindung an eine entsprechende Primer-Bindungsstelle einer Allel-Variante der Zielsequenz und Initiierung einer Synthese eines Primer-Verlängerungsprodutes durch die Polymerase bestimmen das Ausmass der unterschiedlichen Amplifikation. Das Aktivator-Oligonukleotid interagiert lediglich mit dem ersten Primer (erste Gruppe) und mit dem komplementären Sequenzsegment zum 3'-Segment des zweiten Primers (dritte Gruppe). Die erfolgreiche Strangverdrängung durch das Aktivator-Oligonukleotid hängt somit in erster Linie vom entsprechenden komplementären Design dieser Anteile von Primern und des Aktivator-Oligonukleotide ab. Der synthetisierte Segment-Anteil des jeweiligen Primer-Verlängerungsproduktes, welches zwischen beiden Primern liegt repräsentiert eine einheitliche Sequenzzusammensetzung für eine Allel-Varianten einer gemeinsamen Zielsequenz. Aus diesem Grund ist es vorteilhaft, die Amplifikation bei stringenten Hybridisierungs-Bedingungen auszuführen, welche eine allel-spezifische Initiierung einer Synthese unterstützen. Eine Verwendung eines weiteren allel-spezifischen Kompetitor-Pimers kann eine spezifische Initiierung der Synthese weiter verbessern.

Bei der zweiten Gruppe spielt Initiierung der Synthese von Primer-Verlängerungsprodukten eine eher sekundäre Rolle, da verwendete Primer an Primer-Bindungsstellen binden, welche eine einheitliche Zusammensetzung für alle potenziellen Allel-Varianten einer Zielsequenz darstellen. Die Diskriminierung erfolgt lediglich unter Mitwirkung des Aktivator-Oligonukleotides, welches durch sequenzspezifische bzw. vorwiegend spezifische Strangverdrängung bei der Trennung des ersten und des zweiten Primer-Verlängerungsproduktes mitwirkt.

In einer Ausführungsform wird bevorzugt eine voll-komplementäre Sequenz des dritten Bereichs des Aktivator-Oligonukleotides verwendet, welche einen perfekt-match mit mindestens einer Allel-Variante der Zielsequenz bilden kann. Amplifikation von verschiedenen Allel-Varianten erfordert somit bei dieser Ausführungsform eine Kombination aus je einem allel-spezifischen Aktivator-Oligonukleotid.

In der Regel führt eine Abweichung von voll-komplementären Sequenzzusammensetzungen zu abweichenden Amplifikatons-Effizienzen. Am korrespondierenden Sequenz-Segmenten eines Aktivator-Oligonukleotides erfolgt eine Konkurrenz um die Bindung an das erste Primer-Verlängerungsprodukt (bestehend vorwiegend aus DNA) mit dem zweiten Primer-Verlängerungsprodukt (umfasst ebenfalls DNA). Allel-spezifische Sequenz-Gestaltung dieses zu einer Allel-Variante korrekpondierenden Sequenzsegmentes des Aktivator-Oligonukleotides ermöglicht für beide konkurrierende Stränge eine perfekt-match/perfekt-mach Bindung an das erste Primer-Verlängerungsprodukt. Damit ist ein Aktivator-Oligonukleotid in der Lage, mit seinem korrespondierenden Sequenz-Segment das zweite Primer-Verlängerungsprodukt aus der Bindung mit dem ersten Primer-Verlängerungsprodukt zu erfolgreich verdrängen. Die Trennung von beiden Primer-Verlängerungsprodukten ermöglicht eine exponentielle Amplifikation.

Bei einer Abweichung in der Zusammensetzung des korrepondierenden Segmentes des Aktivator-Oligonukleotides (z.B. bei einer Bindung an eine nicht-komplementäre Allel-Variante eines Primer-Verlängerungsprodukte) ändert sich die Situation in der Regel. Die Interaktion zwischen perfekt-match des zweiten Primer-Verlängerungsproduktes (bestehend aus DNA) und des ersten Primer-Verlängerungsproduktes (bestehend aus DNA) kann nur schlecht oder auch gar nicht durch ein mismatch umfassendes Segment eines Aktivator-Oligonukleotides überwunden werden. Durch preferrierte Bindung eines perfekt-match Doppelstranges zwischen beiden Primer-Verlängerungsprodukten erfolgt somit keine hinreichende Strangtrennung unter Mitwirkung eines mismatch-Aktivator-Oligonukleotides, was in der Regel zu einer unzureichenden bzw. eine verlangsamten Amplifikation führt.

In einer bevorzugten Ausführungsform liegt das zu einer Allel-Variante korrespondierende Sequenz-Segment im dritten Bereich des Aktivator-Oligonukleotides, welches vorwiegend oder ausschließlich DNA-Monomeren umfassen.

In einer weiteren Ausführungsform liegt das zu einer Allel-Variante korrespondierende Sequenz-Segment im dritten Bereich des Aktivator-Oligonukleotides, welches sowohl DNA als auch DNA-Modifikationen umfassen.

In einer weiteren Ausführungsform liegt das zu einer Allel-Variante korrespondierende Sequenz-Segment im dritten Bereich des Aktivator-Oligonukleotides, welches vorwiegend oder ausschließlich DNA-Modifikationen umfassen. Beispielsweise bei Lokalisation eines zu einer Allel-Variante korrespondierenden Sequenzsegmentes innerhalb einer zweiten Blockierungs-Einheit, welche mehrere 2'-O-Alkyl-Modifikation von Nukleotiden umfasst, können solche Modifikationen einen Einfluss auf Bindung des Aktivator-Oligonukleotide an allel-spezifischen Variaten von Primer-Verlängerungsprodukten ausüben.

In einer bevorzugten Ausführungsform liegt das zu einer Allel-variante korrekspondierende Segment eines Aktivator-Oligonukleotides im dritten Bereich des Aktivator-Oligonukleotides, wobei dieses Segment des Stranges des Aktivator-Oligonukleotides vorwiegend aus DNA-Nukleotiden zusammengesetzt ist. Vorzugsweise liegt dieses Segment in 5'-Richtung von der zweiten Blockierungs-Einheit. Die Länge dieses zum polymorphen Lokus korrespondierenden Segmentes umfasst vorzugsweise 1 bis 20 Nukleotide. Die Zusammensetzung der Nukleotide in diesem vollkomplementären korrespondierenden Segment des Aktivator-Oligonukleotides umfasst dabei mindestens 70 % DNA Nukleotide, besser 80 %, bevorzugt mehr als 95 % DNA-Nukleotide.

Bevorzugt liegt auch bei einer Allel-Variante, welche nur eine Nukleobase umfasst (z.B. SNP oder eine Punkt-Mutation), die korrespondierende Position im mittleren Bereich des Aktivator-Oligonukleotid-Stranges, welche auf beiden Seiten zu dieser korrespondiernden Position von zu Zielsequenz komplementären DNA-Monomeren umgeben ist, wobei der Strang des Aktivator-Oligonukleotides mindestens 4 DNA-Monomere, besser mindestens 6 DNA-Nukleotide,bevorzugt mindestens 10 in beide Richtungen von der zu erwartenden SNP bzw. Punkt-Mutation umfasst. Solche Anordnung von DNA-Monomeren um eine zu erwartende SNP-Stelle bzw. Punkt-Mutation bzw. Einzelnukleotid-Allel-Variante ermöglicht Aufrechterhaltung einer Einzelstrang-Konformation, welche für B-Form charakteristisch ist (sogenannte single strand persistence length). Damit erfolgt eine Strangverdrängung durch das Aktivator-Oligonukleotid unter Verwendung eines korrespondierenden Segmentes, welches DNA-Monomere umfasst.

In einer Ausführungsform wird ein Aktivator-Oligonukleotid verwendet, welches mit seinem 5'-Ende eine komplementäre Bindung mit dem ersten Primer-Verlängerungsprodukt eingehen kann. Bei Alle-Varianten, welche nur eine Nukleobase umfassen, z.B. SNP oder Punktmutationen, wird das korrespondierende Sequenzsegment bevorzugt in einem Abstand vom 5'-Ende des Aktivator-Oligonukleotides angeordnet, welcher Längen von 10 bis 60 Nukleotide, besser 20 bis 50 Nukleotide, bevozugt 30 bis 40 Nukleotide umfasst.

Verwendung von DNA-Nukleotiden als Monomeren von Aktivator-Oligonukleotiden darf allerdings nicht dazu führen, dass Aktivator-Oligonukleotid als Matrize für eine Primer-Verlängerung unter Verwendung eines der eingesetzten Primers durch eine verwendete Polymerase genutzt wird.

Verwendung von DNA-Nukleotiden (beispielsweie A, C, T oder G) innerhalb des zu einer Allel-Variante korrespondierenden Segments im Aktivator-Oligonukleotid-Strang bringt dadurch einige Vorteile, dass das Diskriminierungsverhalten eines Amplifikations-Systems leichter abzuschätzen ist.

Beispielsweise kann Verhalten von perfekt-match Varianten eines Aktivator-Oligonukleotides zu bestimmten Allel-Varianten bei Punkt-Mutationen leicher abzuschätzen sein. Dabei folgt die Diskriminierung zwischen Allel-Varianten weit anerkannten Regeln von Watson-Crick-Basenpaarung: Aktivator-Oligonukleotide welche komplementäre Basenpaare (A:T, G:C) mit den ersten Primer-Verlängerungsprodukten ausbilden können, führen in der Regel zu Amplifikationsreaktionen in guten Ausbeuten.

Potenzielle Mismatches zwischen einem allel-spezifischen Aktivator-Oligonukleotid und einem ersten Primer-Verlängerungsprodukt (z.B. A:C oder G:G) führen hingegen zu einer unzureichenden oder verzögerten Amplifikation. Beim Vorliegen von Allel-Varianten, welche in einerm potenziellen ersten Primer-Verlängerungsprodukt mehr als ein Nukleotid-Unterschied zum Aktivator-Oligonukleotid ausmachen können (z.B. kurze InDels) wird in der Regel keine hinreichende Amplifikation erreicht.

Die Auswirkung eines Mismatches auf eine Amplifikation kann abgeschätzt werden, indem man Stablitäten von potenziellen Duplexen umfassend ein allel-spezifisches Aktivator-Olgonukleotid mit einer definierten Sequenz und einen potenziellen Primer-Verlängerungsprodukt oder seinen Anteilen, z.B. nur den synthetisierten Anteil eines Primer-Verlängerungsproduktes. Die Duplexe werden in Abwesenheit von zweiten Primer-Verlängerungsprodukten gebildet. Bei Hybridisierung eines allel-spezifischen Aktivator-Oligonukleotides an ein potenzielles Primer-Verlängerungsprodukt mit vollständiger Komplementarität (perfekt-match) ist die Bindung solcher Duplexe stabiler als die Bindung von Duplexen, welche mindsten eine Mismatch-Positionen umfasst. Diese Stabilität kann mittels Schmelztemperatur-Messung erfasst werden. Unterschiede in zwischen Amplifiktionsreaktion von Allel-Varianten sind n der Regel um so größer, je größer die Differenz in der Stabiliät zwischen einer perfekt-match Duplex und einer mismatch Duplex umfassend ein allel-spezifisches Aktivator-Oligonukleotid und ein potenzielles erstes Primer-Verlängerungsprodukt. Solche Unterschiede zwischen Reaktionen können beispielsweise durch die für eine Reaktion erforderliche Zeit bis zum Erreichen einer gewissen Menge an Produkten gemessen werden. Solche Unterschiede in Zeit zwischen Amplifikations-Reaktionen von perfekt-match und mismatch Allel-Varianten können auch als Fähigkeit zur Diskriminierung aufgefasst werden: je größer die Zeitdifferenz, um so höher die Diskriminierung zwischen einzelnen Allel-Varianten.

Aufgrund der Verwendung von Modifikationen im Aktivator-Oligonukleotid, z.B. im Bereich der zweiten Blockierungseinheit zur Verhinderung der Verlängerung des ersten Primers oder eines Kompetitor-Primers, kann eine abweichende Diskriminierung bzw. sogar Toleranz zu bestimmten Mismatches erfolgen. Dabei können beispielsweise 2'-O-Alkyl-Modifikationen innerhalb der zweiten Blockierungs-Einheit verwendet werden. Solche Nukleotid-Modifikationen können Konformation eines Doppelstranges verändern (von

B-Form einer DNA:DNA-Duplex auf A-like-Form eines DNA:RNA oder DNA:mod. DNA-Duplex). Dabei kann es vor allem in der Basenpaarung zwischen G:C und G:U bzw. G:T eine Änderung der Diskriminierung erfolgen.

Beispielsweise führt die Verwendung von Modfikationen im Aktivator-Oligonukleotid zu Änderung im Diskriminierungs-Verhalten bei einzelnen Basen-Paaren. Ein ähnliches Verhalten ist aus der Erforschung von RNA bekannt: so ändert sich das Verhalten der G:U je nach Strang-Form: bei RNA bildet G:U Basenpaar in der Regel eine hinreichend stabile Basenpaarung, Bei DNA führt ein G:T-Mismatch in der Regel zu einer Abschwächung der Bindung von beiden Strängen.

Bei dieser Anwendung werden beispielsweise 2'-Alkyl-Nukleotid umfassende Modifikationen im Aktivator-Oligonukleotid verwendet (z.B. innerhalb der zweiten Blockierungs-Einheit). Dabei wird ein 2'-O-Me Cytosine (C*) oder 2'-O-Me Adenosine (A*) verwendet. Solche modifizierte Nukleotide ermöglichen eine gute Diskriminierung zwischen einzelnen Sequenzvarianten (wobei perfekt match Bindung C*:dG und A*:U oder A*:T umfasst und Mismatch z.B. C*:dA oder A*:dC umfassen).

Hingegen führt die Verwendung von 2'-O-Me-G (G*) oder 2'-O-Me-U (U*) zu abweichenden Ergebnissen. Beispielsweise führt eine Amplifikation unter Verwendung von Aktivator-Oligonukleotides mit einem G* an der korrespondierenden Stelle im Strang zur gleichzeitigen Amplifikation von Allel-Varianten mit dC und dU an korrespondierenden Stellen im ersten Primer-Verlängerungsprodukt.

Somit läßt sich nicht nur eine allel-diskriminierende, sonder auch eine allel-tolerante Funktion eines Aktivator-Oligonukleotides darstellen.

Bei Verwendung von Nukleotid-Modifikationen, welche eine unzureichende Diskriminierung von Allel-Varianten zulassen, können somit mehrere Allel-Varianten gleichzeitig amplifiziert werden. Neben einem 2'-O-Me Guanosine oder 2'-O-Me Uridine gehören auch UniversalBasen, wie Inosine oder 5-Nitro-Indol Monomere zu Kandidaten mit Toleranz zu Allel-Zusammensetzung. Solche Modifikationen eines Aktivator-Oligonukleotid-Stranges können an zu erwartenden Korrespondierenden Positionen zu bestimmten Allel-Varianten angeordnet werden. Beim Vorliegen einer der Allel-Varianten kann eine Amplifikation, welche mindestens zwei unterschiedliche Alle-Varianten vermehrt.
Somit kann diese fehlende Diskriminierung bei einigen Modifikationen wie folgt berücksichtigt werden:
In einer weiteren Ausführungsform liegt das zu einer Allel-Variante korrespondierende Sequenz-Segment im dritten Bereich des Aktivator-Oligonukleotides, welches vorwiegend oder ausschließlich 2'-O-Alkyl-Modifikationen umfasst.

In einer bevorzugten Ausführungsform liegt das zu einer Allel-variante korrespondierende Segment eines Aktivator-Oligonukleotides im dritten Bereich des Aktivator-Oligonukleotides, wobei dieses Segment des Stranges des Aktivator-Oligonukleotides vorwiegend aus 2'-O-Alkyl-Modifikationen zusammengesetzt ist. Vorzugsweise liegt dieses Segment innerhalb der zweiten Blockierungs-Einheit oder in 5'-Richtung von der zweiten Blockierungs-Einheit. Die Länge dieses zum polymorphen Lokus korrespondierenden Segmentes umfasst vorzugsweise 1 bis 20 Nukleotide. Die Zusammensetzung der Nukleotide in diesem vollkomplementären korrespondierenden Segment des Aktivator-Oligonukleotides umfasst dabei mindestens 50 % 2'-O-Alkyl-Modifikationen, besser 80%, bevorzugt mehr als 95 % 2'-O-Alkyl-Modifikationen.

Bevorzugt liegt auch bei einer Allel-Variante, welche nur eine Nukleobase umfasst (z.B. SNP oder eine Punkt-Mutation), die korrespondierende Position im Mittleren Bereich des Aktivator-Oligonukleotid-Stranges, welche auf beiden Seiten zu dieser korrespondiernden Position von zu Zielsequenz komplementären 2'-O-Alkyl-Modifikationen umgeben ist, wobei der Strang des Aktivator-Oligonukleotides mindestens vier 2'-O-Alkyl-Modifikationen, besser mindestens sechs 2'-O-Alkyl-Modifikationen, bevorzugt mindestens 10 2'-O-Alkyl-Modifikationen in beide Richtungen von der zu erwartenden SNP bzw. Punkt-Mutation umfasst. Solche Anordnung von DNA-Monomeren um eine zu erwartende SNP-Stelle bzw. Punkt-Mutation bzw. Einzelnukleotid-Allel-Variante ermöglicht Aufrechterhaltung einer Einzelstrang-Konformation, welche als A-like-Form bezeichnet werden kann. Damit erfolgt eine Strangverdrängung durch das Aktivator-Oligonukleotid unter Verwendung eines korrespondierenden Segmentes, welches Nukleotide mit 2'-O-Alkyl-Modifikationen umfasst.

In einer Ausführungsform wird ein Aktivator-Oligonukleotid verwendet, welches mit seinem 5'-Ende eine komplementäre Bindung mit dem ersten Primer-Verlängerungsprodukt eingehen kann. Bei Alle-Varianten, welche nur eine Nukleobase umfassen, z.B. SNP oder Punktmutationen, wird das korrespondierende Sequenzsegment bevorzugt in einem Abstand vom 5'-Ende des Aktivator-Oligonukleotides angeordnet, welcher Längen von 10 bis 60 Nukleotide, besser 20 bis 50 Nukleotide, bevozugt 30 bis 40 Nukleotide umfasst.

Bei Lokalisation eines zu einer Allel-Variante erwartenden Sequenzsegmenten innerhalb eines solchen mehrere 2'-O-Alkyl-Modifikationen umfassenden Segmentes wird eine ggf. abweichende Basenpaarung berücksichtigt. Bei einem Unterschied zwischen Allel-Varianten von nur einem Nukleotid wird folgende Regel angewandt:
Aktivator-Oligonukleotide umfassend 2'-O-Alkyl-Modifikationen von Cytosine können in der Regel eine hinreichende Basenpaarung mit dG-Nukleotid an korrespondierenden Stellen im ersten Primer-Verlängerungsprodukt ausbilden und somit die Amplifikation solcher Allel-Varianten unterstützen.

Aktivator-Oligonukleotide umfassend 2'-O-Alkyl-Modifikationen von Adenosine können in der Regel eine hinreichende Basenpaarung mit dT- oder dU-Nukleotid an korrespondierenden Stellen im ersten Primer-Verlängerungsprodukt ausbilden und somit die Amplifikation solcher Allel-Varianten unterstützen.

Aktivator-Oligonukleotide umfassend 2'-O-Alkyl-Modifikationen von Guanosine können in der Regel eine hinreichende Basenpaarung mit dC- und dU-Nukleotid an korrespondierenden Stellen im ersten Primer-Verlängerungsprodukt ausbilden und somit die Amplifikation beider Allel-Varianten unterstützen.

Aktivator-Oligonukleotide umfassend 2'-O-Alkyl-Modifikationen von Uridine können in der Regel eine hinreichende Basenpaarung mit dA- und dG-Nukleotid an korrespondierenden Stellen im ersten Primer-Verlängerungsprodukt ausbilden und somit die Amplifikation beider Allel-Varianten unterstützen.

Aufgrund einer Strang-Komplementarität einer doppelsträngigen Zielnukleinsäure, können Strang-Spezifische Amplifikations-Systeme erstellt werden, wobei an korrespondierenden Stellen jeweils entweder 2'-O-Alkyl-Modifikationen von Cytosine oder 2'-O-Alkyl-Modifikationen von Adenosine verwendet wird. Somit kann man fehlende Diskriminierung von G- und U-Nukleotid-Analoga umgehen.

### Reaktionsbedingungen bei Strangverdrängungsreaktion

Die Verdrängung des zweiten Primer-Verlängerungsproduktes aus der Bindung mit dem ersten Primer-Verlängerungsprodukt mittels einer sequenzabhänigen Strangverdängung durch das Aktivator-Oligonukleotid bildet einen Teilschritt in der Amplifikation. Die Reaktionsbedingungen während dieses Schrittes sind entsprechend angepasst. Die Reaktions-Temperatur und die Reaktions-Zeit sind dermaßen gewählt, dass die Reaktion erfolgreich stattfinden kann.

In einer bevorzugten Ausführungsform verläuft die Strangverdrängung durch das Aktivator-Oligonukleotid bis zur Ablösung / Dissoziation des zweiten Primer-Verlängerungsproduktes aus der Bindung mit dem ersten Primer-Verlängerungsprodukt. Eine solche Dissoziation des 3'-Segment des ersten Primer-Verlängrungsproduktes von komplementären Anteilen des zweiten Primerverlängerungsproduktes kann spontant erfolgen im Rahmen einer temperatur-abhängigen/temperatur-bedingten Trennung von beiden Primer-Verlängerungprodukten. Eine solche Dissoziation wirkt sich günstig auf Kinetik der Amplifikationsreaktion und kann durch die Wahl der Reaktionsbedingungen beeinflusst werden, z.B. mittels Temperatur-Bedingungen. Die Temperatur-Bedingungen werden deshalb dermaßen gewählt, dass eine erfolgreiche Strangverdrängung durch komplementäre Bindung des Aktivator-Oligonukleotides eine Dissoziation des zweiten Primer-Verlängerungsproduktes vom 3'-Segment des ersten Primer-Verlängerungsproduktes begünstigt.

In einer weiteren bevorzugten Ausführungsform verläuft die Strangverdrängung durch das Aktivator-Oligonukleotid bis zur Ablösung/Dissoziation eines 3'-Segmentes des zweiten Primer-Verlängerungsproduktes (P2.1-Ext) aus der komplementären Bindung mit dem ersten Primer-Verlängerungsprodukt (P1.1-Ext), wobei dieses 3'-Segment des zweiten Primer-Verlängerungsproduktes (P2.1-Ext) zumindest einen komplemehteren Bereich zum ersten Primer umfasst und ein komplementäres Segment zum ersten Primerverlängerungsprodukt (P1.1-Ext), welches erst bei der enzymatsichen Synthese entstanden ist. Dabei kommt es zu Ausbildung eines Komplexes (C1.1/ P1.1-Ext / P2.1.-Ext) (Fig. 1B), welches sowohl das erste Primer-Verlängerungsprodukt (P1.1-Ext), das zweite Primer-Verlängerungsprodukt (P2.1-Ext) sowie das Aktivator-Oligonukleotid (C1.1) umfasst. In einem solchen Komplex liegt das 3'-Segment des zweiten Primer-Verlängerungsproduktes zumindest vorübergehend in einer einzelsträngiger Form vor, da es vom Aktivator-Oligonukleotid aus seiner Bindung mit dem ersten Primer-Verlängerungsprodukt verdrängt werden kann. Schematisch dargestellt, besteht ein Gleichgewicht zwischen Bindung und Ablösung des P2.1-Ext an das P1.1-Ext. Das 3'-Segment des ersten Primer-Verlängerungsproduktes (P1.1-Ext) liegt in einem solchen Komplex komplementär hybridisiert an das zweite Primer-Verlängerungsprodukt (P2.1-Ext) (Fig. 1B).

Aufgrund einer teilweise / vorübergehend offenen Primer-Bindungsstelle für das erste Primer-Oligonukleotid (3'-Segment des zweiten Primer-Verlängerungsprodukte) kann ein neues Primer-Verlängerungsprodukt (P1.2) an eine dieses einzelsträngigers Sequenzsegment des noch im Komplex liegenden (P2.1-Ext) unter Reaktionsbedingungen binden und somit eine Synthese eines neuen ersten Primer-Verlängerungsproduktes (P1.2-Ext) durch eine Polymerase initiieren. In der Regel verläuft die Initiierung dieser Reaktion mit verminderter Effizienz, da 3'-Segment des P2.1-Ext nicht dauerhaft einzelsträngig vorliegt, sondern im kompetetiven Verhalten mit Aktivator-Oligonukleotid steht und damit abwechselnd einzelsträngige und doppelsträngige Zustände durch Bindung an das P1.1-Ext aufweist.

Fortführung dieser neugestarteten Synthese von P1.2-Ext unter Verwendung des zweiten Primer-Verlängerungsproduktes als Matrize (P2.1-Ext) kann durch Polymerase-bedingte Strandverdrängung ebenfalls zu Dissoziiereung des Komplexes (C1.1/ P1.1-Ext / P2.1.-Ext) führen / beitragen. Dabei wirken Aktivator-Oligonukleotid, temperatur-abhängige doppelstrang-destabilisierung und Strang-Verdrängung durch die Polymerase synnergistisch und komplementär. Es resultiert eine Dissoziation des 3'-Segment des ersten Primer-Verlängrungsproduktes (P1.1-Ext) von komplementären Anteilen des zweiten Primerverlängerungsproduktes (P2.1-Ext).

Eine solche Dissoziation wirkt sich günstig auf Kinetik der Amplifikationsreaktion und kann durch die Wahl der Reaktionsbedingungen beeinflusst werden, z.B. mittels Temperatur-Bedingungen. Das Mitwirken der Polymerase-vermittelten syntheseabhängigen Strangverdrängung an der Dissoziierung von P1.1-Ext und P2.1-Ext hat einen begünstigenden Effekt bei der Strangtrennung.

Die Temperatur in diesem Schritt umfasst beispielsweise Bereiche von 15°C bis 75°C, besser von 30°C bis 70°C, bevorzugt von 50°C bis 70°C.

Bei gegebener Länge des ersten Bereichs des Aktivator-Oligonukleotides und des zweiten Bereichs des ersten Primer-Oligonukleotides (umfassend beispielsweise Bereiche von 3 bis 25 Nukleotid-Monomere, besser von 5 bis 15 Nukleotid-Monomere) kann eine Strangverdrängungsreaktion im allgemeinen erfolgreich initiiert werden. Bei vollständigen Komplementarität des Aktivator-Oligonukleotid zu entsprechenden Anteilen des ersten Primer-Verlängerungsproduktes kann das Aktivator-Oligonukleotid an das erste Primer-Verlängerungsprodukt bis auf das 3'-Segment des ersten Primer-Verlängerungsproduktes binden und das zweite Primer-Verlängerungsprodukt verdrängen. Das zweite Primer-Verlängerungsprodukt verbleibt somit in Verbindung mit dem 3'-Segment des ersten Primer-Verlängerungsproduktes. Diese Stärke dieser Verbindung kann temperaturabhängig beeinflusst werden. Beim Erreichen einer kritischen Temperatur kann diese Verbindung zerfallen und beide Primer-Verlängerungsprodukte dissoziieren. Je kürzer die Sequenz des 3'-Segmentes ist, umso instabiler ist diese Verbindung und umso niedriger kann die Temperatur sein, welche eine spontane Dissoziation herbeiführt.

Eine spontane Dissozialtion kann beispielsweise im Temperatur-Bereich erreicht werden, welcher etwa bei der Schmelztemperatur liegt. In einer Ausführungsform liegt die Temperatur der Schritte der Strangverdrängung durch das Aktivator-Oligonukleotid etwa bei der Schmelztemperatur (Tm +/- 3°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid etwa bei der Schmelztemperatur (Tm +/- 5°C) des Komplexes umfassend das 3'-Segment des ersten Primer-Verlängerungsproduktes, welches nicht von Aktivator-Oligonukleotid gebunden wird, und dem zweiten Primer-Oligonukleotid bzw. dem zweiten Primer-Verlängerungsprodukt.

In einer Ausführungsform liegt die Temperatur der Schritte der Strangverdrängung durch das Aktivator-Oligonukleotid über der Schmelztemperatur des Komplexes umfassend das 3'-Segment des ersten Primer-Verlängerungsproduktes, welches nicht vom Aktivator-Oligonukleotid gebunden wird, und dem zweiten Primer-Oligonukleotid bzw. dem zweiten Primer-Verlängerungsprodukt. Eine solche Temperatur umfasst TemperaturBereiche von etwa Tm + 5°C bis Tm+20°C, besser von Tm+5°C bis Tm +10°C. Durch Verwendung einer höheren Temperatur kann das Gleichgewicht in diesem Reaktionsschritt in Richtung Dissoziation verschoben werden. Dadurch kann die Kinetik der Reaktion günstig beeinflusst werden. Verwendung von zu niedrigen Temperaturen im Schritt der Strangverdrängung mittels Aktivator-Oligonukleotides kann zu einer signifikanten Verlangsamung der Amplifikation führen.

In einer Ausführungsform umfasst ein erstes Primer-Verlängerungsprodukt ein 3'-Segment, welches nicht von Aktivator-Oligonukleotid gebunden wird, und welches Sequenzlängen von 9 bis etwa 18 Nukleotiden umfasst. Bei dieser Ausführungsform kann eine spontane Dissoziation in der Regel bereits bei Temperatur-Bereichen zwischen 40°C und 65°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 15 bis etwa 25 Nukleotiden umfasst. Bei dieser Ausführungsform kann eine spontane Dissoziation in der Regel bereits bei Temperatur-Bereichen zwischen 50°C und 70°C erreicht werden. Auch höhere Temperaturen führen zu einer Dissoziation.

In einer Ausführungsform umfasst ein erstes Primer-Verlängerungsprodukt ein 3'-Segment, welches nicht von Aktivator-Oligonukleotid gebunden wird, und welches Sequenzlängen von 20 bis etwa 40 Nukleotiden umfasst. Bei dieser Ausführungsform kann eine spontane Dissoziation in der Regel bereits bei Temperatur-Bereichen zwischen 50°C und 75°C erreicht werden. Auch höhere Temperaturen führen zu einer Dissoziation.

Die Zusammensetzung des 3'-Segmentes des ersten Primer-Verlängerungsproduktes und ggf. eine Einführung von Schmelztemperatur-beeinflussenden Oligonukleotid-Modifikationen (z.B. MGB) bzw. Reaktionsbedingungen (z.B. TPAC, Betaine) kann die Wahl der Temperatur beeinflussen. Entsprechende Anpassung kann daher vorgenommen werden.

In einer Ausführungsform verlaufen alle Schritte der Amplifikation unter stringenten Bedingungen, welche Ausbildung von unspezifischen Produkten / Nebenprodukten verhindern bzw. verlangsamen. Zu solchen Bedingungen zählen beispielsweise höhere Temperaturen, beispielsweise über 50°C.

In einer Ausführungsform verläufen die Einzelschritte der Strangverdrängung durch Aktivator-Oligonukleotide bei gleicher Temperatur wie die Synthese des ersten und des zweiten Primer-Verlängerungsproduktes. In einer weiteren Ausführungsform verläufen die Einzelschritte der Strangverdrängung durch Aktivator-Oligonukleotide bei Temperatur, welche von der Temperatur der jeweiligen Synthese des ersten und des zweiten Primer-Verlängerungsproduktes abweicht. In einer weiteren Ausführungsform verläuft die Synthese des ersten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei gleicher Temperatur.

Die Konzentration des Aktivator-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

### Bevorzugte Ausführungsformen des zweiten Primer-Oligonukleotides (Primer-2):

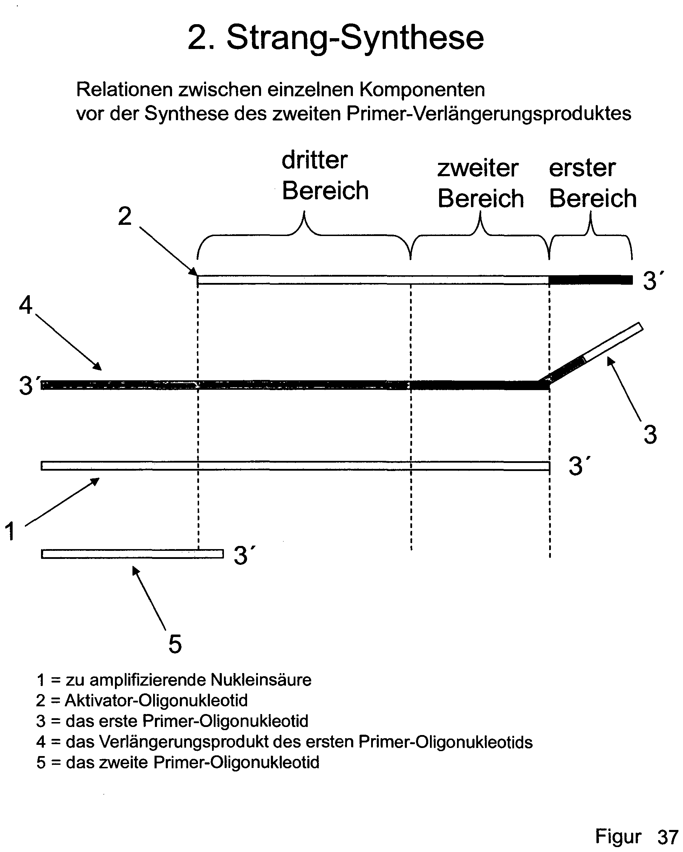

Ein Oligonukleotid, welches mit seinem 3'-Segment in der Lage ist, an eine im Wesentlichen komplementäre Sequenz innerhalb der zu amplifizierenden Nukleinsäure oder ihrer Äquivalente zu binden und eine spezifische zweite Primer-Verlängerungsreaktion zu initiieren (Fig 37). Dieses zweite Primer-Oligonukleotid ist somit in der Lage, an das 3'-Segment eines ersten spezifischen Primer-Verlängerungsproduktes des ersten Primer-Oligonukleotids zu binden und eine polymerase-abhängige Synthese eines zweiten Primer-Verlängerungsproduktes zu initiieren. In einer Ausführungsform ist ein jedes zweite Primer-Oligonukleotid spezifisch für je eine zu amplifizierende Nukleinsäure.

Der zweite Primer-Oligonukleotid soll bei Rücksynthese kopiert werden können und dient auch als selbst Matrize für im Rahmen der Synthese des ersten Primer-Verlängerungsproduktes.

Die Länge des zweiten Primer-Oligonukleotids kann zwischen 15 und 100 Nukleotiden liegen, bevorzugt zwischen 20 und 60 Nukleotiden, besonders bevorzugt zwischen 30 und 50 Nukleotiden. Die Nukleotid-Bausteine sind vorzugsweise untereinander via übliche 5'-3' Phosphodiester-Bindung oder Phosphothioester-Bindung verknüpft. Ein solches Primer-Oligonukleotid kann in gewünscher Form chemisch synthetisiert werden.

In einer Ausführungsform kann das zweite Primer-Oligonukleotid Nukleotid-Monomere einschließen, welche Funktion der Polymerase nicht oder nur unwesentlich beeinflussen, dazu gehöhren beispielsweise:
- natürliche Nukleotide (dA, dT, dC, dG etc.) oder deren Modifikationen ohne veränderte Basen-Paarung
- modifizierte Nukleotide, 2-Amino-dA, 2-thio-dT oder andere Nukleotid-Modifikationen mit abweichender Basen-Paarung (z.B. Universal-Basenpaaren, wie beispielsweise Inosine 5-Nitroindol).

In einer bevorzugten Ausführungsform ist das 3'-OH-Ende dieses Bereichs vorzugsweise frei von Modifikationen und hat eine funktionsfähige 3'-OH Gruppe, welche von Polymerase erkannt und matrizenabhänig verlängert werden kann. In einer weiteren bevorzugten Ausführungsform umfasst Das 3'-Segment des zweiten Primers mindestens eine Phosphorothioat-Verbindung, so dass kein Abbau von 3'-Ende des Primern durch 3'-Exonuclease Aktivität von Polymerasen erfolgen kann.

Das zweite Primer-Oligonukleotid kann in mehreren Teil-Schritten verwendet werden. In erster Linie übernimmt es eine Primer-Funktion in der Amplifikation. Dabei wird Primer-Verlängerungsreaktion unter Verwendung des ersten Primer-Verlängerungsproduktes als Matrize ausgeführt. In einer weiteren Ausführungsform kann das zweite Primer-Oligonukleotid zu Beginn der Amplifikationsreaktion die Start-Nukleinsäurekette als Matrize verwenden. In einer weiteren Ausführungsform kann das zweite Primer-Oligonukleotid bei der Erstellung/Bereitstellung einer Start-Nukleinsäurekette verwendet werden.

Im Rahmen der Amplifikation dient der zweiten Primer als Initiator der Synthese des zweiten Primer-Verlängerungsproduktes unter Verwendung des ersten Primer-Verlängerungsproduktes als Matrize. Das 3'-Segment des zweiten Primers umfasst eine Sequenz, welche vorwiegend komplementär an das erste Primer-Verlängerungsprodukt binden kann. Die enzymatische Verlängerung des zweiten Primer-Oligonukleotid unter Verwendung des ersten Primer-Verlängerungsproduktes als Matrize führt zu Ausbildung des zweiten Primer-Verlängerungsproduktes. Eine solche Synthese erfolgt typischerweise parallel zu Verdrängung des Aktivator-Oligonukleotides aus seiner Bindung mit dem ersten Primer-Verlängerungsprodukt. Diese Verdrängung erfolgt vorwiegend durch Polymerase und kann teilweise durch das zweite Primer-Oligonukleotid erfolgen. Ein solches zweites Verlängerungsprodukt umfasst die Zielsequenz oder ihre Segmente. Im Verlauf der Synthese des zweiten Primer-Verlängerungsproduktes wird die Sequenz des kopierbaren Anteils des ersten Primer-Oligonukleotid von Polymerase als Matrize erkannt und eine entsprechende komplementäre Sequenz synthetisiert wird. Diese Sequenz liegt im 3'-Segment des zweiten Primer-Verlängerungsproduktes und umfasst Primer-Bindungsstelle für das erste Primer-Oligonukleotid. Die Synthese des zweiten Primer-Verlängerungsproduktes erfolgt bis zur Stopp-Position im ersten Primer-Oligonukleotid. Unmittelbar nach der Synthese des zweiten Primer-Verlängerungsproduktes ist dieses Produkt an das erste Primer-Verlängerungsprodukt gebunden und bildet doppelsträngigen Komplex. Das zweite Primer-Verlängerungsprodukt wird aus diesem Komplex sequenzspezifisch durch das Aktivator-Oligonukleotid verdrängt. Nach einer erfolgreichen Strangverdrängung durch Aktivator-Oligonukleotid kann das zweite Primer-Verlängerungsprodukt wiederum selbst als Matrize für die Synthese des ersten Primer-Verlängerungsproduktes dienen.

Weiterhin kann das zweite Primer-Oligonukleotid als Initiator der Synthese des zweiten Primer-Verlängerungsproduktes ausgehend von Start-Nukleinsäurekette zu Begin der Amplifikation dienen. In einer Ausführungsform ist die Sequenz des zweiten Primers vollständig komplementär zum entsprechenden Sequenzsegment einer Start-Nukleinsäurekette. In einer weiteren Ausführungsform ist die Sequenz des zweiten Primer-Oligonukleotides nur teilweise komplementäre zum entsprechenden Sequenzsegment einer Start-Nukleinsäurekette. Diese abweichende Komplementarität soll das zweite Primer-Oligonukleotid allerdings nicht daran hindern, eine vorwiegend sequenzspezifische Primer-Verlängerungsreaktion zu starten. Die jeweiligen Unterschiede in Komplementarität des zweiten Primer-Oligonukleotides zur jeweiligen Position in der Start-Nukleinsäurekette liegen vorzugsweise im 5'-Segment des zweiten Primer-Oligonukleotides, so dass im 3'-Segment vorwiegend komplementäre Basenpaarung und Initiierung der Synthese möglich ist. Für die Initiierung der Synthese sollen beispielsweise besonders die ersten 4-10 Positionen im 3'-Segment vollkomplementär zu Matrize (Start-Nukleinsäurekette) sein. Die restlichen Nukleotidpositionen können von perfekten Komplementarität abweichen. Das Ausmaß einer perfekten Komplementarität im 5'-Segment kann somit Bereiche umfassen zwischen 10 % bis 100 %, besser zwischen 30 % und 100 % der Basenzusammensetzung. Je nach Länge des zweiten Primer-Oligonukleotides, umfassen diese Abweichung von einer vollständigen Komplementarität im 5'-Segment von 1 bis 40, besser 1 bis 20 Nukleotid-Positionen. In einer weiteren Ausführungsform bindet das zweite Primer-Oligonukleotid nur mit seinem 3'-Segment an die Start-Nukleinsäurekette, aber nicht mit seinem 5'-Segment. Die Länge eines solchen zu Start-Nukleinsäurekette vollkomplementären 3'-Segment des zweiten Primer-Oligonukleotides umfasst Bereiche zwischen 6 und 40 Nukleotide, besser, zwischen 6 und 30 Nukleotide, bevorzugt zwischen 6 und 20. Die Länge eines entsprechenden, zu Start-Nukleinsäurekette nicht komplementären 5'-Segments des zweiten Primer-Oligonukleotides umfasst Bereiche zwischen 5 und 60, besser zwischen 10 und 40 Nukleotide. Das zweite Primer-Oligonukleotid kann somit eine Synthese von einer Start-Nukleinsäukette initiieren. Bei einer darauffolgenden Synthese des ersten Primer-Verlängerungsproduktes werden Sequenzabschnitte des zweiten Primer-Oligonukleotides von Polymerase kopiert, so dass wiederum in darauf folgenden Synthese-Zyklen eine vollkomplementäre Primer-Bindungsstelle innerhalb des ersten Primer-Verlängerungsprodukte für die Bindung des zweiten Primer-Oligonukleotides ausgebildet wird und in darauf folgenden Synthese-Zyklen zur Verfügung steht.

In einer weiteren Ausführungsform kann das zweite Primer-Oligonukleotid im Rahmen der Vorbereitung einer Start-Nukleinsäurekette verwendet werden. Dabei kann ein solches zweite Primer-Oligonukleotid an eine Nukleinsäure (z.B. eine einzelsträngige genomische DNA oder RNA oder ihre Äquivalente umfassend eine Zielsequenz) vorwiegend / bevorzugt sequenzspezifisch binden und in Anwesenheit einer Polymerase eine matrizenabhängige Primer-Verlängerungsreaktion initiieren. Die Bindungsposition ist dermaßen gewählt, dass das Primer-Verlängerungsprodukt eine gewünschte Zielsequenz umfasst. Durch die Verlängerung des zweiten Primer-Oligonukleotides resultiert ein Strang, welche zu Matrize komplementäre Sequenz aufweist. Ein solcher Strang kann von der Matrize abgelöst werden (z.B. durch Hitze oder Alkalie) und damit in eine einzelsträngige Form überführt werden. Eine solche einzelsträngige Nukleinsäurekette kann als Start-Nukleinsäurekette zu Beginn der Amplifikation dienen. Eine solche Start-Nukleinsäurekette umfasst in ihrem 5'-Segment die Sequenzanteile des zweiten Primer-Oligonukleotides, weiterhin umfasst sie eine Zielsequenz oder ihre Äquivalente und eine Primer-Bindungsstelle für das erste Primer-Oligonukleotid. Weitere Schritte sind im Abschnitt "Start-Nukleinsäurekette" erläutert.

In einer bevorzugten Ausführungsform umfasst das zweite Primer-Oligonukleotid zumindest in seinem 3'-Segment Sequenzanteile, welche komplementär und sequenzspezifisch an ein Sequenzsegment einer Zielsequenz binden können und eine erfolgreiche Primer-Verlängerungsreaktion durch Polymerase initiieren/unterstützen. Die Länge eines solchen Sequenzsegments umfasst Bereiche von 6 und 40 Nukleotide, besser von 8 bis 30 Nukleotide, bevorzugt von 10 bis 25 Nukleotide.

In einer Ausführungsform umfasst das zweite Primer-Oligonukleotid in seinem 3'- und 5'-Segment kopierbare Sequenzabschnitte, welche bei Synthese des ersten Primer-Verlängerungsproduktes von Polymerase kopiert werden. Somit werden alle Sequenzabschnitte des zweiten Primers von Polymerase kopiert. Das führt zu Ausbildung einer Primer-Bindungsstelle im 3'-Segment des ersten Primer-Verlängerungsproduktes.

In einer Ausführungsform entspricht das zweite Primer-Oligonukleotid mit seinen kopierbaren Anteilen in ihrer Länge dem 3'-Segment des ersten Primer-Verlängerungsproduktes, welches nicht vom Aktivator-Oligonukleotid gebunden wird. Im Komplex umfassend das zweite Primer-Oligonukleotid und das erste Primer-Verlängerungsprodukt grenzt das 3'-Ende eines solchen zweiten Primer-Oligonukleotides an das Aktivator-Oligonukleotid, welches an das erste Primer-Verlängerungsprodukt gebunden ist. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt, abgebildet (Primer-Verlängerungsprodukt).

In einer weiteren Ausführungsform ist das zweite Primer-Oligonukleotid mit seinen kopierbaren Sequenzanteilen kürzer als das 3'-Segment des ersten Primer-Verlängerungsproduktes, welches nicht vom Aktivator-Oligonukleotid gebunden wird. Im Komplex umfassend das zweite Primer-Oligonukleotid und das erste Primer-Verlängerungsprodukt liegt somit zwischen dem 3'-Ende eines solchen Primers und dem an das erste Primer-Verlängerungsprodukt gebundenen Aktivator-Oligonukleotid ein einzelsträngiger Abschnitt des ersten Primer-Verlängerungsproduktes. Verlängerung eines solchen Primers erfolgt unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in in, abgebildet (Primer-Verlängerungsprodukt).

In einer weiteren Ausführungsform ist das zweite Primer-Oligonukleotid mit seinen kopierbaren Anteilen länger als das 3'-Segment des ersten Primer-Verlängerungsproduktes, welches nicht vom Aktivator-Oligonukleotid gebunden wird. Im Komplex aus dem zweiten Primer-Oligonukleotid und dem ersten Primer-Verlängerungsprodukt konkurrieren das 3'-Segment des zweiten Primers und das 5'-Segment des Aktivator-Oligonukleotides um die Bindung an das das erste Primer-Verlängerungsprodukt. Die für eine Initiierung der Synthese erforderliche Bindung des 3'-Segments des zweiten Primers an das erste Primer-Verlängerungsprodukt erfolgt unter gleichzeitiger partiellen Verdrängung des 5'-Segmentes des Aktivator-Oligonukleotides.

Nach Initiierung der Synthese durch Polymerase erfolgt die Verlängerung eines solchen Primers unter Verwendung des ersten Primer-Verlängerungproduktes als Matrize. Bei Verlängerung eines solchen Primers erfolgt die Verdrängung des Aktivator-Oligonukleotides aus der Bindung mit dem ersten Primer-Verlängerungsprodukt mittes polymese-abhängigen Strangverdrängung. Das entsprechende zweite Primer-Verlängerungsprodukt in abgebildet (Primer-Verlängerungsprodukt 6A, 6D, 6E). Die Sequenzlänge des 3'-Segment des zweiten Primer-Oligonukleotides, welches das 5'-Segment des Aktivator-Oligonukleotid verdrängt, kann folgende Bereiche umfassen: 1 bis 50 Nukleotide, besser 3 bis 30 Nukleotide, bevorzugt 5 bis 20 Nukleotide. Verwendung von zweiten Primer-Oligonukleotiden mit einer größeren Länge, welche die Länge des 3'-Segment des ersten Primer-Verlängerungsproduktes übersteigt, ist beispielsweise in einigen Ausführungsformen vorteilhaft. Solche Ausführungsformen umfassen beispielsweise ein erstes Primer-Verlängerungsprodukt mit einem 3'-Segment, welches nicht vom Aktivator-Oligonukleotid gebunden wird, mit einer Länge von 5 bis 40 Nukleotiden, besser von 10 bis 30 Nukleotiden. Besonders bei kürzeren 3'-Segmenten bietet ein längeres zweites Primer-Oligonukleotid eine verbesserte Sequenzspezifität bei Initiierung Synthese.
Die Bindungsstärke des zweiten Primer-Oligonukleotides an seine Primer-Bindungsstelle hängt von der Länge des Primers ab. Im Allgemeinen können längere zweite Primer-Oligonukleotide bei höheren Reaktionstemperaturen eingesetzt werden.

Vorzugsweise sind Sequenzen des ersten, des zweiten Primer-Oligonukleotides und des Aktivator-Oligonukleotides dermaßen aneinander angepasst, dass Nebenreaktionen, z.B. Primer-Dimer-Ausbildung, minimiert sind. Zu diesem Zweck sind beispielsweise die Sequenz des ersten und des zweiten Primer-Oligonukleotides aneinander demassen angepasst, daß beide Primer-Oligonukleotide nicht in der Lage sind, eine Amplifikations-Reaktion in Abwesenheit einer passenden Matrize und/oder einer Zielsequenz und/oder einer Start-Nukleinsäurekette zu starten. Das kann beispielsweise dadurch erreicht werden, dass das zweite Primer-Oligonukleotid keine Primer-Bindungsstelle für das erste Primer-Oligonukleotid umfasst und das erste Primer-Oligonukleotid keine Primer-Bindungsstelle für das zweite Primer-Oligonukleotid umfasst. Weiterhin soll vermieden werden, dass die Primer-Sequenzen ausgedenhnte eigen-komplementäre Strukturen (Self-complement) umfassen.

Die Synthese des zweiten Primer-Verlängerungsproduktes ist eine Primer-Verlängerungsreaktion und bildet einen Teilschritt in der Amplifikation. Die Reaktionsbedingungen während dieses Schrittes sind entsprechend angepasst. Die Reaktions-Temperatur und die Reaktions-Zeit sind dermaßen gewählt, dass die Reaktion erfolgreich stattfinden kann. Die jeweils bevorzugte Temperatur in diesem Schritt hängt von der verwendeten Polymerase ab und der Bindungsstärke des jeweiligen zweiten Primer-Oligonukleotides an seine Primer-Bindungsstelle und umfasst beispielsweise Bereiche von 15°C bis 75°C, besser von 20 bis 65°C, bevorzugt von 25°C bis 65°C. Die Konzentration des zweiten Primer-Oligonukleotides umfasst Bereiche von 0,01 µmol/l bis 50 µmol/l, besser von 0,1 µmol/l bis 20 µmol/l, bevorzugt von 0,1 µmol/l bis 10 µmol/l.

In einer Ausführungsform verlaufen alle Schritte der Amplifikation unter stringenten Bedingungen, welche Ausbildung von unspezifischen Produkten / Nebenprodukten verhindern bzw. verlangsamen. Zu solchen Bedingungen zählen beispielsweise höhere Temperaturen, beispielsweise über 50°C.

Falls mehr als eine spezifische Nukleinsäurekette in einem Ansatz amplifiziert werden müssen, werden in einer Ausführungsform bevorzugt jeweils sequenzspezifische Primer-Oligonukleotide für Amplifikation von entsprechenden jeweiligen Zielsequenzen verwendet.

In einer Ausführungsform verläuft die Synthese des ersten und des zweiten Primer-Verlängerungsproduktes bei gleicher Temperatur. In einer weiteren Ausführungsform verläuft die Synthese des ersten und des zweiten Primer-Verlängerungsproduktes bei unterschiedlichen Temperaturen. In einer weiteren Ausführungsform verläuft die Synthese des zweiten Primer-Verlängerungsproduktes und Strangverdrängung durch Aktivator-Oligonukleotid bei gleicher Temperatur. In einer weiteren Ausführungsform verläuft die Synthese des zweiten Primer-Verlängerungsproduktes und Strangverdrängung durch Aktivator-Oligonukleotid bei unterschiedlichen Temperaturen.

**In ein r Ausführungsform wird in Allel-spezifischer zweit r Primer in Kombination mit einem allel-spezifischen Aktivator-Oligonukleotid verwendet.**

Der zweite Primer kann an die ensprechende komplementäre Position einer Start-Nukleinsäure oder einer zu amplifizierenden Nukleinsäurekette binden. Bevorzugt umfasst ein zweiter Primer zumindest eine Sequenzsegment, welches bevorzugt spezifisch unter verwendeten Reaktionsbedingungen an eine allel-spezifische Sequenzvariante der Zielnukleinsäure binden kann, wobei Polymerase in der Lage ist, einen dabei gebildeten perfekt-match Komplex zu verlängert, so dass dabei ein erstes Primer-Verlängerungsprodukt resultiert.

Die Position einer alle-spezifischen Sequenz im Primer umfasst in einer Ausführungsform das 3'-Terminalen Nukleotid. In einer weiteren Ausführungsfrom umfasst die Position einer allel-spezifischen Sequenz im Primer die mindestens eine der Positionen -1 bis -6 Nukleotide im 3'-terminalen Segment des zweiten Primers. In einer weiteren Ausführungsfrom umfasst die Position einer allel-spezifischen Sequenz im Primer mindestens eine der Positionen von -6 bis mindestens -15 im 3'-terminalen Segment des zweiten Primers.

Ein solcher Primer umfasst weiterhin Sequenzsegmente, welche komplementär an alle Allel-Varianten einer Zielsequenz einheitlich binden können. Somit umfasst der zweite allel-spezifischer Primer Sequenzsegmente, welche sowohl zielsequenzspezifisch sind als auch solche, welche allel-spezifisch sind.

Bevorzugt wird eine Kombination eines allel-spezifischen Primers und eines allel-spezifischen Aktivator-Oligonukleotides verwendet, wobei die zum 3'-Segment des zweiten Primer komplementäre Sequenz an das Aktivator-Oligonukleotid komplementär binden kann.

Einzelne allel-spezifische Primer können zu einer Gruppe zusammengefasst werden, welche sämtliche Varianten einer Zielsequenz abdecken. Eine solche Gruppe von allel-spezifischen Primern umfasst zumindest zwei unterschiedliche allel-spezifische Primer, da ein polymorpher Lokus an einer vorgegeben Position in der Zielsequenz mindestens zwei Sequenzvarianten umfasst. Die allel-spezifischen Primer sind dahingehend konstruiert, dass sie unter stringenten Reaktionsbedingungen vorzugsweise mit ihrer jeweils spezifischen Matrize eine perfekt-match Bindung eingehen und somit diese spezifische perfekt-Match Matrize zur Ausbildung der jeweiligen Primer-Verlängerungsprodukte unter katalytischer Wirkung der Polymerase verwenden. Vorzugsweise können 3'-terminale Nukleotide und/oder 3'-Terminale Segmente von allel-spezifischen Primern zur Diskriminierung von Varianten von Zielsequenzen verwendet werden und dabei dermaßen in ihrer Sequenzzusammensetzung an die jeweiligen Varianten angepasst, dass solche Primer einen perfekt-match Doppelstrang unter stringenten Bedingungen mit der jeweiligen Variante ausbilden. Solche perfekt-match Doppelstränge können in der Regel von einer Polymerase gut erkannt werden und unter geeigneten Reaktionsbedingungen kommt es zu einer Primer-Verlängerung. Bei der Interaktion eines allel-spezifischen Primers mit einer anderen Variante einer Zielsequenz entsteht somit ein Mismatch-Doppelstrang. Solche Mismatches führen in der Regel zu einer Verzögerung der Verlängerung durch eine Polymerase bzw. zu einer Verlangsamung der Gesamtreaktion. In einer Ausführungsform können allel-spezifischen Primer im 3'-Segment mindestens eine phosphoro-thioat-Bindung umfassen, welche allel-spezifische Primer vor 3'-5'-Nuklease-Abbau durch eine Polymerase schützt.

Mehrere Allel-spezifische Primer umfassen somit Sequenzsegmente, welche für eine Gruppe von allel-spezifischen Primern im Wesentlichen identisch bzw. einheitlich sind, sowie Sequenzsegmente, welche unter den Primern einer Gruppe unterschiedlich sind und charakteristisch für jeweilige Sequenzvariante einer Zielsequenz. Unter Einbeziehung von einheitlichen Sequenzsegmenten können solche Primer an die jeweilige Zielsequenz unter Reaktionsbedingungen hybridisieren. Unter Einbeziehung von charakteristischen Sequenzsegmenten kann ein jeweiliger Primer spezifisch an eine Sequenzvariante der Zielsequenz unter Ausbildung eines perfekt-match Doppelstranges binden. Vorzugsweise sind die Primer dermaßen konstruiert, dass unter verwendeten Reaktionsbedingungen die Bindung an eine Zielsequenz unter Ausbildung eines perfekt-match Doppelstranges bevorzugt wird und die Bindung an einer Zielsequenz unter Ausbildung eines Mismatch-Doppelstranges weniger bevorzugt wird.

**In ein r weiteren Ausführungsform wird in Zielsequenz-spezifisch r zweiter Primer, (aber kein allel-spezifischer zweiter Primer) in Kombination mit einem allel-spezifischen Aktivator-Oligonukleotid verwendet.**

Der zweite Primer kann an die ensprechende komplementäre Position einer Start-Nukleinsäure oder einer zu amplifizierenden Nukleinsäurekette binden. Bevozugt umfasst ein zweiter Primer zumindest eine Sequenzsegment, welches bevorzugt sequenz-spezifisch unter verwendeten Reaktionsbedingungen an Sequenzsegmente einer Zielnukleinsäurekette (beispielsweise umfassend eine Start-Nukleinsäure und/oder die zu amplifizierende Nukleinsäurekette) binden kann, wobei diese Bindung im wesentlichen unabhängig von potenziell vorhandenen Sequenzunterschieden im polymorphen Lokus erfolgt, wobei Polymerase in der Lage ist, einen dabei gebildeten perfekt-match Komplex zu verlängert, so dass dabei ein zweites Primer-Verlängerungsprodukt resultiert.

Die Bindung eines solchen Primers erfolgt im Wesentlichen im Sequenzsegment der Zielnukleinsäure, welches für mindestens zwei Allel-Varianten dieser Zielnukleinsäureketten einheitlich ist. Bevorzugt erfolgt die Primer-Bindung im Sequenzsegment der Zielnukleinsäure, welches für alle Allel-Varianten dieser Zielnukleinsäure einheitlich ist.

Die Bindung erfolgt dabei dermaßen, dass der polymorphe Lokus der Zielsequenz in 3'-Richtung vom zweiten Primers liegt, so dass bei einer Primer-Verlängerungsreaktion dieser polymorphe Lokus von Polymerase kopiert wird. Ein resultierendes zweites Primer-Verlängerungsprodukt umfasst somit eine komplementäre Sequenz zum polymorphen Lokus. Diese Sequenz liegt in 3'-Richtung vom zweiten Primer.

Ein solcher Primer umfasst somit Sequenzsegmente, welche bevorzugt komplementär an alle Allel-Varianten einer Zielsequenz einheitlich binden können. Damit eine Differenzierung von allel-Varianten stattfinden kann, muss ein solcher Primer mit mindestens mit einem allel-spezifischen Aktivator-Oligonukleotid kombiniert werden. Die Allel-Diskriminierung erfolgt somit durch Wirkung von Aktivator-Oligonukleotid. Die Positionierung des polymorphen Lokus in 3'-Richtung vom Primer lokalisiert ist, bedingt das seine Lokalisation im dritten und / oder im zweiten Bereich des Aktivator-Oligonukleotides.

### Expon ntielle vs. Lineare Amplifikation

Falls beide komplementäre Stränge (das erste Primer-Verlängerungs-Produkt und das zweite Primer-Verlängerungsprodukt, wobei beide Primer-Verlängerungsprodukte als Matrizen für Synthesen der komplementären Stränge auftreten können) im Wesentlichen parallel zueinander im gleichen Ansatz synthetisiert werden, kann es zu einer exponentiell ablaufenden Vermehrung beider Primer-Verlängerungsprodukte im Rahmen einer solchen Reaktion kommen.

Die während eines Synthese-Vorgangs neu synthetisierten Primer-Verlängerungsprodukte schließen die jeweils komplementäre Sequenzabschnitte zu verwendeten Primern, so dass Primer-Bindungsstellen neu generiert werden. Dadurch können neu synthetisierte Stränge in den darauf folgenden Synthese-Vorgängen ihrerseits als Matrizen dienen.

Falls im Wesentlichen nur ein Primer-Verlängerungs-Produkt als Folge zyklisch wiederholten Synthese-Vorgänge synthetisiert wird, so kommt es zu einer linear ablaufenden Amplifikation des besagten Primer-Verlängerungsproduktes.

In einer vorteilhaften Ausführungsform des Verfahrens werden beide Primer im Wesentlichen in gleich hohen Konzentrationen, oder in Konzentrationsbereichen, welche in etwas gleich hoch sind, eingesetzt.

In einer weiteren vorteilhaften Ausführungsfrom des Verfahrens wird mindestens eines der beiden Primer in einer höheren Konzentration eingesetzt, als sein Partnerprimer. Die Konzentrationsunterschiede können dabei in Bereichen liegen, welche zwischen 1:2 bis 1:50, vorteilhafter zwischen 1:2 bis 1:10 liegen.

Dabei kann eine asymmetrische Amplifikations-Reaktion resultieren, bei welcher Konzentration eines Primer-Verlängerungsproduktes entsprechend höher liegt als die des anderen Stranges.

Die im Folgenden aufgeführten Beispiele sollen lediglich zu Demonstrationszwecken des Verfahrens angeführt werden und nicht als Einschränkung verstanden werden.

Die in den Beispielen angeführten Strukturen, Sequenzen und Reaktiosbedingungen sollten lediglich Funktionsweise des Verfahrens darstellen und anschaulich machen, und dienen nicht als Einschränkung.

### Beispiele:

### Material und Methoden:

Reagenzien wurden von folgenden kommerziellen Anbietern bezogen:
- nicht modifizierte und modifizierte Oligonukleotide (Eurofins MWG, Eurogentec, Biomers, Trilink Technologies, IBA Solutions for Life Sciences)
- Polymerasen NEB (New England Biolabs)
- dNTP's: Jena Bioscience
- interkallierender EvaGreen Farbstoff: Jena Bioscience
- Puffer-Substanzen und andere Chemikalien: Sigma-Aldrich
- Plastikware: Sarstedt

Lösung 1 (Amplifikationsreaktion Lösung 1) :
- Kalium Glutamat, 50 mmol/l, pH 8,0
- Magnesium Acetat, 10 mmol/l
- dNTP (dATP, dCTP, dTTP, dGTP), je 200 µmol/l
- Polymerase (Bst 2.0 WarmStart, 120.000 U/ml NEB), 12 Units / 10 µl
- Triton X-100, 0,1% (v/v)
- EDTA, 0,1 mmol/l
- TPAC (Tetrapropylammonium Chloride), 50 mmol/l, pH 8,0
- EvaGreen Farbstoff (Farbstoff wurde entsprechend Anleitung des Herstellers in Verdünnung 1:50 eingesetzt).

Lösung 2 (Amplifikationsreaktion Lösung 2) :
- 1x Isothermal Puffer (New England Biolabs); in einfacher Konzentration enthält der Puffer:
   20 mM Tris-HCl
   10 mM (NH4)2SO4
   50 mM KCl
   2 mM MgSO4
   0.1% Tween® 20
   pH 8.8@25°C)
- dNTP (dATP, dCTP, dUTP, dGTP), je 200 µmol/l
- EvaGreen Farbstoff (Farbstoff wurde entsprechend Anleitung des Herstellers in Verdünnung 1:50 eingesetzt)

Alle Konzentrationen sind Angaben der Endkonzentrationen in der Reaktion. Abweichungen von der Standard-Reaktion werden entsprechend angegeben.

Die Schmelztemperatur (Tm) von beteiligten Komponenten wurde bei Konzentration von 1 µmol/l von jeweiligen Komponenten in Lösung 1 bestimmt. Abweichende Parameter sind jeweils angegeben.

### Allgemeine Informationen zu Reaktionen

Primer-Verlängerungsreaktionen und Amplifikation wurden standardmäßig bei zwei Reaktionstemperaturen von 55°C und 65°C durchgeführt. Wobei sowohl zyklische Temperatur-Änderungen zwischen 55°C und 65°C als auch isothermale ReaktionsBedingungen verwendet wurden, wie in Beispielen angegeben

Der Reaktionsstart erfolgte durch Erhitzen der Reaktionslösungen auf Reaktionstemperatur, da Bst 2.0 Polymerase Warmstart bei niedrigeren Temperauren größtenteils in ihrer Funktion durch ein temperatur-sensitives Oligonukleotid gehemmt ist (laut Hersteller-Angaben). Die Polymerase wird zunehmend aktiver ab einer Temperatur von ca. 45°C, bei einer Temperatur von 65°C konnten keine Unterschiede zwischen Polymerase Bst 2.0 und Bst 2.0 Warmstart festgestellt werden. Um der extensiven Bildung von Nebenprodukten (z.B. Primer-Dimeren) während der Vorbereitungsphase einer Reaktion vorzubeugen, wurde Polymerase Bst 2.0 Warmstart verwendet. Abweichungen davon werden speziell angegeben.

Reaktionsstopp erfolgte durch Erhitzen der Reaktionslösung auf über 80°C, z.B. 10 min bei 95°C. Bei dieser Temperatur wird Polymerase Bst 2.0 irreversible denaturiert und das Ergebnis der Synthese-Reaktion kann nachträglich nicht geändert werden.

Reaktionen wurden in einem Thermostat mit einer Fluoreszenz-Messvorrichtung ausgeführt. Zu diesem Zweck wurde ein kommerzielles Real-Time PCR Gerät verwendet, StepOne Plus (Applied Biosystems, Thermofischer). Das Reaktionsvolumen betrug standardmäßig 10 µl. Abweichungen davon werden angegeben.

Sowohl Endpunkt-Bestimmung als auch kinetische Beobachtungen wurden vorgenommen. Bei Endpunktbestimmungen wurde das Signal beispielsweise von an Nukleinsäuren gebundenen Farbstoffen registriert, z.B. von TMR (Tetramethyl-Rhodamine, auch TAMRA genannt) oder von FAM (Fluorescein). Die Wellenlängen zur Anregung und Messung der Fluoreszenzsignale von FAM und TMR sind als Werkeinstellungen bei StepOne Plus Real-Time PCR Gerät gespeichert. Ebenfalls wurde ein interkallierender Farbstoff (EvaGreen) bei Endpunktmessungen vorgenommen, z.B. bei Messung einer Schmelzkurve). EvaGreen ist ein interkallierender Farbstoff und ist ein Analogon des häufig eingesetzten Farbstoff Sybrgreen, allerdings mit etwas geringerer Inhibierung von Polymerasen. Die Wellenlängen zur Anregung und Messung der Fluoreszenzsignale von SybreGreen und EvaGreen sind identisch und sind als Werkeinstellungen bei StepOne Plus Real-Time PCR Gerät gespeichert. Die Fluoreszenz kann mittels eingebauter Detektoren fortlaufend, d.h. "online" oder "Real-Time" detektiert werden. Da die Polymerase während ihrer Synthese einen Doppelstrang synthetisiert, konnte diese Technik zu kinetischen Messungen (Real-Time Monitoring) der Reaktion verwendet werden. Aufgrund von einem gewissen Cross-Talk zwischen Farb-Kanälen bei StepOne Plus Gerät wurde teilweise erhöhte basale Signal-Intensität bei Messungen beobachtet, bei welchen z.B. TMR-markierte Primer in Konzentration von über 1 µmol/l (z.B. 10 µmol/l) eingesetzt waren. Es wurde beobachtet, dass TMR-Signal in Sybre-Green-Kanal zu erhöhten Grundwerten führt. Diese erhöhten Grundwerte wurden bei Berechnungen berücksichtigt.

Die kinetischen Beobachtungen von Reaktionsverläufen wurden routinemäßig mittels Fluoreszenzsignalen von Fluoreszein (FAM-TAMRA Fret-Paar) bzw. interkallierenden Farbstoffen (EvaGreen) aufgenommen. Zeitabhängigkeit des Signal-Verlaufs wurde registriert (Real-Time Signal Erfassung bei StepOne plus PCR Gerät). Ein Anstieg des Signals während einer Reaktion verglichen mit einer Kontroll-Reaktion wurde je nach Aufbau des Ansatzes interpretiert. Beispielsweise, eine Zunahme des Signals bei Verwendung von Evagreen-Farbstoff wurde als Hinweise auf Zunahme der Menge an doppelsträngigen Nukleinsäureketten während der Reaktion interpretiert, und somit als Ergebnis einer stattfindenden Synthese durch DNA-Polymerase gewertet.

Bei einigen Reaktionen wurde im Anschluss an die Reaktion eine Schmelzkurvenbestimmung durchgeführt. Solche Messungen erlauben Rückschlüsse auf das Vorliegen von Doppelsträngen, welche beispielsweise interkallierende Farbstoffe aufnehmen können und dadurch die Signalintensität von Farbstoffen deutlich verstärken. Mit der steigenden Temperatur sinkt der Anteil von Doppelsträngen und die Signal-Intensität sinkt ebenfalls. Das Signal ist von der Länge von Nukleinsäureketten und von der Sequenzzusammensetzung abhängig. Diese Technik ist einem Fachmann hinreichend bekannt.

Bei Verwendung der Schmelzkurven-Analyse im Zusammenhang mit Reaktionen, welche signifikante Anteile von modifizierten Nukleinsäureketten (z.B. Aktivator-Oligonukleotide oder Primer) enthielten, wurde festgestellt, dass sich das Signal von Farbstoff EvaGreen beispielsweise unterschiedlich zwischen B-Form der DNA und A-Form von modifizierten Nukleinsäureketten verhalten kann. Beispielsweise wurde bei B-Form der doppelsträngigen Nukleinsäureketten (gewöhnlich angenommen für klassische DNA-Abschnitte) eine höhere Signal-Intensität beobachtet, als bei doppelsträngigen Nukleinsäureketten mit der gleichen Sequenz von Nukleobasen, welche eine A-Form ähnliche Konformation annehmen können (z.B. durch mehrere 2'-O-Me Modifikationen von Nukleotiden). Diese Beobachtung wurde beim Einsatz von interkallierenden Farbstoffen berücksichtigt.

Bei Bedarf wurde die Reaktion mittels Kapillarelektrophorese analysiert und die Länge von gebildeten Fragmenten mit einem Standard verglichen. Als Vorbereitung auf die Kapillarelektrophorese wurde die Reaktionsmischung in einem Puffer (Tris-HCl, 20 mmol/l, pH 8,0, und EDTA, 20 mmol/l, pH 8,0) dermaßen verdünnt, dass die Konzentration von markierten Nukleinsäuren ca. 20 nmol/l betrug. Die Kapillar-Elektrophorese wurde bei Firma GATC-Biotech (Konstanz, Deutschland) als Auftragsleistung durchgeführt. Nach Angaben des Anbieters wurde die Kapillarelektrophorese auf einem ABI 3730 Cappilary Sequencer unter Standard-Bedingungen für eine Sanger-Sequenzierung unter Verwendung von POP7 Gelmatrix, bei ca. 50°C durchgeführt und konstanter Spannung (ca. 10 kV) durchgeführt. Die verwendeten Bedingungen führten zu Denaturierung von Doppelsträngen, so dass in der Kapillarelektrophorese die einzelsträngige Form von Nukleinsäureketten separiert wurde. Die Elektrophorese ist eine Standardtechnik in der genetischen Analyse. Die automatisierte Kapillar-Elektrophorese wird heute routinemäßig bei Sanger-Sequenzierung eingesetzt. Das Fluoreszenz-Signal wird während der Kapillar-Elektrophorese kontinuierlich aufgezeichnet (gewöhnlich unter Verwendung von virtuellen Filtern), so dass ein Elektrophorogramm entsteht, bei welchem die Signal-Intensität mit der Dauer der Elektrophorese korreliert. Bei kürzeren Fragmenten, z.B. nicht verbrauchte Primer, beobachtet man einen frühren Signal-Peak, bei verlängerten Fragmenten kommt es zu einer zeitlichen Verschiebung der Signale proportional der Länge des extendierten Bereichs. Dank mitgeführten Kontrollen mit bekannten Längen lässt sich die Länge von extendierten Fragmenten ausmessen. Diese Technik ist einem Fachmann bekannt und wird ebenfalls standardmäßig bei Fragment-Längen-Polymorphismus eingesetzt.

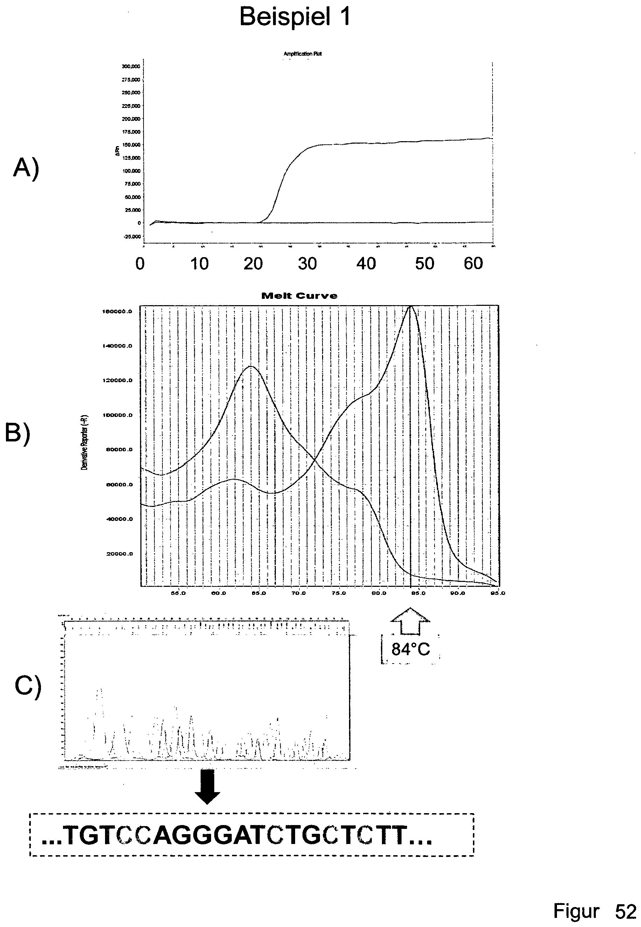

### Beispiel 1 (Fig. 52)

### Verwendung humaner genomischer DNA als Quelle von Zielsequenz

In diesem Beispiel wird Verwendung humaner genomischer DNA (hgDNA) als Quelle einer Zielsequenz gezeigt. Als Zielsequenz wurde ein Sequenzsegment des Faktor-V-Leiden Genes (Homo sapiens coagulation factor V (F5), mRNA, hier als FVL-Gen bezeichnet) gewählt.

Zielsequenz:
5' GTAA GAGCAGATCC CTGGACAGGC AA GGAATACAGGTA - 3' (SEQ ID NO:1)
Die Bindungssequenz für das erste Primer-Oligonukleotid ist unterstrichen. Das zweite Primer-Oligonukleotid bindet mit seinem 3'-Segment an das Reverse-Complement der doppelt unterstrichenen Sequenz.
Der erste Primer, der zweite Primer, sowie Aktivator-Oligonukletid wurden für FVL-Mutation Variante des Genes designet und synthetisiert.

Das erste Primer-Oligonukleotid (SEQ ID NO:2):
P1F5-200-AE2053
   5' AACTCAGACAAGATGTGATTTTTTTACCTGTAT [CUCU GAUGCUUC] 1TACCTGTATTCC 3'
Der als Primer in der Reaktion verwendetes Segment ist unterstrichen.
A = 2'-deoxy-Adenosin
C = 2'-deoxy-Cytosin
G = 2'-deoxy-Guanosin
T = 2'-deoxy-Thymidin (Thymidin)

Dieses Oligonukleotid umfasst folgende Modifikationen:
1= C3-Linker diente zur Terminierung der Synthese des zweiten Primer-Verlängerungsproduktes.
Segment des Primers [CUCU GAUGCUUC] umfasste 2'-O-Me-Modifikationen und diente als zweiter Primer-Bereich zur Bindung des ersten Bereich des Aktivator-Oligonukleotides:
A = (2'-O-Methyl-Adenosine)
G = (2'-O-Methyl-Guanosine)
C = (2'-O-Methyl-Cytosine)
U = (2'-O-Methyl-Uridine)

### Primer 2:

P2G3-5270-7063 
5′ CTACAGAACTCAGACAAGATGTGAACTACAATGTT 6
GCTCATACTACAATGTCACTTACTGTAAGAGCAGA 3′ (SEQ ID NO:3)
Der als Primer in der Reaktion verwendetes Segment ist unterstrichen.

Dieses Oligonukleotid umfasst folgende Modifikationen:
6 = HEG-Linker
A = 2'-deoxy-Adenosin
C = 2'-deoxy-Cytosin
G = 2'-deoxy-Guanosin
T = 2'-deoxy-Thymidin (Thymidin)

Folgendes Aktivator-Oligonukleotid (SEQ ID NO:4) wurde verwendet:
AD-F5-1001-503
   5'[UAAUCUGUAA GAGCAGAUCC CUGGACAGGC AA GGAAUAC]AGGTAGAAGCATC AGAG X3'
   A = 2'-deoxy-Adenosin
   C = 2'-deoxy-Cytosin
   G = 2'-deoxy-Guanosin
   T = 2'-deoxy-Thymidin (Thymidin)

Das 5'-Segment des Oligonukleotids [UAAUCUGUAA GAGCAGAUCC CUGGACAGGC AA GGAAUAC] umfasste 2'-O-Me-Nukleotid-Modifikationen:
Modifikationen:
   A = (2'-O-Methyl-Adenosine)
   G = (2'-O-Methyl-Guanosine)
   C = (2'-O-Methyl-Cytosine)
   U = (2'-O-Methyl-Uridine)
   X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende des Aktivator-Oligonukleotdies ist mit einer Phosphat-Gruppe blockiert, um eine mögliche Verlängerung durch die Polymerase zu verhindern.

Der erste Primer umfasst in seinem ersten Bereich eine Sequenz, welche spezifisch an die Sequenz des Faktor-V-Leiden Genes innerhalb der genomischen DNA binden kann, so dass eine Synthese durch eine Polymerase gestartet werden kann. Der zweite Bereich des ersten Primers umfasst eine Sequenz, welche nicht an die Sequenz von FVL-Genes spezifisch hybridisiert. Weiterhin umfasst der erste Primer ein weiteres Sequenzsegment, welches an das 5'-Ende des zweiten Bereichs anknüpft. Dieses Segment nimmt an der spezifischen Amplifikation des Faktor-5-Leiden Segments nicht teil. Die Funktion dieses Segments wird vor allem Verzögerung von Nebenreaktionen gesehen.
Der zweite Primer umfasst ein Segment in seinem 3'-Segment, welches spezifisch an die genomiche DNA binden kann, so dass eine Synthese durch eine Polymerase gestartet werden kann. Das 5'-Segment des zweiten Primers umfasst eine Sequenz, welche nicht an die Sequenz von FVL-Genes spezifisch hybridisiert. Während der Rücksynthese kann dieses Sequenzsegment kopiert werden. Der zweite Primer umfasst ein weiteres Sequenzsegment, welches weder mit dem Aktivator-Oligonukleotid, noch mit dem erten Primer, noch mit dem zweiten Primer spezifisch hybridisieren kann. Dieses Segment wurde an das 5'-Ende des zweiten Primer lokalisiert und vom 5'-Ende des Primers durch eine HEG-Linker getrennt ist. Dieses Segment nimmt nicht an der spezifischen Amplifikation teil. Die Funktion dieses Segments wird vor allem in der Verzögerung von Nebenreaktionen gesehen.

Aktivator-Oligonukleotid wurde dermaßen konstruiert, dass eine Perfekt-Match-Situation zur Sequenz der Faktor-V-Leiden Mutation des FVL-Gens resultiert. Das Aktivator-Oligonukleotid umfasst einen ersten, zweiten und dritten Bereich.

Als genomische DNA wurde WHO-Standard für FVL-Mutation verwendet. Vor der Verwendung in der Reaktion wurde DNA durch Erhitzung denaturiert (5 min bei 95°C) und damit aus dem doppelsträngigen Zustand in einzelsträngigen Zustand überführt. Anhang dieser einzelsträngiger hgDNA wurde zunächst eine Start-Nukleinsäurekette durch eine Primer-Verlängerng erstellt. Anschließend erfolgte eine exponentielle Amplifikation ausgehend von dieser Start-Nukleinsäurekette. Der Nachweis der Spezifität der Amplifikation erfolgte mittels Schmelzkurven-Analyse und Sanger-Sequenzierung mit einem Sequenzierungprimer.

Alle Reaktionen wurden in Amplifikations-Lösung 1 durchgeführt.
Die verwendeten dNTPs umfassten: dATP, dGTP, dCTP, dUTP (anstatt von dTTP).
Als Polymerase wurde Bst 2.0 Warm-Start Polymerase von NEB verwendet.

Die Start-Nukleinsäurekette wurde wie folgt erstellt:
Etwa 50000 haploider genom Äquivalente (HGE), 150 ng hgDNA, wurden in Kontakt mit dem zweiten Primer (0,5 µmol/l) und Bst-2.0 Warm Start Polymerase (etwa 1 unit), sowie dNTPs (ca. 250 µmol/l) unter Hybridierungsbedingungen (Amplifikationslösung 1, Temperatur von etwa 60°C) in 50 µl Reaktionsvolumen gebracht und ca. 10 min inkubiert. Während dieser Phase erfolgt eine Verlängerung des zweiten Primers, wobei die genomische DNA als Matrize dient. Es resultiert ein Primer-Verlängerungsprodukt, welches als Start-Nukleinsäure dienen kann. Nach Abschluss dieser Reaktion wurde Reaktionsgemisch erhitzt auf 95°C für ca. 10 min, um diese Start-Nukleinsäurekette Matrize zu trennen. Dieses Reaktionsgemisch wurde eingefroren und als Quelle der Start-Nukleinsäurekette nach Bedarf verwendet.

Die spezifische Amplifikation der Zielsequenz des FVL-Gens erfolgte unter Verwendung von 5 µl des Reaktionsgemisches mit der Start-Nukleinsäurekette (entspricht ca. 5000 HGE).

Die anderen Reaktionskomponenten (erster Primer, zweiter Primer, Aktivator-Oligonukleotid, Eva-Green Farbstoff, Polymerase Bst.2.0 Warm Start, dNTPs) wurden dazugegeben, so dass ein Reaktionsendvolumen von ca. 10 µl resultierte. Die Endkonzentrationen der Komponenten betrugen: erster Primer: 5µmol/l, zweiter Primer: 2 µmol/l, Aktivator-Oligonukleotid: 1 µmol/l, Eva-Green Farbstoff (1:50), Polymerase Bst.2.0 Warm Start (ca. 8 Units), dNTPs: ca. 250 µmol/l.

Im Kontroll-Ansatz wurde keine hgDNA zugegeben.

Die Reaktion wurde in einem Step-One Plus Gerät (Thermofisher Scientific) durchgeführt.

Die Reaktionstemperatur wurde initial durch zyklische Änderungen (30 Zyklen) geändert zwischen 65°C (5 min, einschließlich Detektionsschritt) und 55°C (1 min) und anschließend für 1 hr bei 65°C konstant gehalten (Detektionsschritt alle 2 min). Der Verlauf der Reaktion wurde durch Signal-Erfassung des EvaGreen Farbstoffs verfolgt. Nach Abschluss der Reaktion wurde das Reaktionsgemisch zunächst auf 95°C für 10 min gebracht und anschließend wurde eine Schmelzkurve der gebildeten Produkte gemessen.

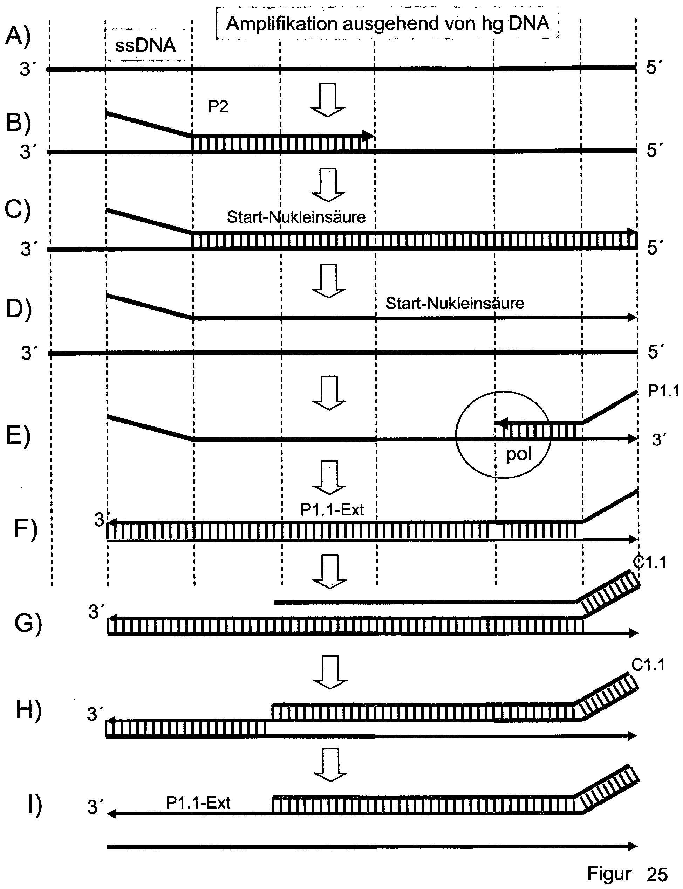

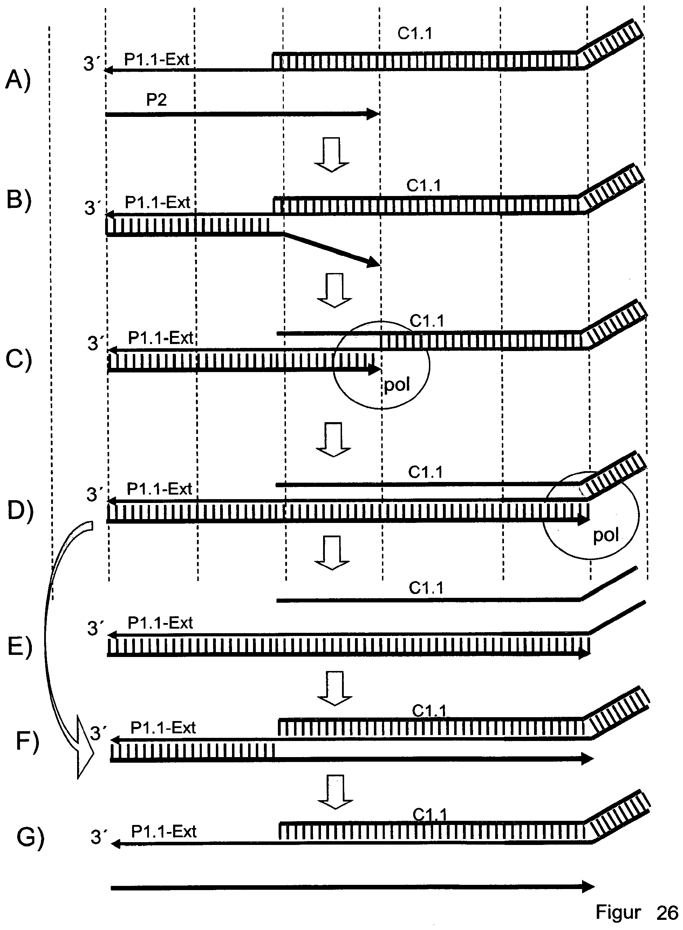

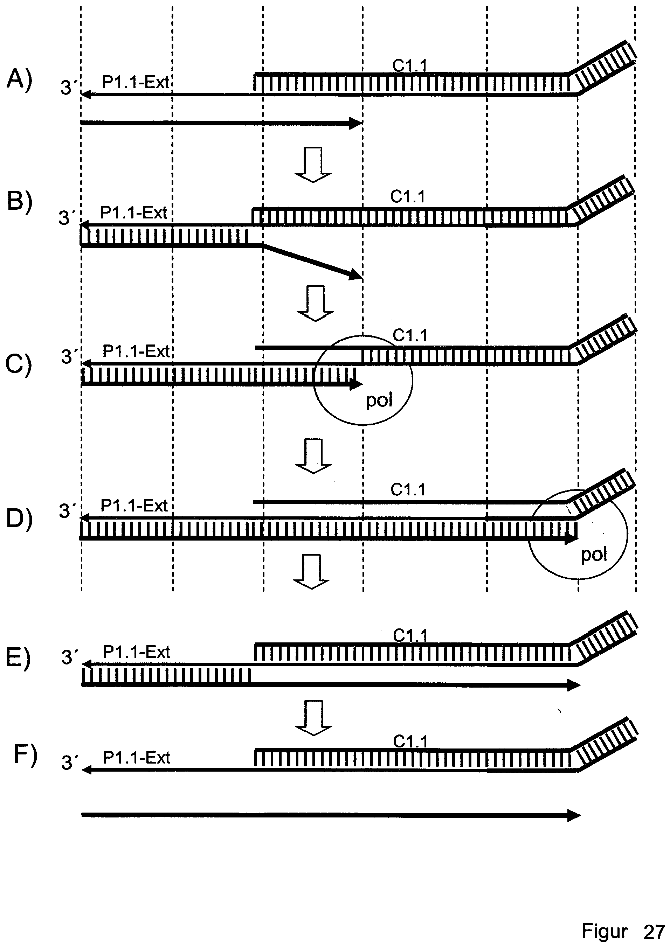

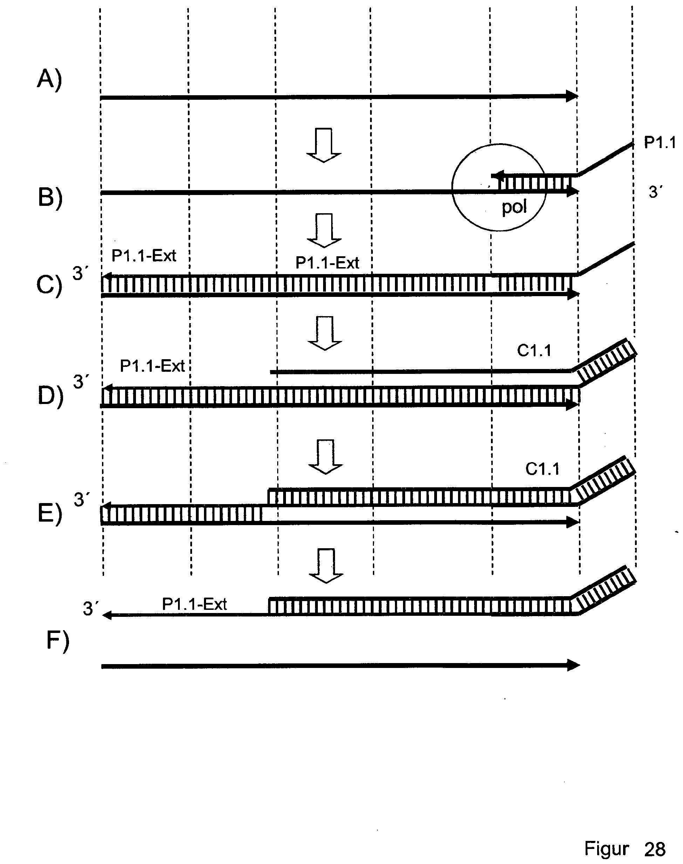

Ein schematischer Ablauf der Amplifikation ist in Fig. 25 bis Fig. 28 dargestellt.

Zunächst erfolgte eine Erstellung einer Start-Nukleinsäurekette (Fig.25, Schritte A bis D). Anschließend erfolgte eine exponentielle Amplifikation unter Verlängerung des ersten Primers und des zweiten Primers und des Aktivator-Oligonukleotids (Fig. 25E bis Fig. 28).

Als Ergebnis der Amplifikation kommt es zu Akkumulierung von Amplifikationsfragmenten. Das Ergebnis der Detektion der Amplifikation ist in Fig. 52 zu sehen. Man sieht, dass ca. nach 2 hr der Reaktion eine sichtbare Zunahme der Fluoreszenz erfolgt (52 A). Anschließende Schmelzkurven-Analyse zeigte eine spezifische Schmelzkurve mit Tm von 84°C (Fig. 52 B).

Sequenzüberprüfung (Fig.52C):
Die Sequenzüberprüfung erfolgte mittels eines Sequenzierungsprimers:
   SEQP1F5-35-X03 
5´- AAG CTCGACAAAGAAC TCAG AG TGTG CTCGAC AC TACCTGTATTCC
TTGCC 3´ (SEQ ID NO:5)
      A = 2'-deoxy-Adenosin
      C = 2'-deoxy-Cytosin
      G = 2'-deoxy-Guanosin
      T = 2'-deoxy-Thymidin (Thymidin)

Zur Sequenzüberprüfung wurde das Reaktionsgemisch (nach Messung der Schmelzkurve) mit Wasser verdünnt (von ca. 1:10 bis ca. 1:100) und jeweils erhaltenen Aliquoten mit einem Sequenzierungsprimer (zugegeben in Konzentration von 2 µmol/l) gemischt. Dieses Gemisch wurde durch einen kommerziellen Sequenzierungs-Anbieter versendet (GATC-Biotec) und mittels Sanger-Sequenzierung als Auftrags-Sequenzierung sequenziert. Die erhaltenen Elektropherogramme wurden auf Übereinstimmung mit der FVL-Sequenz Gens geprüft. Als Ergebnis der Reaktion wurde Sequenz des FVL-Gens identifiziert.

### Beispiel 2:

### Selektive Amplifikations-Reaktion von Sequenz-Varianten einer Zielsequenz.

In diesem Beispiel wurde Einfluss einer Sequenzänderung in der Matrize auf die Amplifikation untersucht. Bei Verlängerung des ersten Primer-Oligonukleotides wird ein komplementärer Strang gebildet, welcher eine komplementäre Sequenz zur Matrize aufweist und somit diese Abweichungen in der Sequenz umfasst. Auf diese Weise sollte überprüft werden, welche Auswirkung ein solcher Mismatch zwischen einem dabei entstehenden ersten Primer-Verlängerungsprodukt und einem Aktivator-Oligonukleotid auf die Amplifikation hat. Die Position des Mismatches liegt in 3'-Richtung vom ersten Primer und wird somit nicht vom Primer, sonder durch das Aktivator-Oligonukleotid überprüft. Diskriminierung zwischen einzelnen Sequenz-Varianten der Zielsequenz erfolgt somit mittels Aktivator-Oligonukleoitides unter Verwendung eines einheitlichen ersten und zweiten Primers.

### Folgende Matrizen wurden verwendet:

Matrize (SEQ ID NO 6) mit Sequenzzusammensetzung, welche zu einem ersten Primer-Verlängerungsprodukt mit einem Perfekt-Match Übereinstimmung mit Aktivator-Oligonukleotid führt:
M2SF5-M001-200 
5′ GCT CATA CTACAATGTCA CT TA CTGTAA GAGCAGATCC CTGGACAGGC AA
GGAATACAGGTA AAAAA 3′ (SEQ ID NO:6)
   A = 2'-deoxy-Adenosin
   C = 2'-deoxy-Cytosin
   G = 2'-deoxy-Guanosin
   T = 2'-deoxy-Thymidin (Thymidin)

Die Bindungssequenz für das erste Primer-Oligonukleotid ist unterstrichen. Das zweite Primer-Oligonukleotid bindet an das Reverse-Complement der doppelt unterstrichen Sequenz.

Matrize (SEQ ID NO 7) mit Sequenzzusammensetzung, welche zu einem ersten Primer-Verlängerungsprodukt führt, das an einer einzelnen Basenposition (fett gedruckt) ein Mismatch mit dem Aktivator-Oligonukleotid bildet:
M2SF5-WT01-200 
5′ GCT CATA CTACAATGTCA CT TA CTGTAA GAGCAGATCC CTGGACAGGC GA
GGAATACAGGTA AAAAA 3′ (SEQ ID NO:7)
   A = 2'-deoxy-Adenosin
   C = 2'-deoxy-Cytosin
   G = 2'-deoxy-Guanosin
   T = 2'-deoxy-Thymidin (Thymidin)

Die Bindungssequenz für das erste Primer-Oligonukleotid ist unterstrichen. Das zweite Primer-Oligonukleotid bindet an das Reverse-Complement der doppelt unterstrichenen Sequenz.

Folgende Primer wurden verwendet:
Das erste Primer-Oligonukleotid (SEQ ID NO:2):
   P1F5-200-AE2053
      5' AACTCAGACAAGATGTGATTTTTTTACCTGTAT [CUCU GAUGCUUC] 1TACCTGTATTCC 3'
   Der als Primer in der Reaktion verwendetes Segment ist unterstrichen.
   A = 2'-deoxy-Adenosin
   C = 2'-deoxy-Cytosin
   G = 2'-deoxy-Guanosin
   T = 2'-deoxy-Thymidin (Thymidin)

Dieses Oligonukleotid umfasst folgende Modifikationen:
1= C3-Linker diente zur Terminierung der Synthese des zweiten Primer-Verlängerungsproduktes.
Segment des Primers [CUCU GAUGCUUC] umfasste 2'-O-Me-Modifikationen und diente als zweiter Primer-Bereich zur Bindung des ersten Bereich des Aktivator-Oligonukleotides:
A = (2'-O-Methyl-Adenosine)
G = (2'-O-Methyl-Guanosine)
C = (2'-O-Methyl-Cytosine)
U = (2'-O-Methyl-Uridine)

### Primer 2:

P2G3-5270-7063 
5′ CTACAGAACTCAGACAAGATGTGAACTACAATGTT 6
GCTCATACTACAATGTCACTTACTGTAAGAGCAGA 3′ (SEQ ID NO:8)
Der als Primer in der Reaktion verwendetes Segment ist unterstrichen.

Dieses Oligonukleotid umfasst folgende Modifikationen:
6 = HEG-Linker
A = 2'-deoxy-Adenosin
C = 2'-deoxy-Cytosin
G = 2'-deoxy-Guanosin
T = 2'-deoxy-Thymidin (Thymidin)

Folgendes Aktivator-Oligonukleotid (SEQ ID NO:4) wurde verwendet:
AD-F5-1001-503 
5′[UAAUCUGUAA GAGCAGAUCC CUGGACAGGC AA GGAAUAC]AGGTAGAAGCATC AGAG
X 3′
   A = 2'-deoxy-Adenosin
   C = 2'-deoxy-Cytosin
   G = 2'-deoxy-Guanosin
   T = 2'-deoxy-Thymidin (Thymidin)

Das 5'-Segment des Oligonukleotids [UAAUCUGUAA GAGCAGAUCC CUGGACAGGC AA GGAAUAC] umfasste 2'-O-Me-Nukleotid-Modifikationen:
Modifikationen:
   A = (2'-O-Methyl-Adenosine)
   G = (2'-O-Methyl-Guanosine)
   C = (2'-O-Methyl-Cytosine)
   U = (2'-O-Methyl-Uridine)
   X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende des Aktivator-Oligonukleotdies ist mit einer Phosphat-Gruppe blockiert, um eine mögliche Verlängerung durch die Polymerase zu verhindern.

Es wurden vier Ansätze vorbereitet:
Ansatz 1 enthält als Start-Nukleinsäurekette die Matrize M2SF5-M001-200 (Perfect Match Situation) in Konzentration von 300 fmol/l (entspricht ca. 2x10^6 Kopien/ Ansatz).
Ansatz 2 enthält als Start-Nukleinsäurekette die Matrize M2SF5-M001-200 (Perfect Match Situation) in Konzentration von 300 amol/l (entspricht ca. 2x10^3 Kopien/ Ansatz).
Ansatz 3 enthält keine Matrize und bildet somit eine Kontrolle.
Ansatz 4 enthält als Start-Nukleinsäurekette die Matrize M2SF5-WT01-200 (single Mismatch Situation) in 300 µmol/l Konzentration (ca. 2x 10^9 Kopien pro Ansatz).

Primer 1 wurde mit 5 µmol/l, das Aktivator-Oligonukleotid mit 2 µmol/l und Primer 2 mit 1 µmol/l eingesetzt.

Die weiteren Reaktionsbedingungen waren: Amplifikations-Lösung 2.

Um die Anwesenheit von genomischer DNA im Assay nachzustellen wurden pro Reaktion 100 ng frisch denaturierte Fisch-DNA (Lachs-DNA) zugegeben.

Die thermischen Reaktionsbedingungen waren zyklisch, alternierende Temperaturänderungen, wobei jeweils auf ein 2 min Zeitintervall bei 55 °C ein 5 min Zeitintervall bei 65 °C folgte. Die Amplifikation wurde über 100 Zyklen verfolgt. Detektion erfolgte bei 65°C für EvaGreen-Fluoreszenzsignal.

Die erfolgreiche Amplifikation konnte durch einen Anstieg des EvaGreen-Fluoreszenzsignals im Laufe der Zeit festgestellt werden.

Die Temperatur-Änderungen und Real-Time Monitoring wurde mit StepPne Plus Real-Time PCR Gerät von Thermofisher durchgeführt.

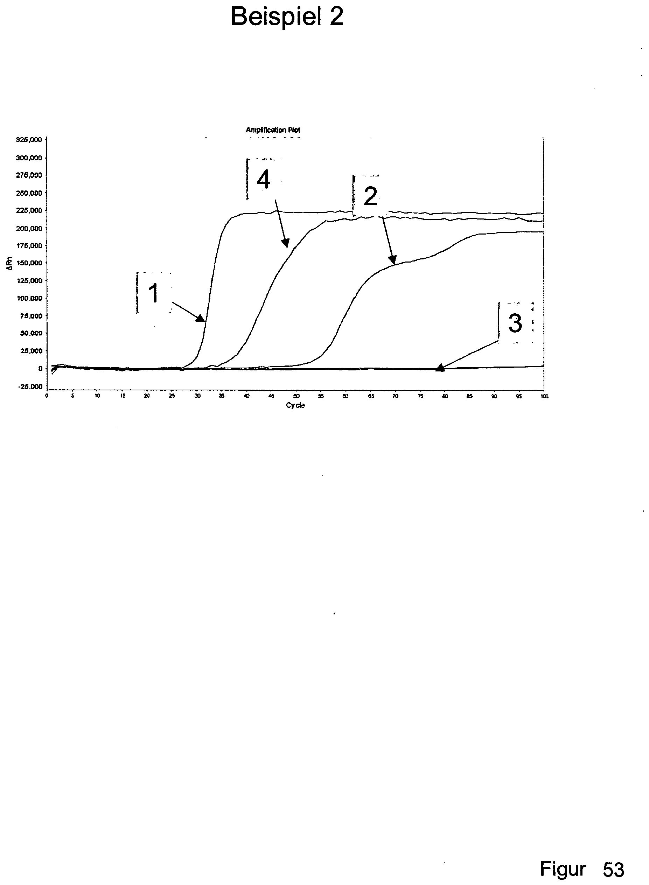

Fig. 53A zeigt einen typischen Verlauf des EvaGreen-Signals. Auf der Y-Achse ist die Zunahme des Fluoreszenzsignal (Delta Rn) und auf der X-Achse ist die Reaktionszeit (als Zyklusnummer) aufgetragen. Pfeile markieren einzelne Reaktionsansätze. Dabei gehören die markierten Positionen zu folgenden Ansätzen:

| | |
|---|---|
| Pfeil 1: 300 fmol/l perfekt-Match Matrize | (ca. 2x10^6 Kopien/Ansatz) |
| Pfeil 2: 300 amol/l perfekt-Match Matrize | (ca. 2x10^3 Kopien/Ansatz) |
| Pfeil 3: keine Matrize | |
| Pfeil 4: 300 µmol/l Mismatch Matrize | (ca. 2x 10^9 Kopien pro Ansatz). |

Man sieht den Anstieg des Fluoreszenz-Signals sowohl bei perfekt-Match als auch bei Mismatch-Variante der Matrize, wobei das Signal der Mismatch-Variante (4) trotz 100fachen Überschußes später erscheint. Es ist erkennbar, dass die Mismatch-Amplifikationssignale gegenüber dem Perfekt-Match Amplifikationssignal deutlich verzögert sind. Beim Single Mismatch wird eine Verzögerung von ca. 15 Zyklen beobachtet. Die zeitliche Verzögerung (=Zyklusanzahl) ist ein unmittelbares Maß für die Diskriminierung in der Amplifikation. Eine weitere Quantifizierung der Diskriminierung in der Amplifikation kann über den Vergleich mit einer Matrizen-Konzentrationsreihe unter Perfekt-Match Amplifikation erzielt werden.

Bei Verwendung einer Perfect-Match Matrize kommt es zur Synthese eines komplementären Stranges eines Primer-Verlängerungsproduktes. Dieses Verlängerungsprodukt ist sowohl zu Perfect-Match-Matrize komplementär als auch zum verwendeten Aktivator-Oligonukleotid.

Im Gegensatz dazu, unter Verwendung einer Mismatch-Sequenz kommt es bei der Synthese des ersten Primer-Verlängerungsproduktes zu Generierung eines komplementären Stranges des Verlängerungsproduktes, welches zwar eine vollständige Komplementarität zu Mismatch-Matrize aufweist, aber dadurch von der Komplementarität mit dem dritten Bereich des Aktivator-Oligonukleotid abweicht. Diese Abweichung findet im 5'-ständigen Segment des Verlängerungsproduktes statt, welches mit Aktivator-Oligonukleotid reagieren sollte, damit Strangverdrängungsvorgang voranschreiten kann. Wie im vorliegenden Beispiel gezeigt wird, stört das Mismatch eine Strangverdrängung durch das Aktivator-Oligonukleotid.

Die Kontrollreaktionen mit einer Perfect Match Matrize (Pfeile 1 und 2) zeigten eine Konzentrationsabhängigkeit der Amplifikation. Mit sinkender Konzentration brauchte Reaktion längere Zeit, um eine ausreichende Menge der zu amplifizierenden Nukleinsäure zu synthetisieren, damit das Signal über Niveau der Baseline ansteigt.

Dieses Ergebnis veranschaulicht die Bedeutung der Basenzusammensetzung im Aktivator-Oligonukleotid: Abweichungen von der Komplementarität zwischen Aktivator-Oligonukleotid und dem Primer-Verlängerungsprodukt können zu Verlangsamung oder sogar Unterbrechung der Amplifikation führen.

In diesem Beispiel wurde gezeigt, dass obwohl Sequenz-Enden von Perfect-Match-Matrize und Mismatch-Matrize komplett übereinstimmten und somit das Potenzial zu Bindung von beiden Primer-Oligonukleotide gleich war, sind beide Reaktionen vollkommen unterschiedlich gelaufen: bei einer vollständigen Komplementarität zwischen dem Aktivator-Oligonukleotid und dem 5'-Segment des Verlängerungsproduktes des ersten Primer-Oligonukleotides verlief die Amplifikation planmäßig. Eine Unterbrechung der Strangverdrängung durch eine Sequenzabweichung (in diesem Fall durch ein Mismatch) führte zur Unterdrückung der Amplifikation.

### B ispiel 3:

### Demonstration einer selektiven Amplifikationsreaktion unter Verwendung eines perfekt-match Primers und unterschiedlicher Aktivator-Oligonukleotide: eines perfekt match und eines mismatch Aktivator-Oligonukleotides.

In diesem Ausführungsbeispiel demonstrieren wir wie durch ein geschicktes Aktivator-Oligonukleotid-Design eine allel-spezifische Amplifikationsreaktion umgesetzt werden kann. Dazu wurde eine Zielsequenz jeweils nach Zugabe von Perfekt-Match- und Mismatch-Aktivator-Oligonukleotid unter zyklischen Temperaturbedingungen amplifiziert. Die Detektion der entstehenden Amplifikationsprodukte erfolgte in diesem Beispiel mit dem interkalierenden Farbstoff Eva Green.

Folgende Matrize wurde verwendet:
- Matrize (SEQ ID NO 9) mit Sequenzzusammensetzung, welche zu einem ersten Primer-Verlängerungsprodukt mit einer Perfekt-Match-Übereinstimmung mit dem Aktivator-Oligonukleotid führt:
   M2SF5-M001-200 
5′ GCT CATA CTACAATGTCA CT TA CTGTAA GAGCAGATCC CTGGACAGGC AA
GGAATACAGGTA AAAAA 3′ (SEQ ID NO:9)
Die Bindungssequenz für das erste Primer-Oligonukleotid ist unterstrichen.
Das zweite Primer-Oligonukleotid bindet an das Reverse-Complement der doppelt unterstrichen Sequenz.

Folgende Primer wurden verwendet:
Das erste Primer-Oligonukleotid (SEQ ID NO:2): 
5´
AACTCAGACAAGATGTGATTTTTTACCTGTAT[CUCUGAUGCUUC]1TACCTGTATTCC
3´
Der als Primer in der Reaktion verwendetes Segment ist unterstrichen.
A = 2'-deoxy-Adenosin
C = 2'-deoxy-Cytosin
G = 2'-deoxy-Guanosin
T = 2'-deoxy-Thymidin (Thymidin)

Dieses Oligonukleotid umfasst folgende Modifikationen:
1= C3-Linker diente zur Terminierung der Synthese des zweiten Primer-Verlängerungsproduktes.
Segment des Primers [CUCU GAUGCUUC] umfasste 2'-O-Me-Modifikationen und diente als zweiter Primer-Bereich zur Bindung des ersten Bereich des Aktivator-Oligonukleotides:
A = (2'-O-Methyl-Adenosine)
G = (2'-O-Methyl-Guanosine)
C = (2'-O-Methyl-Cytosine)
U = (2'-O-Methyl-Uridine)

### • Das zweite Primer-Oligonukleotid (SEQ ID NO 10):

P2G3-5270-7063

5′ CTACAGAACTCAGACAAGATGTGAACTACAATGTT 6
GCTCATACTACAATGTCACTTACTGTAAGAGCAGA 3′ (SEQ ID NO:10)

Modifikationen:
6 = HEG-Linker

Sowohl erster Primerals auch zweiter Primer sind in der Lage, eine Amplifikation mit einem perfekt-match Aktivator-Oligonukleotid zu unterstützen.

Folgendes Perfekt-Match-Aktivator-Oligonukleotid (SEQ ID NO:4) wurde verwendet:
AD-F5-1001-503 
5′[UAAUCUGUAA GAGCAGAUCC CUGGACAGGC AA GGAAUAC]AGGTAGAAGCATC
AGAG X 3′
   A = 2'-deoxy-Adenosin
   C = 2'-deoxy-Cytosin
   G = 2'-deoxy-Guanosin
   T = 2'-deoxy-Thymidin (Thymidin)

Das 5'-Segment des Oligonukleotids [UAAUCUGUAA GAGCAGAUCC CUGGACAGGC AA' GGAAUAC] umfasste 2'-O-Me-Nukleotid-Modifikationen:
Modifikationen:
   A = (2'-O-Methyl-Adenosine)
   G = (2'-O-Methyl-Guanosine)
   C = (2'-O-Methyl-Cytosine)
   U = (2'-O-Methyl-Uridine)
   X = 3'-Phosphat-Gruppe zur Blockade einer möglichen Verlängerung durch Polymerase.

Folgendes Mismatch-Aktivator-Oligonukleotid (SEQ ID NO 11) wurde verwendet:
MD2AD-F5-011-5 
5′- GCTC TAATCTGTAA GAGCAGATCC CTG [GAC AGGC AA AGAAUACAGG] TA
GAAGCATC AGAG X 3′ (SEQ ID NO:11)
   A = 2'-deoxy-Adenosin
   C = 2'-deoxy-Cytosin
   G = 2'-deoxy-Guanosin
   T = 2'-deoxy-Thymidin (Thymidin)

Das mittlere Segment des Oligonukleotids [GAC AGGC AA ***A***GAAUACAGG] umfasste 2'-O-Me-Nukleotid-Modifikationen:
Modifikationen:
   A = (2'-O-Methyl-Adenosine)
   G = (2'-O-Methyl-Guanosine)
   C = (2'-O-Methyl-Cytosine)
   U = (2'-O-Methyl-Uridine)
   X = 3'-Phosphat-Gruppe zur Blockade einer möglichen Verlängerung durch Polymerase.

Die Nukleotide und Nukleotid-Modifikationen sind untereinander mit Phosphodiesterbindungen verknüpft. Das 3'-Ende ist mit einer Phosphat-Gruppe blockiert, um eine mögliche Verlängerung durch die Polymerase zu verhindern.

Im Mismatch Aktivator-Oligonukleotid wurde in der Position -24 (vom 3'-Ende) wurde ein A-Nukleotid (Mismatch-Variante) anstatt eines G-Nukleotides (perfekt Match Variante) eingesetzt. Das Nukleotid ist durch Fettschrift hervorgehoben. Diese Position bildet ein Mismatch mit dem 3'-Terminalen Nukleotide des eingesetzten ersten Primers.

Die Matrize wurde in Konzentrationen von 1-3 pmol/l eingesetzt. In der Kontroll-Reaktion wurde keine Matrize eingesetzt (= Negativkontrolle). Primer 1 wurde mit 5 pmol/l, das jeweilige Aktivator-Oligonukleotid mit 2 pmol/l und Primer 2 mit 1 pmol/l eingesetzt.

Die weiteren Reaktionsbedingungen waren:
- Amplifikations-Lösung 2
- Um die Anwesenheit von genomischer DNA im Assay nachzustellen wurden pro Reaktion 100 ng frisch denaturierte Fisch-DNA (Lachs-DNA) zugegeben.

Die thermischen Reaktionsbedingungen waren zyklisch, alternierende Temperaturänderungen, wobei jeweils auf ein 1 min Zeitintervall bei 55 °C ein 5 min Zeitintervall bei 65 °C folgte. Die Amplifikation wurde typischerweise über 120 Zyklen, d.h. 120 x (1 min 55°C + 5 min 65°C) = 120 x 6 min = 12 h verfolgt. Die erfolgreiche Amplifikation konnte durch einen Anstieg des EvaGreen-Fluoreszenzsignals im Laufe der Zeit festgestellt werden.

### Analyse der Allel-spezifischen Amplifikationsreaktionen

Zur Analyse der Amplifikationsreaktion und zur Bewertung der entstandenen Amplifikationsprodukte wurden folgende Techniken genutzt:
- Fluoreszenzsignal eines interkalierenden Farbstoffes (EvaGreen)
- Schmelzkurvenanalyse von den entstandenen Amplifikationsprodukten

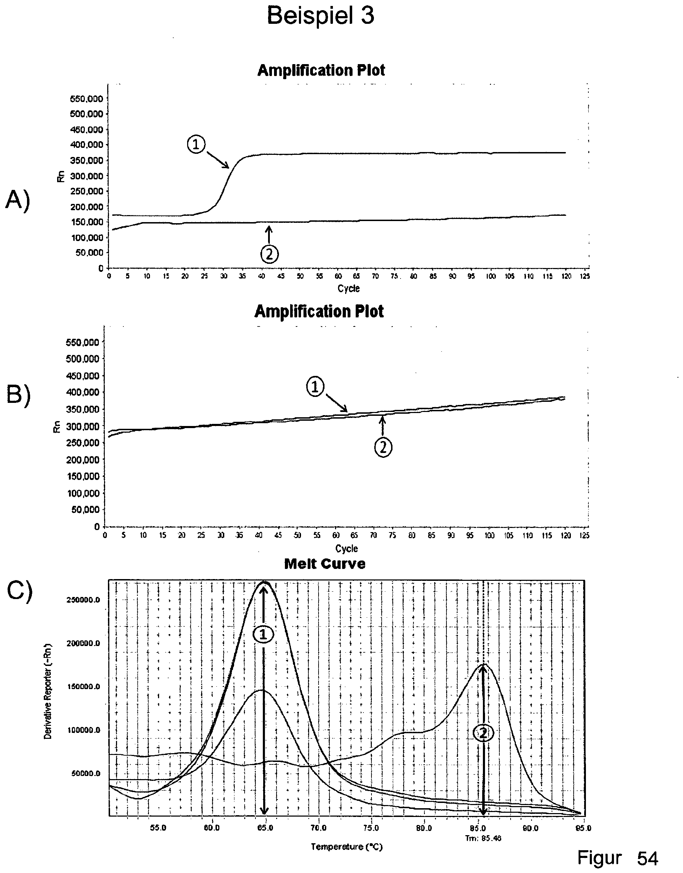

Fig. 54A zeigt einen typischen Verlauf des EvaGreen-Fluoreszenzsignals im Laufe einer Allel-spezifischen Amplifikationsreaktion unter Verwendung eines Perfekt-Match-Aktivator-Oligonukleotids. Auf der Y-Achse ist das Fluoreszenzsignal des EvaGreen-Farbstoffes und auf der X-Achse ist die Reaktionszeit (als Zyklusnummer) aufgetragen. Pfeil 1 markiert in Fig.54 einen Perfekt-Match-Amplifikationsansatz. Aufgrund des Perfekt-Match-Aktivator-Oligonukleotids kann die Matrize amplifiziert werden und es kommt ab Zyklus 27 zu einem Anstieg des Fluoreszenzsignals. Pfeil 2 markiert einen Negativkontrolle-Ansatz, der keine Matrize enthält und daher im Laufe des Experiments auch kein Amplifikationssignal generiert.

Fig. 54B zeigt einen typischen Verlauf des EvaGreen-Fluoreszenzsignals im Laufe einer Allel-spezifischen Amplifikationsreaktion unter Verwendung eines Mismatch-Aktivator-Oligonukleotids. Auf der Y-Achse ist das Fluoreszenzsignal des EvaGreen-Farbstoffes und auf der X-Achse ist die Reaktionszeit (als Zyklusnummer) aufgetragen. Pfeil 1 markiert einen Mismatch-Amplifikationsansatz. Aufgrund des Mismatch-Aktivator-Oligonukleotids kann die Matrize nicht amplifiziert werden. Pfeil 2 markiert einen Negativkontrolle-Ansatz, der keine Matrize enthält und daher im Laufe des Experiments auch kein Amplifikationssignal generiert. Das Fluoreszenzsignal von Mismatch-Aktivator-Oligonukleotid-Ansatz und Negativkontrolle sind nahezu deckungsgleich, was zeigt, wie stark die Amplifikation in der Mismatch-Situation unterdrückt wird.

Fig. 54C zeigt die Schmelzkurven der Allel-spezifischen Amplifikationsprodukte aus Fig. 54A und 54B. Auf der Y-Achse ist die Ableitung des Fluoreszenzsignals gegen die Temperatur und auf der X-Achse ist die Temperatur aufgetragen. Es wurden zwei charakteristische Peaks gefunden, markiert mit Pfeil 1 und 2. Der Peak bei Position 1 mit einem Schmelzpunkt in der Nähe von 65 °C gehört zum Mismatch-Aktivator-Oligonukleotid-Ansatz. Der Signalverlauf ist nicht von Negativkontroll-Ansätzen (= Amplifikationsreaktion ohne Matrize) zu unterscheiden. Dem gegenüber zu stellen ist der Peak bei Position 2 mit einem Schmelzpunkt in der Nähe von etwa 85 °C. Dieser Peak gehört zum Perfekt-Match-Aktivator-Oligonukleotid-Ansatz und ist spezifisch für das gebildete Amplifikationsprodukt. Die Ergebnisse legen nahe, dass in der Perfekt-Match-Situation spezifische Amplifikationsprodukte mit einem definierten Schmelzpunkt in der Nähe von etwa 85°C entstehen. Dagegen wird bei Mismatch-Aktivator-Oligonukleotiden die Amplifiktionsreaktion unterdrückt. Die Fluoreszenzsignale der Mismatch-Aktivator-Oligonukleotid-Ansätze waren in diesem Beispiel nicht von der Negativ-Kontrolle zu unterscheiden. Dies zeigt wie stark die Amplifikationsunterdrückung in der Mismatch-Situation ist.

Zusammenfassend wird festgehalten: ein Nukleotid-Mismatch im Aktivator-Oligonukleotid positioniert im zweiten Bereich des Aktivator-Oligonukleotides kann bei eine Amplifikation verhindern.

Ausgehend von diesem Beispiel kann man erkennen, dass Sequenz-Varianten-spezifische Amplifikations-Systeme umfassend ein Sequenz-Variante-spezifischen Primer und einem entsprechenden komplementären Sequenz-Variante -spezifisches Aktivator-Oligonukleotid zusammengestellt werden können.

## Patentansprüche

1. Verfahren zur Amplifikation einer Nukleinsäure,das folgende Schritte umfasst:
a) Hybridisierung eines ersten Primer-Oligonukleotids an das 3'-Segment eines Stranges einer zur amplifizierenden Nukleinsäurekette, wobei die zu amplifizierende Nukleinsäurekette eine Zielsequenz umfasst,
wobei das erste Primer-Oligonukleotid umfasst:
• Einen ersten Primer-Bereich im 3'-Segment des ersten Primer-Oligonukleotides, der an einen Strang einer zu amplifizierenden Nukleinsäurekette sequenzspezifisch binden kann
• Einen zweiten Bereich, gekoppelt direkt oder via einen Linker an das 5'-Ende des ersten Primer-Bereichs des ersten Primer-Oligonukleotides, welcher einen Polynukleotid-Schwanz umfasst, welcher zur Bindung eines Aktivator-Oligonukleotids und Unterstützung der Strangverdrängung (Schritt c) durch Aktivator-Oligonukleotids geeignet ist, wobei der Polynukleotid-Schwanz von Polymerase unter den gewählten Reaktionsbedinungen im Wesentlichen unkopiert bleibt.
b) Extension des ersten Primer-Oligonukleotids mit Hilfe einer Polymerase zur Bildung eines ersten Primer-Verlängerungsproduktes, das eine zur Zielsequenz der zu amplifizierenden Nukleinsäurekette (a) komplementäre Sequenz umfasst,
c) Bindung des Aktivator-Oligonukleotides an den Polynukleotid-Schwanz des zweiten Bereichs des ersten verlängerten Primer-Oligonukleotids, wobei das Aktivator-Oligonukleotid umfasst:
• Einen ersten einzelsträngigen Bereich, welcher an den Polynukleotid-Schwanz des zweiten Bereichs des ersten Primer-Oligonukleotides binden kann
• Einen zweiten einzelsträngigen Bereich, welcher zu dem ersten Bereich des ersten Primer-Oligonukleotides im Wesentlichen komplementär ist und an diesen binden kann
• Einen dritten einzelsträngigen Bereich, welcher zumindest zu einem Segment des von Polymerase synthetisierten Verlängerungsproduktes des ersten Primer-Verlängerungsprodukt im Wesentlichen komplementär ist,
wobei das Aktivator-Oligonukleotid nicht als Matrize für eine Primerverlängerung des ersten Primer-Oligonukleotid dient,
d) Bindung des Aktivator-Oligonukleotides an den ersten Primer-Bereich des ersten verlängerten Primer-Oligonukleotids, unter Verdrängung des zu diesem ersten Primer-Bereich komplementären Stranges der zur amplifizierenden Nukleinsäurekette
e) Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, wobei das 3'-Segment des ersten Primer-Verlängerungsproduktes einzelsträngig wird,
f) Hybridisierung eines zweiten Oligonukleotid-Primers an das erste Primer-Verlängerungsprodukt, wobei das 3'-Segment des zweiten Oligonukleotid-Primers eine Sequenz umfasst, die an das erste Primer-Verlängerungsprodukt hybridisieren kann, und
g) Verlängerung des zweiten Oligonukleotid-Primers mit Polymerase zur Bildung eines zweiten Primer-Verlängerungsproduktes, wobei Verlängerung bis einschließlich des ersten Primer-Bereichs des ersten Primer-Oligonukleotids erfolgt und dieser erste Primer-Bereich von der Polymerase kopiert wird, wobei der Polynukleotid-Schwanz des zweiten Bereichs unkopiert bleibt,
h) Wiederholung der Schritte a)-g) bis das gewünschte Ausmaß der Amplifikation erreicht ist, **dadurch gekennzeichnet, dass** wenigstens eine der Komponenten, erster Primer, Aktivator-Oligonukleotid und zweiter Primer eine Abweichung von wenigstens einem Oligonukleotid zu der Perfect-Match-Sequenz der Zielsequenz aufweist, so dass keine perfekte Anlagerung dieser Komponente an die Zielsequenz erfolgt.

2. Verfahren nach Anspruch 1, **dadurch gekennzeichnet, dass** für wenigstens eine der Komponenten, erstes Primer-Oligonukleotid, Aktivator-Oligonukleotid und zweiter Oligonukleotid-Primer sowohl solche vorhanden sind, die perfekt komplementär zu den Zielsequenzen sind, wie auch solche, die sich in wenigstens einem Nukleotid von der komplementären Sequenz der Zielsequenz unterscheiden, so dass der Nachweis von Punktmutationen durch Amplifikation oder nicht erfolgte Amplifikation nachgewiesen werden kann.

3. Verfahren nach einem der Ansprüche 1 oder 2, **dadurch gekennzeichnet, dass** von wenigstens einem der Komponenten, erstes Primer-Oligonukleotid, Aktivator-Oligonukleotid und zweitem Primer-Oligonukleotid wenigstens drei Varianten vorhanden sind, wobei sich jede dieser Varianten von den anderen Varianten durch mindestens ein Nukleotid unterscheidet.

4. Verfahren nach Anspruch 3, **dadurch gekennzeichnet, dass** Verfahren nach einem der Ansprüche 1 bis 3, **dadurch gekennzeichnet, dass** von wenigstens zwei der Komponenten ausgewählt aus erstes Primer Oligonukleotid, Aktivator Oligonukleotid und zweites Primer Oligonukleotid wenigstens zwei Varianten vorhanden sind, wobei sich jede dieser Varianten von der Zielsequenz, zu der die Varianten komplementär sind, durch mindestens ein Oligonukleotid unterscheidet.

5. Verfahren nach einem der Ansprüche 1 bis 4, **dadurch gekennzeichnet, dass** das Verfahren im Wesentlichen isothermal durchgeführt wird.

6. Verfahren nach einem der Ansprüche 1 bis 5, **dadurch gekennzeichnet, dass** der dritte einzelsträngige Bereich des Aktivator-Oligonukleotids zum Segment des von der Polymerase synthetisierten Verlängerungsproduktes des ersten Primer-Verlängerungsprodukts, welches sich unmittelbar an den ersten Primer-Bereich anschließt, im Wesentlichen komplementär ist.

7. Verfahren nach Anspruch 1, **dadurch gekennzeichnet, dass** Schritt (e) des Verfahrens dahingehend modifiziert wird, dass es umfasst die Bindung des Aktivator-Oligonukleotides an das komplementäre Segment des Verlängerungsproduktes des ersten verlängerten Primer-Oligonukleotids, unter Verdrängung des zu diesem Verlängerungsprodukt komplementären Stranges der zur amplifizierenden Nukleinsäurekette, bis dieser komplementäre Strang der zu amplifizierenden Nukleinsäure vom ersten Primer-Verlängerungsprodukt abgelöst wird, wobei das 3'-Segment des ersten Primer-Verlängerungsproduktes einzelsträngig wird.

8. Verfahren nach Anspruch 1, **dadurch gekennzeichnet, dass** Schritt (f) des Verfahrens dahingehend modifiziert wird, dass es umfasst die Hybridisierung eines zweiten Oligonukleotid-Primers an das erste Primer-Verlängerungsprodukt, wobei gleichzeitig zumindest eine partielle Verdrängung des Aktivator-Oligonukleotides aus der Bindung mit dem ersten Verlängerungsprodukt durch Strangverdrängung erfolgt.

9. Verfahren nach Anspruch 1, **dadurch gekennzeichnet, dass** Schritt (g) des Verfahrens so modifiziert wird, dass er eine Verdrängung des Aktivator-Oligonukleotides aus der Bindung mit dem ersten Primer-Verlängerungsprodukt unter Mitwirkung der Polymerase umfasst.

10. Verfahren nach Anspruch 1, **dadurch gekennzeichnet, dass** Schritt (h) des Verfahrens dahingehend modifiziert wird, dass es die Bindung des Aktivator-Oligonukleotides an den unkopierten Polynukleotid-Schwanz des ersten verlängerten Primer-Oligonukleotids und eine Verdrängung des zweiten Primer-Verlängerungsprodukts aus der Bindung an das erste Primer-Verlängerungsprodukt unter gleichzeitiger Ausbildung eines komplementären Doppelstranges mit einem Segment des ersten spezifischen Verlängerungsproduktes des ersten Primer-Oligonukleotides umfasst.

11. Verfahren nach Anspruch 1, **dadurch gekennzeichnet, dass** die Wiederholung der Schritte unter solchen Bedingungen durchgeführt wird, die die Wiederholung der Schritte (a) bis (g) zulassen.

12. Verfahren nach einem der vorhergehenden Ansprüche, **dadurch gekennzeichnet, dass** es die gleichzeitige Amplifikation des ersten und zweiten Primer-Verlängerungsproduktes in einer exponentiellen Reaktion unter Verwendung der ersten und zweiten Primer-Oligonukleotide und des Aktivator-Oligonukleotides umfasst, wobei die gebildeten Primer-Verlängerungsprodukte als Matrizen für die gegenseitige Synthese fungieren.

13. Verwendung eines Kits zur Durchführung eines Verfahrens nach einem der Ansprüche 1 bis 9, **dadurch gekennzeichnet, dass** der Kit wenigstens ein erstes Primer-Oligonukleotid, wenigstens ein Aktivator-Oligonukleotid und wenigstens eine Polymerase zur Amplifikation enthält.

14. Verwendung eines Kits nach Anspruch 13, **dadurch gekennzeichnet, dass** es weiterhin einen zweiten Oligonukleotid-Primer enthält.
